(19) Europäisches Patentamt
European Patent Office
Office européen des brevets

(11) EP 4 410 790 A1

(12) **EUROPEAN PATENT APPLICATION**
published in accordance with Art. 153(4) EPC

(43) Date of publication:
**07.08.2024 Bulletin 2024/32**

(21) Application number: **22875011.3**

(22) Date of filing: **28.09.2022**

(51) International Patent Classification (IPC):
***C07D 471/04*** $^{(2006.01)}$ ***C07D 487/04*** $^{(2006.01)}$
***A61K 31/519*** $^{(2006.01)}$ ***A61P 35/00*** $^{(2006.01)}$

(52) Cooperative Patent Classification (CPC):
**A61K 31/519; A61P 35/00; C07D 471/04; C07D 487/04**

(86) International application number:
**PCT/CN2022/122205**

(87) International publication number:
**WO 2023/051628 (06.04.2023 Gazette 2023/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.09.2021 CN 202111155906**
**20.09.2022 CN 202211141499**

(71) Applicant: **Haihe Biopharma Co., Ltd.**
**Shanghai 201203 (CN)**

(72) Inventors:
- **LI, Dongsheng**
  **Shanghai 201203 (CN)**
- **GAO, Shanyun**
  **Shanghai 201203 (CN)**
- **LIU, Cailu**
  **Shanghai 201203 (CN)**
- **CAI, Yalei**
  **Shanghai 201203 (CN)**
- **YANG, Maozhi**
  **Shanghai 201203 (CN)**
- **TU, Wangyang**
  **Shanghai 201203 (CN)**
- **YU, Bing**
  **Shanghai 201203 (CN)**
- **XIE, Qing**
  **Shanghai 201203 (CN)**
- **ZHANG, Yixiang**
  **Shanghai 201203 (CN)**
- **LI, Leping**
  **Shanghai 201203 (CN)**

(74) Representative: **Sagittarius IP**
**Marlow International**
**Parkway**
**Marlow SL7 1YL (GB)**

# (54) SOS1 INHIBITORS HAVING PYRIDO SIX-MEMBERED RING STRUCTURE

(57) The present invention relates to an SOS1 inhibitor with a pyrido-fused-six-membered ring structure, a preparation method therefor, and use thereof. The SOS1 inhibitor has the structural formula shown in formula (I) and an inhibitory activity on SOS1, and can be used to treat head and neck cancer, lung cancer, a mediastinal tumor, a gastrointestinal tumor, prostate cancer, testicular cancer, a gynecological tumor, breast cancer, renal and bladder cancer, an endocrine tumor, soft tissue sarcoma, osteosarcoma, rhabdomyosarcoma, mesothelioma, skin cancer, a peripheral nerve tumor, a central nervous system tumor, lymphoma, leukemia, Noonan syndrome, cardio-facio-cutaneous syndrome, and hereditary gingival fibromatosis and a related syndrome thereof.

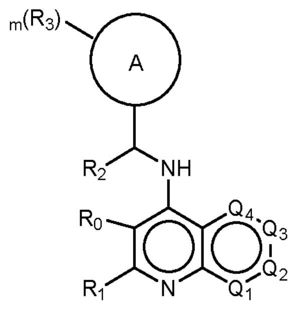

(I)

EP 4 410 790 A1

## Description

### TECHNICAL FIELD

The present invention belongs to the field of medicinal chemistry. Particularly, the present invention relates to a novel class of compounds, a stereoisomer, a racemate, a geometric isomer, a tautomer, a prodrug, a hydrate, a solvate, or a pharmaceutically acceptable salt thereof, and a pharmaceutical composition containing same, which have an activity of an SOS1 inhibitor.

### BACKGROUND ART

A RAS protein includes V-Ki-ras2 Kirsten rat sarcoma viral oncogene homolog (KRAS), neuroplastoma RAS viral oncogene homolog (HRAS), and Harvey murine sarcoma virus oncogene (NRAS). The KRAS has two variable cleavage isomers, KRAS4A and KRAS4B. The RAS protein is mainly distributed inside the cell membrane, and thus membrane localization is a key step in activating the RAS. The membrane localization of the RAS protein requires prenylation and palmitoylation at its C-terminus. However, since the KRAS4B lacks a palmitoylation site, the membrane localization relies on the electrostatic interaction between a polybase region composed of lysine and a plasma membrane (Ahearn et al., 2011; Wright and Philips, 2006). The RAS protein belongs to the small GTPase family and exists in cells in either GTP-binding or GDP-binding mode. The activation of the RAS protein requires its transition from GDP-binding to GTP-binding. The process is catalyzed by guanine nucleotide exchange factors (GEFs), such as Son of Sevenless 1 (SOS1) (Chardin et al., 1993). The RAS activation promotes the activation of downstream effector molecules RAF, phosphoside 3-kinase (PI3K), and Ral guanine nucleotide dissociation stimulator (RalGDS) to influence biological processes such as cell proliferation, growth, metabolism, migration, angiogenesis, etc. (Rodriguez-Viciana and McCormick, 2005; Young et al., 2009). The RAS protein has an intrinsic hydrolytic activity and can convert GTP to GDP. GTPase activating proteins (GAPs), such as NF1, increase the hydrolytic rate to inactivate the RAS. Under normal conditions, the GAPs and the GEFs strictly regulate RAS protein inactivation and activation, but after the RAS protein mutation, the regulatory mechanisms are deregulated. RAS mutations occur predominantly at positions G12, G13, and Q61 in tumor cells, the mutations at these positions attenuate endogenous and GAPs-mediated hydrolytic activity, and the mutations at positions G13 and Q61 also increase the GEFs-mediated GTP exchange rate (Simanshu et al., 2017; Smith et al., 2013). Biochemical data analysis in recent years has shown that the mutated RAS still has a certain intrinsic hydrolytic activity, and that the stronger intrinsic hydrolytic activity of the RAS mutant protein indicates inhibition of its upstream protein SHP2 has a stronger obstruction on its activity (Hunter et al., 2015; Mainardi et al., 2018).

The SOS1 protein has two important motifs, a RAS exchanger motif (REM) and a CDC25 homology domain, an allosteric binding site and a catalytic binding site, respectively. The CDC25 binds RAS-GDP to facilitate exchange of GDP and GTP, and the REM binds RAS-GTP to further increase the catalytic activity of SOS1 (Freedman et al., 2006; pierce et al., 2011). The SOS1 has a key role in KRAS mutant tumors. The knockdown of the SOS1 decreases proliferation and viability of KRAS mutant tumor cells, but does not affect KRAS wild-type cells (Jeng et al, 2012). The SOS1 plays an important role in activating a RAS signaling pathway. Activation of receptor tyrosine kinases (RTKs) activates SHP2, enables the SHP2 to bind to an adaptor protein Grb2, promotes the formation of a Grb2 and SOS1 complex to activate the SOS1, thereby activating the RAS protein (Baltanas et al., 2020). SOS1 mutations are present in tumor cells, such as embryonal rhabdomyosarcoma, lung adenocarcinoma, etc. (Denayer et al., 2010), while the SOS1 is highly expressed in bladder cancer and prostate cancer (Timofeeva et al., 2009; Watanabe et al., 2000). In addition, the SOS1 also has mutations in Noonan syndrome (NS), cardio-facio-cutaneous syndrome (CFC), and hereditary gingival fibromatosis and a related syndrome thereof (Pierre et al., 2011).

SOS2, a homologue of SOS1, also acts as the GEF to activate the RAS protein, with functional redundancy. The knockout of the SOS1 in mice results in embryonic lethality (Qian et al., 2000), while the conditional knockout of the SOS1 in adult mice, the mice can survive (Baltanas et al., 2013). However, the knockout of the SOS2 in mice does not have an obvious phenotype (Esteban et al., 2000). If the SOS1 and the SOS2 are knocked out simultaneously in the adult mice, the mice die soon (Baltanas et al., 2013). Therefore, selective inhibition of a single SOS subtype, such as SOS1, may be more effective in treating an SOS1-RAS activated disease. Inhibition of the binding of a catalytic site of the SOS1 to the RAS can inhibit the RAS signaling pathway by preventing the production of SOS1-mediated RAS-GTP. In a RAS-dependent tumor, such compound can theoretically disrupt the binding of RAS and SOS, and inhibit phosphorylation of cellular ERK to play an anti-tumor effect. The compound, inhibiting the interaction of the SOS1 and the RAS, can inhibit the RAS activity, and can be used to treat head and neck cancer, lung cancer, a mediastinal tumor, a gastrointestinal tumor, prostate cancer, testicular cancer, a gynecological tumor, breast cancer, renal and bladder cancer, an endocrine tumor, soft tissue sarcoma, osteosarcoma, rhabdomyosarcoma, mesothelioma, skin cancer, a peripheral nerve tumor, a central nervous system tumor, lymphoma, leukemia, Noonan syndrome, cardio-facio-cutaneous syndrome, and hereditary gingival fibromatosis and a related syndrome thereof.

## SUMMARY

The present invention provides a compound of formula (I), and an enantiomer, a diastereoisomer, a racemate, a prodrug, a hydrate, a solvate, or a pharmaceutically acceptable salt thereof,

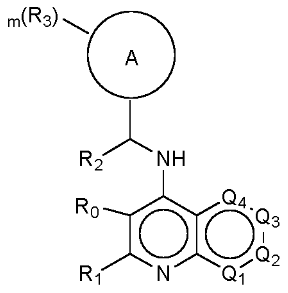

(I)

wherein,

ring A is $C_{6-10}$ aryl, 5-10 membered heteroaryl, or 4-10 membered saturated or unsaturated heterocyclyl;
$m(R_3)$ represents m identical or different $R_3$ substituents at any position of the ring A;
m is 0-5; preferably, m is 1, 2, or 3; and more preferably, m is 1 or 2;
each $R_3$ substituent is independently selected from: unsubstituted or substituted $C_{1-4}$ alkyl, unsubstituted or substituted $C_{1-4}$ alkoxy, unsubstituted or substituted $C_{2-4}$ alkynyl, unsubstituted or substituted $C_{3-6}$ cycloalkyl, unsubstituted or substituted 4-6 membered saturated or unsaturated heterocyclyl, halogen, cyano, amino, and a divalent substituent =O; the substitution refers to being substituted by one or more substituents selected from halogen, hydroxy, cyano, and amino; and when ring A is $C_{6-10}$ aryl or 5-10 membered heteroaryl, $R_3$ is not a divalent substituent =O;
$R_0$ is hydrogen, halogen, $C_{1-4}$ alkyl, cyano, or cyclopropyl;
$R_1$ is hydrogen, halogen, unsubstituted or substituted $C_{1-6}$ alkyl, unsubstituted or substituted $C_{3-6}$ cycloalkyl, unsubstituted or substituted 4-10 membered saturated or unsaturated heterocyclyl, unsubstituted or substituted $C_{1-6}$ alkoxy, -CN, -COOH, $-CONH_2$, $-CONH-C_{1-6}$ alkyl, amino, or $-NH-C_{1-6}$ alkyl;
$R_2$ is hydrogen, unsubstituted or substituted $C_{1-6}$ alkyl, or unsubstituted or substituted $C_{3-6}$ cycloalkyl;
$Q_1$ is N, $NR_4$, -C(O)-, or $CR_4$, $Q_2$ is N, $NR_5$, -C(O)-, or $CR_5$, $Q_3$ is N, $NR_6$, -C(O)-, or $CR_6$, $Q_4$ is N or CH; at most two N atoms are contained in $Q_1$, $Q_2$, $Q_3$, and $Q_4$; and a ring where $Q_1$, $Q_2$, $Q_3$, and $Q_4$ located and a fused ring thereof both remain aromaticity;
$R_4$ is selected from hydrogen, unsubstituted or substituted $C_{1-4}$ alkyl, unsubstituted or substituted $C_{1-4}$ alkoxy, unsubstituted or substituted $C_{2-4}$ alkynyl, unsubstituted or substituted $C_{3-6}$ cycloalkyl, unsubstituted or substituted 4-6 membered saturated or unsaturated heterocyclyl, halogen, cyano, and amino;
$R_5$ and $R_6$ are each independently hydrogen, unsubstituted or substituted $C_{1-10}$ alkyl, unsubstituted or substituted $C_{6-10}$ aryl, unsubstituted or substituted 5-10 membered heteroaryl, unsubstituted or substituted $C_{3-6}$ cycloalkyl, unsubstituted or substituted 4-10 membered saturated or unsaturated heterocyclyl, unsubstituted or substituted 5-10 membered heteroarylo-fused 4-10 membered heterocyclyl, halogen, -CN, -COOH, $-OR_7$, $-NH-R_7$, $-CONH-R_7$, - $NHCO-R_7$, $-SO_2-R_7$, or $-SO_2NH-R_7$;
$R_7$ is selected from hydrogen, unsubstituted or substituted $C_{1-10}$ alkyl, unsubstituted or substituted $C_{6-10}$ aryl, unsubstituted or substituted 5-10 membered heteroaryl, unsubstituted or substituted $C_{3-6}$ cycloalkyl, and unsubstituted or substituted 4-10 membered saturated or unsaturated heterocyclyl;
the substitution in $R_1$, $R_2$, $R_4$, $R_5$, $R_6$, and $R_7$ refers to being substituted by one or more group A substituents, which include: $C_{1-6}$ alkyl, $C_{1-6}$ halogenated alkyl, hydroxy-substituted $C_{1-6}$ alkyl, amino-substituted $C_{1-6}$ alkyl, cyano-substituted $C_{1-6}$ alkyl, $C_{1-6}$ alkoxy-substituted $C_{1-6}$ alkyl, $C_{1-6}$ alkoxy, hydroxy, halogen, cyano, amino, carboxy, $-NH-C_{1-6}$ alkyl, $-NH-C_{3-6}$ cycloalkyl, unsubstituted or group B substituent-substituted $C_{6-10}$ aryl, unsubstituted or group B substituent-substituted 5-10 membered heteroaryl, unsubstituted or group B-substituted $C_{3-6}$ cycloalkyl, unsubstituted or group B substituent-substituted 4-10 membered saturated or unsaturated heterocyclyl, $-C(O)-R_8$, -C(O)O-Rs, $-C(O)-CH_2-R_8$, $-C(O)-NH-R_8$, $-NH-C(O)-R_8$, $-SO_2-R_8$, and $-SO_2NH-R_8$; $R_8$ is selected from hydrogen, unsubstituted or group B substituent-substituted $C_{1-10}$ alkyl, unsubstituted or group B substituent-substituted $C_{1-10}$ alkoxy, unsubstituted or group B substituent-substituted $C_{6-10}$ aryl, unsubstituted or group B substituent-substituted 5-10 membered heteroaryl, unsubstituted or group B substituent-substituted $C_{3-6}$ cycloalkyl, and unsubstituted or group B substituent-substituted 4-10 membered saturated or unsaturated heterocyclyl; and the group B substituent includes: unsubstituted or halogenated $C_{1-6}$ alkyl, unsubstituted or halogenated $C_{1-6}$ alkoxy, hydroxy, halogen, cyano,

amino, carboxy, and -NH-$C_{1-6}$ alkyl.

In one embodiment, the present invention provides the compound of formula (I), and the enantiomer, the diastereoisomer, the racemate, the prodrug, the hydrate, the solvate, or the pharmaceutically acceptable salt thereof, wherein $R_2$ is methyl or ethyl.

In one embodiment, the present invention provides the compound of formula (I), and the enantiomer, the diastereoisomer, the racemate, the prodrug, the hydrate, the solvate, or the pharmaceutically acceptable salt thereof, wherein ring A is phenyl.

In one embodiment, the present invention provides the compound of formula (I), and the enantiomer, the diastereoisomer, the racemate, the prodrug, the hydrate, the solvate, or the pharmaceutically acceptable salt thereof,

wherein m is 1 or 2; and
each $R_3$ substituent is independently selected from: substituted or unsubstituted $C_{1-4}$ alkyl, substituted or unsubstituted $C_{2-4}$ alkynyl, substituted or unsubstituted 4-6 membered saturated or unsaturated heterocyclyl, halogen, cyano, and amino; the substitution refers to being substituted by one or more substituents selected from halogen, hydroxy, cyano, and amino; and preferably, $R_3$ is selected from substituted or unsubstituted $C_{1-4}$ alkyl and halogen, more preferably, $R_3$ is selected from halogenated $C_{1-4}$ alkyl and halogen, and further preferably, $R_3$ is selected from fluorine and difluoromethyl.

In one embodiment, the present invention provides the compound of formula (I), and the enantiomer, the diastereoisomer, the racemate, the prodrug, the hydrate, the solvate, or the pharmaceutically acceptable salt thereof, wherein $R_4$ is selected from Hand $C_{1-4}$ alkyl.

In one embodiment, the present invention provides the compound of formula (I), and the enantiomer, the diastereoisomer, the racemate, the prodrug, the hydrate, the solvate, or the pharmaceutically acceptable salt thereof, wherein $R_5$ is selected from hydrogen and $C_{1-10}$ alkoxy, for example, $C_{1-4}$ alkoxy.

In one embodiment, the present invention provides the compound of formula (I), and the enantiomer, the diastereoisomer, the racemate, the prodrug, the hydrate, the solvate, or the pharmaceutically acceptable salt thereof,

wherein $R_6$ is selected from unsubstituted or substituted $C_{1-10}$ alkyl, unsubstituted or substituted $C_{6-10}$ aryl, unsubstituted or substituted 5-10 membered heteroaryl, unsubstituted or substituted $C_{3-6}$ cycloalkyl, unsubstituted or substituted 4-10 membered saturated or unsaturated heterocyclyl, unsubstituted or substituted 5-10 membered heteroarylo-fused 4-10 membered heterocyclyl, halogen, -CN, -COOH, -$OR_7$, -NH-$R_7$, -CONH-$R_7$, -NHCO-$R_7$, -$SO_2$-$R_7$, and - $SO_2$NH-$R_7$; and
$R_7$ is selected from hydrogen, unsubstituted or substituted $C_{1-10}$ alkyl, unsubstituted or substituted $C_{6-10}$ aryl, unsubstituted or substituted 5-10 membered heteroaryl, unsubstituted or substituted $C_{3-6}$ cycloalkyl, and unsubstituted or substituted 4-10 membered saturated or unsaturated heterocyclyl.

In one embodiment, the present invention provides the compound of formula (I), and the enantiomer, the diastereoisomer, the racemate, the prodrug, the hydrate, the solvate, or the pharmaceutically acceptable salt thereof,

wherein the compound of formula (I) has the structures of formulas (I-1-1)-(I-1-7):

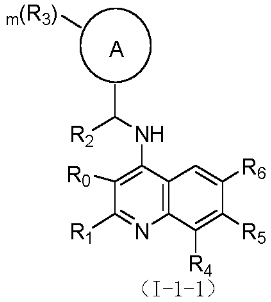
(I-1-1)

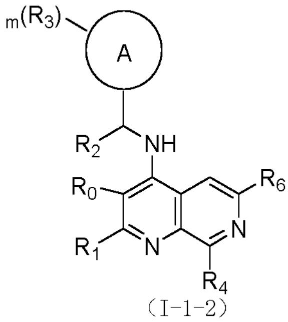
(I-1-2)

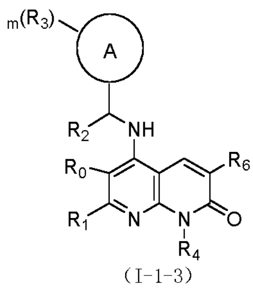
(I-1-3)

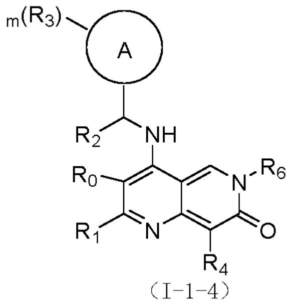
(I-1-4)

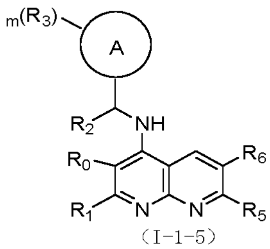
(I-1-5)

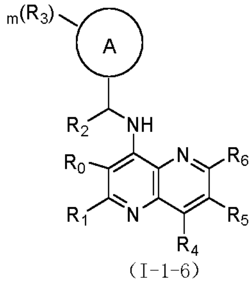
(I-1-6)

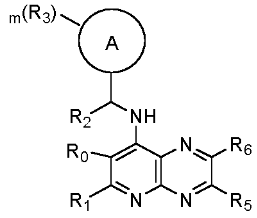
(I-1-7)

wherein,
ring A, $R_0$, $R_1$, $R_2$, $R_3$, $R_4$, $R_5$, $R_6$, and m are as defined previously.

Preferably, the compound of formula (I) has the structures of formulas (I-1-1), (I-1-2), (I-1-3), and (I-1-4).

In one embodiment, the present invention provides the compound of formula (I), and the enantiomer, the diastereoisomer, the racemate, the prodrug, the hydrate, the solvate, or the pharmaceutically acceptable salt thereof,

wherein,
wherein $R_5$ and $R_6$ are each independently hydrogen, unsubstituted or substituted $C_{1-10}$ alkyl, unsubstituted or substituted $C_{6-10}$ aryl, unsubstituted or substituted 5-10 membered heteroaryl, unsubstituted or substituted $C_{3-6}$ cycloalkyl, unsubstituted or substituted 4-10 membered saturated or unsaturated heterocyclyl, unsubstituted or substituted 5-membered heteroarylo-fused 6-membered heterocyclyl, halogen, -CN, -COOH, -$OR_7$, -NH-$R_7$, -CONH-$R_7$, -NHCO-$R_7$, -$SO_2$-$R_7$, or -$SO_2$NH-$R_7$;
$R_7$ is selected from hydrogen, unsubstituted or substituted $C_{1-10}$ alkyl, unsubstituted or substituted $C_{6-10}$ aryl, unsubstituted or substituted 5-10 membered heteroaryl, unsubstituted or substituted $C_{3-6}$ cycloalkyl, and unsubstituted or substituted 4-10 membered saturated or unsaturated heterocyclyl; and
the substitution in $R_5$, $R_6$, and $R_7$ refers to being substituted by one or more group A substituents, which include: $C_{1-6}$ alkyl, $C_{1-6}$ halogenated alkyl, hydroxy-substituted $C_{1-6}$ alkyl, amino-substituted $C_{1-6}$ alkyl, cyano-substituted $C_{1-6}$ alkyl, $C_{1-6}$ alkoxy-substituted $C_{1-6}$ alkyl, $C_{1-6}$ alkoxy, hydroxy, halogen, cyano, amino, carboxy, -NH-$C_{1-6}$ alkyl, -NH-$C_{3-6}$ cycloalkyl, unsubstituted or group B substituent-substituted $C_{6-10}$ aryl, unsubstituted or group B substituent-substituted 5-10 membered heteroaryl, unsubstituted or group B-substituted $C_{3-6}$ cycloalkyl, unsubstituted or group B substituent-substituted 4-10 membered saturated or unsaturated heterocyclyl, -C(O)-$R_8$, -C(O)O-Rs, -C(O)-$CH_2$-$R_8$, -C(O)-NH-$R_8$, -NH-C(O)-$R_8$, -$SO_2$-$R_8$, and -$SO_2$NH-$R_8$; $R_8$ is selected from hydrogen, unsubstituted or group B substituent-substituted $C_{1-10}$ alkyl, unsubstituted or group B substituent-substituted $C_{6-10}$ aryl, unsubstituted or group B substituent-substituted 5-10 membered heteroaryl, unsubstituted or group B substituent-substituted $C_{3-6}$ cycloalkyl, and unsubstituted or group B substituent-substituted 4-10 membered saturated or unsaturated heterocyclyl; and the group B substituent includes: unsubstituted or halogenated $C_{1-6}$ alkyl, unsubstituted or halogenated $C_{1-6}$ alkoxy, hydroxy, halogen, cyano, amino, carboxy, and -NH-$C_{1-6}$ alkyl;
the heteroaryl in $R_5$, $R_6$, $R_7$, and the group A substituent is selected from

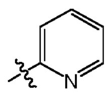,

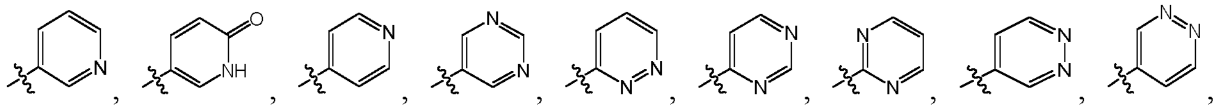

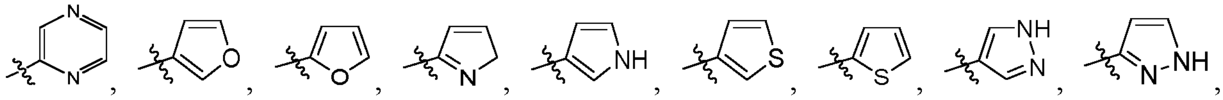

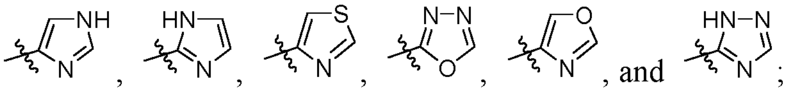

, and ;

the heterocyclyl in $R_5$, $R_6$, $R_7$, and the group A substituent is selected from

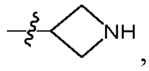

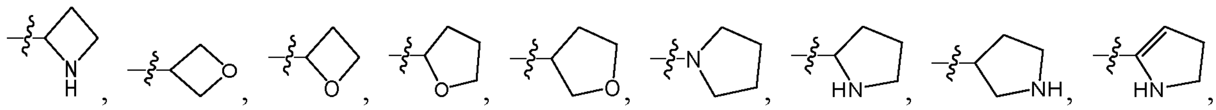

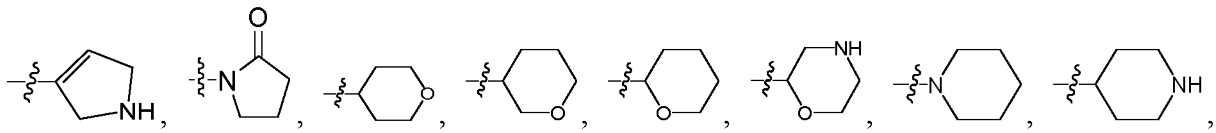

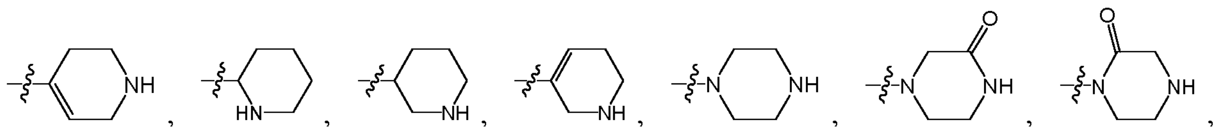

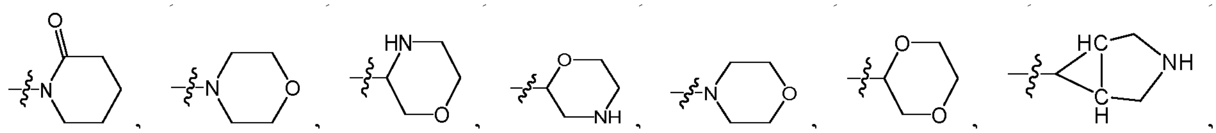

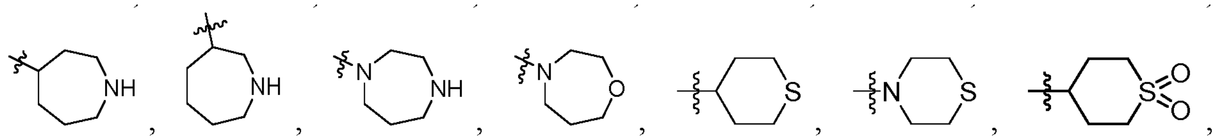

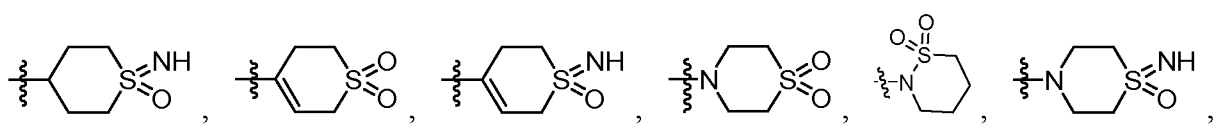

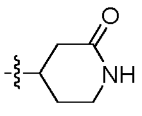, 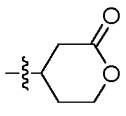, 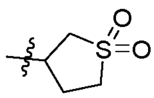, 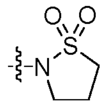, 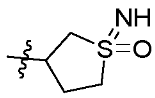, 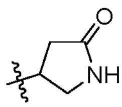, 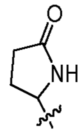, 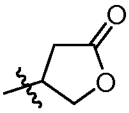,

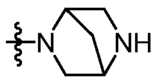, 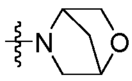, 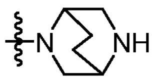, 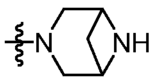, 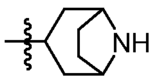, 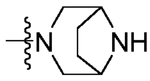,

and

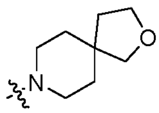;

and

the 5-membered heteroarylo-fused 6-membered heterocyclyl is selected from

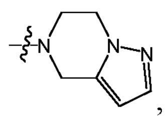, 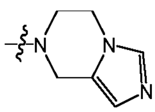, and 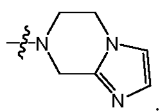.

In one embodiment, the present invention provides the compound of formula (I), and the enantiomer, the diastereoisomer, the racemate, the prodrug, the hydrate, the solvate, or the pharmaceutically acceptable salt thereof,

wherein,

wherein $R_5$ is hydrogen or $C_{1-10}$ alkoxy;

$R_6$ is selected from unsubstituted or substituted $C_{1-10}$ alkyl, unsubstituted or substituted $C_{6-10}$ aryl, unsubstituted or substituted 5-10 membered heteroaryl, unsubstituted or substituted $C_{3-6}$ cycloalkyl, unsubstituted or substituted 4-10 membered saturated or unsaturated heterocyclyl, unsubstituted or substituted 5-10 membered heteroarylo-fused 4-10 membered heterocyclyl, halogen, -CN, -COOH, -$OR_7$, -NH-$R_7$, -CONH-$R_7$, -NHCO-$R_7$, -$SO_2$-$R_7$, and -$SO_2$NH-$R_7$;

$R_7$ is selected from hydrogen, unsubstituted or substituted $C_{1-10}$ alkyl, unsubstituted or substituted $C_{6-10}$ aryl, unsubstituted or substituted $C_{3-6}$ cycloalkyl, and unsubstituted or substituted 4-10 membered saturated or unsaturated heterocyclyl; and

the substitution in $R_6$ and $R_7$ refers to being substituted by one or more group A substituents, which include: $C_{1-6}$ alkyl, $C_{1-6}$ halogenated alkyl, hydroxy-substituted $C_{1-6}$ alkyl, amino-substituted $C_{1-6}$ alkyl, cyano-substituted $C_{1-6}$ alkyl, $C_{1-6}$ alkoxy-substituted $C_{1-6}$ alkyl, $C_{1-6}$ alkoxy, hydroxy, halogen, cyano, amino, carboxy, -NH-$C_{1-6}$ alkyl, -NH-$C_{3-6}$ cycloalkyl, unsubstituted or group B substituent-substituted $C_{6-10}$ aryl, unsubstituted or group B substituent-substituted 5-10 membered heteroaryl, unsubstituted or group B-substituted $C_{3-6}$ cycloalkyl, unsubstituted or group B substituent-substituted 4-10 membered saturated or unsaturated heterocyclyl, -C(O)-$R_8$, -C(O)O-Rs, -C(O)-$CH_2$-$R_8$, -C(O)-NH-$R_8$, -NH-C(O)-$R_8$, -$SO_2$-$R_8$, and -$SO_2$NH-$R_8$; $R_8$ is selected from hydrogen, unsubstituted or group B substituent-substituted $C_{1-10}$ alkyl, unsubstituted or group B substituent-substituted $C_{6-10}$ aryl, unsubstituted or group B substituent-substituted 5-10 membered heteroaryl, unsubstituted or group B substituent-substituted $C_{3-6}$ cycloalkyl, and unsubstituted or group B substituent-substituted 4-10 membered saturated or unsaturated heterocyclyl; and the group B substituent includes: unsubstituted or halogenated $C_{1-6}$ alkyl, unsubstituted or halogenated $C_{1-6}$ alkoxy, hydroxy, halogen, cyano, amino, carboxy, and -NH-$C_{1-6}$ alkyl.

In one embodiment, the present invention provides the compound of formula (I), and the enantiomer, the diastereoisomer, the racemate, the prodrug, the hydrate, the solvate, or the pharmaceutically acceptable salt thereof,

wherein,

$R_5$ and $R_6$ are each independently unsubstituted or substituted $C_{1-10}$ alkyl, unsubstituted or substituted $C_{6-10}$ aryl, unsubstituted or substituted 5-10 membered heteroaryl, unsubstituted or substituted $C_{3-6}$ cycloalkyl, unsubstituted or substituted 4-10 membered saturated or unsaturated heterocyclyl, unsubstituted or substituted 5-membered

heteroarylo-fused 6-membered heterocyclyl, halogen, -CN, -COOH, $-OR_7$, $-NH-R_7$, $-CONH-R_7$, $-SO_2-R_7$, or $-SO_2NH-R_7$;

$R_7$ is selected from hydrogen, unsubstituted or substituted $C_{1-10}$ alkyl, unsubstituted or substituted $C_{6-10}$ aryl, unsubstituted or substituted 5-10 membered heteroaryl, unsubstituted or substituted $C_{3-6}$ cycloalkyl, and unsubstituted or substituted 4-10 membered saturated or unsaturated heterocyclyl;

the substitution in $R_5$, $R_6$, and $R_7$ refers to being substituted by one or more group A substituents, which include: $C_{1-6}$ alkyl, $C_{1-6}$ halogenated alkyl, hydroxy-substituted $C_{1-6}$ alkyl, amino-substituted $C_{1-6}$ alkyl, cyano-substituted $C_{1-6}$ alkyl, $C_{1-6}$ alkoxy-substituted $C_{1-6}$ alkyl, $C_{1-6}$ alkoxy, hydroxy, halogen, cyano, amino, carboxy, $-NH-C_{1-6}$ alkyl, $-NH-C_{3-6}$ cycloalkyl, unsubstituted or group B substituent-substituted $C_{6-10}$ aryl, unsubstituted or group B substituent-substituted 5-10 membered heteroaryl, unsubstituted or group B-substituted $C_{3-6}$ cycloalkyl, unsubstituted or group B substituent-substituted 4-10 membered saturated or unsaturated heterocyclyl, $-C(O)-R_8$, -C(O)O-Rs, $-C(O)-CH_2-R_8$, $-C(O)-NH-R_8$, $-NH-C(O)-R_8$, $-SO_2-R_8$, and $-SO_2NH-R_8$; $R_8$ is selected from hydrogen, unsubstituted or group B substituent-substituted $C_{1-10}$ alkyl, unsubstituted or group B substituent-substituted $C_{6-10}$ aryl, unsubstituted or group B substituent-substituted 5-10 membered heteroaryl, unsubstituted or group B substituent-substituted $C_{3-6}$ cycloalkyl, and unsubstituted or group B substituent-substituted 4-10 membered saturated or unsaturated heterocyclyl; and the group B substituent includes: unsubstituted or halogenated $C_{1-6}$ alkyl, unsubstituted or halogenated $C_{1-6}$ alkoxy, hydroxy, halogen, cyano, amino, carboxy, and $-NH-C_{1-6}$ alkyl;

the heteroaryl in $R_5$, $R_6$, $R_7$, and the group A substituent is selected from

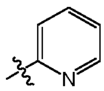,

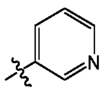, 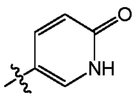, 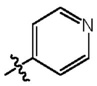, 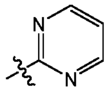, 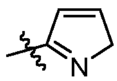, 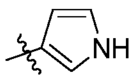, 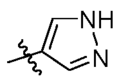, 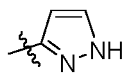, 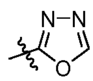,

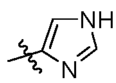, and 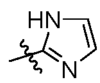;

The heterocyclyl in $R_5$, $R_6$, $R_7$, and the group A substituent is selected from

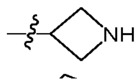,

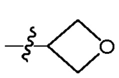, 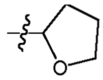, 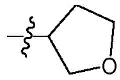, 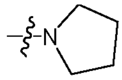, 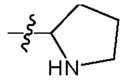, 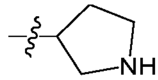, 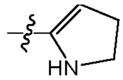, 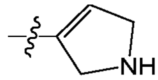,

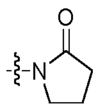, 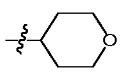, 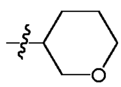, 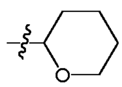, 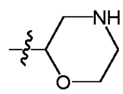, 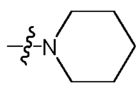, 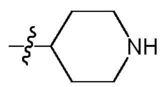, 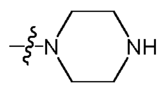,

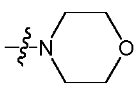, 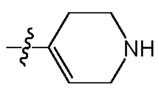, 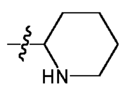, 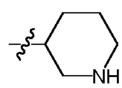, 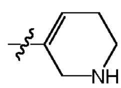, 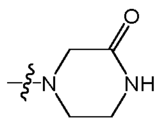, 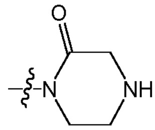,

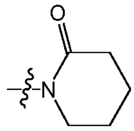, 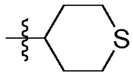, 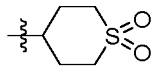, 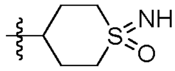, 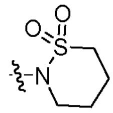, 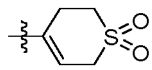, 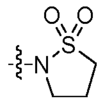,

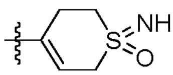, 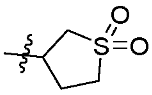, 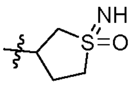, 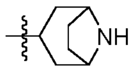, 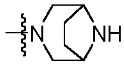, and 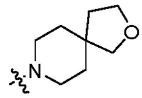;

and
the 5-membered heteroarylo-fused 6-membered heterocyclyl is selected from

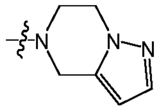, 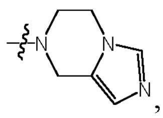, and 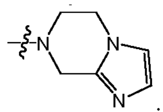.

In one embodiment, the present invention provides the compound of formula (I), and the enantiomer, the diastereoisomer, the racemate, the prodrug, the hydrate, the solvate, or the pharmaceutically acceptable salt thereof, wherein the compound of formula (I) has the structure of formula (I-2) or formula (I-3), and each symbol and variable are as defined previously provided that $R_2$ is not hydrogen:

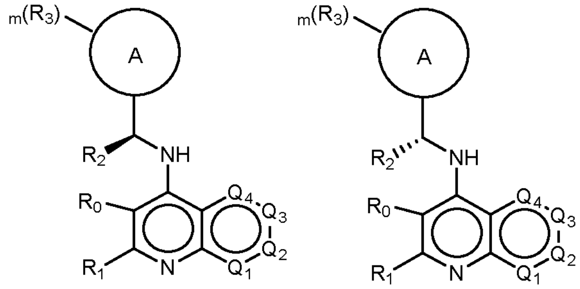

(I-2) (I-3)

.

In one embodiment, the compound of formula (I) has the structure of formula (I-4):

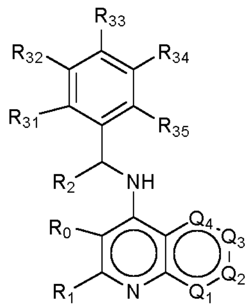

(I-4)

wherein $R_0$, $R_1$, $R_2$, $Q_1$, $Q_2$, $Q_3$, and $Q_4$ are as defined previously; $R_{31}$, $R_{32}$, $R_{33}$, $R_{34}$, and $R_{35}$ are each independently selected from: hydrogen, substituted or unsubstituted $C_{l-4}$ alkyl, substituted or unsubstituted $C_{2-4}$ alkynyl, substituted or unsubstituted 4-6 membered saturated or unsaturated heterocyclyl, halogen, cyano, and amino; the substitution refers to being substituted by one or more substituents selected from halogen, hydroxy, cyano, and amino; and preferably, $R_{31}$ is halogen, $R_{32}$ is halogenated $C_{1-6}$ alkyl, and $R_{33}$, $R_{34}$, and $R_{35}$ are all hydrogen.

In one embodiment, $R_1$ is hydrogen or $C_{1-6}$ alkyl, preferably, hydrogen or methyl.

The compound of formula (I) is selected from the following compounds:

| No. | Structure | No. | Structure |
|---|---|---|---|
| A-1 | 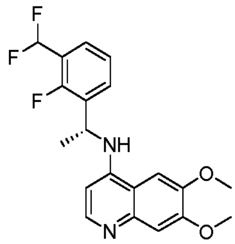 | A-2 | 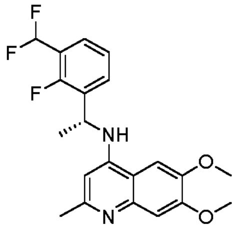 |
| A-3 | 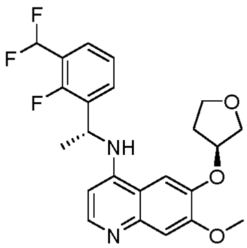 | A-4 | 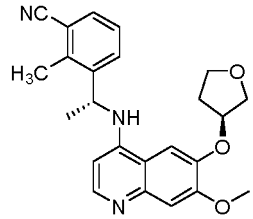 |
| A-5 | 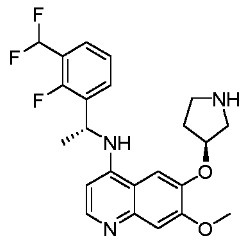 | A-6 | 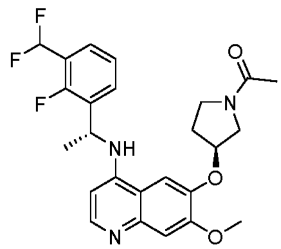 |
| A-7 | 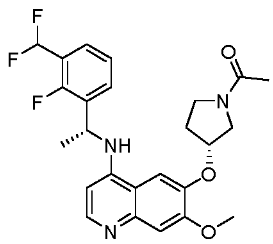 | A-8 | 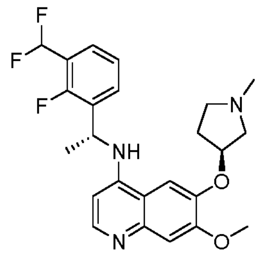 |
| A-9 | 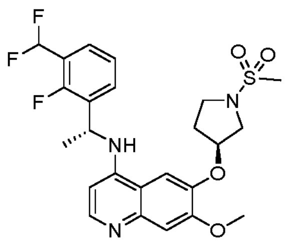 | A-10 | 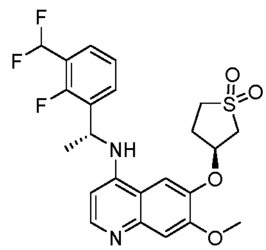 |
| A-11 | 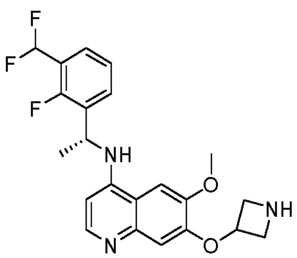 | A-12 | 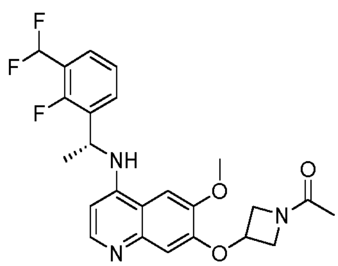 |

(continued)

| No. | Structure | No. | Structure |
|---|---|---|---|
| A-13 | 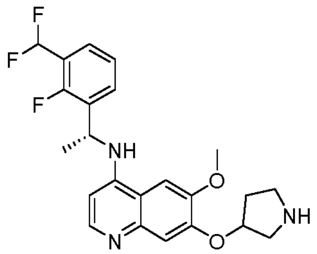 | A-14 | 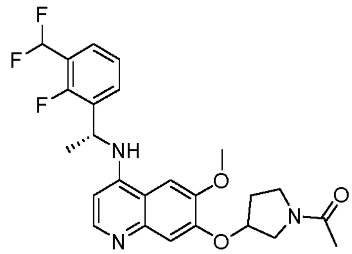 |
| A-15 | 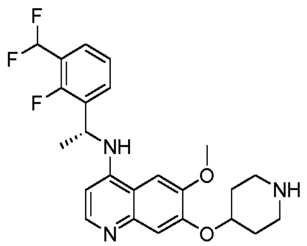 | A-16 | 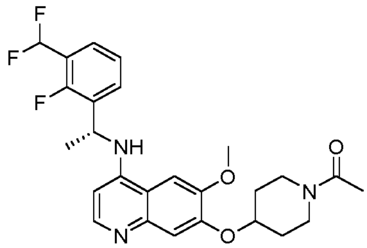 |
| A-17 | 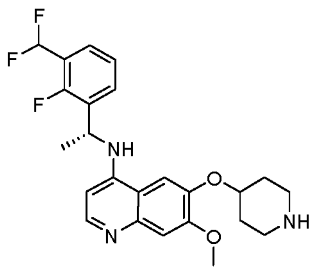 | A-18 | 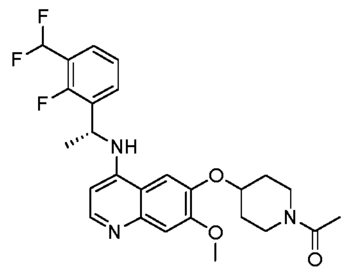 |
| A-19 | 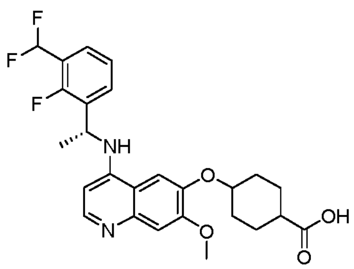 | A-20 | 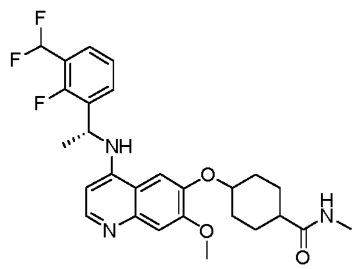 |
| A-21 | 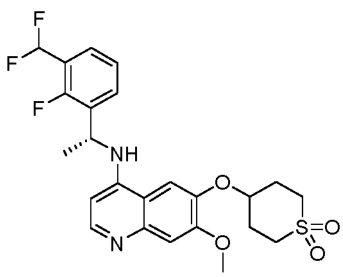 | A-22 | 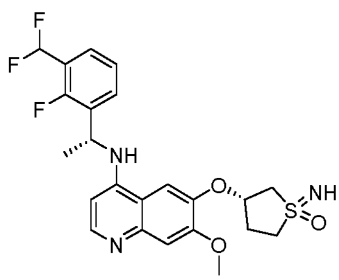 |
| A-23 | 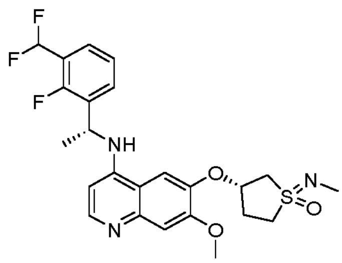 | A-24 | 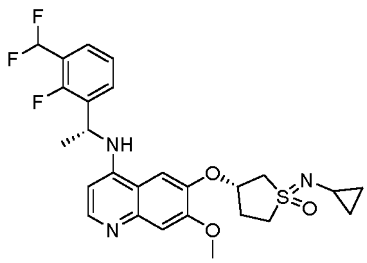 |

(continued)

| No. | Structure | No. | Structure |
|---|---|---|---|
| A-25 | 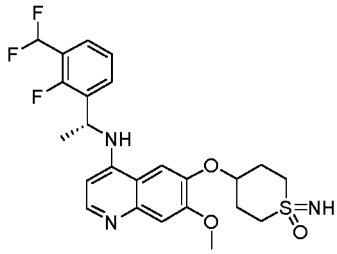 | A-26 | 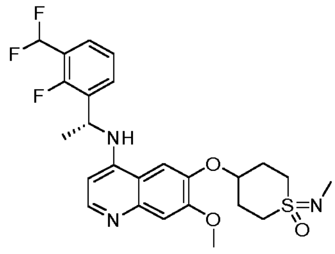 |
| A-27 | 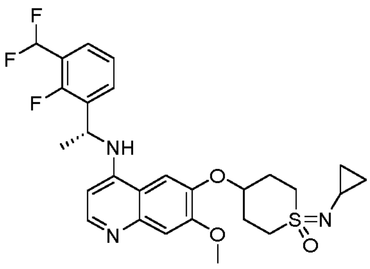 | A-28 | 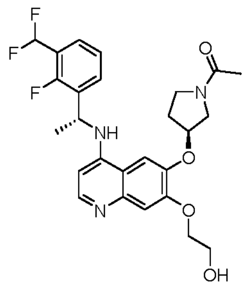 |
| A-29 | 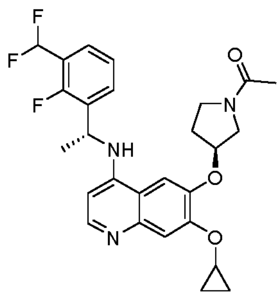 | A-30 | 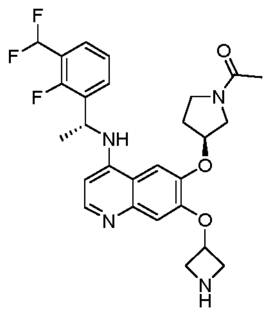 |
| A-31 | 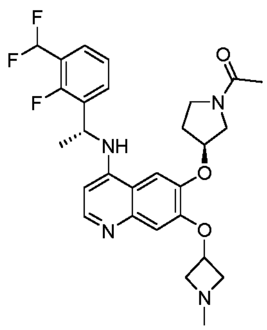 | A-32 | 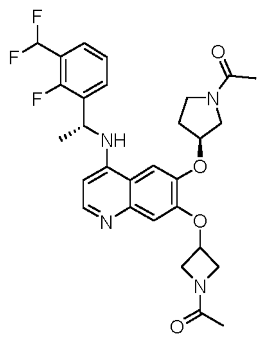 |
| A-33 | 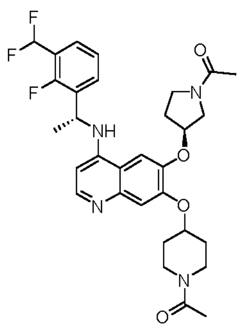 | A-34 | 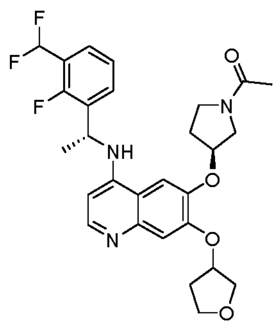 |

(continued)

| No. | Structure | No. | Structure |
|---|---|---|---|
| A-3 5 | 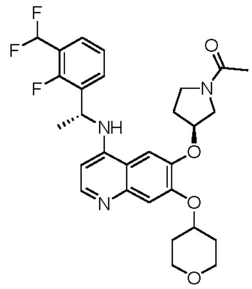 | A-36 | 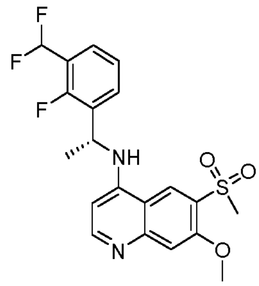 |
| A-37 | 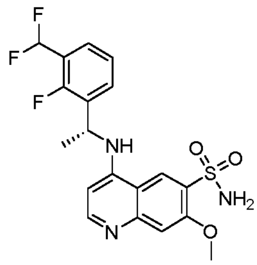 | A-38 | 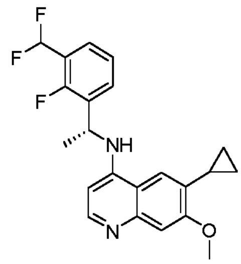 |
| A-39 | 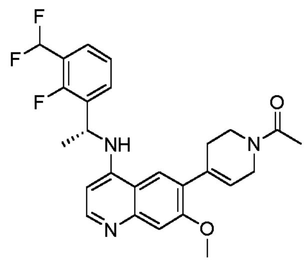 | A-40 | 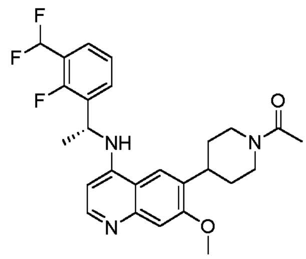 |
| A-41 | 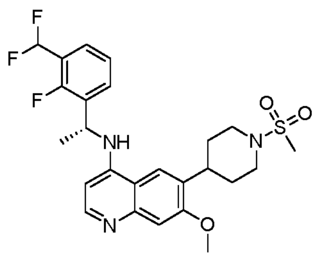 | A-42 | 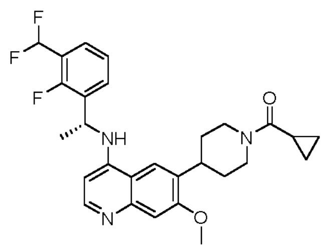 |
| A-43 | 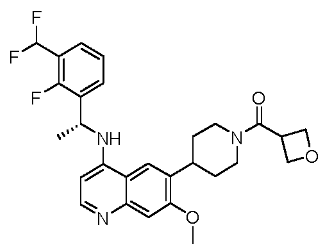 | A-44 | 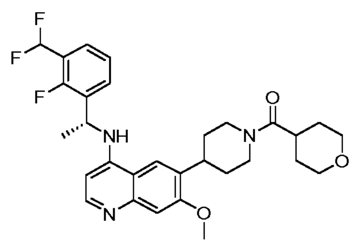 |
| A-45 | 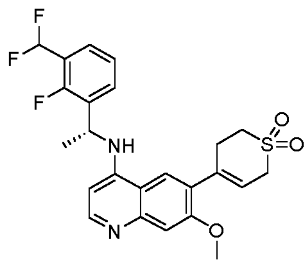 | A-46 | 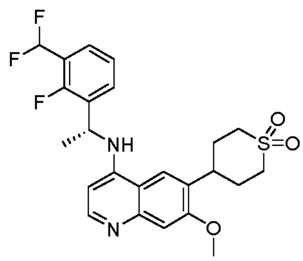 |

(continued)

| No. | Structure | No. | Structure |
|---|---|---|---|
| A-47 | 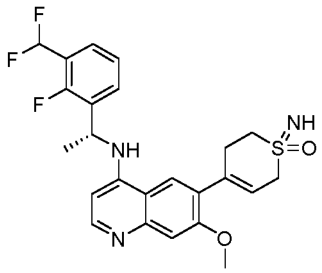 | A-48 | 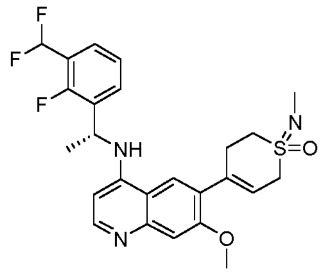 |
| A-49 | 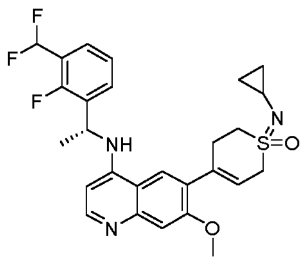 | A-50 | 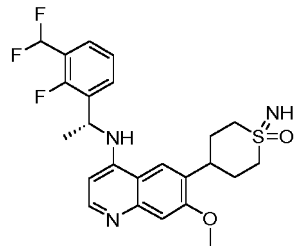 |
| A-51 | 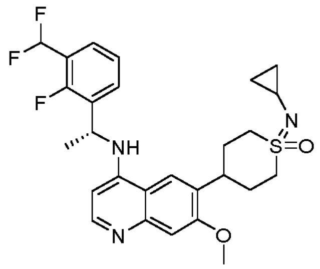 | A-52 | 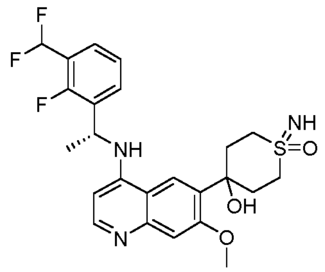 |
| A-53 | 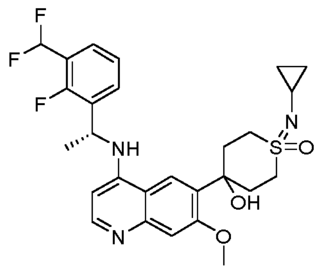 | A-54 | 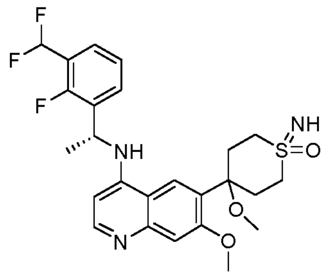 |
| A-55 | 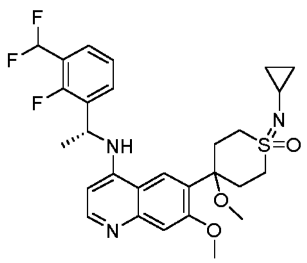 | A-56 | 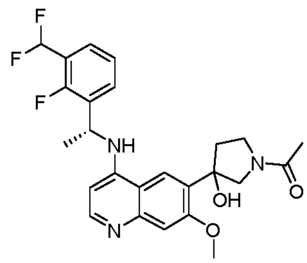 |

(continued)

| No. | Structure | No. | Structure |
|---|---|---|---|
| A-57 | 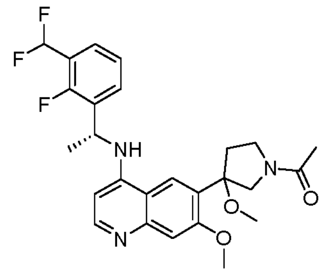 | A-58 | 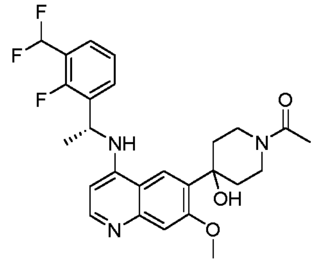 |
| A-59 | 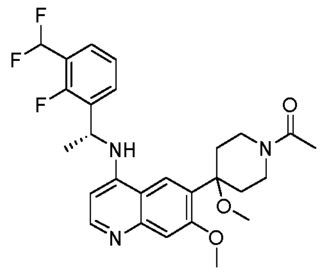 | A-60 | 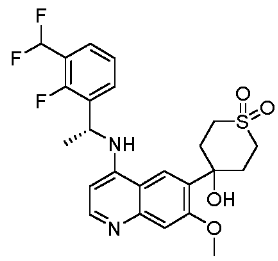 |
| A-61 | 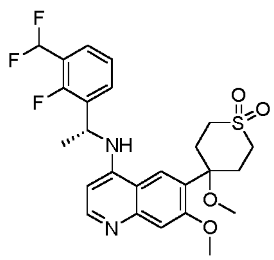 | A-62 | 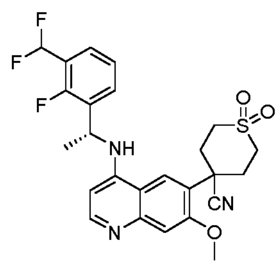 |
| A-63 | 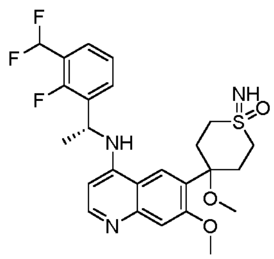 | A-64 | 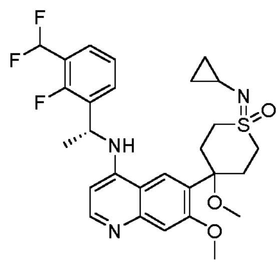 |
| A-65 | 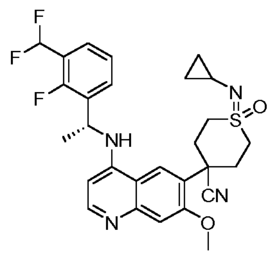 | A-66 | 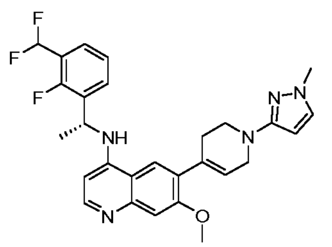 |
| A-67 | 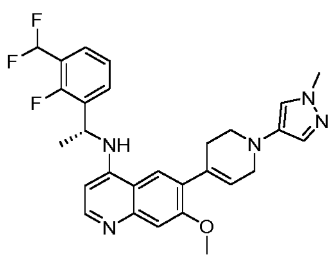 | A-68 | 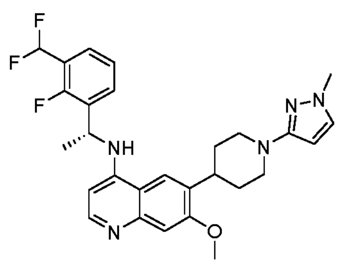 |

(continued)

| No. | Structure | No. | Structure |
|---|---|---|---|
| A-69 | 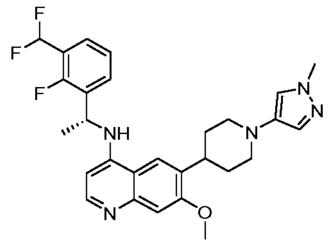 | A-70 | 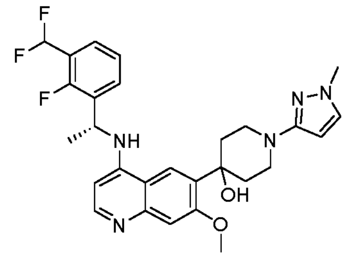 |
| A-71 | 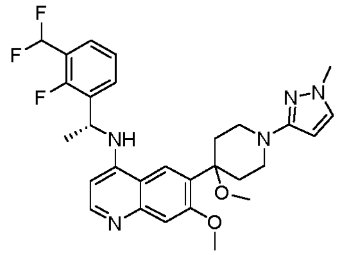 | A-72 | 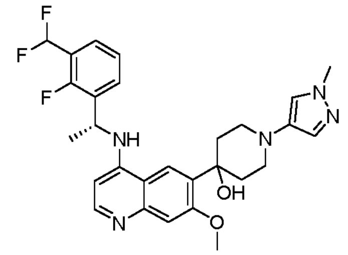 |
| A-73 | 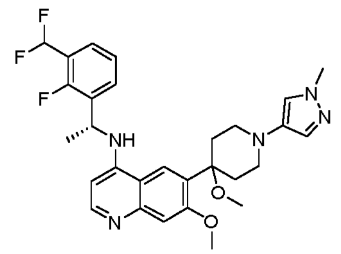 | A-74 | 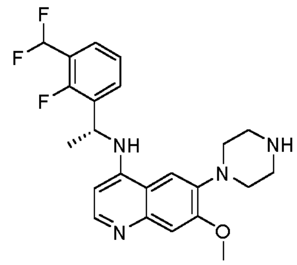 |
| A-75 | 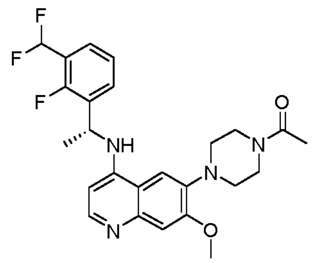 | A-76 | 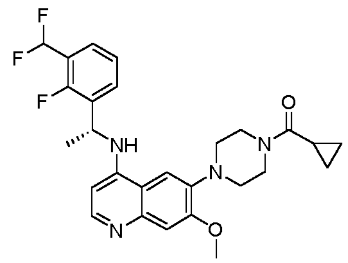 |
| A-77 | 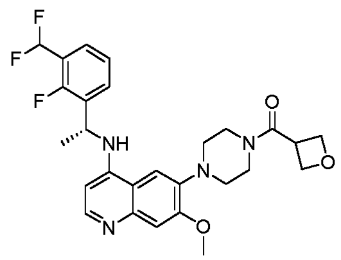 | A-78 | 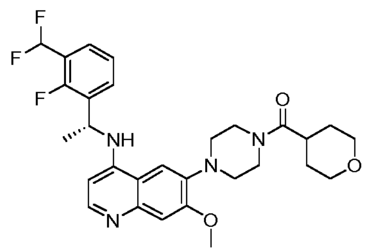 |
| A-79 | 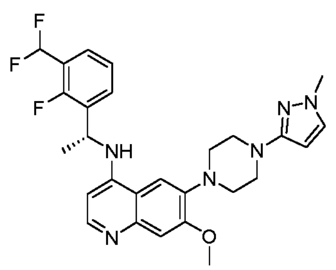 | A-80 | 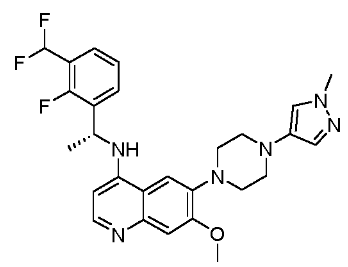 |

(continued)

| No. | Structure | No. | Structure |
|---|---|---|---|
| A-81 | 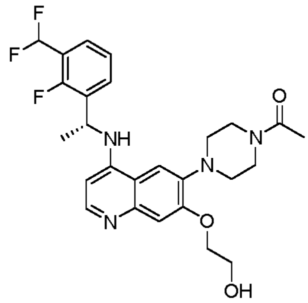 | A-82 | 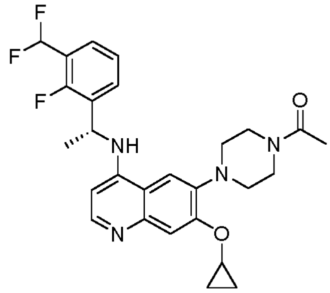 |
| A-83 | 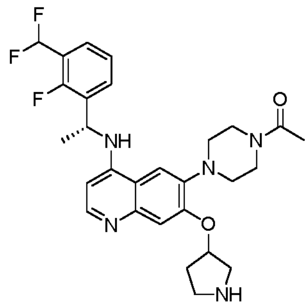 | A-84 | 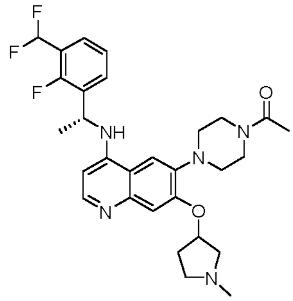 |
| A-85 | 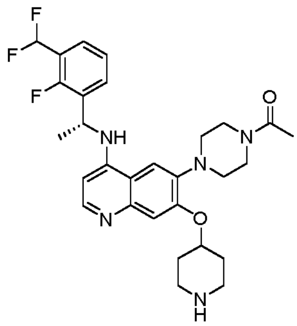 | A-86 | 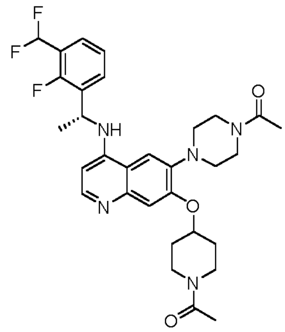 |
| A-87 | 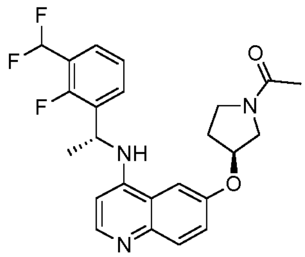 | A-88 | 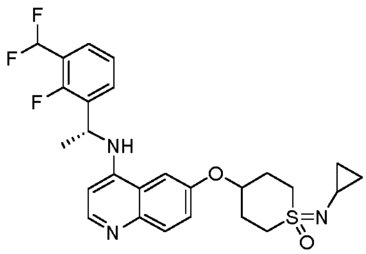 |
| A-89 | 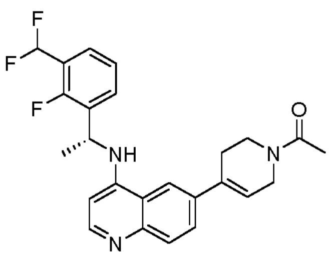 | A-90 | 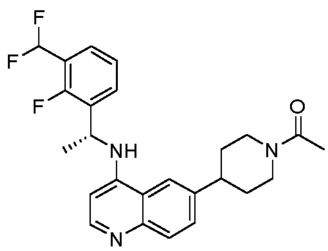 |

(continued)

| No. | Structure | No. | Structure |
|---|---|---|---|
| A-91 | 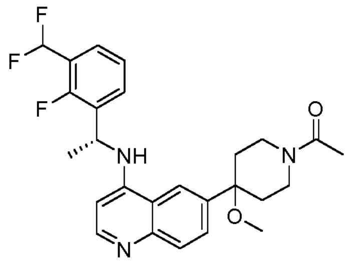 | A-92 | 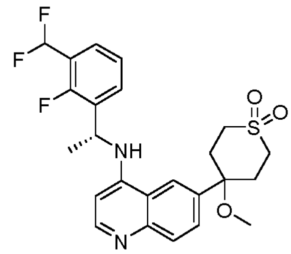 |
| A-93 | 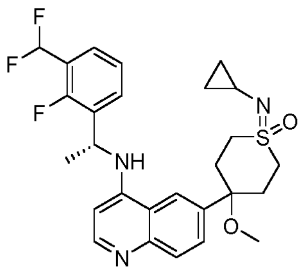 | A-94 | 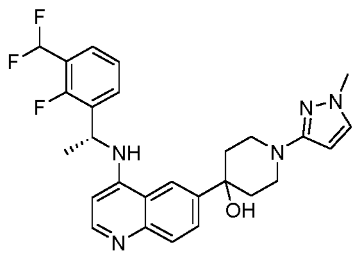 |
| A-95 | 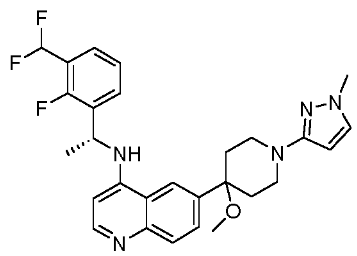 | A-96 | 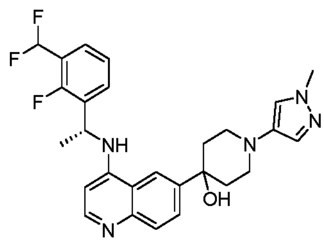 |
| A-97 | 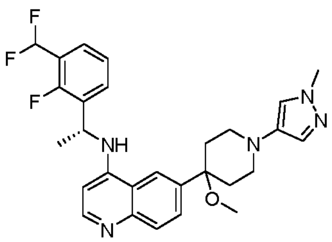 | A-98 | 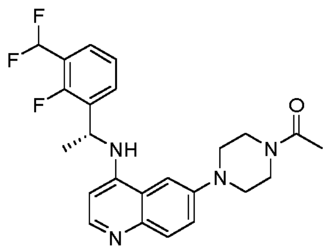 |
| A-99 | 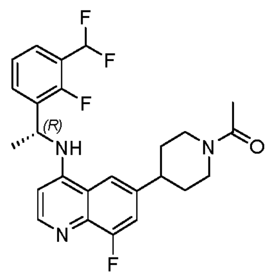 | A-100 | 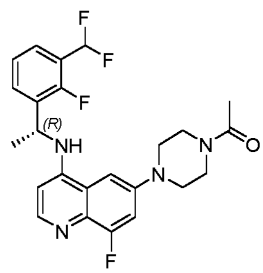 |

(continued)

| No. | Structure | No. | Structure |
|---|---|---|---|
| A-101 | 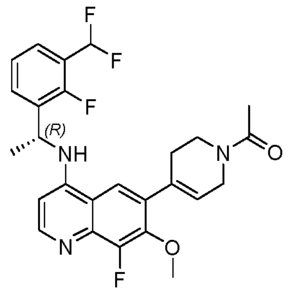 | A-102 | 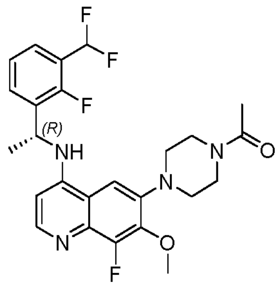 |
| B-1 | 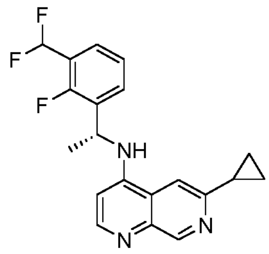 | B-2 | 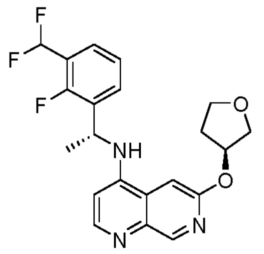 |
| B-3 | 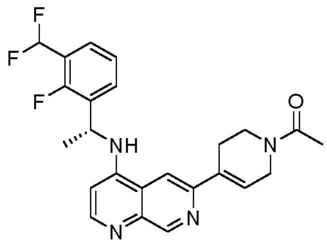 | B-4 | 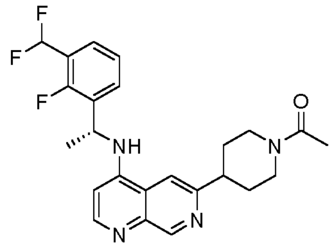 |
| B-5 | 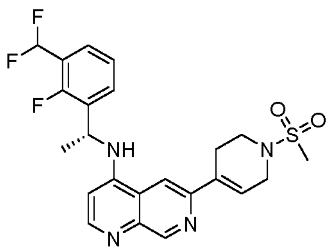 | B-6 | 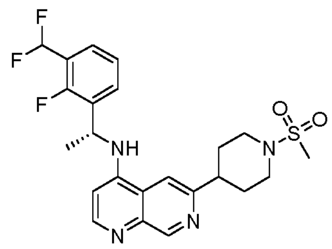 |
| B-7 | 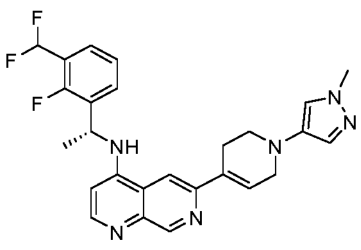 | B-8 | 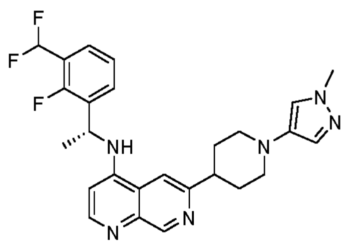 |
| B-9 | 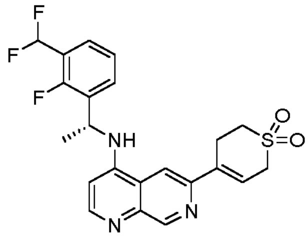 | B-10 | 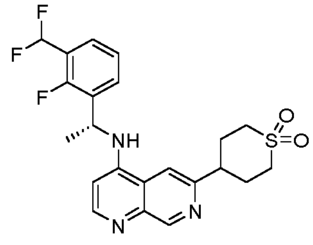 |

(continued)

| No. | Structure | No. | Structure |
|---|---|---|---|
| B-11 | 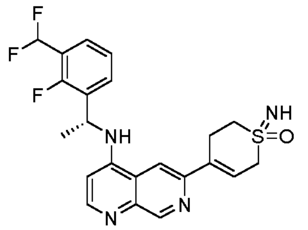 | B-12 | 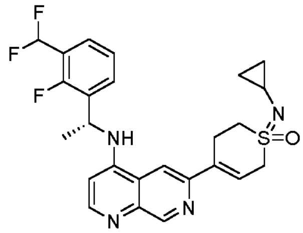 |
| B-13 | 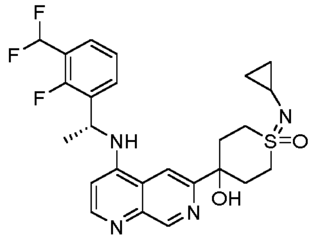 | B-14 | 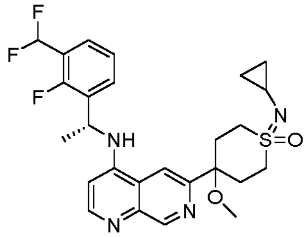 |
| B-15 | 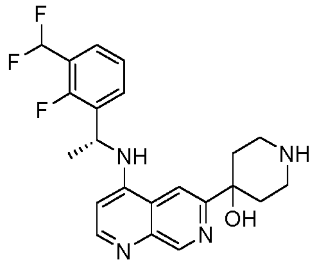 | B-16 | 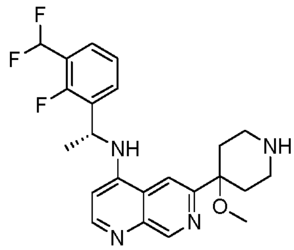 |
| B-17 | 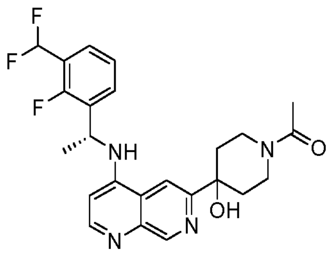 | B-18 | 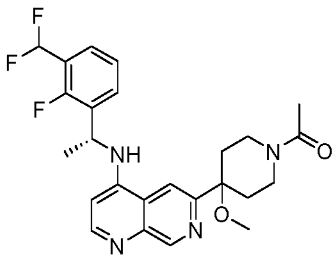 |
| B-19 | 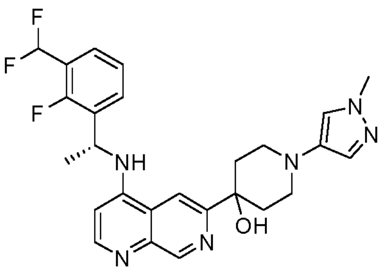 | B-20 | 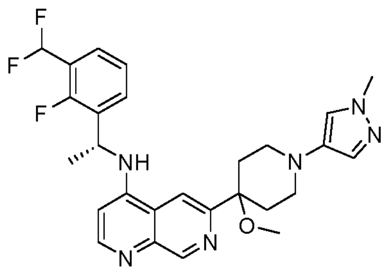 |
| B-21 | 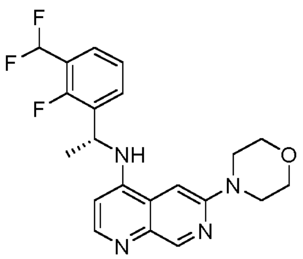 | B-22 | 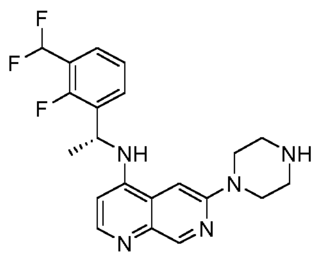 |

(continued)

| No. | Structure | No. | Structure |
|---|---|---|---|
| B-23 | 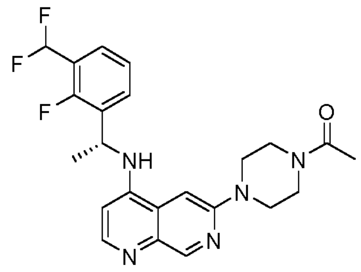 | B-24 | 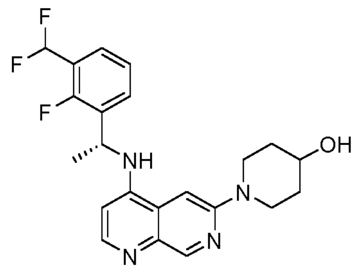 |
| B-25 | 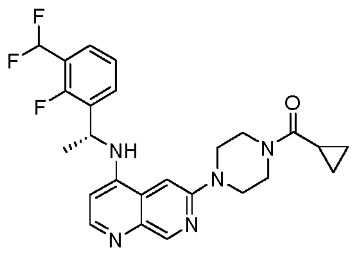 | B-26 | 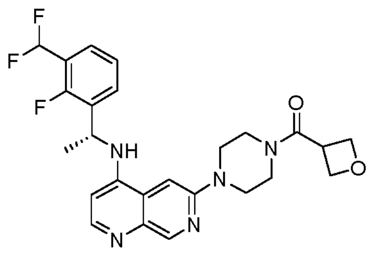 |
| B-27 | 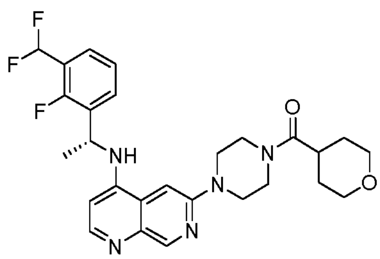 | B-28 | 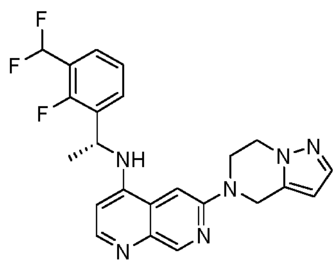 |
| B-29 | 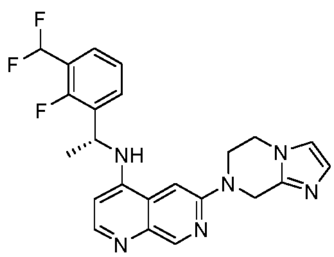 | B-30 | 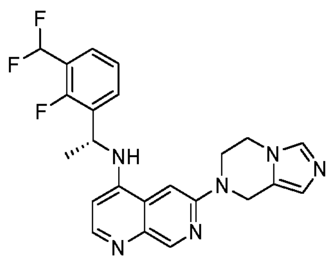 |
| B-31 | 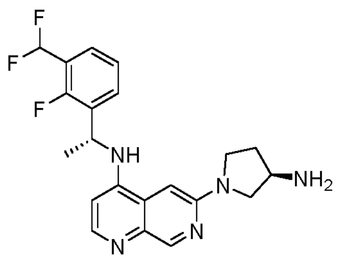 | B-32 | 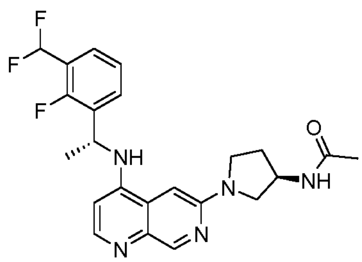 |
| B-33 | 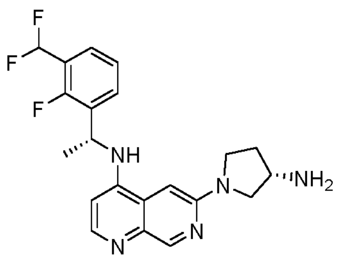 | B-34 | 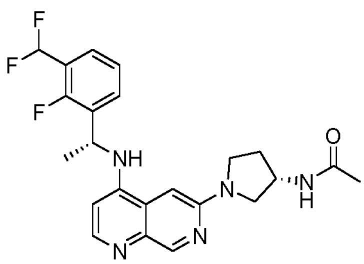 |

(continued)

| No. | Structure | No. | Structure |
|---|---|---|---|
| B-35 | 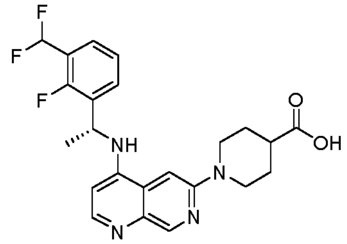 | B-36 | 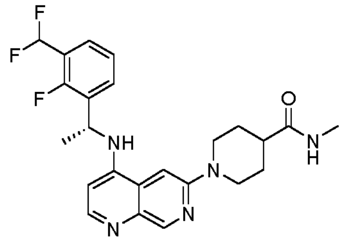 |
| B-37 | 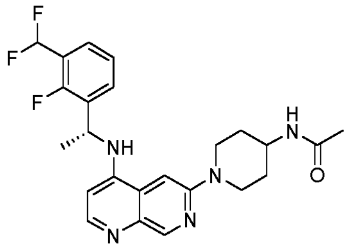 | B-38 | 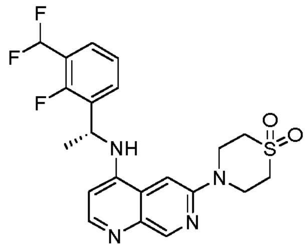 |
| B-39 | 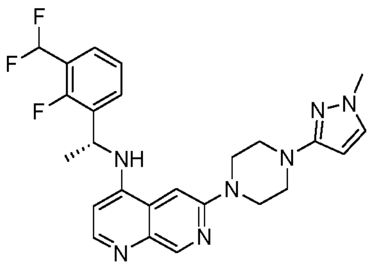 | B-40 | 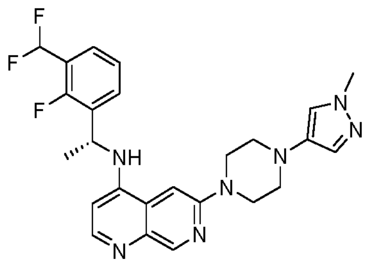 |
| B-41 | 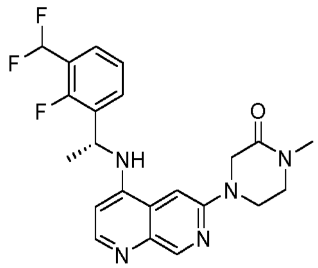 | B-42 | 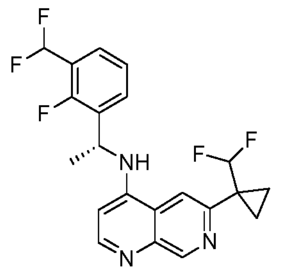 |
| B-43 | 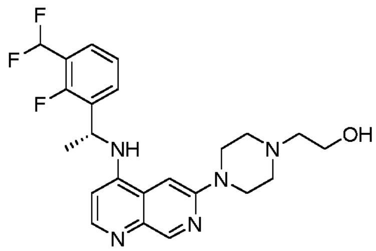 | B-44 | 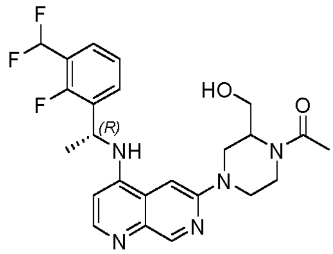 |
| B-45 | 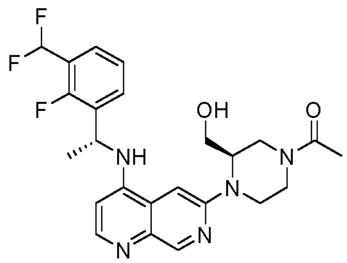 | B-46 | 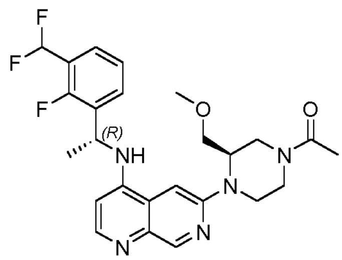 |

(continued)

| No. | Structure | No. | Structure |
|---|---|---|---|
| B-47 | 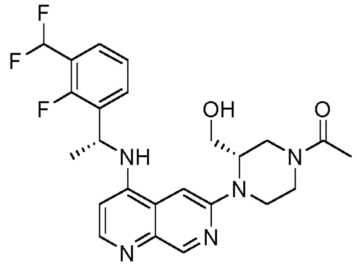 | B-48 | 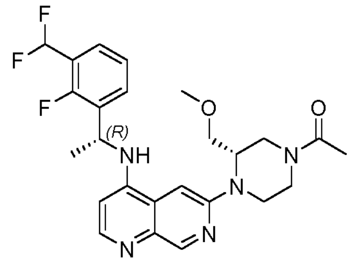 |
| B-49 | 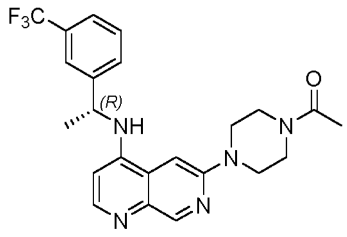 | B-50 | 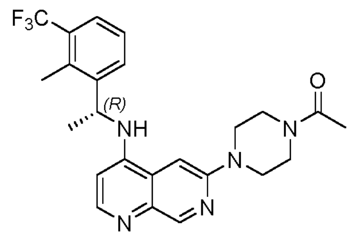 |
| B-51 | 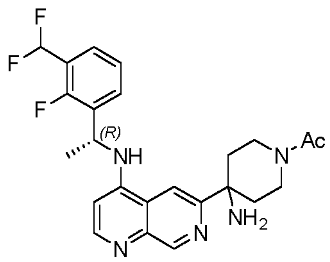 | B-52 | 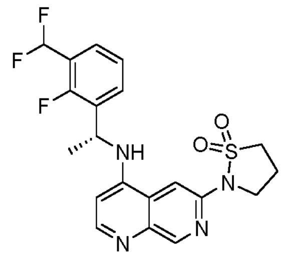 |
| B-53 | 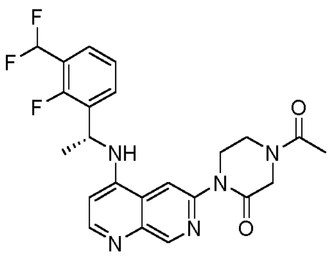 | B-54 | 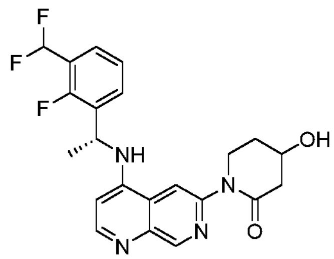 |
| B-55 | 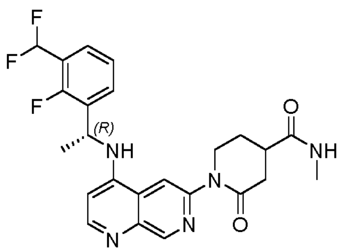 | B-56 | 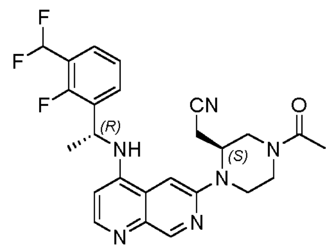 |
| B-57 | 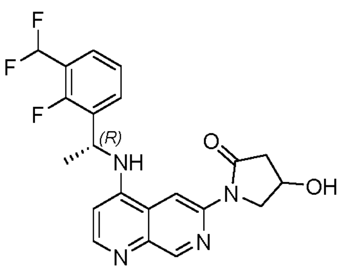 | B-58 | 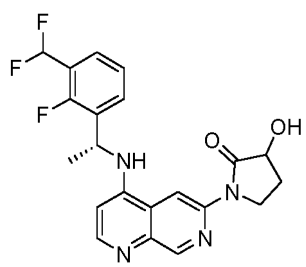 |

(continued)

| No. | Structure | No. | Structure |
|---|---|---|---|
| B-59 | 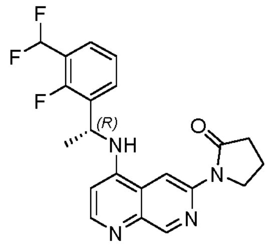 | B-60 | 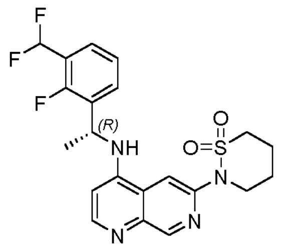 |
| B-61 | 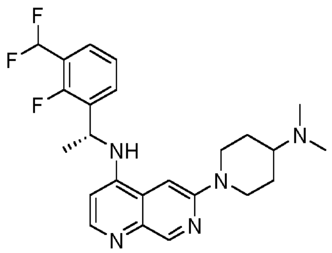 | B-62 | 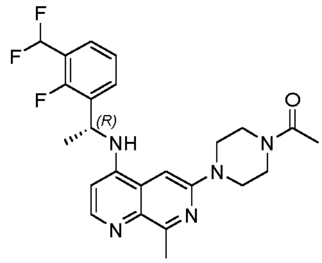 |
| B-63 | 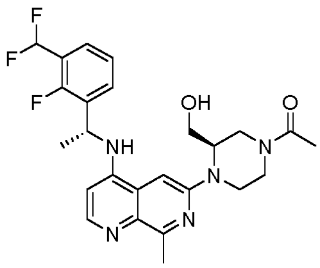 | B-64 | 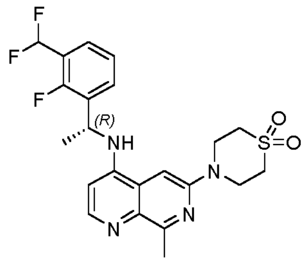 |
| B-65 | 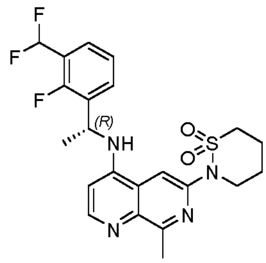 | B-66 | 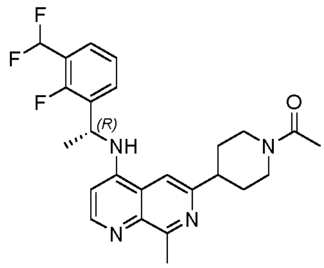 |
| B-67 | 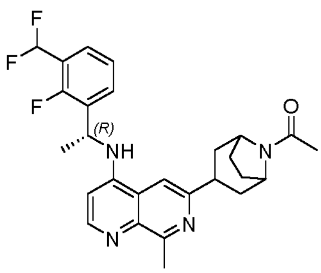 | B-68 | 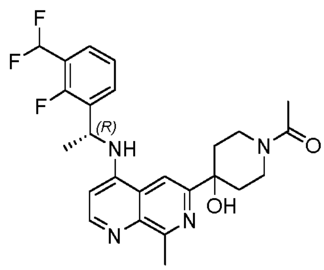 |
| B-69 | 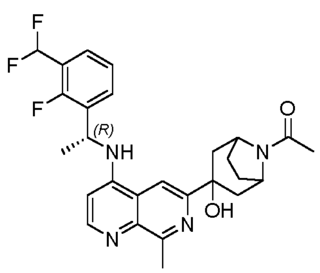 | B-70 | 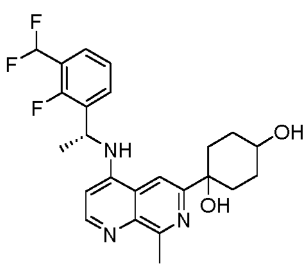 |

(continued)

| No. | Structure | No. | Structure |
|---|---|---|---|
| B-71 | 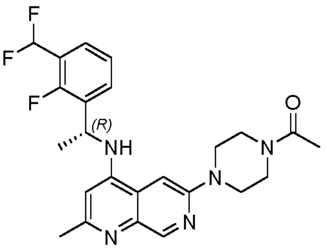 | B-72 | 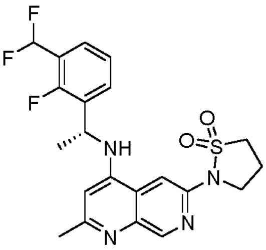 |
| B-73 | 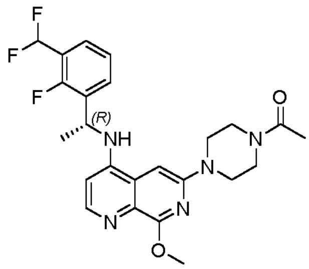 | B-74 | 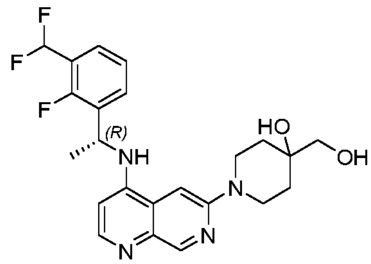 |
| B-75 | 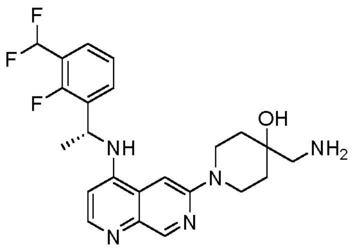 | B-76 | 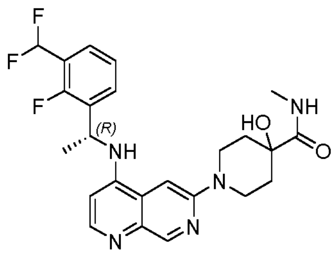 |
| B-77 | 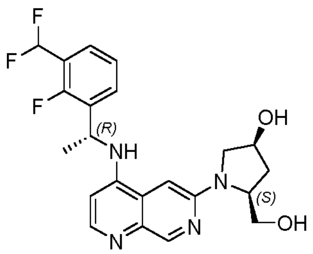 | B-78 | 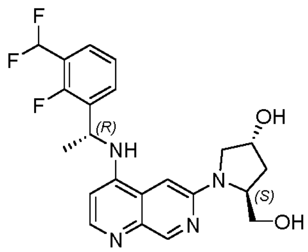 |
| B-79 | 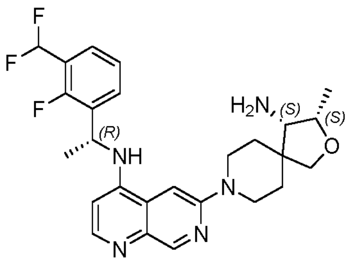 | B-80 | 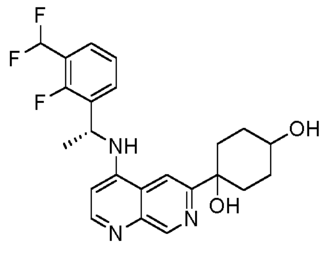 |
| B-81 | 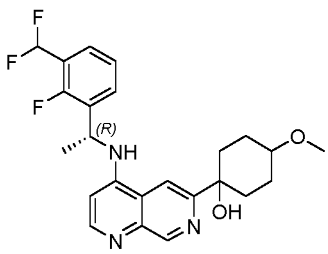 | B-82 | 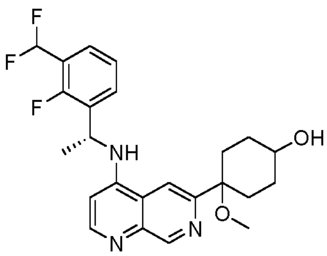 |

(continued)

| No. | Structure | No. | Structure |
|---|---|---|---|
| B-83 | 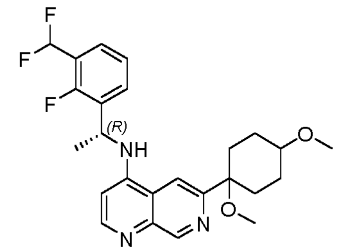 | B-84 | 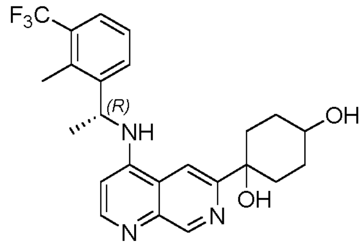 |
| B-85 | 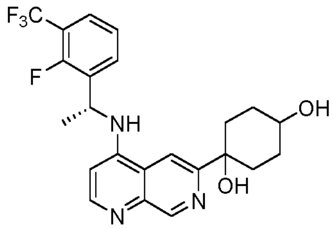 | B-86 | 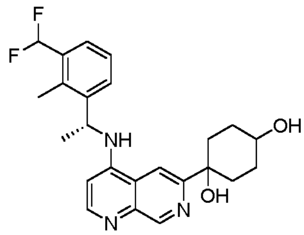 |
| B-87 | 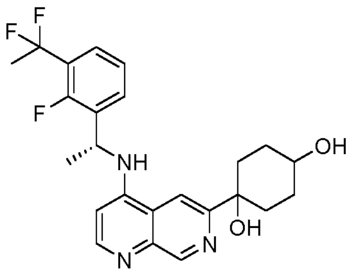 | B-88 | 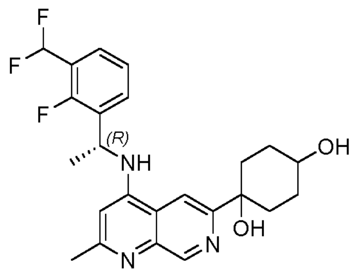 |
| B-89 | 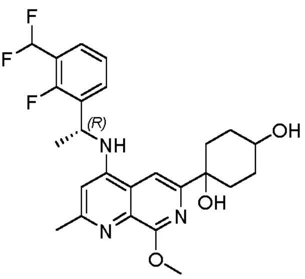 | B-90 | 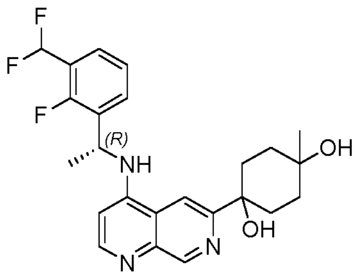 |
| B-91 | 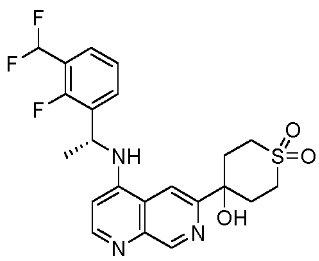 | B-92 | 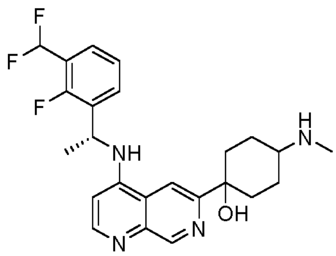 |
| B-93 | 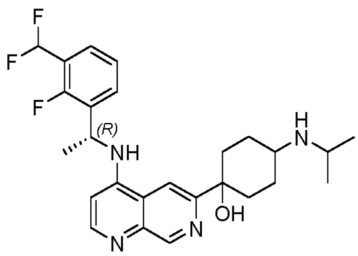 | B-94 | 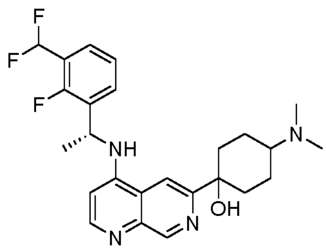 |

(continued)

| No. | Structure | No. | Structure |
|---|---|---|---|
| B-95 | 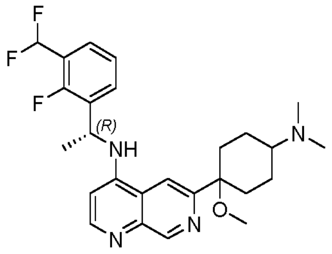 | B-96 | 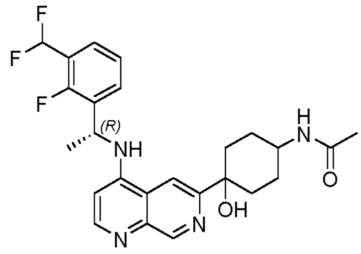 |
| B-97 | 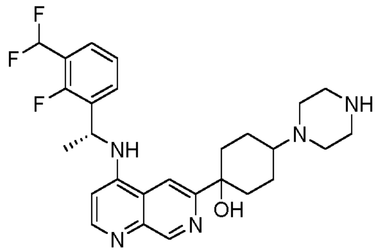 | B-98 | 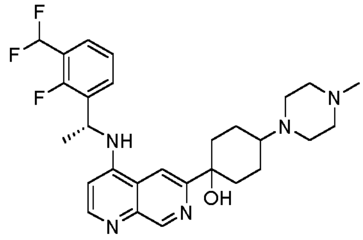 |
| B-99 | 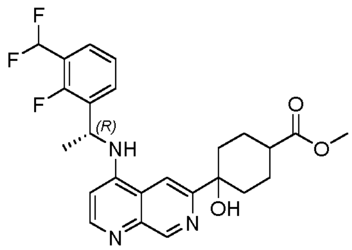 | B-100 | 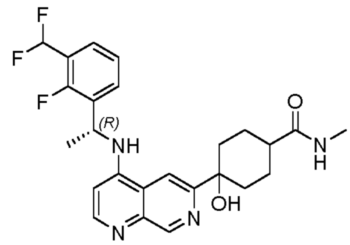 |
| B-101 | 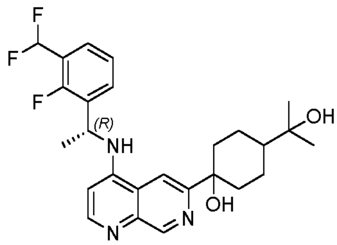 | B-102 | 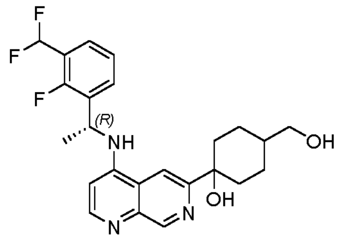 |
| B-103 | 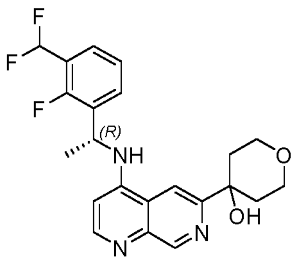 | B-104 | 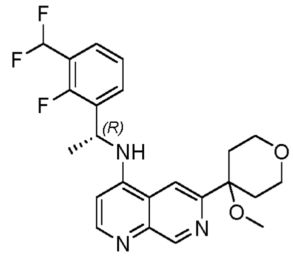 |
| B-105 | 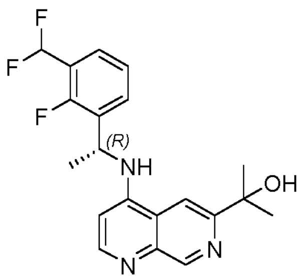 | B-106 | 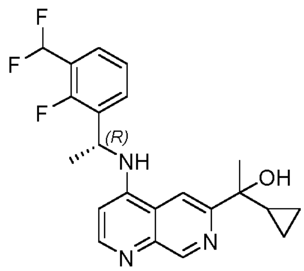 |

(continued)

| No. | Structure | No. | Structure |
|---|---|---|---|
| B-107 | 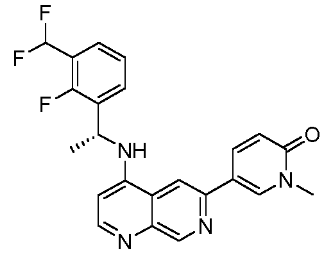 | B-108 | 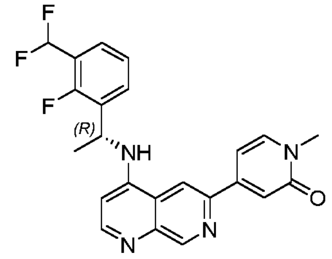 |
| B-109 | 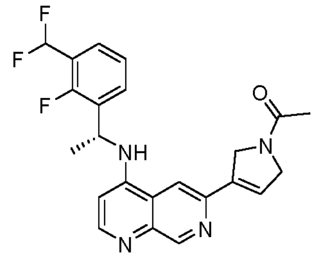 | B-110 | 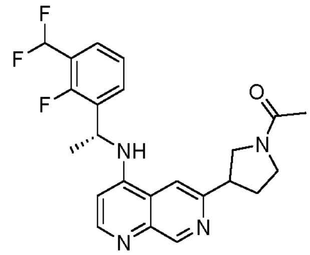 |
| B-111 | 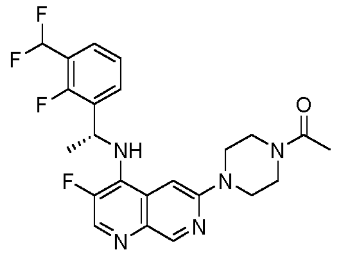 | B-112 | 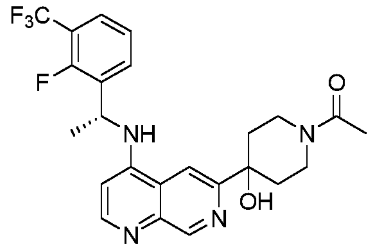 |
| B-113 | 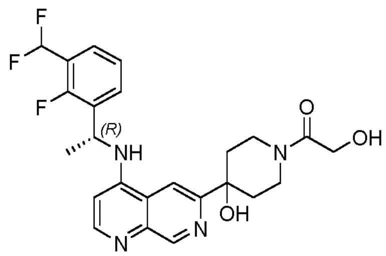 | B-114 | 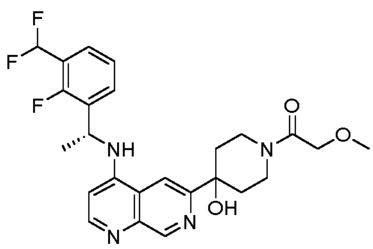 |
| B-115 | 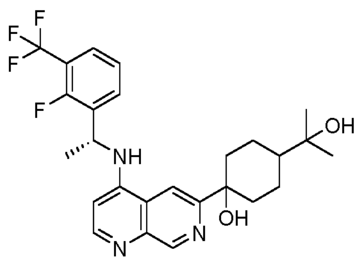 | B-116 | 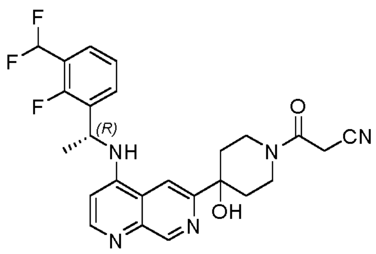 |
| B-117 | 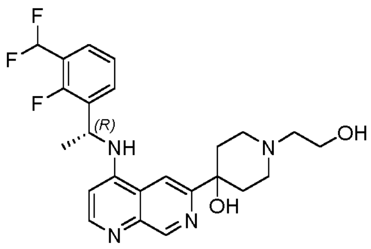 | B-118 | 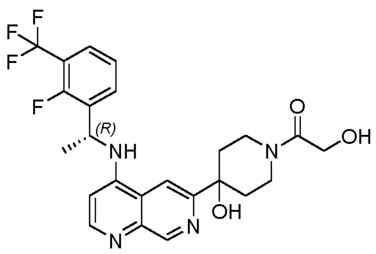 |

(continued)

| No. | Structure | No. | Structure |
|---|---|---|---|
| B-119 | 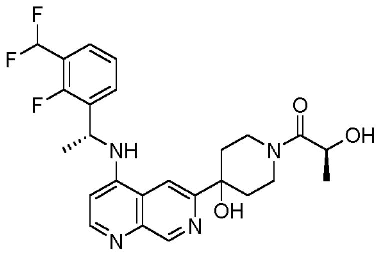 | B-120 | 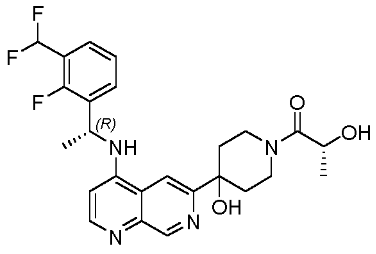 |
| B-121 | 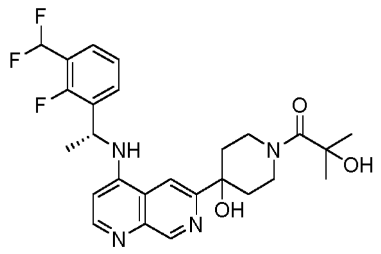 | B-122 | 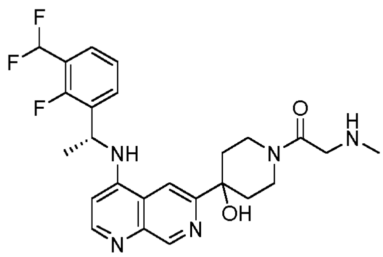 |
| B-123 | 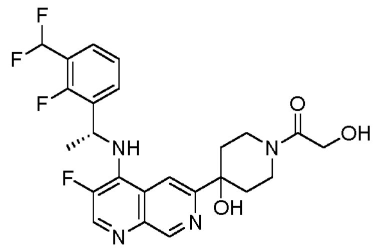 | B-124 | 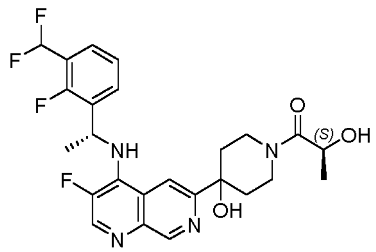 |
| B-125 | 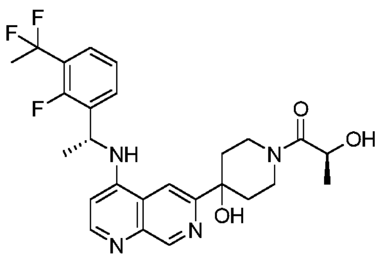 | B-126 | 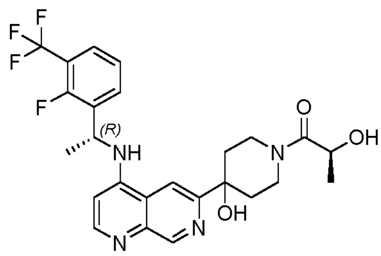 |
| B-127 | 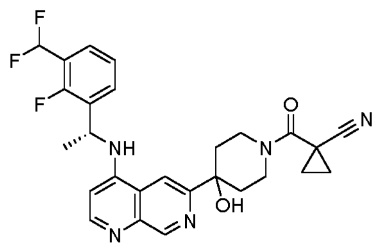 | B-128 | 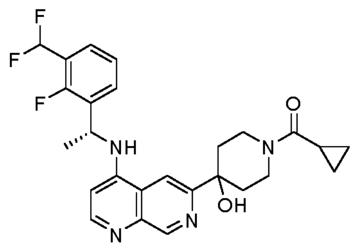 |
| B-129 | 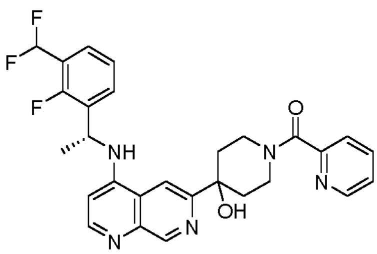 | B-130 | 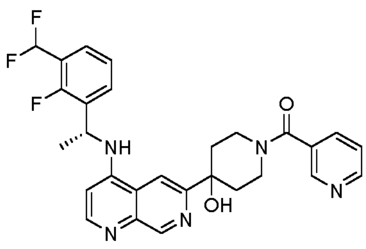 |

(continued)

| No. | Structure | No. | Structure |
|---|---|---|---|
| B-131 | 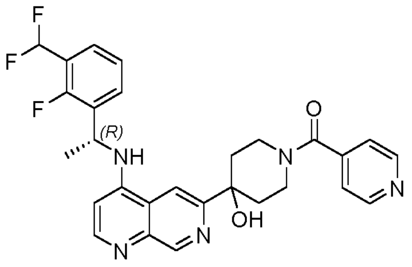 | B-132 | 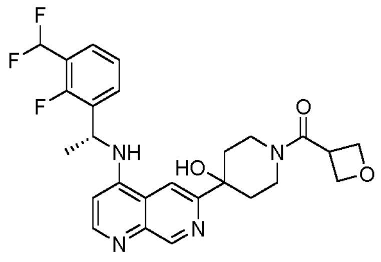 |
| B-133 | 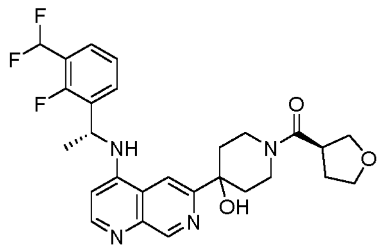 | B-134 | 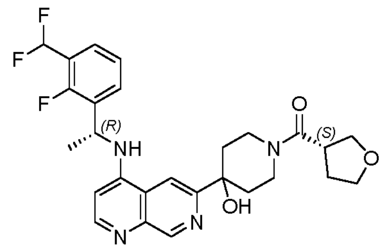 |
| B-135 | 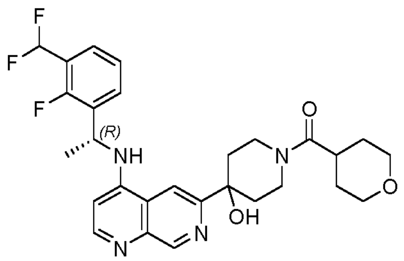 | B-136 | 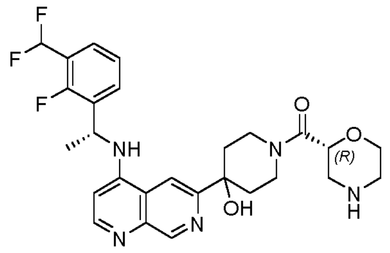 |
| B-137 | 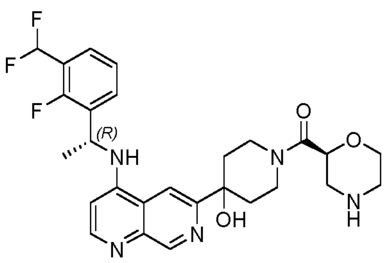 | B-138 | 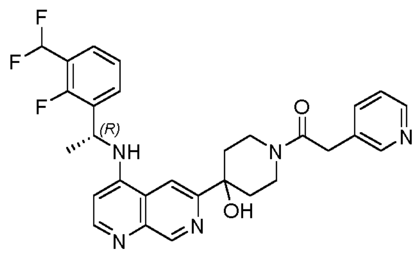 |
| B-139 | 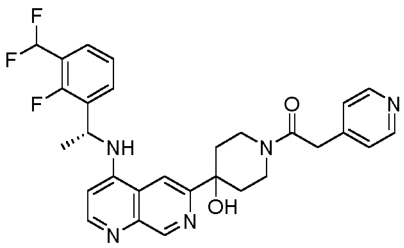 | B-140 | 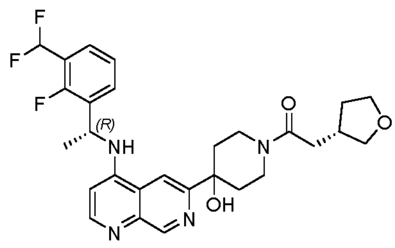 |
| B-141 | 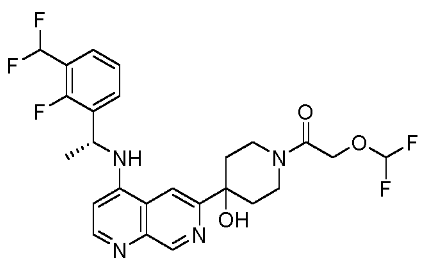 | B-142 | 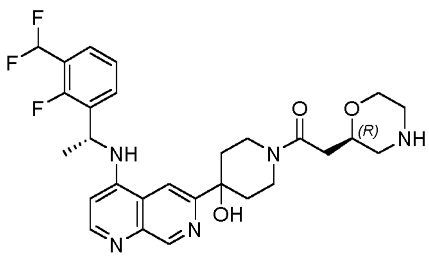 |

(continued)

| No. | Structure | No. | Structure |
|---|---|---|---|
| B-143 | 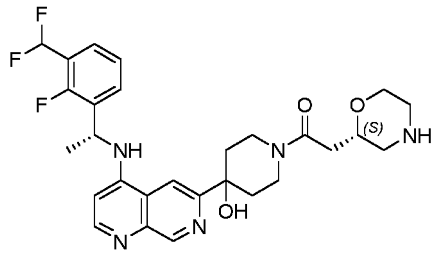 | B-144 | 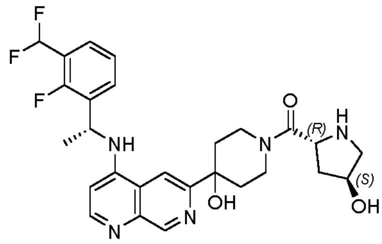 |
| B-145 | 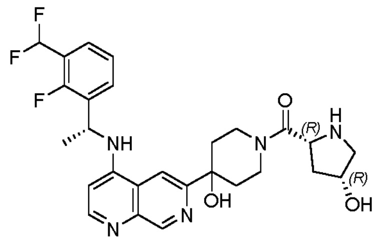 | B-146 | 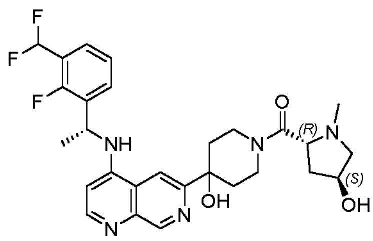 |
| B-147 | 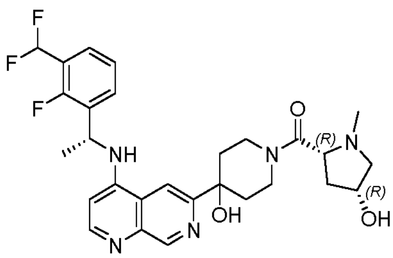 | B-148 | 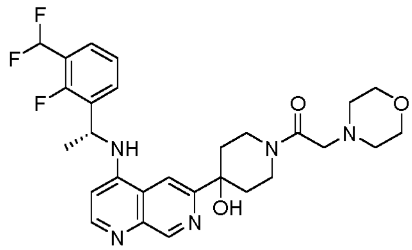 |
| B-149 | 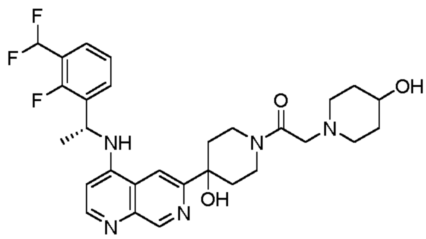 | B-150 | 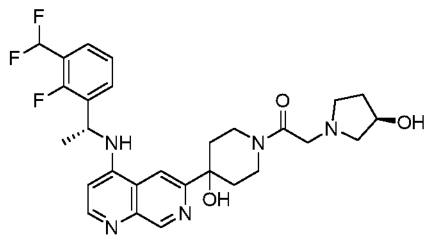 |
| B-151 | 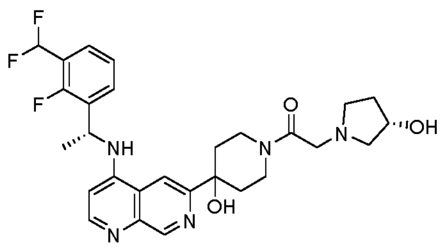 | B-152 | 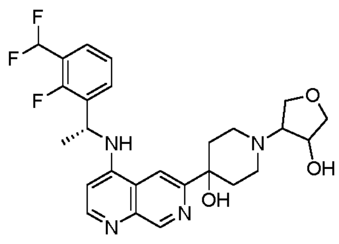 |
| B-153 | 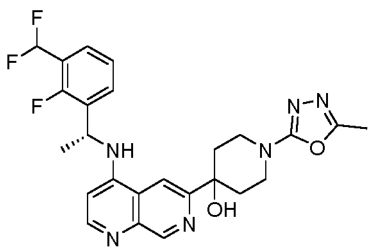 | B-154 | 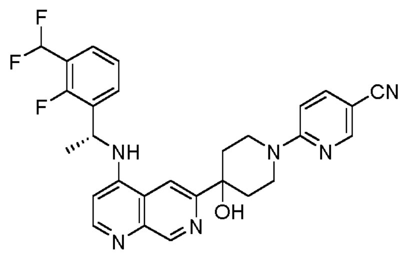 |

(continued)

| No. | Structure | No. | Structure |
|---|---|---|---|
| B-155 | 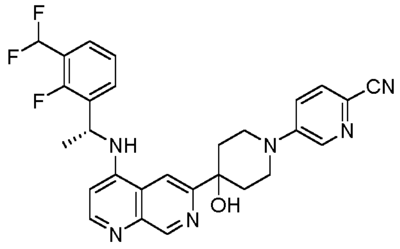 | B-156 | 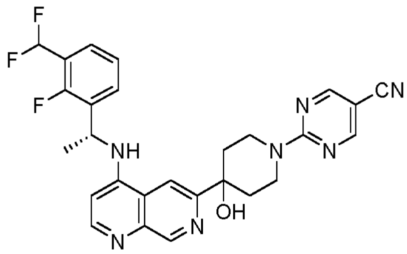 |
| B-157 | 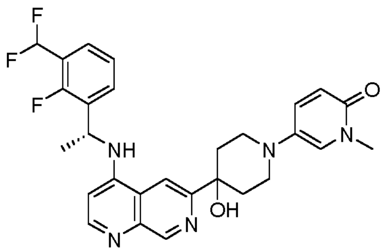 | B-158 | 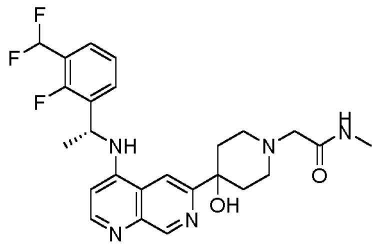 |
| B-159 | 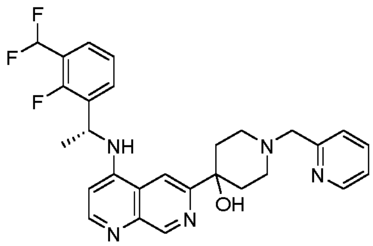 | B-160 | 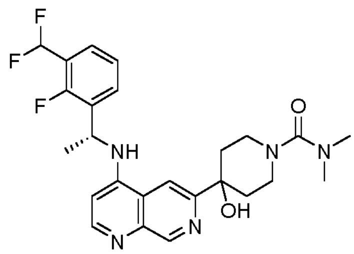 |
| B-161 | 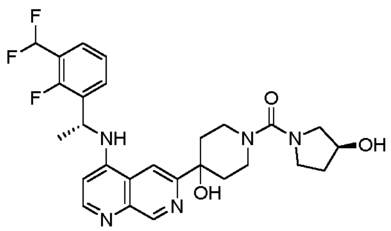 | B-162 | 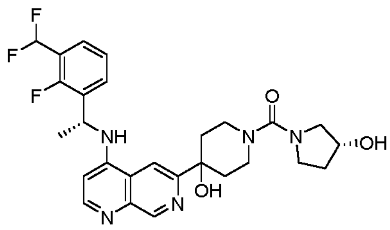 |
| B-163 | 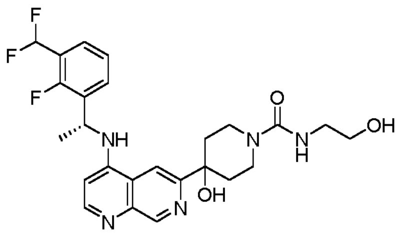 | B-164 | 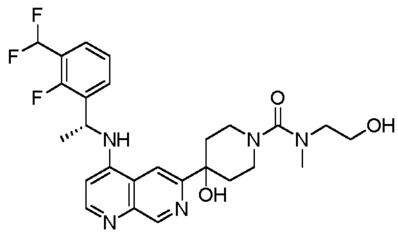 |
| B-165 | 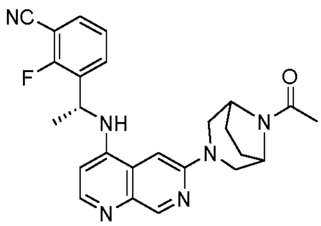 | B-166 | 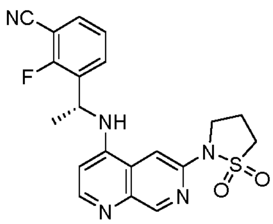 |

(continued)

| No. | Structure | No. | Structure |
|---|---|---|---|
| B-167 | 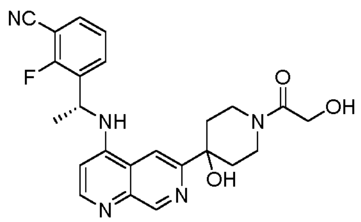 | B-168 | 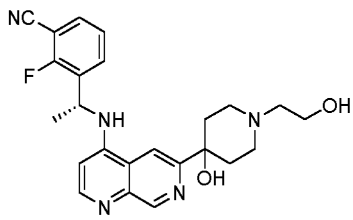 |
| B-169 | 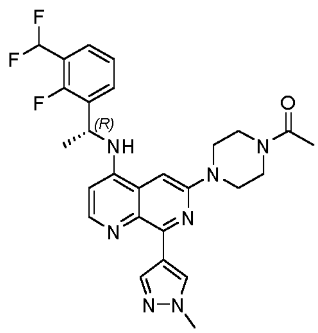 | B-170 | 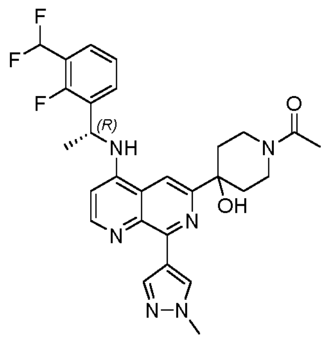 |
| B-171 | 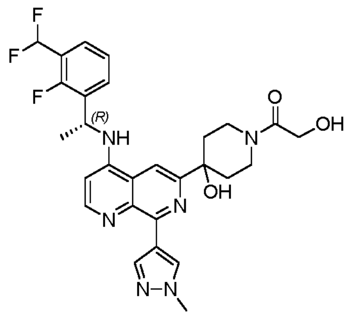 | B-172 | 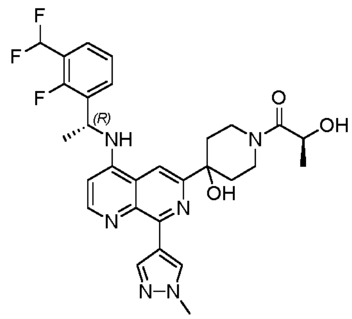 |
| B-173 | 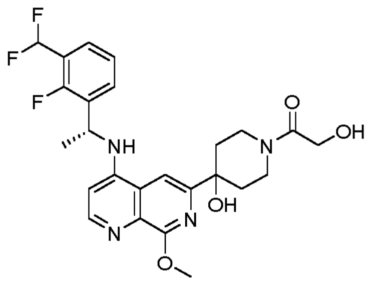 | B-174 | 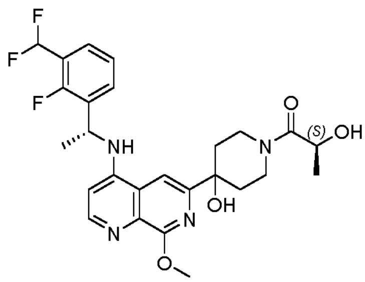 |
| B-175 | 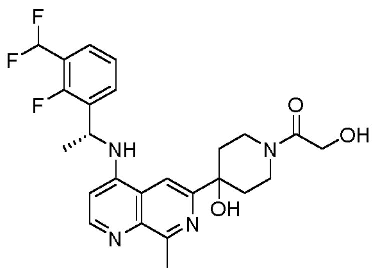 | B-176 | 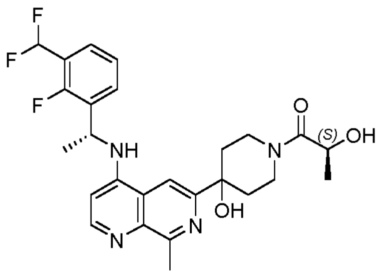 |

(continued)

| No. | Structure | No. | Structure |
|---|---|---|---|
| B-177 | 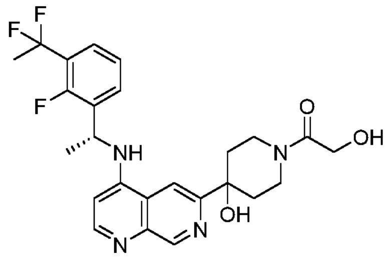 | B-178 | 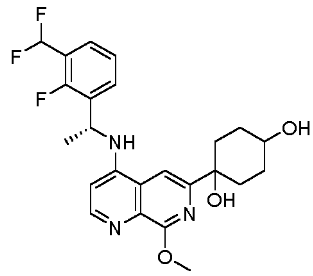 |
| C-1 | 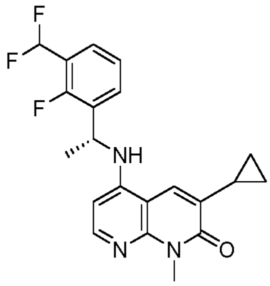 | C-2 | 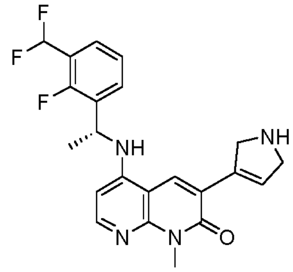 |
| C-3 | 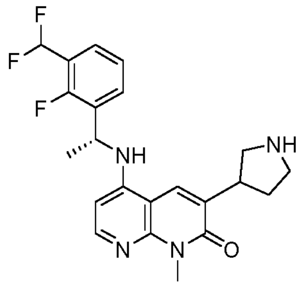 | C-4 | 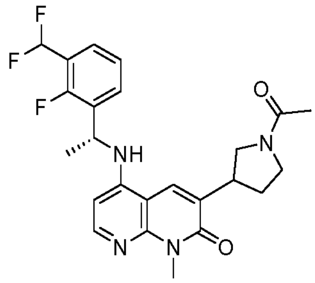 |
| C-5 | 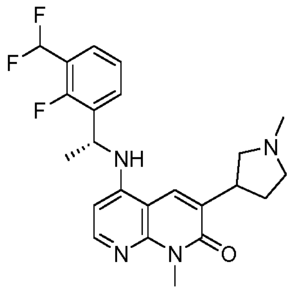 | C-6 | 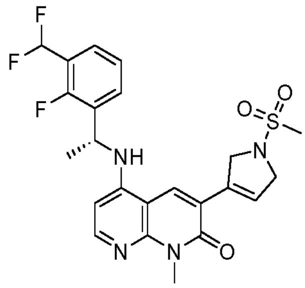 |
| C-7 | 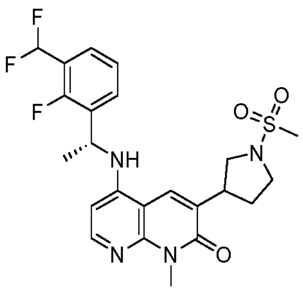 | C-8 | 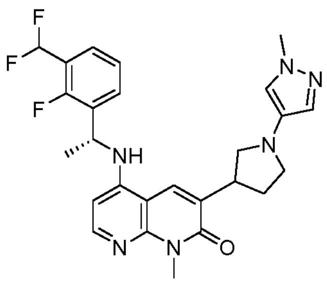 |

(continued)

| No. | Structure | No. | Structure |
|---|---|---|---|
| C-9 | 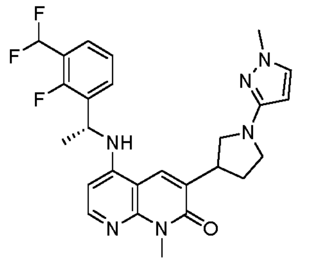 | C-10 | 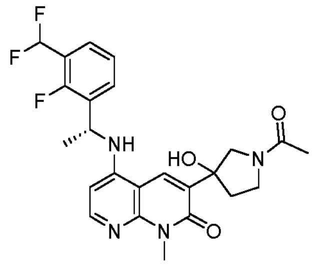 |
| C-11 | 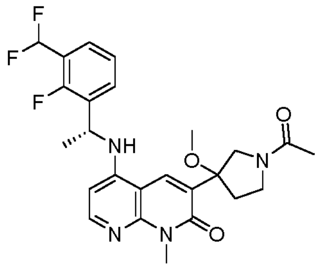 | C-12 | 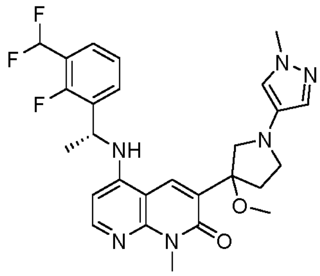 |
| C-13 | 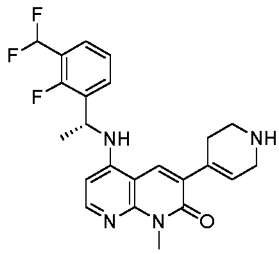 | C-14 | 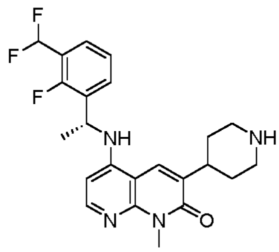 |
| C-15 | 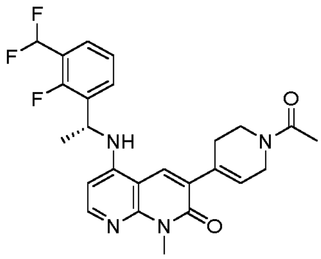 | C-16 | 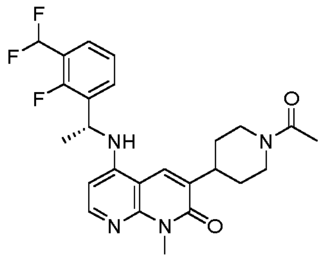 |
| C-17 | 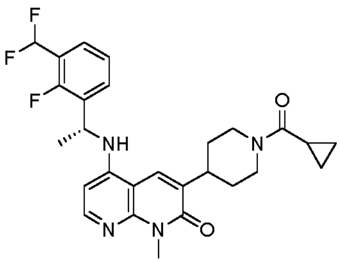 | C-18 | 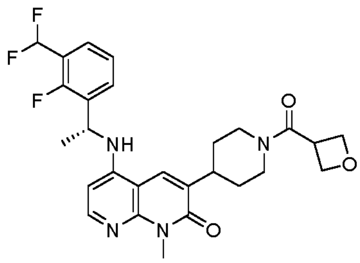 |

(continued)

| No. | Structure | No. | Structure |
|---|---|---|---|
| C-19 | 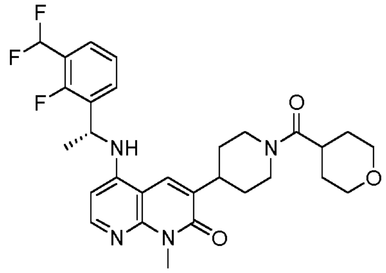 | C-20 | 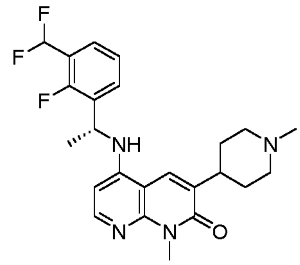 |
| C-21 | 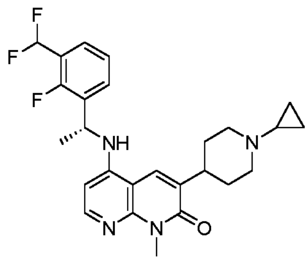 | C-22 | 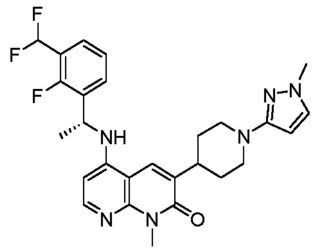 |
| C-23 | 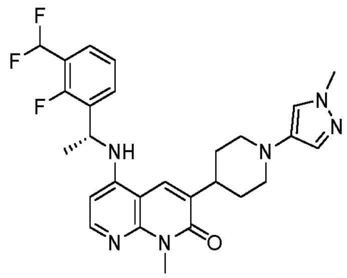 | C-24 | 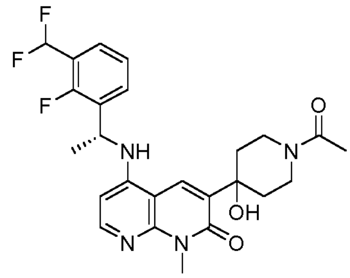 |
| C-25 | 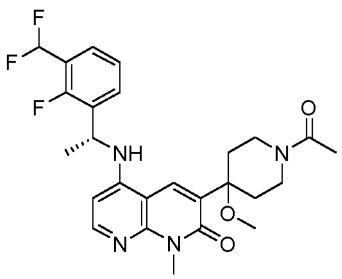 | C-26 | 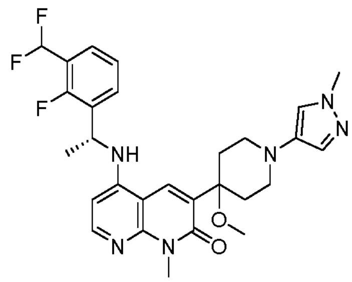 |
| C-27 | 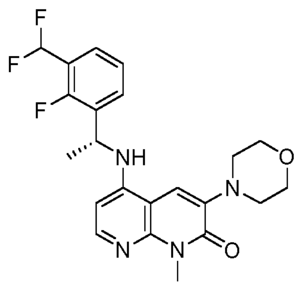 | C-28 | 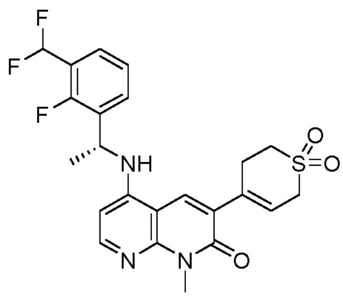 |

(continued)

| No. | Structure | No. | Structure |
|---|---|---|---|
| C-29 | 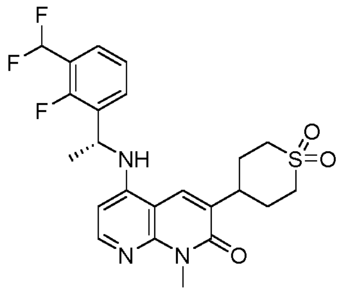 | C-30 | 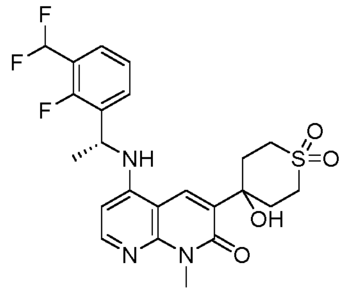 |
| C-31 | 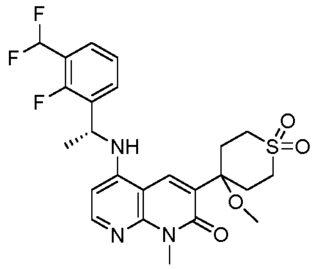 | C-32 | 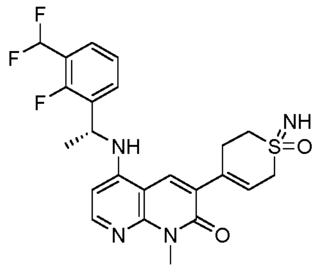 |
| C-33 | 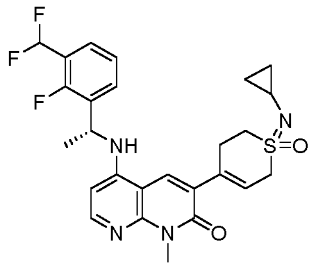 | C-34 | 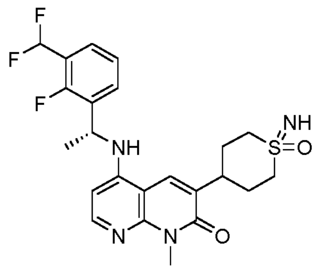 |
| C-3 5 | 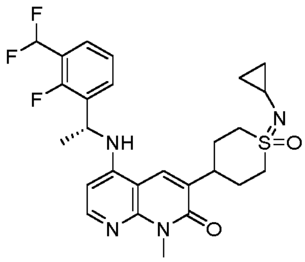 | C-36 | 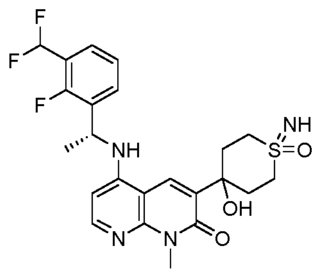 |
| C-37 | 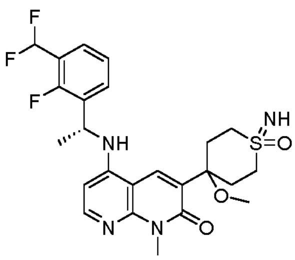 | C-38 | 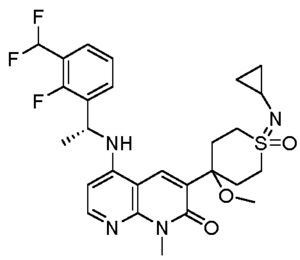 |

(continued)

| No. | Structure | No. | Structure |
|---|---|---|---|
| D-1 | 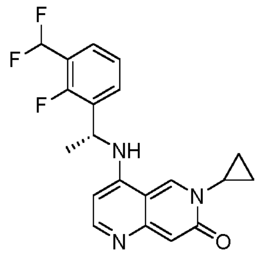 | D-2 | 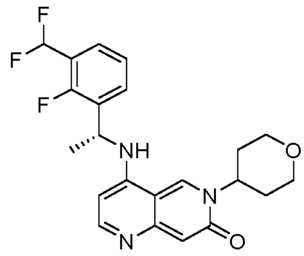 |
| D-3 | 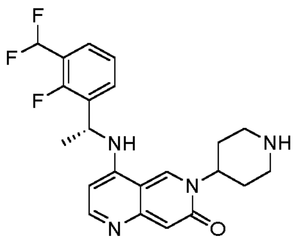 | D-4 | 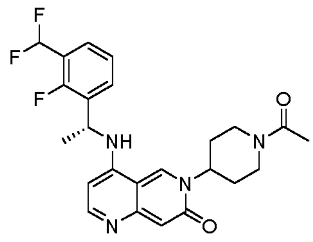 |
| D-5 | 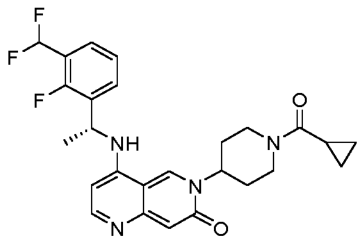 | D-6 | 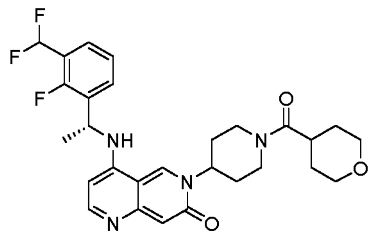 |
| D-7 | 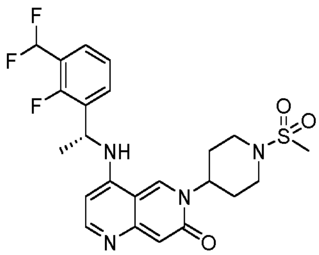 | D-8 | 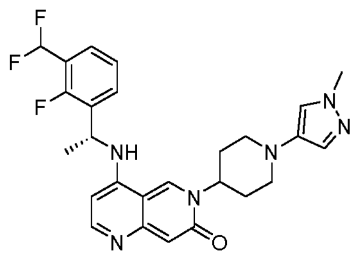 |
| D-9 | 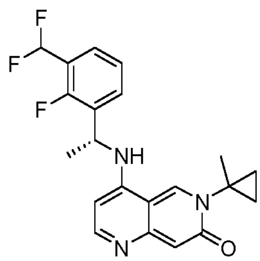 | D-10 | 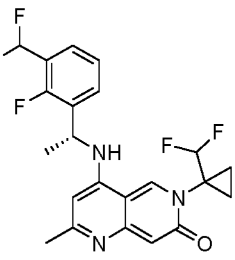 |
| D-11 | 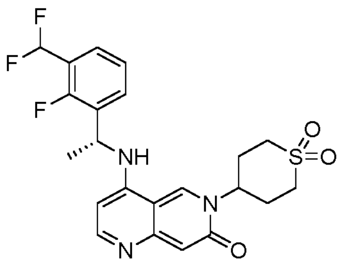 | D-12 | 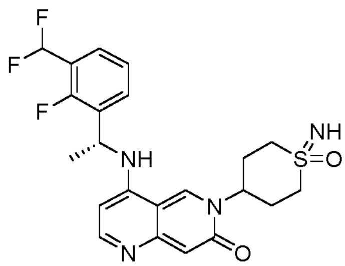 |

(continued)

| No. | Structure | No. | Structure |
|---|---|---|---|
| D-13 | 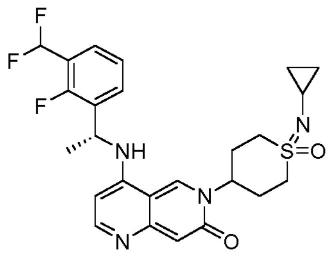 | | |

Another aspect of the present invention provides a pharmaceutical composition, comprising:

(1) a therapeutically effective amount of the compound of formula (I), and the enantiomer, the diastereoisomer, the racemate, the prodrug, the hydrate, the solvate, or the pharmaceutically acceptable salt thereof as an active ingredient; and
(2) a pharmaceutically acceptable carrier.

Another aspect of the present invention provides use of the compound of formula (I), and the enantiomer, the diastereoisomer, the racemate, the prodrug, the hydrate, the solvate, or the pharmaceutically acceptable salt thereof, or the pharmaceutical composition in the preparation of an SOS1 inhibitor.

Another aspect of the present invention provides use of the compound of formula (I), and the enantiomer, the diastereoisomer, the racemate, the prodrug, the hydrate, the solvate, or the pharmaceutically acceptable salt thereof, or the pharmaceutical composition in the preparation of a drug for preventing and/or treating a disease associated with mutation, activity, or expression amount of SOS1.

The disease associated with mutation, activity, or expression amount of SOS1 includes head and neck cancer, lung cancer, a mediastinal tumor, a gastrointestinal tumor, prostate cancer, testicular cancer, a gynecological tumor, breast cancer, renal and bladder cancer, an endocrine tumor, soft tissue sarcoma, osteosarcoma, rhabdomyosarcoma, mesothelioma, skin cancer, a peripheral nerve tumor, a central nervous system tumor, lymphoma, leukemia, Noonan syndrome, cardio-facio-cutaneous syndrome, and hereditary gingival fibromatosis and a related syndrome thereof.

It is to be understood that within the scope of the present invention, the technical features of the present invention and those specifically described below (e.g., examples) may be combined with each other to form a new or preferred technical solution. Each feature disclosed in the description may be replaced by any alternative feature serving the same, equivalent, or similar purpose. Limited by space, it is not repeated herein.

Terms

In the present invention, when a group valence bond has a wavy line

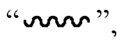

for example, in

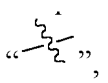

the wavy line indicates a linking site of the group to the rest of the molecule.

In the present invention, the halogen is F, Cl, Br, or I.

The term "substituted" or "substituted with" used herein means that one or more hydrogen atoms on a given atom or group are replaced with one or more substituents selected from a given group of substituents, provided that the normal valence of the given atom is not exceeded.

In the present invention, unless otherwise specified, the used terms have the ordinary meaning known to those skilled in the art.

In the present invention, the term "$C_{1-6}$" means having 1, 2, 3, 4, 5, or 6 carbon atoms, and "$C_{1-8}$" means having

1, 2, 3, 4, 5, 6, 7, or 8 carbon atoms, and so on. "3-8 membered" heterocyclyl means that there are 3-8 ring atoms in the heterocyclyl, and "4-10 membered heterocyclyl" and the like have the similar meaning.

In the present invention, the term "alkyl" indicates a saturated linear or branched-chain hydrocarbyl moiety, for example, the term "$C_{1-10}$ alkyl" means a straight-chain or branched-chain alkyl group having 1 to 10 carbon atoms, including, but not limited to, methyl, ethyl, propyl, isopropyl, butyl, isobutyl, sec-butyl, tert-butyl, pentyl, hexyl and the like, preferably, methyl, ethyl, propyl, isopropyl, butyl, isobutyl, sec-butyl, and tert-butyl. In the present invention, unless otherwise specified, the $C_{1-10}$ alkyl is preferably $C_{1-6}$ alkyl, more preferably $C_{1-4}$ alkyl.

In the present invention, the term "alkoxy" indicates the alkyl defined herein is linked to an oxygen atom. For example, the term "$C_{1-6}$ alkoxy" indicates a straight-chain or branched-chain alkoxy group having 1 to 6 carbon atoms, i.e., -O-($C_{1-6}$ alkyl), including, but not limited to methoxy, ethoxy, propoxy, isopropoxy, butoxy and the like.

In the present invention, the term "alkenyl" indicates a straight-chain or branched-chain hydrocarbyl moiety containing at least one double bond, for example, the term "$C_{2-6}$ alkenyl" refers to a straight-chain or branched-chain alkenyl group having 2 to 6 carbon atoms containing at least one double bond, including, but not limited to ethenyl, propenyl, butenyl, isobutenyl, pentenyl, hexenyl and the like.

In the present invention, the term "cycloalkyl" indicates a saturated cyclic hydrocarbyl moiety, for example, the term "$C_{3-8}$ cycloalkyl" refers to a cyclic alkyl group having 3 to 8 carbon atoms in the ring, including, but not limited to cyclopropyl, cyclobutyl, cyclopentyl, cyclohexyl, cycloheptyl, cyclooctyl, cyclodecyl and the like.

The term "aryl" used herein refers to a carbocyclic hydrocarbyl group consisting of one ring or multiple, e.g., two, fused rings, wherein at least one ring is aromatic, e.g., $C_{6-10}$ aryl. Examples of the aryl includes, but is not limited to, phenyl, naphthyl and the like.

In the present application, the term "heterocyclyl" indicates a non-aromatic cyclic group containing at least one carbon atom and at least one (e.g. 1-3) heterocyclic atom selected from N, O, and S, wherein the sulfur atom may optionally be oxidized. Examples of the "heterocyclyl" are specifically groups formed by replacement of one or more cyclic carbons of cycloalkyl as defined herein by a moiety selected from-O-, -N=, -NR-, -C(O)-, -S(O)-, $-S(O)_2-$,

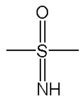 , and 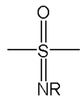 ,

wherein the R is hydrogen, $C_{1-4}$ alkyl, $C_{3-6}$ cycloalkyl, or a nitrogen protecting group (e.g. benzyloxycarbonyl, p-methoxybenzylcarbonyl, t-butoxycarbonyl, acetyl, benzoyl, benzyl, p-methoxy-benzyl, p-methoxy-phenyl, 3,4-dimethoxybenzyl, etc.). The term "heterocyclyl" comprises a monocyclic ring and a fused ring, a bridged ring, a spiro ring, and other bicyclic structures and can be partially or fully saturated, e.g., 4-10 membered saturated or unsaturated heterocyclyl, 4-6 membered saturated or unsaturated heterocyclyl, 3-8 membered heterocyclyl, 3-6 membered heterocyclyl and the like, such as tetrahydrofuranyl, pyrrolidinyl, oxacyclobutyl, oxacyclohexyl, azacyclobutyl, oxiranyl, aziridinyl, thiacyclobutyl, 1,2-dithiacyclobutyl, 1,3-dithiacyclobutyl, azacycloheptyl, oxacycloheptyl and the like. For example, the heterocyclyl described herein may be selected from the following group:

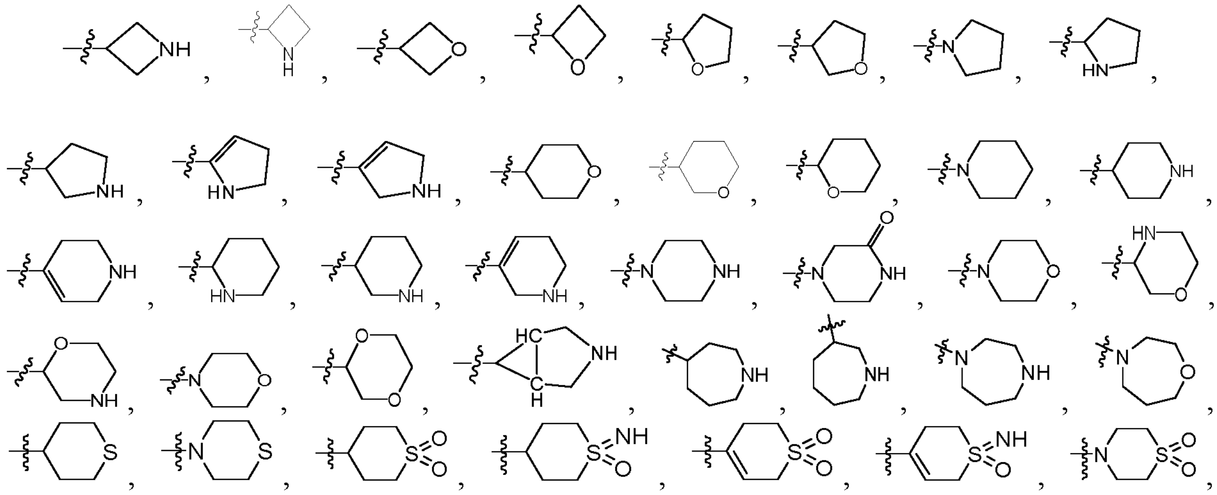

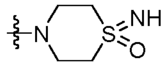, 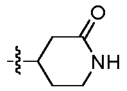, 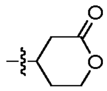, 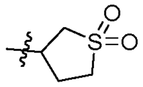, 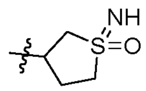, 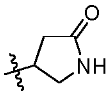, 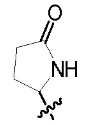, 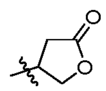, 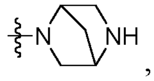, 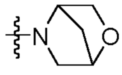, 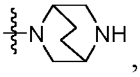, 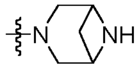 and 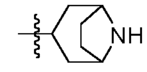.

In the present application, the term "5-10 membered heteroaryl" refers to a monocyclic or bicyclic or fused polycyclic ring aromatic hydrocarbon group having 5-10 ring atoms, for example 5, 6, or 7 ring atoms (i.e., 5-7 membered heteroaryl), which contains at least one (e.g., 1-3) heterocyclic atom independently selected from N, O, and S (e.g., N) in the ring, and the remaining ring atoms being a carbon atom, such as imidazolyl, pyridyl, pyrrolyl, thiazolyl, furyl, oxazolyl, isoxazolyl, pyrazolyl, thienyl, pyrimidinyl, 1,2,4-triazolyl, etc., preferably five-membered heteroaryl, such as imidazolyl, isoxazolyl, and 1,2,4-triazolyl. Bicyclic heteroaryl comprises, for example, benzoxazolyl, imidazopyridinyl, triazolopyridinyl, benzofuranyl, pyrazolopyrimidinyl, benzodioxolyl, indolyl, quinolyl, isoquinolyl and the like.

In the present invention, the substitution is mono-substitution or multi-substitution, and the multi-substitution is di-substitution, tri-substitution, tetra-substitution, or penta-substitution. The di-substitution means having two substituents, and so on. In the case of the multi-substitution, the substituents may be identical to or different from each other.

The pharmaceutically acceptable salt of the present invention may be a salt formed by an anion with a positively charged group on the compound of formula (I). A suitable anion is a chloride ion, a bromide ion, an iodide ion, sulfate, nitrate, phosphate, citrate, methylsulfonate, trifluoroacetate, acetate, malate, tosylate, tartrate, fumarate, glutamate, glucuronate, lactate, glutarate, or maleate. Similarly, the salt may be formed by a cation with a negatively charged group on compound of formula I. A suitable cation comprises a sodium ion, a potassium ion, a magnesium ion, a calcium ion, and an ammonium ion, for example a tetramethylammonium ion.

In another preferred embodiment, the term "pharmaceutically acceptable salt" refers to a salt formed by the compound of formula (I) with an acid selected from the group consisting of: hydrofluoric acid, hydrochloric acid, hydrobromic acid, phosphoric acid, acetic acid, oxalic acid, sulfuric acid, nitric acid, methanesulfonic acid, amino sulfonic acid, salicylic acid, trifluoromethanesulfonic acid, naphthalenesulfonic acid, maleic acid, citric acid, acetic acid, lactic acid, tartaric acid, succinic acid, oxalis corniculata acid, pyruvic acid, malic acid, glutamic acid, p-toluenesulfonic acid, naphthalenesulfonic acid, ethanesulfonic acid, naphthalenedisulfonic acid, malonic acid, fumaric acid, propionic acid, oxalic acid, trifluoroacetic acid, stearic acid, pamoic acid, hydroxymaleic acid, phenylacetic acid, benzoic acid, glutamic acid, ascorbic acid, p-aminobenzenesulfonic acid, 2-acetoxybenzoic acid, isethionic acid and the like; or a salt formed by the compound of formula (I) with an inorganic base, such as a sodium salt, a magnesium salt, a potassium salt, a calcium salt, an aluminium salt, a manganese salt, or an ammonium salt; or a salt formed by the compound of formula (I) with an organic base, such as a methylamine salt, an ethylamine salt, or an ethanolamine salt.

The term "safe effective amount" means the amount of an active ingredient is sufficient to obviously improve the condition without causing serious side effects. Typically, a pharmaceutical composition contains 1-2,000 mg of the active ingredient per dose, more preferably, 10-200 mg of the active ingredient per dose. Preferably, the "one dose" is a tablet.

The term "pharmaceutically acceptable carrier" refers to one or more compatible solid or liquid fillers or gel substances which are suitable for human and must be of sufficient purity and sufficiently low toxicity. The "compatible" herein refers to that each component in a composition is capable of intermixing with and between the active ingredient of the present invention without obviously reducing the efficacy of the active ingredient. Examples of the pharmaceutically acceptable carrier moiety are cellulose and a derivative thereof (e.g., sodium carboxymethylcellulose, sodium ethylcellulose, cellulose acetate, etc.), gelatin, talc, a solid lubricant (e.g., stearic acid, magnesium stearate), calcium sulfate, vegetable oil (e.g., soybean oil, sesame oil, peanut oil, olive oil, etc.), a polyol (e.g., propylene glycol, glycerin, mannitol, sorbitol, etc.), an emulsifier (e.g., Tween®), a wetting agent (e.g., sodium lauryl sulfate), a colorant, a flavor, a stabilizer, an antioxidant, a preservative, pyrogen-free water and the like.

The mode of administration of the active ingredient or pharmaceutical composition of the present invention is not particularly limited, and the representative mode of administration includes (but is not limited to): oral, intratumoral, rectal, parenteral (intravenous, intramuscular, or subcutaneous) administration and the like.

A solid formulation for the oral administration comprises a capsule, a tablet, a pill, a powder, and a granule.

A liquid formulation for the oral administration comprises a pharmaceutically acceptable emulsion, solution, suspension, syrup or tincture. In addition to the active ingredient, the liquid formulation may contain an inert diluent commonly employed in the art, such as water or other solvents, a solubilizer, and an emulsifier, such as ethyl alcohol, isopropyl alcohol, ethyl carbonate, ethyl acetate, propylene glycol, 1,3-butylene glycol, dimethylformamide, and oil, particularly cottonseed oil, peanut oil, corn germ oil, olive oil, castor oil, and sesame oil or a mixture of such substances and the like. In addition to these inert diluents, the composition can also contain an adjuvant such as a wetting agent,

an emulsifying agent and a suspending agent, a sweetening agent, a flavoring agent, and a spice.

The suspension, in addition to the active ingredient, may contain the suspending agent such as ethoxylated isostearyl alcohol, polyoxyethylene sorbitol and sorbitan ester, microcrystalline cellulose, aluminum methoxide and agar, or a mixture of these substances and the like.

The composition for parenteral injection may contain a physiologically acceptable sterile aqueous or anhydrous solution, a dispersion, a suspension, or an emulsion, and a sterile powder to be redissolved into a sterile injectable solution or dispersion. Suitable aqueous and nonaqueous carriers, a diluent, a solvent, or an excipient comprise water, ethanol, a polyol, and a suitable mixture thereof.

The compound of the present invention may be administered alone or in combination with other therapeutic drugs (e.g. an anti-tumor drug).

When the pharmaceutical composition is used, a safe effective amount of the compound of the present invention is administrated to a mammal (such as human) to be treated, wherein the administration dose is a pharmaceutically-considered effective administration dose, and for a human body with the weight of 60 kg, the daily administration dose is usually 1-2,000 mg, preferably 20-500 mg. Of course, the specific dose will also take into account such factors as a route of administration, health of a patient and the like, which are within a skill range of a skilled physician.

The present invention will be further described in combination with specific examples. It should be understood that these examples are only used to illustrate the present invention and not to limit the scope of the present invention. The experimental methods, where specific conditions are not specified, are generally performed according to conventional conditions (e.g., conditions described in Sambrook et al., Molecular Cloning: A Laboratory Manual (New York: Cold Spring Harbor Laboratory Press, 1989)) or according to conditions recommended by the manufacturer. Unless otherwise indicated, percentages and parts are by weight.

Unless otherwise defined, all technical and scientific terms used herein have same meaning as commonly understood by those skilled in the art. In addition, any methods and materials similar or equivalent to those described herein can be used in the methods of the present invention. The preferred embodiments and materials described herein are exemplary only.

In the present application, when the name of the compound is inconsistent with the structural formula, the structural formula of the compound is taken as the criterion.

**Example 1 Synthesis of compound Int-1**

Synthetic route:

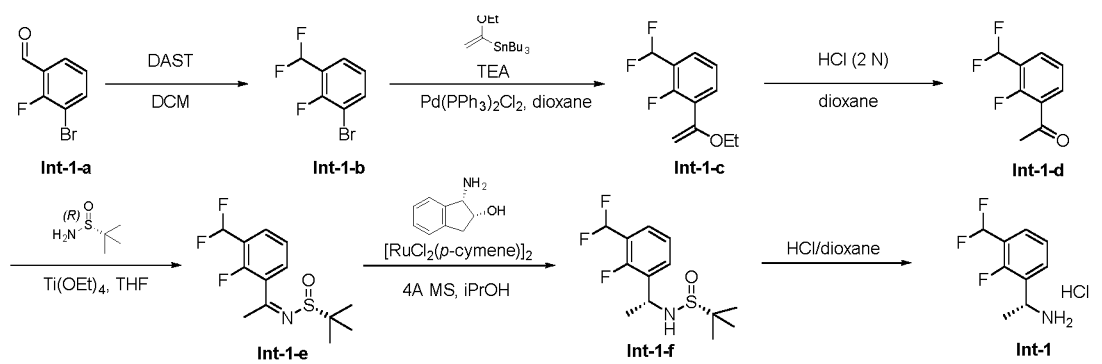


Int-1-a, Int-1-b, Int-1-c, Int-1-d, Int-1-e, Int-1-f, Int-1

<u>Step one:</u>

To a dry 3-L round-bottom flask, compound **Int-1-a** (100 g, 0.49 mol) and anhydrous tetrahydrofuran (1 L) were added. The solution was cooled to 0°C and diethylaminosulfur trifluoride (120 g, 0.17 mol) was dropwise added under nitrogen protection. After the addition was completed, the reaction temperature was raised to room temperature and stirring was continued for 16 hours. After the reaction was completed monitored by TLC, the reaction solution was poured into ice water and extracted with ethyl acetate (500 mL×3). The organic phases were combined, dried, filtered, and concentrated under reduced pressure. The resulting residue was purified by a silica gel column chromatography (petroleum ether/ethyl acetate = 30/1) to obtain 1-bromo-3-(difluoromethyl)-2-fluorobenzene **Int-1-b** (90 g, light yellow oil) with the yield of 82%. $^1H$ NMR ($CD_3Cl$, 400 MHz): $\delta$ 7.69-7.65 (m, 1H), 7.56-7.52 (m, 1H), 7.14 (t, $J$ = 8.0 Hz, 1H), 6.88 (t, $J$ = 54.8 Hz, 1H).

Step two:

To a dry 2-L single-neck round-bottom flask, compound **Int-1-b** (90 g, 0.40 mol), tributyl(1-ethoxyvinyl)stannane (173 g, 0.48 mol), and anhydrous dioxane (900 mL) were successively added, and triethylamine (101 g, 1.0 mol) and bis(triphenylphosphine)palladium(II) chloride (2.8 g, 4.0 mmol) were added while stirring. The reaction system was replaced by argon, heated to 80°C, and stirred for reaction for 12 hours. After the reaction was completed monitored by TLC, the reaction solution was concentrated. The residue was added into a saturated potassium fluoride solution (300 mL), stirred for 1 hour, and filtered. The filtrate was extracted with ethyl acetate (300 mL×3). The organic phases were combined, dried over anhydrous sodium sulfate, and concentrated. The residue was purified by the silica gel column chromatography (petroleum ether/ethyl acetate = 20/1) to obtain 1-(difluoromethyl)-3-(1-ethoxyvinyl)-2-fluorobenzene **Int-1-c** (100 g, brown oil). The crude product was directly used in the next step.

Step three:

To a dry 1-L single-neck round-bottom flask, compound **Int-1-c** (100 g, crude product) and anhydrous dioxane (200 mL) were successively added. The solution was cooled to 0°C and dilute hydrochloric acid (200 mL, 0.40 mol, 2 M) was dropwise added under nitrogen protection. After the dropwise addition was completed, the reaction temperature was raised to room temperature and stirring was continued for 12 hours. After the reaction was completed monitored by TLC, the reaction solution was poured into water, the filtrate was extracted with dichloromethane (300 mL×3). The organic phases were combined, dried over anhydrous sodium sulfate, and concentrated under reduced pressure. The resulting residue was purified by the silica gel column chromatography (petroleum ether/ethyl acetate = 10/1) to obtain 1-(3-(difluoromethyl)-2-fluorophenyl)ethan-1-one **Int-1-d** (53 g, light yellow oil) with the yield of 50% in two steps. $^1$H NMR ($CD_3Cl$, 400 MHz): $\delta$ 7.69-7.98 (m, 1H), 7.81-7.77 (m, 1H), 7.36-7.33 (m, 1H), 6.95 (t, *J*= 54.8 Hz, 1H), 2.68 (d, *J*= 4.2 Hz, 3H).

Step four:

To a dry 1-L single-neck round-bottom flask, compound **Int-1-d** (17 g, 90 mmol), (R)-(+)-2-methylpropan-2-sulfinylamine (16 g, 0.14 mol), and anhydrous tetrahydrofuran (200 mL) were added successively, tetraethoxy titanium (62 g, 0.27 mol) was added while stirring, and the reaction was performed while stirring at 80°C for 16 hours under argon protection. After the reaction was completed monitored by TLC, the reaction solution was concentrated. The residue was added into a saturated salt solution (100 mL) and the solution was extracted with ethyl acetate (300 mL×3). The organic phases were combined, dried over anhydrous sodium sulfate, and concentrated. The resulting residue was purified by the silica gel column chromatography (petroleum ether/ethyl acetate = 3/1) to obtain (*R*)-N-(1-(3-(difluoromethyl)-2-fluorophenyl)ethylene)-2-methylpropan-2-sulfinylamine **Int-1-e** (23.7 g, yellow oil) with the yield of 90%. LCMS (ESI): *m/z* 292.1 $[M+H]^+$.

Step five:

To a dry 1-L three-neck flask, a dichloro(p-methylisopropylbenzene)ruthenium (II) dimer (1.4 g, 2.3 mmol), (1S,2R)-1-amino-2,3-dihydro-1H-inden-2-ol (0.70 g, 4.5 mmol), 4A molecular sieve (50 g), and isopropanol (100 mL) were successively added, and the reaction was performed while stirring at 90°C for 20 minutes under argon protection. The reaction was cooled to 40°C, an isopropanol solution (450 mL) of compound **Int-1-e** (13 g, 45 mmol) and an isopropanol solution of potassium tert-butoxide (113 mL, 11 mmol, 0.1 M) were successively added, and the reaction was performed at 40°C for 2 hours. After the reaction was completed monitored by TLC, the system was concentrated, the residue was added into a saturated salt solution (100 mL), and the solution was extracted with ethyl acetate (100 mL×3). The organic phases were combined, dried over anhydrous sodium sulfate, filtered, and concentrated. The resulting residue was purified by the silica gel column chromatography (petroleum ether/ethyl acetate = 1/2) to obtain (R)-N-((R)-1-(3-(difluoromethyl)-2-fluorophenyl)ethyl)-2-methylpropan-2-sulfoxide-amide **Int-1-f** (12.5 g, yellow oil) with the yield of 95%. $^1$H- NMR ($CD_3Cl$, 400 MHz): $\delta$ 7.54-7.51 (m, 2H), 7.24-7.23 (m, 1H), 6.90 (t, *J* = 54.8 Hz, 1H), 4.87-4.80 (m, 1H), 3.55 (d, *J* = 5.2 Hz, 1H), 1.55 (t, *J* = 6.4 Hz, 3H), 1.23 (s, 9H).

Step six:

To a dry 1-L single-neck round-bottom flask, compound **Int-1-f** (12.5 g, 43 mmol) and anhydrous dioxane (100 mL) were successively added. The solution was cooled to 0°C and a dioxane solution of dilute hydrochloric acid (50 mL, 0.20 mol, 4 M) was dropwise added under nitrogen protection. After the dropwise addition was completed, the reaction temperature was raised to room temperature and stirring was continued for 12 hours. After the reaction completed

monitored by LC-MS, the reaction solution was concentrated. Methyl t-butyl ether (200 mL) was added to the residue and stirred for 2 hours. The precipitated product was filtered and dried to obtain (*R*)-1-(3-(difluoromethyl)-2-fluorophenyl)ethan-1-amine hydrochloride **Int-1** (8.7 g, white solid) with the yield of 92%. $^1$H NMR ($CD_3Cl$, 400 MHz): $\delta$ 8.87 (s, 3H), 7.98-7.94 (m, 1H), 7.67-7.64 (m, 1H), 7.45-7.41 (m, 1H), 7.25 (t, *J* = 54.8 Hz, 1H), 4.64 (m, 1H), 1.55 (t, *d* = 6.4 Hz, 3H). Chiral HPLC: 98.5%.

**Example 2 Synthesis of compound Int-2**

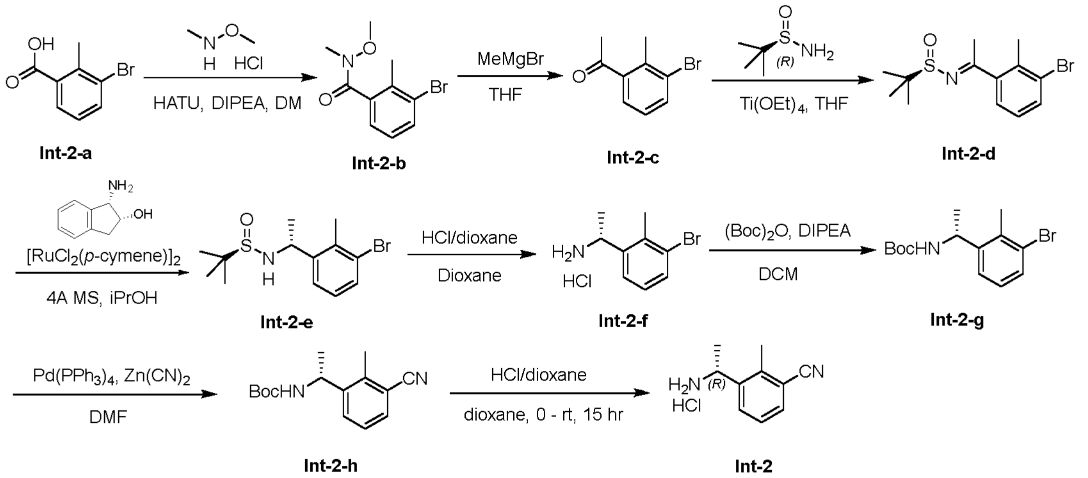


Int-2-a, Int-2-b, Int-2-c, Int-2-d, Int-2-e, Int-2-f, Int-2-g, Int-2-h, Int-2

Step one:

To a dry 3-L three-neck flask, compound **Int-2-a** (100 g, 465 mmol), dry N,N-dimethylformamide (1.5 L) and 2-(7-azabenzotriazole)-N,N,N',N'-tetramethyluronium hexafluorophosphate (195 g, 512 mmol) were added, the reaction was performed while stirring at room temperature for 30 minutes, methoxymethylamine hydrochloride (69 g, 512 mmol) and N,N-diisopropylethylamine (180 g, 1.4 mol) were added, and the reaction was performed while stirring at room temperature for 3 hours. After the reaction was completed monitored by LCMS, the reaction was lowered to 0°C, 2 N hydrochloric acid was added, then water (200 mL) was added for dilution, and the solution was extracted with ethyl acetate (500 mL×3). The organic phases were combined, washed with a saturated salt solution (200 mL×2), dried over anhydrous sodium sulfate, and filtered. The filtrate was concentrated under reduced pressure. The resulting residue was purified by a silica gel column chromatography (petroleum ether/ethyl acetate = 20/1) to obtain 3-bromo-N-methoxy-N,2-dimethylbenzamide **Int-2-b** (110 g, light yellow oily liquid) with the yield of 92%. $^1$H NMR ($CD_3Cl$, 400 MHz): $\delta$ 7.58 (d, *J* = 8.0 Hz, 1H), 7.21 (d, *J* = 7.6 Hz, 1H), 7.11-7.07 (m, 1H), 3.41(s, 3H), 3.37 (s, 3H), 2.37 (s, 3H).

Step two:

To a dry 1-L three-neck flask, compound **Int-2-b** (20 g, 77 mmol) and anhydrous tetrahydrofuran (300 mL) were added, the solution was cooled to 0°C in an ice bath, methyl magnesium bromide (51 mL, 154 mmol, 3.0 M) was dropwise added, the reaction temperature was slowly raised to room temperature, and stirring was continued for 3 hours. After the reaction was completed monitored by TLC, the reaction was lowered to 0°C, a hydrochloric acid solution was added (75 mL, 6 N) was added, after stirring was performed for 30 minutes, water (200 mL) was added for dilution, and the solution was extracted with ethyl acetate (200 mL×3). The organic phases were combined, washed with a saturated salt solution (200 mL×3), dried over anhydrous sodium sulfate, and filtered. The filtrate was concentrated under reduced pressure. The resulting residue was purified by the silica gel column chromatography (petroleum ether/ethyl acetate = 20/1) to obtain 1-(3-bromo-2-methylphenyl)ethan-1-one **Int-2-c** (9.3, orange oily liquid) with the yield of 56%. $^1$H NMR ($CD_3Cl$, 400 MHz): $\delta$ 7.65 (d, *J* = 8.0 Hz, 1H), 7.47 (d, *J*= 7.6 Hz, 1H), 7.11 (t, *J*= 8.0 Hz, 1H), 2.56 (s, 3H), 2.50 (s, 3H).

Step three:

To a dry 500-mL single-neck flask, compound **Int-2-c** (9.3 g, 47 mmol), anhydrous tetrahydrofuran (250 mL), (*R*)-(+)-tert-butylsulfinamide (6.4 g, 52 mmol), and titanium tetraethoxide (50 g, 218 mmol) were added successively,

and the reaction was performed while stirring at 80°C for 12 hours under argon protection. After the reaction was completed monitored by LCMS, water (200 mL) was added for dilution, and the solution was extracted with ethyl acetate (200 mL×3). The organic phases were combined, washed with a saturated salt solution (200 mL×3), dried over anhydrous sodium sulfate, and filtered. The filtrate was concentrated under reduced pressure. The resulting residue was purified by the silica gel column chromatography (petroleum ether/ethyl acetate = 10/1 to 5/1) to obtain (*R,E*)-N-(1-(3-bromo-2-methylphenyl)ethylene)-2-methylpropan-2-sulfinamide **Int-2-d** (13 g, yellow oily liquid) with the yield of 95%. LCMS (ESI): *m*/*z* 318 $[M+H]^+$.

Step four:

To a 500-mL three-neck flask, a dichloro(p-methylisopropylbenzene)ruthenium (II) dimer (0.73 g, 1.2 mmol), (1S,2R)-1-amino-2,3-dihydro-1H-inden-2-ol (0.35 g, 2.4 mmol), 4A molecular sieve (29 g), and isopropanol (60 mL) were successively added, the reaction was performed at 90°C for 20 minutes under argon protection, and the system turned from yellow to dark red. The reaction was cooled to 40°C, an isopropanol solution (250 mL) of (*R*,*E*)-N-(1-(3-bromo-2-methylphenyl)ethylene)-2-methylpropan-2-sulfinamide **Int-2-d** (7.5 g, 24 mmol) and an isopropanol solution of potassium tert-butoxide (60 mL, 2.4 mmol, 0.1 M) were successively added, and the reaction was performed at 40°C for 15 hours under argon protection. After the reaction was completed monitored by LCMS, the reaction solution was concentrated. Water (200 mL) was added to the residue for dilution and the solution was extracted with ethyl acetate (200 mL×3). The organic phases were combined, washed with a saturated salt solution (200 mL), dried over anhydrous sodium sulfate, and filtered. The filtrate was concentrated under reduced pressure. The resulting residue was purified by the silica gel column chromatography (petroleum ether/ethyl acetate/dichloromethane = 2/1/1) to obtain (R)-N-((R)-1-(3-bromo-2-methylphenyl)ethyl)-2-methylpropan-2-sulfinamide **Int-2-e** (2.0 g, brown oily liquid) with the yield of 27%. LCMS (ESI): *m*/*z* 318 $[M+H]^+$.

Step five:

To a dry 250-mL single-neck flask, compound **Int-2-e** (6.1 g, 19 mmol) and dioxane (50 mL) were added, the solution was cooled to 0°C in an ice bath, a hydrochloric acid/1,4-dioxane (25 mL, 100 mmol, 4 M) solution was dropwise added, and stirring was performed at room temperature for 12 hours. After the reaction completed monitored by LCMS, the reaction solution was concentrated. Petroleum ether (200 mL) was added into the crude product and stirred for 12 hours. The product was filtered and dried to obtain (R)-1-(3-bromo-2-methylphenyl)ethan-1-amine hydrochloride **Int-2-f** (4.8 g, dark gray solid) with the yield of 100%. LCMS (ESI): *m*/*z* 214, 216 $[M+H]^+$.

Step six:

To a dry 250-mL single-neck flask, compound **Int-2-f** (6.5 g, 30 mmol), di-tert-butyl dicarbonate (1.3 g, 33 mmol), N,N-diisopropylethylamine (12 g, 31 mmol), and anhydrous dichloromethane (150 mL) were successively added, and stirring was performed at room temperature for 3 hours. After the reaction was completed monitored by LCMS, water (100 mL) was added for dilution, and the solution was extracted with ethyl acetate (200 mL×3). The organic phases were combined, washed with a saturated salt solution (100 mL×2), dried over anhydrous sodium sulfate, and filtered. The filtrate was concentrated under reduced pressure. The resulting residue was purified by the silica gel column chromatography (petroleum ether/ethyl acetate = 5/1) to obtain (R)-(1-(3-bromo-2-methylphenyl)ethyl)tert-butyl carbamate **Int-2-g** (7.1 g, white solid) with the yield of 75%. LCMS (ESI): *m*/*z* 316 $[M+H]^+$.

Step seven:

To a dry 250-mL single-neck flask, compound **Int-2-g** (7.1 g, 23 mmol), zinc cyanide (3.2 g, 27 mmol), tetrtriphenylphosphine palladium (2.6 g, 2.3 mmol), and anhydrous N,N-dimethylformamide (100 mL) were added, and the reaction was performed while stirring at 110°C for 3 hours under argon protection. After the reaction was completed monitored by LCMS, water (150 mL) was added for dilution, and the solution was extracted with ethyl acetate (200 mL×3). The organic phases were combined, washed with a saturated salt solution (100 mL×3), dried over anhydrous sodium sulfate, and filtered. The filtrate was concentrated under reduced pressure. The resulting residue was purified by the silica gel column chromatography (petroleum ether/ethyl acetate = 5/1) to obtain ((*R*)-(1-(3-cyano-2-methylphenyl)ethyl)tert-butyl carbamate **Int-2-h** (5.0 g, white solid) with the yield of 85%. LCMS (ESI): *m*/*z* 261 $[M+H]^+$.

Step eight:

To a dry 250-mL single-neck flask, compound **Int-2-h** (5.0 g, 16 mmol) and dioxane (50 mL) were added, the

solution was cooled to 0°C in an ice bath, a hydrochloric acid/1,4-dioxane (25 mL, 100 mmol, 4 M) solution was dropwise added, the reaction temperature was raised to room temperature, and stirring was performed for 15 hours. After the reaction completed monitored by LC-MS, the reaction solution was concentrated. Methyl t-butyl ether (200 mL) was added, and the solution was stirred for 12 hours, filtered, and dried to obtain (R)-3-(1-aminoethyl)-2-methylbenzonitrile hydrochloride **Int-2** (3.0 g, white solid) with the yield of 80%. LCMS (ESI): *m/z* 161 $[M+H]^+$; $^1H$ NMR (DMSO-$d_6$, 400 MHz): $\delta$8.75 (s, 3H), 8.00 (d, *J* = 7.6 Hz, 1H), 7.80 (d, *J* = 7.6 Hz, 1H), 7.51 (t, *J* = 8.0 Hz, 1H), 4.67-4.61 (m, 1H), 2.56 (s, 3H), 1.51 (d, *J* = 6.4 Hz, 3H).

**Example 3 Synthesis of compound Int-3**

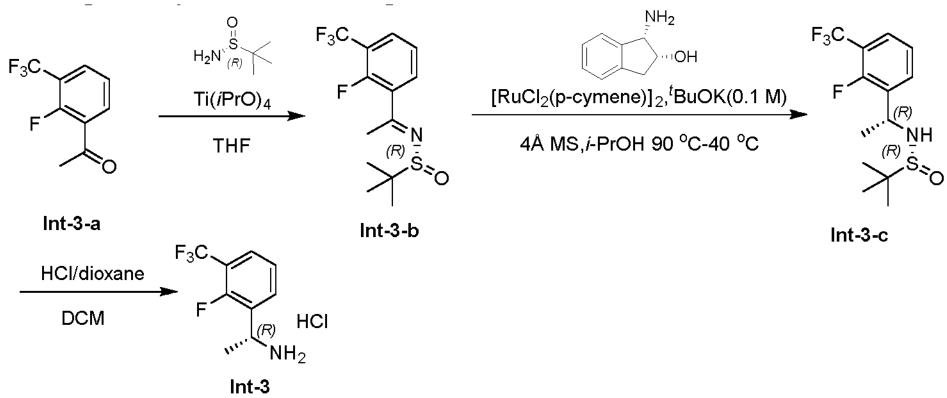


Int-3-a, Int-3-b, Int-3-c, Int-3

Step one:

To a 500-mL flask, compound **Int-3-a** (10 g, 48.51 mmol), (*R*)-(+)-tert-butylsulfinamide (8.82 g 72.77 mmol), and titanium tetraisopropoxide (41.33g, 145.53 mmol) were added, followed by 150 mL of tetrahydrofuran. Nitrogen was pumped and exchanged three times and the mixture was stirred at 80°C for 8 hours. The reaction was cooled to room temperature and a large amount of a solid precipitated by addition of ice water. The solid was removed by filtration and the filtrate was extracted with 100 mL of ethyl acetate for three times. The organic phases were combined, and subjected to rotary evaporation and a column chromatography to obtain compound **Int-3-b.** LCMS (ESI): *m/z* 309.9 $[M+H]^+$.

Step two:

To a dry 1-L three-neck flask, a dichloro(p-methylisopropylbenzene)ruthenium (II) dimer (0.35 g, 1.05 mmol 0.1 eq), (1S,2R)-1-amino-2,3-dihydro-1H-inden-2-ol (0.70 g, 2.1 mmol 0.05 eq), 4A molecular sieve (7 g), and isopropanol (50 mL) were successively added, and the reaction was performed while stirring at 90°C for 20 minutes under argon protection. The reaction was cooled to 40°C, an isopropanol solution (150 mL) of compound **Int-3-b** (6.5 g, 21 mmol) and an isopropanol solution of potassium tert-butoxide (20 mL, 2.1 mmol) were successively added, and the reaction was performed at 40°C for 8 hours. After the reaction was completed monitored by TLC, the system was concentrated, the residue was added into a saturated salt solution (100 mL), and the solution was extracted with ethyl acetate (100 mL×3). The organic phases were combined, dried over anhydrous sodium sulfate, filtered, and concentrated. The resulting residue was purified by the silica gel column chromatography to obtain compound **Int-3-c.** LCMS (ESI): *m/z* 312.0 $[M+H]^+$.

Step three:

To a dry 250-mL single-neck round-bottom flask, compound **Int-3-c** (2.2 g, 7.07 mmol) and 50 mL of dichloromethane were successively added. The solution was cooled to 0°C and a dioxane solution of dilute hydrochloric acid (10 mL, 40 mol, 4 M) was added dropwise under nitrogen protection. After the dropwise addition was completed, the reaction temperature was raised to room temperature and stirring was continued for 12 hours. After the reaction was completed monitored by LC-MS, the reaction solution was concentrated to obtain the crude product. The crude product was washed with 20 mL of ethyl acetate and filtered to obtain compound **Int-3** as a white solid. $^1H$ NMR ($CD_3OD$, 400 MHz): $\delta$7.91-7.85 (m, 1H), 7.82 (t, *J* = 7.9 Hz, 1H), 7.52 (t, *J* = 7.9 Hz, 1H), 4.85 (d, *J* = 6.9 Hz, 1H), 1.71 (d, *J* = 6.9 Hz, 3H).

**Example 4 Synthesis of compound Int-4**

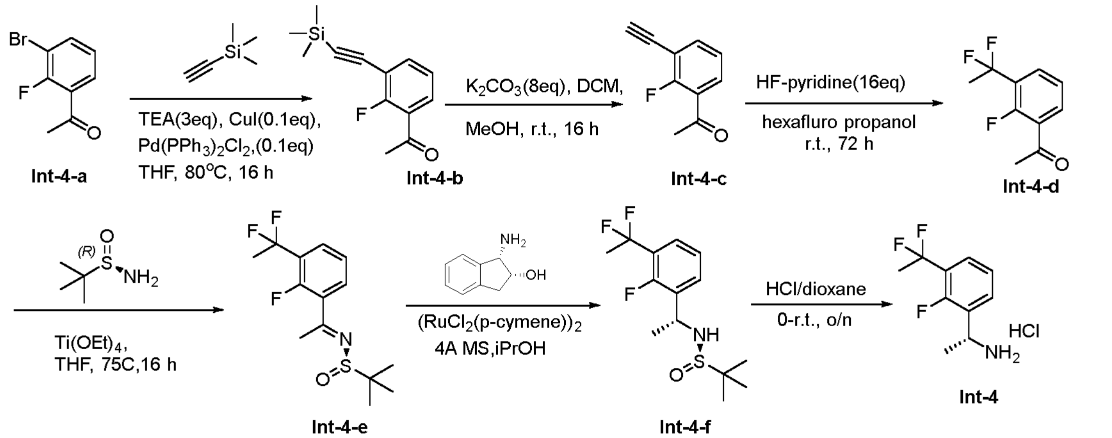


Step one:

To a single-neck flask containing 100 mL of tetrahydrofuran, compound Int-4-a (7.0 g, 32.26 mmol) was added, followed by trimethylethynylsilicon (4.74 g, 48.39 mmol), cuprous iodide (0.61 g, 3.23 mmol), $Pd(PPh_3)_2Cl_2$ (2.26 g, 3.23 mmol), and triethylamine (9.77 g, 96.77 mmol), and the reaction was performed while stirring at 80°C for 16 hours under argon protection. After the reaction was completed, the reaction solution was subjected to rotary evaporation and water was added. The solution was extracted with ethyl acetate. The organic phase was dried, concentrated, and purified by a column to obtain compound **Int-4-b** (6 g) with the yield of 79%. LCMS (ESI): m/z 235.2 $[M+H]^+$. $^1H$ NMR (400 MHz, $CDCl_3$) $\delta$7.81 (ddd, J = 7.9, 6.9, 1.9 Hz, 1H), 7.62 (ddd, J = 7.6, 6.5, 1.9 Hz, 1H), 7.16 (t, J = 7.7 Hz, 1H), 2.65 (d, J = 5.2 Hz, 3H), 0.32-0.23 (m, 9H).

Step two:

To a single-neck flask containing 50 mL of dichloromethane and 50 mL of methanol, compound **Int-4-b** (6 g, 25.641 mmol) was added, followed by potassium carbonate (28.31 g, 205.128 mmol), and the reaction was performed while stirring at room temperature for 16 hours under argon protection. After the reaction was completed, the reaction solution was subjected to rotary evaporation and water was added. The solution was extracted with ethyl acetate. The organic phase was dried, concentrated, and purified by the column to obtain compound **Int-4-c** (3.2 g) with the yield of 77%. LCMS (ESI): *m/z* 163.2 $[M+H]^+$; $^1H$ NMR (400 MHz, $CDCl_3$) $\delta$7.85 (ddd, *J* = 7.9, 7.0, 1.9 Hz, 1H), 7.69-7.62 (m, 1H), 7.20 (t, *J* = 7.7 Hz, 1H), 3.37 (s, 1H), 2.66 (d, *J* = 5.1 Hz, 3H).

Step three:

To a single-neck flask containing 20 mL of hexafluoroisopropanol, compound **Int-4-c** (3 g, 18.518 mmol) was added, followed by HF-pyridine (70%) (7.65 mL, 296.296 mmol), and the reaction was performed while stirring at room temperature for 16 hours. After the reaction was completed, the reaction solution was added into ice water. The solution was extracted with ethyl acetate and washed over sodium bicarbonate. The organic phase was dried, concentrated, and purified by the column to obtain compound **Int-4-d** (1.4 g) with the yield of 37%. LCMS (ESI): *m/z* 203.2 $[M+H]^+$; $^1H$ NMR (400 MHz, $CDCl_3$) $\delta$7.97-7.90 (m, 1H), 7.77-7.69 (m, 1H), 7.28 (dd, *J* = 9.7, 5.8 Hz, 1H), 2.67 (d, *J* = 5.1 Hz, 3H), 2.09-1.98 (m, 3H).

Step four:

To a single-neck flask containing 50 mL of tetrahydrofuran, compound **Int-4-d** (1.4 g, 6.90 mmol) was added, followed by compound (A)-2-methylpropan-2-sulfinamide (1.09 g, 9.0 mmol), and tetraethyl titanate (3.16 g, 13.85 mmol) at room temperature, and after the reaction temperature was raised to 75°C, the reaction was performed while stirring for 16 hours under argon protection. After the reaction was completed, ethyl acetate and diatomite were added, water was dropwise added, and the solution was filtered. The filtrate was extracted with ethyl acetate, dried over sodium sulfate, and subjected to rotary evaporation and column chromatography to obtain compound **Int-4-e** (1.67 g) with the

yield of 76%. LCMS (ESI): *m/z* 306.1 $[M+H]^+$.

Step five:

To a three-neck flask, a dichloro(p-methylisopropylbenzene)ruthenium (II) dimer (242 mg, 0.40 mmol), (1S,2R)-1-amino-2,3-dihydro-1H-inden-2-ol (118 mg, 0.79 mmol), 4A molecular sieve (8.00 g), and isopropanol (30 mL) were successively added, and the reaction was performed while stirring at 90°C for 20 minutes under argon protection. After the temperature was lowered to 40°C, an isopropanol solution of compound **Int-4-e** (2.42 g, 7.92 mmol) and an isopropanol solution of potassium tert-butoxide (19.8 mL, 0.1 N) were added, and the reaction was performed at 40°C overnight. The solution was subjected to rotary evaporation and water was added. The solution was extracted with ethyl acetate, dried over sodium sulfate, concentrated, and subjected to column chromatography to obtain compound **Int-4-f** (1.10 g) with yield of 41%. LCMS (ESI): *m/z* 308.1 $[M+H]^+$.

Step six:

To a single-neck flask containing 10 mL of 1,4-dioxane, compound 6 (1.03 g, 3.26 mmol) was added, followed by HCl/dioxane (3.26 mL, 13.03 mmol, 4.0 N), and the reaction was performed while stirring at room temperature for 12 hours under argon protection. After the reaction was completed, the reaction solution was subjected to rotary evaporation, washed with ethyl methyl acetate and petroleum ether, and air-dried to obtain compound **Int-4** (0.61 mg) as a white solid with the yield of 77%. LCMS (ESI): *m/z* 204.1 $[M+H]^+$; $^1H$ NMR (600 MHz, $CD_3OD$) $\delta$7.73-7.63 (m, 2H), 7.42 (t, *J* = 7.8 Hz, 1H), 4.83 (d, *J* = 6.9 Hz, 1H), 2.03 (t, *J* = 18.6 Hz, 3H), 1.70 (d, *J* = 6.9 Hz, 3H).

**Example 5 Synthesis of compound Int-5**

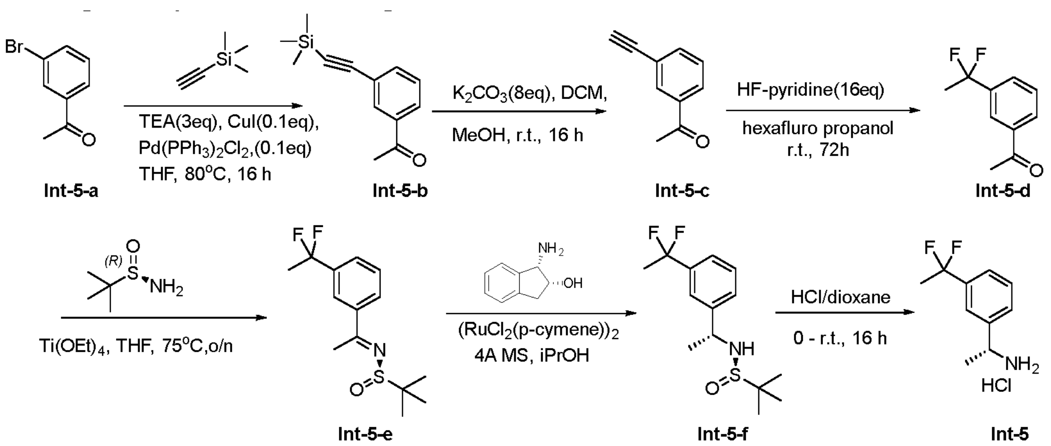


Step one:

To a single-neck flask containing 100 mL of tetrahydrofuran, compound **Int-5-a** (8.00 g, 40.19 mmol) was added, followed by trimethylethynylsilicon (5.91 g, 60.28 mmol), cuprous iodide (765 mg, 4.02 mmol), $Pd(PPh_3)_2Cl_2$ (2.82 g, 4.02 mmol), and triethylamine (12.18 g, 120.57 mmol), and the reaction was performed while stirring at 80°C for 16 hours under argon protection. After the reaction was completed, the reaction solution was subjected to rotary evaporation and water was added. The solution was extracted with ethyl acetate. The organic phase was dried, concentrated, and purified by a column to obtain compound **Int-5-b** (7.05 g) with the yield of 81%. LCMS (ESI): *m/z* 217.2 $[M+H]^+$.

Step two:

To a single-neck flask containing 50 mL of dichloromethane and 50 mL of methanol, compound **Int-5-b** (7.00 g, 32.35 mmol) was added, followed by potassium carbonate (35.72 g, 258.83 mmol), and the reaction was performed while stirring at room temperature for 16 hours under argon protection. After the reaction was completed, the reaction solution was subjected to rotary evaporation and water was added. The solution was extracted with ethyl acetate. The organic phase was dried, concentrated, and purified by the column to obtain compound **Int-5-c** (3 g) with the yield of 64.3%. LCMS (ESI): m/z =145.1 $[M+H]^+$; $^1H$ NMR (600 MHz, $CDCl_3$) $\delta$8.07 (d, *J* = 1.2 Hz, 1H), 7.93 (dd, *J* = 7.9, 1.1

Hz, 1H), 7.67 (dd, $J$ = 7.6, 1.1 Hz, 1H), 7.48-7.40 (m, 1H), 3.15 (s, 1H), 2.61 (d, $J$ = 2.4 Hz, 3H).

Step three:

To a single-neck flask containing 20 mL of hexafluoroisopropanol, compound **Int-5-c** (3.00 g, 20.81 mmol) was added, followed by HF-pyridine (70%) (8.54 mL, 332.96 mmol), and the reaction was performed while stirring at room temperature for 72 hours. After the reaction was completed, the reaction solution was added into ice water. The solution was extracted with ethyl acetate and washed over sodium bicarbonate. The organic phase was dried, concentrated, and purified by the column to obtain compound **Int-5-d** (2.01 g) with the yield of 52%. LCMS (ESI): $m/z$ 185.1 $[M+H]^+$; $^1H$ NMR (600 MHz, $CDCl_3$) $\delta$ 8.10 (s, 1H), 8.02 (d, $J$ = 7.8 Hz, 1H), 7.71 (dd, $J$ = 7.7, 0.5 Hz, 1H), 7.54 (t, $J$ = 7.8 Hz, 1H), 2.64 (s, 3H), 1.95 (t, $J$ = 18.2 Hz, 3H).

Step four:

To a single-neck flask containing 50 mL of tetrahydrofuran, compound **Int-5-d** (2.00 g, 10.86 mmol) was added, followed by compound (A)-2-methylpropan-2-sulfinamide (1.71 g, 14.115 mmol), and tetraethyl titanate (4.95 g, 21.72 mmol) at room temperature, and after the reaction temperature was raised to 75°C, the reaction was performed while stirring for 16 hours under argon protection. After the reaction was completed, ethyl acetate and diatomite were added, water was dropwise added, and the solution was filtered. The filtrate was extracted with ethyl acetate, dried over sodium sulfate, and subjected to rotary evaporation and column chromatography to obtain compound **Int-5-e** (2.08 g) with the yield of 65%. LCMS (ESI): $m/z$ 288.1 $[M+H]^+$.

Step five:

To a three-neck flask, a dichloro(p-methylisopropylbenzene)ruthenium (II) dimer (213 mg, 0.3479 mmol), (1S,2R)-1-amino-2,3-dihydro-1H-inden-2-ol (103 mg, 0.70 mmol), 4A molecular sieve (8.00 g), and isopropanol (30 mL) were successively added, and the reaction was performed while stirring at 90°C for 20 minutes under argon protection. After the temperature was lowered to 40°C, an isopropanol solution of compound **Int-5-e** (2.00 g, 6.96 mmol) and an isopropanol solution of potassium tert-butoxide (17.39 mL, 0.1 N) were added, and the reaction was performed at 40°C overnight. The solution was subjected to rotary evaporation and water was added. The solution was extracted with ethyl acetate, dried over sodium sulfate, concentrated, and subjected to column chromatography to obtain compound **Int-5-f** (0.98 g) as a white solid with yield of 50%. LCMS (ESI): $m/z$ 290.1 $[M+H]^+$.

Step six:

To a single-neck flask containing 10 mL of 1,4-dioxane, compound **Int-5-f** (0.98 g, 3.46 mmol) was added, followed by HCl/dioxane (3.45 mL, 13.82 mmol, 4.0 N), and the reaction was performed while stirring at room temperature for 16 hours under argon protection. After the reaction was completed, the reaction solution was subjected to rotary evaporation, washed with ethyl methyl acetate and petroleum ether, and air-dried to obtain compound **Int-5** (0.41 g) with the yield of 52%. LCMS (ESI): $m/z$ 186.1 $[M+H]^+$; $^1H$ NMR (600 MHz, $CD_3OD$) $\delta$ 7.68 (s, 1H), 7.62-7.53 (m, 3H), 4.55 (d, $J$ = 5.2 Hz, 1H), 1.94 (t, $J$ = 18.4 Hz, 3H), 1.66 (dd, $J$ = 6.9, 1.9 Hz, 3H).

**Example 6 Synthesis of compound Int-6**

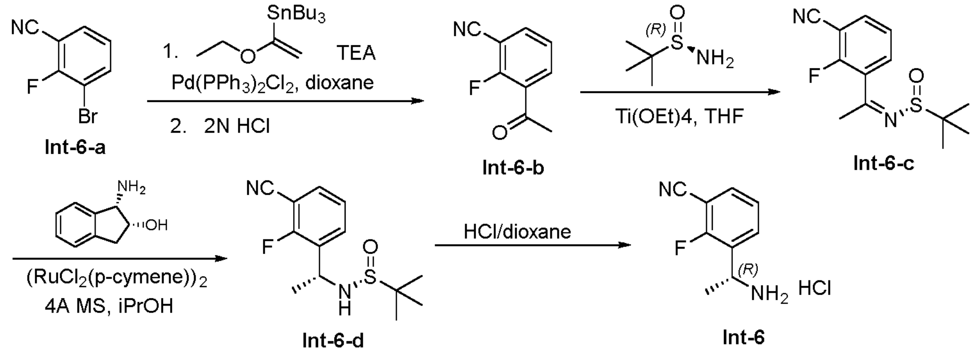


Int-6-a, Int-6-b, Int-6-c, Int-6-d, Int-6

Step one:

To a single-neck flask containing 150 mL of dioxane, compound **Int-6-a** (12 g, 200 mmol) was added, followed by tributyl(1-ethoxyvinyl)stannane (25.992 g, 72 mmol), $Pd(PPh_3)_2Cl_2$ (4.21 g, 6 mmol), and triethylamine (18.18 g, 180 mmol), and the reaction was performed while stirring at 80°C for 16 hours under argon protection. After the reaction was completed, the reaction solution was subjected to rotary evaporation and 100 mL of dioxane and 150 mL of 4 N hydrochloric acid aqueous solution were added. Stirring was performed at room temperature for 3 hours. The solution was subjected to rotary evaporation and extracted with ethyl acetate. The organic phase was dried, concentrated, and purified by a column to obtain compound **Int-6-b** (8 g) with the yield of 81.7%. LCMS (ESI): *m/z* = 164.2 $[M+H]^+$.

Step two:

To a single-neck flask containing 100 mL of tetrahydrofuran, compound **Int-6-b** (7 g, 49.944 mmol) was added, followed by compound (*R*)-2-methylpropan-2-sulfinamide (6.755 g, 55.83 mmol), and tetraethyl titanate (19.582 g, 85.889 mmol) at room temperature, and after the reaction temperature was raised to 75°C, the reaction was performed while stirring for 16 hours under argon protection. After the reaction was completed, ethyl acetate and diatomite were added, water was dropwise added, and the solution was filtered. The filtrate was extracted with ethyl acetate, dried over sodium sulfate, and subjected to rotary evaporation and column chromatography to obtain compound **Int-6-c** (9.1 g) with the yield of 79.6%. LCMS (ESI): *m/z* = 267.0 $[M+H]^+$.

Step three:

To a three-neck flask, a dichloro(p-methylisopropylbenzene)ruthenium (II) dimer (920 mg, 1.5 mmol), compound (1*S*,2*R*)-1-amino-2,3-dihydro-1H-inden-2-ol (448 mg, 3 mmol), 4A molecular sieve (40 g), and isopropanol (100 mL) were successively added, and the reaction was performed while stirring at 90°C for 20 minutes under argon protection. After the temperature was lowered to 40°C, an isopropanol solution of compound **Int-6-c** (8 g, 30.08 mmol) and an isopropanol solution of potassium tert-butoxide (75 mL, 0.1 N) were added, and the reaction was performed at 40°C overnight. The solution was subjected to rotary evaporation and water was added. The solution was extracted with ethyl acetate, dried over sodium sulfate, concentrated, and subjected to column chromatography to obtain compound **Int-6-d** (7 g) with yield of 86.8%. LCMS (ESI): m/z =269.1 $[M+H]^+$.

Step four:

To a single-neck flask containing 50 mL of 1,4-dioxane, compound **Int-6-d**(7 g, 26.114 mmol) was added, followed by HCl/dioxane (26.8 mL, 104.477 mmol, 4 N), and the reaction was performed while stirring at room temperature for 12 hours under argon protection. After the reaction was completed, the reaction solution was subjected to rotary evaporation, washed with ethyl methyl acetate and petroleum ether, and air-dried to obtain compound **Int-6** (4 g) with the yield of 76.6%. LCMS (ESI): m/z = 165.1 $[M+H]^+$; 1H NMR (400 MHz, $CD_3OD$) $\delta$ 7.96-7.92 (m, 1H), 7.86 -7.86 (m, 1H), 7.54 -7.50 (m, 1H), 4.87-4.82 (m, 1H), 1.71 (d, *J*= 6.8 Hz, 3H).

**Example 7 Synthesis of compound A-1**

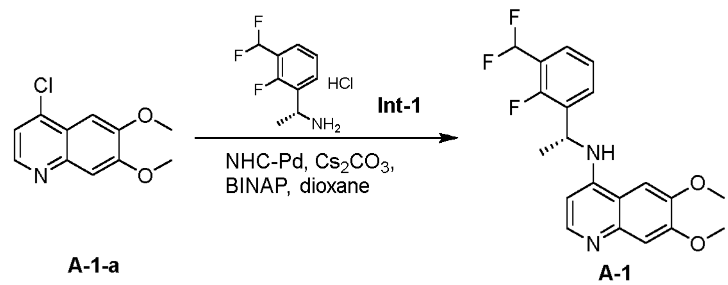


**A-1-a** **A-1**

To a three-neck flask containing 1,4-dioxane (3 mL), compound **A-1-a** (50 mg, 0.22 mmol), cesium carbonate (219 mg, 0.67 mmol), and compound Int-1(85 mg, 0.22 mmol) were successively added. NHC-Pd (10 mg, 0.022 mmol) and 1,1'-binaphthyl-2,2'-bis-diphenylphosphine (14 mg, 0.022 mmol) were added under nitrogen protection, and after the addition, the reaction was performed overnight at 110°C. After the reaction was completed, the reaction solution

was cooled to room temperature and then filtered, and the filtrate was concentrated under reduced pressure. The residue was subjected to reverse-phase preparative purification to obtain compound **A-1** (23.6 mg, white solid) with the yield of 28%. LCMS (ESI): *m/z* =377.2 $[M+H]^+$. $^1H$ NMR (400 MHz, $CD_3OD$): $\delta$ 7.98 (d, *J* = 8.0 Hz, 1H), 7.61 (s, 1H), 7.46-7.38 (m, 2H), 7.13-7.08 (m, 2H), 6.94 (d, *J*= 56.0 Hz, 1H), 6.06 (d, *J*= 8.0 Hz, 1H), 5.06-5.05 (m, 1H), 3.94 (s, 3H), 3.86 (s, 3H), 1.65 (d, *J*= 8.0 Hz, 3H).

The synthesis method of the following compounds was the same as that of A-1.

| No. | Structure | Character | LCMS (ESI): *m/z* $[M+H]^+$ | $^1H$ NMR |
|---|---|---|---|---|
| A-2 | 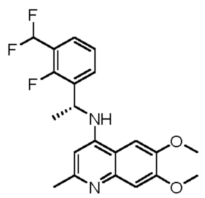 | White solid | 391.2 | $^1H$ NMR (400 MHz, $CD_3OD$): $\delta$ 7.92 (s, 1H), 7.57-7.61 (m, 2H), 7.30-7.32 (m, 1H), 6.90-7.19 (m, 1H), 6.42 (s, 1H), 5.38-5.40 (m,1H), 4.01 (s, 3H), 4.05 (s, 3H), 2.54 (s, 3H), 1.80-1.82 (d, *J* = 6.8 Hz, 3H). |

**Example 8 Synthesis of compound A-3**

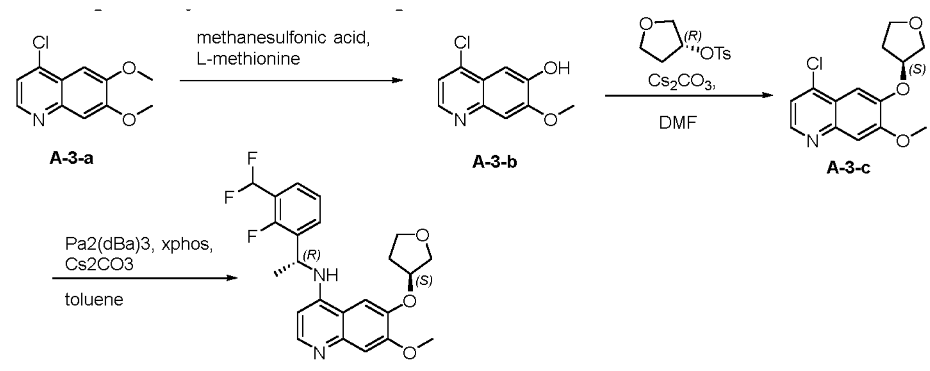


A-3-a A-3-b A-3-c A-3

Step one:

Compound **A-3-a** (3 g, 13.5 mmol) and methanesulfonic acid (10 mL) was added to a sealed tube, followed by L-methionine (5.0 g, 34 mmol) to the system. After the sealing, stirring was performed at 150°C for 12 hours. After the reaction was completed detected by TLC, the reaction mixture was cooled, poured into ice, basified with an ammonium hydroxide solution (pH of 9), and extracted with EtOAc (100 mL×3). The combined organic layers were dried over sodium sulfate and concentrated under reduced pressure to afford the crude product. The crude compound was purified by a silica gel column to obtain **A-3-b** (1.2 g, yield of 43%).

Step two:

Compound **A-3-b** (100 mg, 0.48 mmol), (R)-tetrahydrofuran-3-yl-4-methylbenzenesulfonate (172 mg, 0.71 mmol), and cesium carbonate (390 mg, 1.2 mmol) were dissolved in DMF (5 mL) and reacted overnight at 80°C. After the reaction was completed, the reaction solution was extracted with EtOAc (10 mL×3) The combined organic layers were dried over sodium sulfate and concentrated under reduced pressure to afford the crude product. The crude compound was purified by a silica gel column to obtain **A-3-c** (100 mg, yield of 75%).

Step three:

Compound **A-3-c** (70 mg, 0.25 mmol), (R)-1-(3-(difluoromethyl)-2-fluorophenyl)ethylamine hydrochloride (compound **Int-1**, 56 mg, 0.25 mmol), $Pd_2(dba)_3$ (23 mg, 0.025 mmol), X-phos (24 mg, 0.05 mmol), and cesium carbonate (204 mg, 0.63 mmol) were dissolved in toluene, stirred overnight at 110°C. After the reaction was completed, the reaction

solution was cooled and extracted with EtOAc (10 mL×3). The combined organic layers were dried over sodium sulfate and concentrated under reduced pressure to afford the crude product. The crude product was subjected to Prep-HPLC to obtain compound A-3 (17 mg, yield of 16%). LCMS (ESI): *m/z* 433.2 $[M+H]^+$; $^1H$ NMR (400 MHz, $CD_3OD$): $\delta$ 8.08 (d, *J* = 5.6 Hz, 1H), 7.74 (s, 1H), 7.54-7.50 (m, 2H), 7.24-7.21 (m, 2H), 7.04 (t, *J* = 54.8 Hz, 1H), 6.16 (d, *J*= 5.6 Hz, 1H), 5.31 (d, *J* = 2.0 Hz, 1H), 5.17-5.15 (m, 1H), 4.05 (t, *J* = 3.2 Hz, 3H), 3.96 (t, *J* = 5.2 Hz, 4H), 2.31-2.27 (m, 2H), 1.73 (d, *J* = 6.8 Hz, 3H).

The synthesis method of the following compounds was the same as that of A-3.

| No. | Structure | Character | LCMS (ESI): *m/z* $[M+H]^+$ | $^1H$ NMR |
|---|---|---|---|---|
| A-4 | 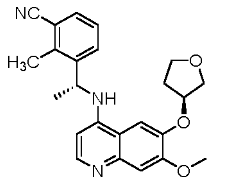 | White solid | 404.3 | $^1H$ NMR (400 MHz, $CD_3OD$): $\delta$ 8.43 (s, 1H), 8.03 (d, *J* = 6.8 Hz, 1H), 7.77-7.89 (m, 1H), 7.46-7.60 (m, 2H), 7.30-7.04 (m, 2H), 6.18 (t, *J* = 6.5 Hz, 1H), 5.02-5.33 (m, 2H), 4.01-3.90 (m, 6H), 3.84 (ddd, *J* = 14.1, 9.1, 5.0 Hz, 1H), 2.62 (s, 3H), 2.05-2.36 (m, 2H), 1.57-1.76 (m, 3H). |

**Example 9 Synthesis of compound A-5**

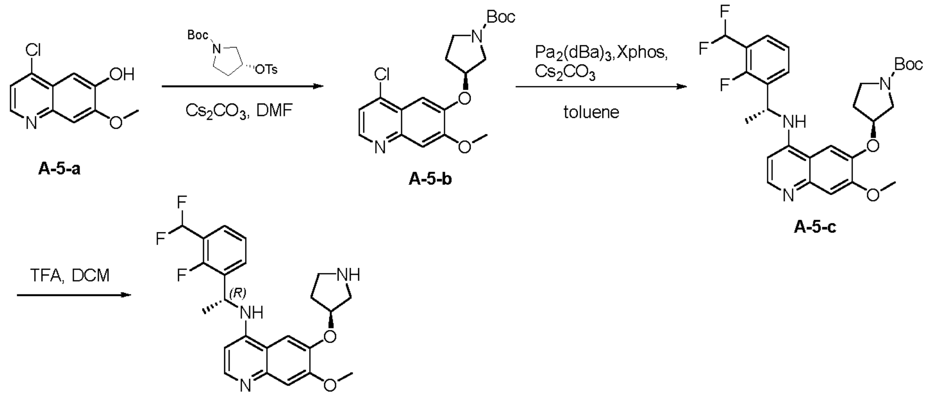


A-5-a A-5-b A-5-c A-5

Step one:

Compound **A-5-a** (300 mg, 1.43 mmol), (R)-3-(tosyloxy)pyrrolidin-1-carboxylic acid tert-butyl ester (734 mg, 2.15 mmol), and cesium carbonate (1,169 mg, 3.6 mmol) were dissolved in DMF (15 mL) and reacted overnight at 80°C. After the reaction was completed, the reaction solution was cooled and extracted with EtOAc (10 mL×3) The combined organic layers were dried over sodium sulfate and concentrated under reduced pressure to afford the crude product. The crude product was purified by a silica gel column to obtain compound A-5-b (500 mg, yield of 92%).

Step two:

Compound **A-5-b** (500 mg, 1.32 mmol), (R)-1-(3-(difluoromethyl)-2-fluorophenyl)ethylamine hydrochloride (compound **Int-1**, 446 mg, 1.98 mmol), $Pd_2(dba)_3$ (121 mg, 0.13 mmol), X-phos (124 mg, 0.26 mmol), and cesium carbonate (1,076 mg, 3.3 mmol) were dissolved in toluene, and stirred overnight at 100°C under nitrogen protection. After the reaction was completed, the reaction solution was cooled and extracted with EtOAc (10 mL×3). The combined organic layers were dried over sodium sulfate and concentrated under reduced pressure to obtain the crude product. The crude product was purified by the silica gel column to obtain **A-5-c** (200 mg, yield of 28%).

Step three:

Compound **A-5-c** (200 mg, 0.38 mmol) was dissolved in DCM (10 mL) and TFA (0.5 mL) was added under ice bath. Stirring was performed at room temperature for 2 hours. After the reaction was completed, the reaction solution was extracted with EtOAc (10 mL×3). The combined organic layers were dried over sodium sulfate and concentrated under reduced pressure to afford the crude product. The compound was subjected to Prep-HPLC to obtain compound **A-5** (6.3 mg, yield of 4%). LCMS (ESI): *m/z* = 432.2 $[M+H]^+$, $^1H$ NMR (400 MHz, $CD_3OD$): $\delta$ 8.20 (d, *J* = 7.2 Hz, 1H), 8.14 (s, 1H), 7.66-7.55 (m, 2H), 7.32-7.28 (m, 2H), 7.03 (t, *J* = 54.4 Hz, 1H), 6.58 (d, *J* = 7.6 Hz, 1H), 5.46-5.37 (m, 2H), 4.03 (s, 3H), 3.74-3.55 (m, 4H), 2.49-2.36 (m, 2H), 1.82 (d, *J*= 7.2 Hz, 3H).

**Example 10 Synthesis of compound A-6**

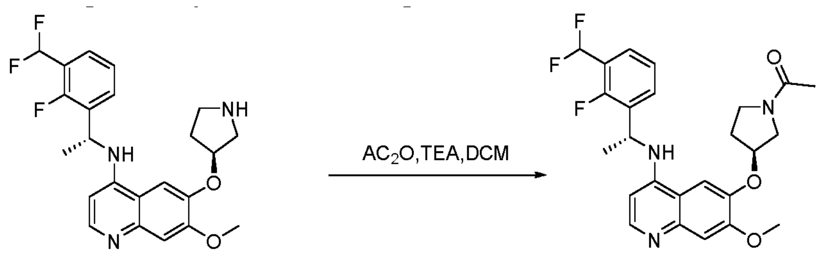


A-5 A-6

Step one:

Compound A-5 (50 mg, 0.12 mmol) and TEA (18 mg, 0.17 mmol) were dissolved in DCM (5 mL), and AczO (12 mg, 0.12 mmol) was added under ice bath. Stirring was performed at room temperature for 12 h. After the reaction was completed, the reaction solution was extracted with EtOAc (10 mL×3). The combined organic layers were dried over sodium sulfate and concentrated under reduced pressure to afford the crude product. The crude product was subjected to Prep-HPLC to obtain compound **A-6** (15.3 mg, yield of 28%). LCMS (ESI): *m/z* = 474.2 $[M+H]^+$, $^1H$ NMR (400 MHz, DMSO_$d_6$): $\delta$ 8.12-8.14 (m, 1H), 7.81-7.87 (m, 1H), 7.50-7.56 (m,2H), 7.12-7.40 (m, 4H), 6.06 (d, *J* = 5.6 Hz, 1H), 5.24-5.31 (m, 1H), 5.08 (t, *J* = 6.8 Hz, 1H), 3.58-3.88 (m, 7H), 2.01-2.27 (m, 2H), 1.68-1.97 (m, 3H), 1.67 (d, *J*= 6.8 Hz, 3H).

The synthesis method of the following compounds was the same as that of A-6.

| No. | Structure | Character | LCMS (ESI): *m/z* $[M+H]^+$ | $^1H$ NMR |
|---|---|---|---|---|
| A-7 | 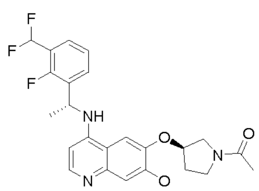 | White solid | 474.2 | $^1H$ NMR (400 MHz, DMSO-$d_6$): $\delta$ 8.13 (dd, *J* = 5.3, 3.2 Hz, 1H), 7.84 (d, *J* = 24.9 Hz, 1H), 7.47-7.61 (m, 2H), 7.04-7.41 (m, 4H), 6.04 (t, *J* = 5.1 Hz, 1H), 5.28 (d, *J* = 26.4 Hz, 1H), 4.98-5.11 (m, 1H), 3.34-4.04 (m, 7H), 2.19-2.39 (m, 1H), 2.14 (s, 1H), 2.00 (d, *J* = 12.5 Hz, 3H), 1.67 (d, *J* = 6.8 Hz, 3H). |

**Example 11 Synthesis of compound A-16**

Synthetic route:

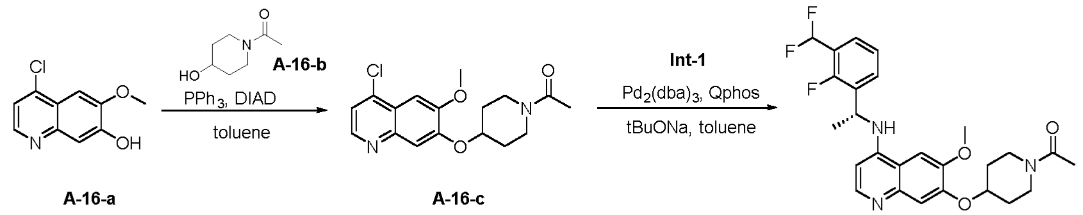


A-16-a A-16-c A-16

Step one:

To a dry 50-mL three-neck flask, compound **A-16-a** (0.10 g, 0.48 mmol), 1-(4-hydroxypiperidin-1-yl)ethanone **A-16-b** (68 mg, 0.48 mmol), triphenylphosphine (0.16 g, 0.62 mmol), and anhydrous toluene (10 mL) were successively added, and nitrogen replacement was performed for three times. The reaction was performed while stirring at 120°C for 10 minutes, diisopropyl azodicarboxylate (0.12 g, 0.62 mmol) was added, and the reaction was performed while stirring for 3 hours. After the reaction was completed monitored by LCMS, water was added for dilution, and the solution was extracted with ethyl acetate (50 mL×3). The organic phases were combined, washed with a saturated salt solution (50 mL×2), dried over anhydrous sodium sulfate, and filtered. The filtrate was concentrated under reduced pressure. The resulting residue was purified by a silica gel column chromatography (dichloromethane/methanol = 50/1-30/1) to obtain (1-(4-((4-chloro-6-methoxyquinolin-7-yl)oxy)piperidin-1-yl)ethanone **A-16-c** (123 mg, light yellow solid) with the yield of 77%. LCMS (ESI): *m/z* = 335.1 [M+H]$^+$.

Step two:

To a dry 50-mL single-neck flask, compound **A-16-c** (0.12 mg, 0.37 mmol), intermediate **Int-1** (83 mg, 0.37 mmol), sodium t-butoxide (0.14 g, 1.5 mmol), trisdibenzylideneacetone dipalladium (67 mg, 0.07 mmol), 1,2,3,4,5-pentylphenyl-1'-(di-t-butylphosphinyl)ferrocene (26 mg, 0.04 mmol), and anhydrous toluene (8 mL) were successively added. The reaction was performed while stirring at 100°C for 16 hours under nitrogen protection. After the reaction was completed monitored by LCMS, water was added for dilution, and the solution was extracted with ethyl acetate (50 mL×3). The organic phases were combined, washed with a saturated salt solution (50 mL×2), dried over anhydrous sodium sulfate, and filtered. The filtrate was concentrated under reduced pressure. The resulting residue was purified by the silica gel column chromatography (dichloromethane/methanol = 50/1-10/1) to obtain the crude product. The crude product was beat using n-hexane, filtered, and dried to obtain A-16 (58 mg, light yellow solid) with the yield of 32%. LCMS: *m/z* 488.2 [M+H]$^+$. $^1$H NMR (DMSO-$d_6$, 400 MHz): $\delta$ 8.16 (d, *J*= 5.6 Hz, 1H), 7.83 (s, 1H), 7.52-7.62 (m, 3H), 7.12-7.39 (m, 3H), 6.15 (d, *J*=5.6 Hz, 1H), 5.13-5.16 (m, 1H), 4.76-4.78 (m, 1H), 3.99 (s, 3H), 3.89-3.91 (m, 1H), 3.69-3.72 (m, 1H), 3.38-3.40 (m, 1H), 3.22-3.28 (m, 1H), 2.03-1.97 (m, 4H), 1.91-1.95 (m, 1H), 1.68-1.70 (m, 4H), 1.55-1.58 (m, 1H).

**Example 12 Synthesis of compound A-17**

Synthetic route:

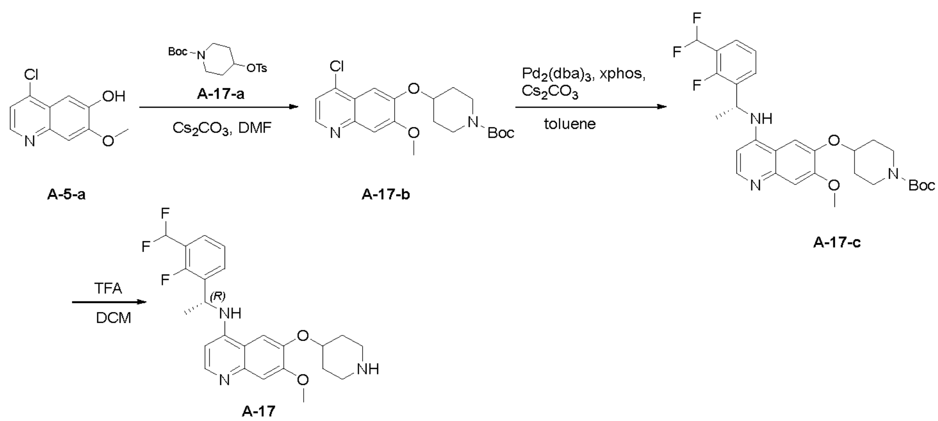


A-5-a A-17-b A-17-c A-17

Step one:

Compound **A-5-a** (300 mg, 1.4 mmol), cesium carbonate (936 mg, 2.8 mmol), and compound **A-17-a** (612 mg, 1.7 mmol) were added into an eggplant-shaped flask containing dimethylformamide (10 mL) to be mixed uniformly and then reacted at room temperature for 7 hours. After the reaction was completed, the reaction solution was cooled and extracted with ethyl acetate, the upper layer organic phase was collected, dried, and then concentrated under reduced pressure, and the residue was purified by a column chromatography to obtain a yellow solid compound **A-17-b**. LCMS (ESI): *m*/*z* = 393.1 $[M+H]^+$.

Step two:

Compound **A-17-b** (500 mg, 1.28 mmol), compound **Int-1** (344 mg, 1.53 mmol), and xphos (243 mg, 0.51 mmol) were dissolved in toluene (15 mL), and cesium carbonate (832 mg, 2.6 mmol) and $Pd_2(dba)_3$ (117 mg, 0.13 mmol) were added under nitrogen protection, and the mixture was reacted overnight at 100°C after replacement of nitrogen. After the reaction was completed, the reaction solution was cooled and extracted with ethyl acetate, the organic phase was concentrated under reduced pressure, and the residue was purified by the column chromatography to obtain a yellow oily compound **A-17-c** (60 mg, yield of 14%). LCMS (ESI): *m*/*z* = 546.2 $[M+H]^+$.

Step three:

Compound **A-17-c** (60 mg, 0.13 mmol) was dissolved in dichloromethane (3 mL) in an eggplant-shaped flask, and trifluoroacetic acid (1 mL) was added thereto under ice bath, followed by uniform mixing. The reaction solution reacted at room temperature for 1 hour. After the reaction was completed, the reaction solution was concentrated under reduced pressure to obtain compound A-17 (23.3 mg, yield of 50%). LCMS (ESI): *m*/*z* =446.2 $[M+H]^+$. $^1H$ NMR (400 MHz, $CD_3OD$): $\delta$ 8.19 (d, *J* = 7.0 Hz, 2H), 7.63 (t, *J* = 7.4 Hz, 1H), 7.57 (t, *J* = 7.0 Hz, 1H), 7.34-7.24 (m, 2H), 7.03 (t, *J* = 54.7 Hz, 1H), 6.56 (d, *J* = 7.2 Hz, 1H), 5.39 (q, *J* = 6.8 Hz, 1H), 4.95-5.03 (m, 1H), 4.05 (s, 3H), 3.54-3.41 (m, 2H), 3.30-3.22 (m, 2H), 2.32-2.11 (m, 4H), 1.82 (d, *J* = 6.8 Hz, 3H).

**Example 13 Synthesis of compound A-18**

Synthetic route:

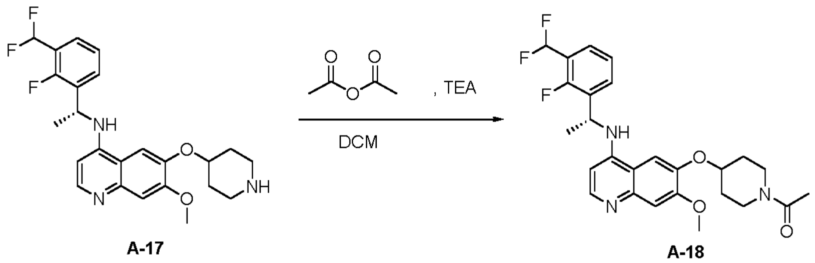


A-17 A-18

Compound **A-17** (16 mg, 0.04 mmol) and triethylamine (7.3 mg, 0.08 mmol) were dissolved in dichloromethane (2 mL) in an eggplant-shaped flask, and acetic anhydride (3.7 mg, 0.04 mmol) was added thereto under ice bath, followed by uniform mixing. The reaction solution reacted at room temperature for 2 hours. After the reaction was completed, the reaction solution was concentrated under reduced pressure. The residue was subjected to reverse-phase preparative purification to obtain a white solid compound **A-18** (10.8 mg, yield of 6.6%). LCMS (ESI): *m*/*z* =488.3 $[M+H]^+$. $^1H$ NMR (400 MHz, $CD_3OD$): $\delta$ 8.17 (d, *J* = 7.1 Hz, 1H), 8.11 (s, 1H), 7.51-7.65 (m, 2H), 7.30 (t, *J*= 7.7 Hz, 1H), 7.24 (s, 1H), 7.03 (t, *J*= 54.7 Hz, 1H), 6.56 (dd, *J*= 7.1, 2.2 Hz, 1H), 5.27-5.46 (m, 1H), 4.93-4.99 (m, 1H), 4.04 (s, 3H), 3.75-3.85 (m, 2H), 3.64-3.73 (m, 1H), 3.52-3.61 (m, 1H), 2.15 (s, 3H), 2.06-2.11 (m, 1H), 1.92-2.05 (m, 2H), 1.85-1.90 (m, 1H), 1.82 (d, *J* = 6.8 Hz, 3H).

**Example 14 Synthesis of compound A-39**

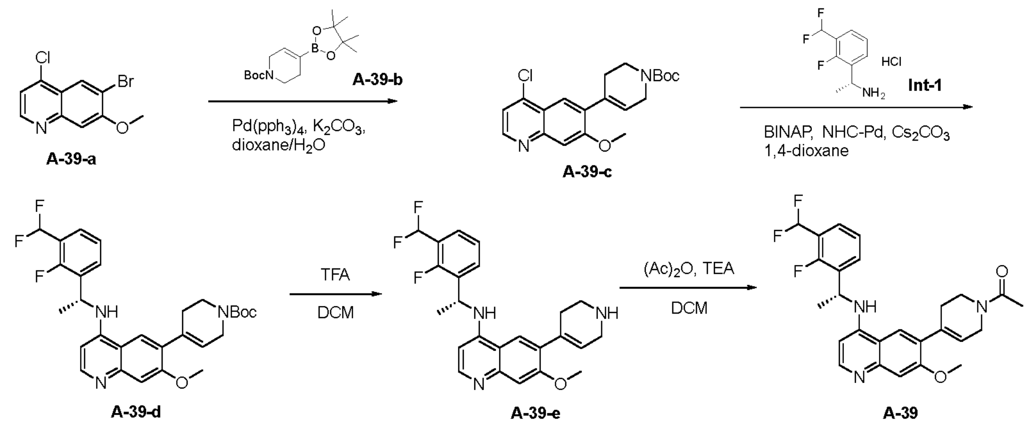


Step one:

To a dry flask, compound **A-39-a** (500 mg, 1.83 mmol), compound **A-39-a** (1,130 mg, 3.67 mmol), potassium carbonate (761 mg, 5.50 mmol), and 1,4-dioxane and water (16 mL, V/V = 15/1) were successively added, and after nitrogen replacement for several times, tetrakis(triphenylphosphine) palladium (424 mg, 0.37 mmol) was added. The reaction was performed at 100°C for 3 hours under nitrogen protection. After the reaction was completed, the reaction solution was cooled and concentrated under reduced pressure. The concentrated product was purified by a glass silica gel column to obtain a light yellow oily compound A-39-c (420 mg, yield of 61.1%).

Step two:

To a dry flask, compound **A-39-c** (300 mg, 0.80 mmol), compound **Int-1** (180 mg, 0.80 mmol), cesium carbonate (780 mg, 2.39 mmol), BINAP (500 mg, 0.80 mmol), and 1,4-dioxane (20 mL) were successively added, and after nitrogen replacement for several times, NHC-Pd (50 mg, 0.08 mmol) was added. The reaction was performed at 110°C for 3 hours under nitrogen protection. After the reaction was completed, the reaction solution was cooled and concentrated under reduced pressure. The concentrated product was purified by the glass silica gel column to obtain a yellow oily compound **A-39-d** (173 mg, yield of 41.0%).

Step three:

Compound **A-39-d** (173 mg, 3.9 mmol) was dissolved in trifluoroacetic acid/dichloromethane (10 mL, 1:4) and reacted at room temperature for two hours under nitrogen protection. After the reaction was completed, the reaction solution was concentrated under reduced pressure to obtain compound A-39-e (290 mg, yellow oil).

Step four:

Compound **A-39-e** (29 mg, 0.07 mmol, crude product) was dissolved in dichloromethane (2 mL), acetic anhydride (6.9 mg, 0.07 mmol) and triethylamine (20.5 mg, 0.20 mmol) were dropwise added at 0°C respectively, and the reaction solution reacted at room temperature for 3 hours. After the reaction was completed, the reaction solution was quenched in ice water and extracted with dichloromethane. The organic phase was backwashed with a saturated salt solution, dried over sodium sulfate, filtered, and concentrated under reduced pressure. The residue was subjected to neutral preparative purification to obtain compound A-**39** (4.2 mg, white solid) with the yield of 13.2%. LCMS (ESI): $m/z$ = 470.2 $[M+H]^+$; $^1H$ NMR (400 MHz, $CDCl_3$): $\delta$ 8.33 (d, $J$= 5.4 Hz, 1H), 7.54-7.49 (m, 2H), 7.46 (t, $J$= 7.0 Hz, 1H), 7.35 (s, 1H), 7.17 (t, $J$ = 7.7 Hz, 1H), 6.92 (t, $J$ = 37.5 Hz, 1H), 6.09 (d, $J$ = 5.4 Hz, 1H), 5.89 (d, $J$ = 9.8 Hz, 1H), 5.20 (d, $J$ = 16.7 Hz, 1H), 5.07 (dd, $J$ = 10.8, 5.0 Hz, 1H), 4.31 (d, $J$ = 2.8 Hz, 1H), 4.19 (d, $J$ = 2.8 Hz, 1H), 3.94 (s, 3H), 3.85 (t, $J$ = 5.7 Hz, 1H), 3.70 (t, $J$ = 5.6 Hz, 1H), 2.62 (d, $J$ = 13.9 Hz, 2H), 2.19 (d, $J$ = 4.1 Hz, 3H), 1.75 (dd, $J$ = 6.6, 3.3 Hz, 3H).

**Example 15 Synthesis of compound A-40**

Synthetic route:

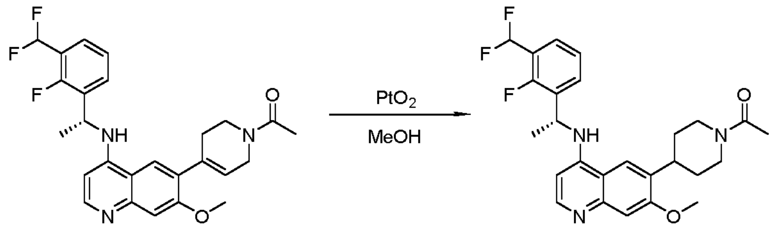


A-39 A-40

Compound A-39(9.2 mg) was dissolved in methanol (3 mL), platinum dioxide (4.6 mg, 50%wt) was added, and the reactants reacted under a hydrogen atmosphere for 3 hours. After the reaction was completed, the reaction solution was filtered, the filtrate was concentrated under reduced pressure, and the residue was purified by a column chromatography to obtain a white solid compound **A-40** (5.3 mg, yield of 57.4%). LCMS (ESI): *m*/*z* = 472.3 [M+H]$^+$; $^1$H NMR (400 MHz, $CD_3OD$): $\delta$ 8.12 (dd, *J* = 14.2, 8.5 Hz, 2H), 7.52 (dt, *J* = 18.5, 7.2 Hz, 2H), 6.83-7.30 (m, 3H), 6.15 (dd, *J* = 5.8, 2.6 Hz, 1H), 5.07-5.18 (m, 1H), 4.75 (d, *J* = 13.3 Hz, 1H), 4.11 (t, *J* = 12.6 Hz, 1H), 3.97 (s, 3H), 3.41 (ddd, *J* = 12.0, 7.8, 3.6 Hz, 1H), 3.35-3.32 (m, 1H), 2.78 (dd, *J* = 12.9, 10.4 Hz, 1H), 2.18 (d, *J* = 7.1 Hz, 3H), 1.96-2.04 (m, 1H), 1.80-1.94 (m, 3H), 1.66-1.79 (m, 3H).

**Example 16 Synthesis of compound A-41**

Synthetic route:

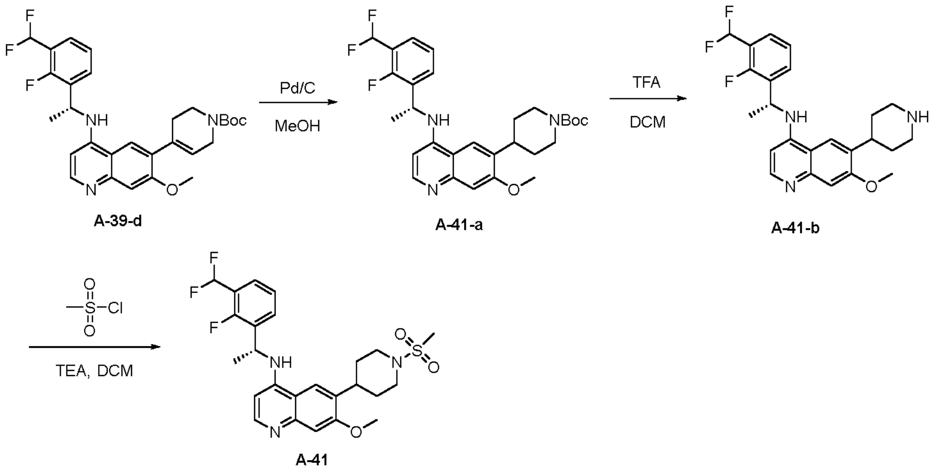


A-39-d A-41-a A-41-b A-41

Step one:

Compound **A-39-d** (150 mg, 0.28 mmol) was dissolved in methanol (5 mL) in an eggplant-shaped flask, and palladium on carbon (60 mg) was added thereto. The reaction was performed at room temperature for 2 hours under a hydrogen condition. After the reaction was completed, the reaction solution was filtered and concentrated under reduced pressure to obtain compound **A-41-a** (80 mg, yield of 53%). LCMS (ESI): *m*/*z* = 530.1 [M+H]$^+$.

Step two:

Compound **A-41-a** (80 mg, 0.15 mmol) was dissolved in dichloromethane (5 mL) in an eggplant-shaped flask, and trifluoroacetic acid (2 mL) was added thereto under ice bath. The reaction was performed at room temperature for 1 hour. After the reaction was completed, the reaction solution was concentrated under reduced pressure. The residue was subjected to reverse-phase preparative purification to obtain a white solid compound **A-41-b** (40 mg, yield of 62%). LCMS (ESI): *m*/*z* =430.2 [M+H]$^+$; $^1$H NMR (400 MHz, $CD_3OD$): $\delta$ 8.59 (s, 1H), 8.23 (d, *J*= 7.1 Hz, 1H), 7.75 (t, *J*= 7.3 Hz, 1H), 7.56 (t, *J*= 7.0 Hz, 1H), 7.30 (dd, *J*= 15.5, 7.7 Hz, 2H), 7.04 (t, *J* = 54.7 Hz, 1H), 6.58 (d, *J* = 7.2 Hz, 1H), 5.43 (q, *J* = 6.7 Hz, 1H), 4.06 (s, 3H), 3.53 -3.65 (m, 2H), 3.38-3.50 (m, 1H), 3.15-3.29 (m, 2H), 2.06-2.40 (m, 4H), 1.88 (d, *J*= 6.8 Hz, 3H).

Step three:

Compound **A-41-b** (20 mg, 0.05 mmol) and triethylamine (9 mg, 0.09 mmol) were dissolved in dichloromethane (2 mL) in an eggplant-shaped flask, and methylsufonyl chloride (4 mg, 0.05 mmol) was added thereto under ice bath, followed by uniform mixing. The reaction solution reacted at room temperature for 2 hours. After the reaction was completed, the reaction solution was concentrated under reduced pressure. The residue was subjected to a reverse-phase preparative purification to obtain a white solid compound **A-41** (7.3 mg, yield of 49%).

LCMS (ESI): *m/z* = 508.3 $[M+H]^+$; $^1H$ NMR (400 MHz, MeOD): $\delta$ 8.18 (s, 1H), 8.12 (d, *J*= 5.7 Hz, 1H), 7.52 (dt, *J*= 21.3, 7.0 Hz, 2H), 7.22 (t, *J*= 7.7 Hz, 1H), 6.86-7.19 (m, 2H), 6.14 (d, *J* = 5.8 Hz, 1H), 5.15 (q, *J* = 6.8 Hz, 1H), 3.96 (s, 3H), 3.91 (d, *J* = 11.6 Hz, 2H), 3.26 (t, *J* = 6.9 Hz, 1H), 2.90-2.98 (m, 2H), 2.89 (s, 3H), 1.96-2.08 (m, 4H), 1.74 (d, *J* = 6.8 Hz, 3H).

**Example 17 Synthesis of compounds A-45 and A-46**

Synthetic route:

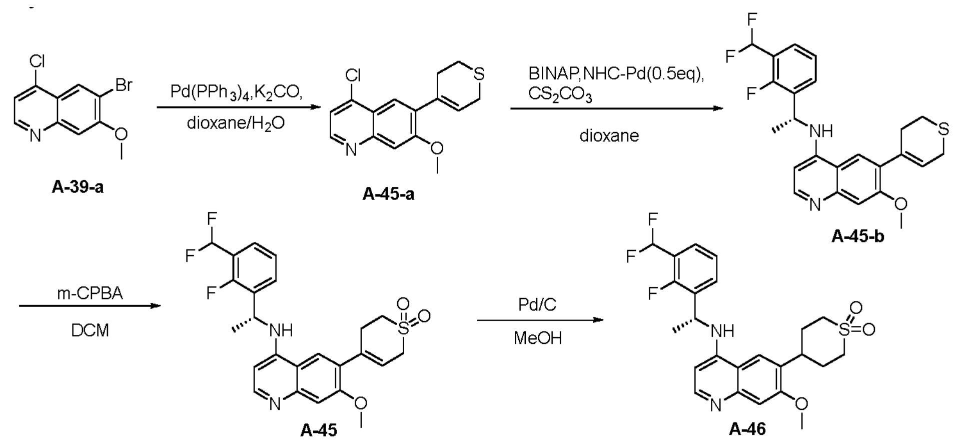


Step one:

To a dry flask, compound **A-39-a** (400 mg, 1.47 mmol), (3,6-dihydro-2H-thiopyran-4-yl)-boronic acid (430 mg, 1.90 mmol), potassium carbonate (606 mg, 4.39 mmol), and 1,4-dioxane and water (12 mL, V/V = 5/1) were successively added, and after nitrogen replacement for several times, tetrakis(triphenylphosphine) palladium (338 mg, 0.29 mmol) was added. The reaction was performed at 100°C for 6 hours under nitrogen protection. After the reaction was completed, the reaction solution was cooled, poured into a saturated ammonium chloride solution, extracted with ethyl acetate, and dried over sodium sulfate. The organic phase was filtered and concentrated under reduced pressure. The concentrated product was purified by a glass silica gel column to obtain a light yellow oily compound **A-45-a** (420 mg, yield of 98%).

Step two:

To a dry flask, compound **A-45-a** (100 mg, 0.34 mmol), compound **Int-1** (77 mg, 0.34 mmol), cesium carbonate (336 mg, 1.03 mmol), BINAP (107 mg, 0.17 mmol), and 1,4-dioxane (10 mL) were successively added, and after nitrogen replacement for several times, NHC-Pd (117 mg, 0.17 mmol) was added. The reaction was performed at 110°C for 12 hours under nitrogen protection. After the reaction was completed, the reaction solution was cooled, poured into a saturated ammonium chloride solution, extracted with ethyl acetate, and dried over sodium sulfate. The organic phase was filtered and concentrated under reduced pressure. The concentrated product was purified by the glass silica gel column to obtain a yellow oily compound **A-45-b** (120 mg, yield of 80%).

Step three:

Compound **A-45-b** (100 mg, 0.25 mmol) and m-CPBA (97 mg, 0.56 mmol) were dissolved in dichloromethane (10 mL) and reacted overnight at room temperature. After the reaction was completed, the reaction solution was poured into a saturated sodium bicarbonate solution, extracted with ethyl acetate, and dried over sodium sulfate. The organic

phase was filtered and concentrated under reduced pressure. The concentrated product was purified by the glass silica gel column to obtain a yellow compound A-45 (35 mg, yield of 35%).

Step four:

Compound A-45 (35 mg, 0.07 mmol) and Pd/C (15 mg) were dissolved in methanol (5 mL) and reacted at room temperature for 12 hours under hydrogen protection. After the reaction was completed, the reaction solution was filtered, the filtrate was concentrated under reduced pressure, and the residue was purified by Prep-HPLC to obtain a white solid compound **A-46** (7.5 mg, yield of 21.4%). LCMS (ESI): *m/z* = 479.3 $[M+H]^+$; $^1H$ NMR (400 MHz, $CD_3OD$): $\delta$ 8.27-8.30 (m,1H), 8.14 (d, *J* = 6.0Hz, 1H), 7.50-7.58 (m, 2H), 7.20-7.25 (m, 2H), 7.03 (t, *J* = 55.2 Hz, 1H), 6.21-6.22 (m, 1H), 4.81-5.20 (m, 1H), 3.99 (s, 3H), 3.47-3.53 (m, 1H), 3.39-3.44 (m, 2H), 3.12-3.18 (m, 2H), 2.560-2.56 (m, 2H), 2.00-2.30 (m, 2H), 1.75 (d, *J* = 6.8Hz, 3H).

**Example 18 Synthesis of compounds A-47 and A-48**

Synthetic route:

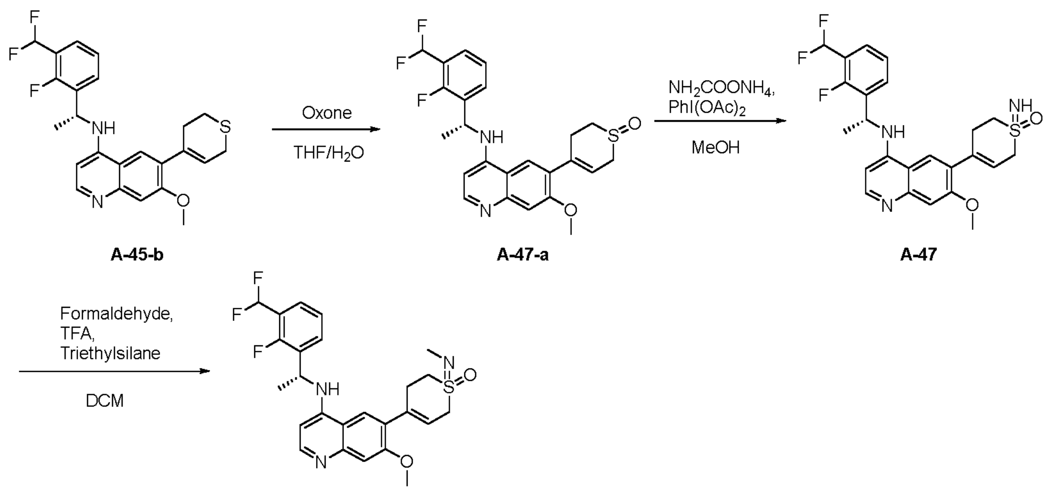


A-45-b A-47-a A-47

A-48

Step one:

To a single neck flask, compound **A-45-b** (445 mg, 1.0 mmol) was added and dissolved with 10 mL of THF, 2.5 mL of water was added to the system, and after the system was cooled to 0°C, an oxidizing agent potassium peroxymonosulfonate (320 mg, 0.52 mmol) was slowly added. After the addition was complete, stirring was continued at this temperature for 3 hours and the reaction was monitored by LCMS. After the reaction was completed, saturated sodium thiosulfate (about 5 mL) was added and continuously stirred for 10 min. The system was extracted with 20 mL of ethyl acetate, followed by washing with water and saturated brine. The organic phase was dried and subjected to a column separation to obtain a target compound A-47-a (light yellow solid, 320 mg, yield of 69%). LCMS (ESI): *m/z* = 461.2 $[M+H]^+$.

Step two:

This operation was performed with reference to the synthesis method described on page 361 of patent WO2021092115. To a single neck flask, **A-47-a** (1.0 eq) was added and dissolved in an appropriate amount of methanol, and iodobenzene diacetate (3.0 eq) and ammonium carbamate (4.0 eq) were added. Stirring was performed overnight at room temperature and the concentrated crude product was separated by a silica gel column to obtain a target compound **A-47.**

Step three:

This operation was performed with reference to the synthesis method described on page 365 of patent WO2021092115. To a single neck flask, compound A-47 (1.0 eq) was added, and dissolved in an appropriate amount of dichloromethane. An aqueous solution of formaldehyde (4.0 eq) and trifluoroacetic acid (4.0 eq) were added at room temperature and stirred at this temperature for 3 hours. Then triethylsilane (4.0 eq) was added and after the addition was completed, the mixture was stirred at room temperature overnight. After the reaction was completed, water, dichloromethane, and sodium carbonate (the pH of the aqueous phase was adjusted to 9) were added and stirred for 30 min, and the organic phase was separated. The organic phase was dried and separated by the silica gel column to obtain a target compound A-48.

**Example 19 Synthesis of compound A-58**

Synthetic route:

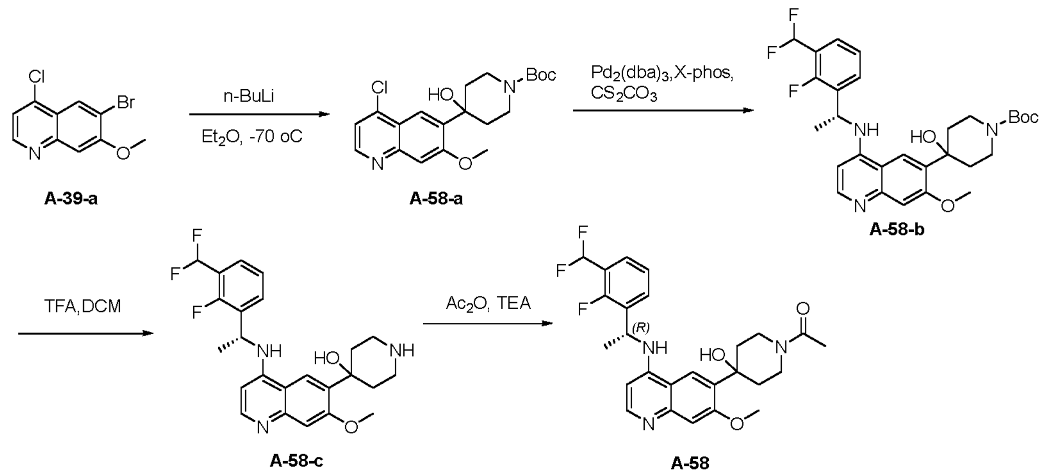


A-39-a A-58-a A-58-b A-58-c A-58

Step one:

Compound **A-39-a** (500 mg, 1.85 mmol) was dissolved in anhydrous ether, and n-BuLi (0.9 mL, 2.22 mmol, 2.5M in a hexane solution) was slowly dropwise added at -70°C and stirred at this temperature for 1 hour. Then tert-butyl 4-oxopiperidin-1-carboxylate (550 mg, 2.76 mmol) was dissolved in anhydrous ether, added to the reaction system at -70°C, and stirred for 3 hours. After the reaction was completed, the reaction solution was quenched with a saturated $NH_4Cl$ solution at low temperature and extracted with EtOAc (10 mL×3). The combined organic layers were dried over sodium sulfate and concentrated under reduced pressure to afford the crude product. The crude product was purified by a silica gel column to obtain compound **A-58-a** (200 mg, yield of 28%).

Step two:

Compound **A-58-a** (200 mg, 0.5 mmol), (R)-1-(3-(difluoromethyl)-2-fluorophenyl)ethylamine hydrochloride (compound **Int-1,** 172 mg, 0.77 mmol), $Pd_2(dba)_3$ (47 mg, 0.05 mmol), X-phos (48 mg, 0.1 mmol), and cesium carbonate (500 mg, 1.53 mmol) were dissolved in toluene, stirred overnight at 110°C under nitrogen protection. After the reaction was completed, the reaction solution was cooled and extracted with EtOAc (10 mL×3). The combined organic layers were dried over sodium sulfate and concentrated under reduced pressure to afford the crude product. The crude product was purified by the silica gel column to obtain compound **A-58-b** (50 mg, yield of 18%).

Step three:

Compound **A-58-b** (50 mg, 0.09 mmol) was dissolved in DCM (10 mL) and TFA (0.5 mL) was added under ice bath. Stirring was performed at room temperature for 2 hours. After the reaction was completed, the reaction solution was extracted with EtOAc (10 mL×3). The combined organic layers were dried over sodium sulfate and concentrated under reduced pressure to obtain compound **A-58-c** (30 mg, yield of 75%).

Step four:

Compound **A-58-c** (30 mg, 0.07 mmol) and TEA (10 mg, 0.1 mmol) were dissolved in DCM (3 mL), and $Ac_2O$ (7 mg, 0.07 mmol) was added under ice bath. Stirring was performed at room temperature for 2 hours. After the reaction was completed, the reaction solution was extracted with EtOAc (10 mL×3). The combined organic layers were dried over sodium sulfate and concentrated under reduced pressure to afford the crude product. The crude product was subjected to Prep-HPLC to obtain compound **A-58** (10.3 mg, yield of 32%). LCMS (ESI): m/z = 488.2 $[M+H]^+$; $^1H$ NMR (400 MHz, $CD_3OD$): $\delta$ 8.36 (d, *J* = 2.4 Hz, 1H), 8.16 (d, *J* = 5.6 Hz, 1H), 7.49-7.59 (m,, 2H), 7.17-7.24 (m,, 2H), 7.03 (t, *J*= 54.8 Hz, 1H), 6.20 (t, *J*= 5.2 Hz, 1H), 5.17-5.18 (m, 1H), 4.48-4.51 (m, 1H), 3.98 (s, 3H), 3.86 (d, *J* = 12.8 Hz, 1H), 3.70-3.73 (m, 1H), 3.19-3.20 (m,,1H), 3.48-3.56 (m, 2H), 2.18 (s, 3H), 1.81-1.91 (m, 2H), 1.73 (d, *J*= 6.45 Hz, 3H).

**Example 20 Synthesis of compound A-59**

Synthetic route:

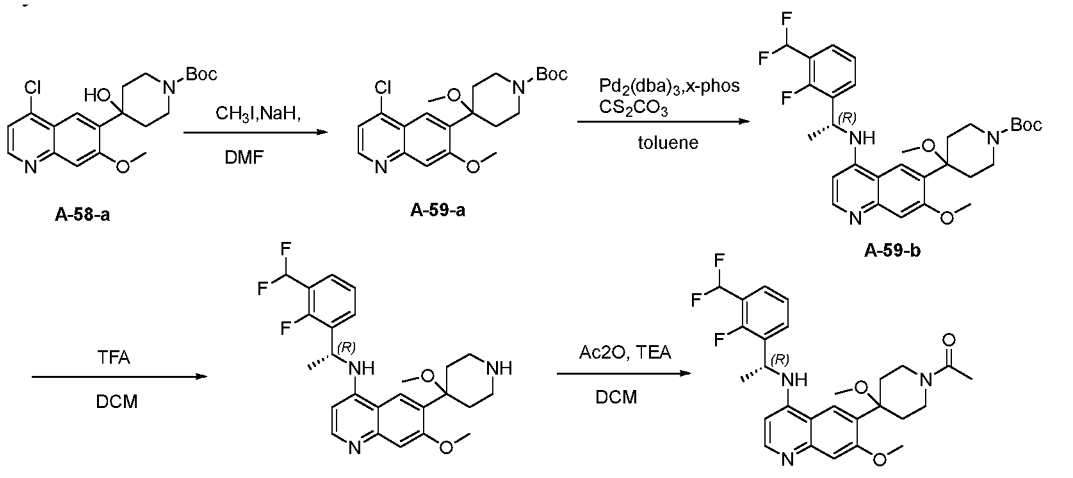


A-58-a A-59-a A-59-b A-59-c A-59

Step one:

Compound **A-58-a** (460 mg, 1.17 mmol) was dissolved in DMF, and NaH (94 mg, 3.90 mmol) was added under ice bath and stirred for 1 hour. Methyl iodide (250 mg, 1.76 mmol) was added in the reaction under ice bath. Stirring was performed at room temperature for 12 hours. After the reaction was completed, the reaction solution was cooled and extracted with EtOAc (10 mL×3). The combined organic layers were dried over sodium sulfate and concentrated under reduced pressure to afford the crude product. The crude product was purified by a silica gel column to obtain compound **A-59-a** (400 mg, yield of 84%).

Step two:

Compound **A-59-a** (400 mg, 1.0 mmol), (R)-1-(3-(difluoromethyl)-2-fluorophenyl)ethylamine hydrochloride (330 mg, 1.5 mmol), $Pd_2(dba)_3$ (90 mg, 0.1 mmol), X-phos (90 mg, 0.2 mmol), and cesium carbonate (960 mg, 3.0 mmol) were dissolved in toluene, stirred overnight at 110°C under nitrogen protection. After the reaction was completed, the reaction solution was cooled and extracted with EtOAc (10 mL×3). The combined organic layers were dried over sodium sulfate and concentrated under reduced pressure to afford the crude product. The crude product was purified by the silica gel column to obtain compound A-59-b (70 mg, yield of 13%).

Step three:

Compound **A-59-b** (50 mg, 0.09 mmol) was dissolved in DCM (10 mL) and TFA (0.5 mL) was added under ice bath. Stirring was performed at room temperature for 12 hours. After the reaction was completed, the solution was extracted with EtOAc (10 mL×3). The combined organic layers were dried over sodium sulfate and concentrated under

reduced pressure to obtain compound **A-59-c** (60 mg, yield of 98%).

Step four:

Compound **A-59-c** (50 mg, 0.11 mmol) and TEA (15 mg, 0.15 mmol) were dissolved in DCM (3 mL), and AczO (10 mg, 0.11 mmol) was added under ice bath. Stirring was performed at room temperature for 2 hours. After the reaction was completed, the reaction solution was extracted with EtOAc (10 mL×3). The combined organic layers were dried over sodium sulfate and concentrated under reduced pressure to afford the crude product. The crude product was subjected to Prep-HPLC to obtain compound **A-59** (20.5 mg, yield of 37%). LCMS (ESI): *m/z* =502.2 $[M+H]^+$; $^1H$ NMR (400 MHz, $CD_3OD$): $\delta$ 8.15-8.17 (m, 2H), 7.48-7.58 (m, 2H), 7.17-7.25 (m, 2H), 7.04 (t, *J* = 54.8Hz, 1H), 6.17-6.18 (m, 1H), 5.15-5.17 (m, 1H), 4.46-4.49 (m,,1H), 3.95 (s, 3H), 3.83 -3.86 (m, 1H), 3.61-3.64 (m, 1H), 3.08-3.14 (m, 4H), 2.51-2.60 (m, 2H), 2.10-2.21 (m, 5H), 1.74 (d, *J* = 6.8 Hz, 3H).

**Example 21 Synthesis of compound A-99**

Synthetic route:

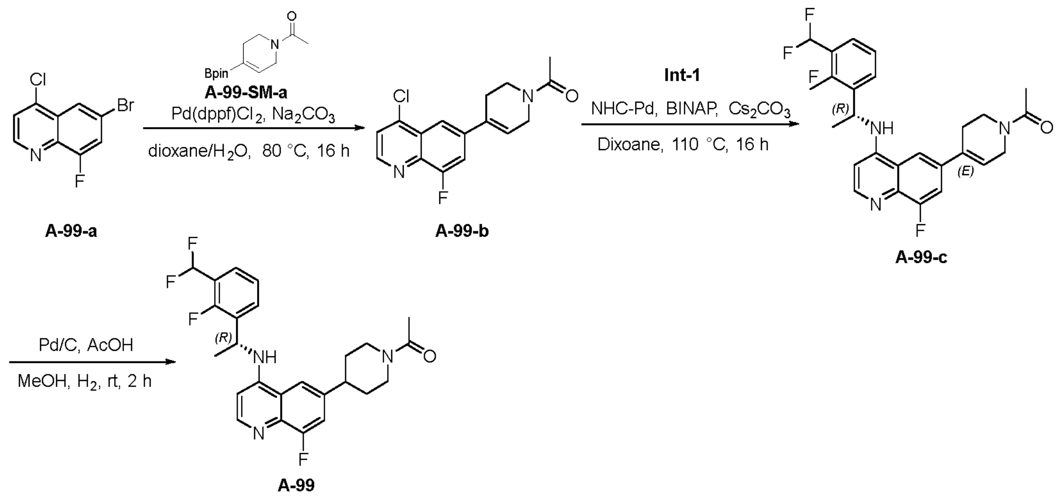

A-99-a
A-99-b
A-99-c
A-99

Step one:

Compound **A-99-a** (100 mg, 0.38 mmol) and compound **A-99-SM-a** (96 mg, 0.38 mmol) were dissolved in dioxane (10 mL) and water (2 mL), sodium carbonate (82 mg, 0.76 mmol) and $Pd(dppf)Cl_2$ (56 mg, 0.07 mmol) were added to the reaction system successively, then heated to 80°C in a sealed tube under nitrogen protection, and reacted for 16 hours. After the reaction was completed, the reaction solution was cooled and quenched with an ammonium chloride solution and then extracted with ethyl acetate. Then the organic phase was backwashed with a saturated salt solution, dried over sodium sulfate, filtered, and concentrated under reduced pressure. The residue was purified by a column chromatography to obtain compound **A-99-b** (108 mg, yellow solid) with the yield of 92%. LCMS (ESI): *m/z* = 305.7 $[M+H]^+$

Step two:

To a dry flask, compound **A-99-b** (108 mg, 0.34 mmol), **Int-1** (189 mg, 0.39 mmol), 1,4-dioxane (10 mL), cesium carbonate (323 mg, 0.99 mmol), BINAP (21 mg, 0.03 mmol), and NHC-Pd (23 mg, 0.03 mmol) were successively added, then heated to 110°C in a sealed tube and reacted for 16 hours under nitrogen protection. After the reaction was completed, the reaction solution was quenched with an ammonium chloride solution and the extracted with ethyl acetate. Then the organic phase was backwashed with a saturated salt solution, dried over sodium sulfate, filtered, and concentrated under reduced pressure. The residue was purified by a rapid column chromatography to obtain the crude product and then the crude product was purified by prep-HPLC to obtain a brown solid **A-99-c** (143 mg, yield of 80%). LCMS (ESI): *m/z* = 458.2 $[M+H]^+$

Step three:

To a dry flask, compound **A-99-c** (143 mg), Pd/C (50 mg), AcOH (20 mg), and MeOH (20 mL) were successively added and reacted under hydrogen at room temperature for 2 hours. After the reaction was completed, the reaction solution was concentrated under reduced pressure. The residue was purified by rapid column chromatography to obtain the crude product and then the crude product was purified by prep-HPLC to obtain a white solid **A-99** (22.7 mg, yield: 23%). LCMS (ESI): *m/z* = 458.2 $[M+H]^+$, $^1H$ NMR (400 MHz, DMSO-$d_6$) $\delta$ 8.27 (d, J = 5.3 Hz, 1H), 8.12 (s, J = 1.7 Hz, 1H), 7.59 (t, J = 7.5 Hz, 1H), 7.52 (q, J = 8.1, 7.6 Hz, 2H), 7.47-7.08 (m, 3H), 6.20 (d, J = 5.4 Hz, 1H), 5.09 (m, 1H), 4.70-4.57 (m, 1H), 4.00 (d, J = 13.1 Hz, 1H), 3.18 (t, J = 13.1 Hz, 1H), 2.94 (m, 1H), 2.63 (m, 1H), 2.06 (s, 3H), 1.90 (t, J = 14.4 Hz, 2H), 1.80-1.51 (m, 5H).

**Example 22 Synthesis of compound A-100**

Synthetic route:

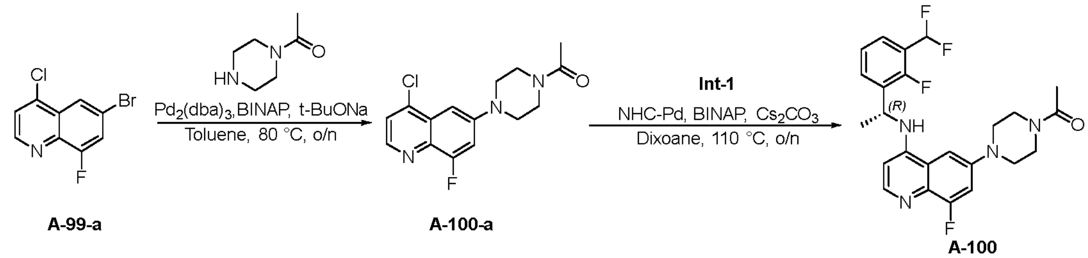


A-99-a A-100-a A-100

Step one:

To a dry flask, compound **A-99-a** (260 mg, 1.0 mmol), compound acetylpiperazine (128 mg, 1.0 mmol), toluene (10 mL), sodium t-butoxide (192 mg, 2.0 mmol), BINAP (124 mg, 0.2 mmol), and $Pd_2(dba)_3$ (183 mg, 0.2 mmol) were successively added. Stirring was performed overnight at 80°C under nitrogen protection. After the reaction was completed, the reaction solution was cooled and concentrated under reduced pressure. The residue was purified by a rapid column chromatography to obtain a yellow solid **A-100-a** (200 mg, yield of 65%). LCMS (ESI): m/z = 308.1 $[M+H]^+$

Step two:

To a dry flask, compound **A-100-a** (180 mg, 0.59 mmol), compound **Int-1** (159 mg, 0.70 mmol), 1,4-dioxane (10 mL), cesium carbonate (576 mg, 1.76 mmol), BINAP (36 mg, 0.06 mmol), and NHC-Pd (40 mg, 0.06 mmol) were successively added. Stirring was performed overnight at 110°C under nitrogen protection. After the reaction was completed, the reaction solution was cooled and concentrated under reduced pressure. The residue was purified by the rapid column chromatography to obtain the crude product and then the crude product was purified by prep-HPLC to obtain a white solid **A-100** (123.5 mg, yield of 64%). LCMS (ESI): *m/z* = 461.2 $[M+H]^+$, $^1H$ NMR (400 MHz, DMSO-$d_6$) $\delta$ 8.12 (d, J = 5.2 Hz, 1H), 7.59-7.50 (m, 2H), 7.44-7.35 (m, 2H), 7.31-7.07 (m, 3H), 6.14 (d, J = 5.3 Hz, 1H), 5.08 (p, J = 6.8 Hz, 1H), 3.70-3.61 (m, 4H), 3.38-3.31 (m, 4H), 2.08 (s, 3H), 1.67 (d, *J*= 6.8 Hz, 3H).

**Example 23 Synthesis of compound A-101**

Synthetic route:

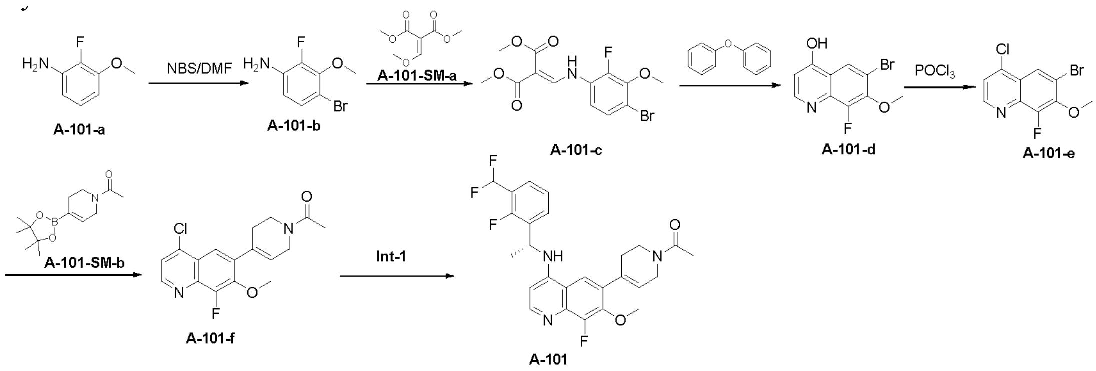


Step one:

To a dry flask, compound **A-101-a** (1.0 g, 7.7 mmol), NBS (1.38 g, 7.8 mmol), and DMF (50 mL) were successively added and reacted at room temperature for three hours. After the reaction was completed, the reaction solution was poured into ice water. The reaction solution was extracted with ethyl acetate and the extract was dried over anhydrous sodium sulfate. The dried product was purified by a glass silica gel column (petroleum ether/ethyl acetate = 1/1) to obtain a white solid compound **A-101-b** (1.42 g, yield of 89%). LCMS (ESI): *m*/*z* = 220 $[M+H]^+$

Step two:

To a dry flask, compound **A-101-b** (1.3 g, 6.0 mmol) and compound **A-101-SM-a** (1.3 g, 7.2 mmol) were added, dissolved in ethanol (30 mL), and reacted overnight at 130°C in a sealed tube. After the reaction was completed, the reaction solution was filtered to obtain a white solid compound **A-101-c** (1.1 g, yield of 52%). LCMS (ESI): *m*/*z* = 362.1 $[M+H]^+$

Step three:

To a dry flask, compound **A-101-c** (300 mg, 0.8 mmol) and diphenyl ether (20 mL) were added, and heated to 260°C with a heating jacket to react for 2 hours. After the reaction was completed, the reaction solution was cooled and purified by the glass silica gel column (petroleum ether/ethyl acetate = 1/1) to obtain a white solid compound **A-101-d** (50 mg, yield of 22%). LCMS (ESI): *m*/*z* = 271.2 $[M+H]^+$

Step four:

To a dry flask, compound **A-101-d** (50 mg, 0.18 mmol) and phosphorus oxychloride (12 mL) were added and reacted at 120°C for 3 hours. After the reaction was completed, the reaction solution was cooled, concentrated under reduced pressure, and then diluted with water. The pH was adjusted to 7 with a sodium carbonate aqueous solution. The reaction solution was extracted with ethyl acetate and the extract was dried over anhydrous sodium sulfate. The dried extract was concentrated under reduced pressure to obtain a white solid compound **A-101-e** (50 mg, yield of 93%). LCMS (ESI): *m*/*z* =: *m*/*z* =290.2 [M+H]+

Step five:

To a dry flask, compound **A-101-e** (25 mg, 0.086 mmol), compound **A-101-SM-b** (24 mg, 0.095 mmol), $Pd(dppf)Cl_2$ (7 mg, 0.01 mmol), sodium carbonate (27 mg, 0.25 mmol), 1,4-dioxane (5 mL), and water (1 mL) were successively added, and reacted at 80°C for 5 hours in a sealed tube under nitrogen protection. After the reaction was completed, the reaction solution was cooled and filtered. The filter residue was beat with petroleum ether to obtain a white solid compound **A-101-f** (20 mg, yield of 69%). LCMS (ESI): *m*/*z* = 335.3 $[M+H]^+$

Step six:

To a dry flask, compound **A-101-f** (20 mg, 0.06 mmol), compound **Int-1** (16 mg, 007 mmol), NHC-Pd (2 mg, 0.007 mmol), BINAP (2 mg, 0.006 mmol), cesium carbonate (58 mg, 0.18 mmol), and 1,4-dioxane (5 mL) were successively added, and reacted at 110°C for 5 hours in a sealed tube under nitrogen protection. After the reaction was completed, the reaction solution was cooled and concentrated under reduced pressure. The concentrated product was

purified by a reverse-phase column to obtain a white solid compound **A-101** (6.5 mg, yield of 25%). LCMS (ESI): *m/z* = 488.1 $[M+H]^+$. $^1H$ NMR (400 MHz, $CD_3OD$): δ 8.20 (d, J = 5.6 Hz, 1H), 8.01 (d, J = 1.6 Hz, 1H), 7.48-7.54 (m, 2H), 7.219 (t, J = 7.6 Hz, 1H), 7.032 (t, J = 54.8 Hz, 1H), 6.24-6.23 (t, J = 5.6 Hz, 1H), 6.03-6.02 (m, 1H), 5.17-5.11 (m, 1H), 4.27-4.24 (m, 2H), 4.03-4.02 (m, 3H), 3.83 (t, J = 5.6 Hz, 1H), 3.77 (t, J = 5.6 Hz, 1H), 2.70-2.61 (m, 2H), 2.20-2.17 (m, 3H), 1.72-1.70 (m, 3H).

**Example 24 Synthesis of compound A-102**

Synthetic route:

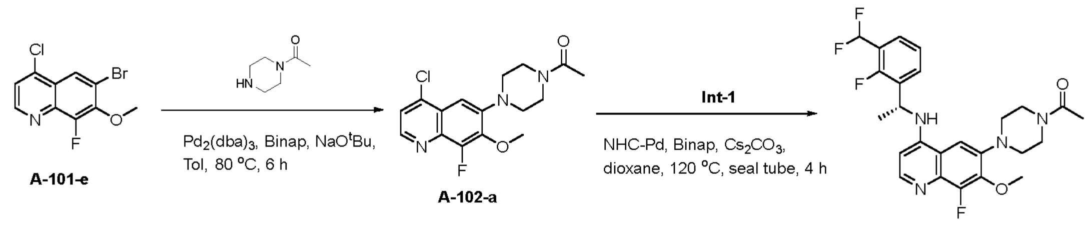


A-101-e A-102-a A-102

Step one:

To a dry sealed tube, compound **A-101-e** (50 mg, 0.18 mmol), compound acetylpiperazine (24 mg, 0.18 mmol), $Pd_2(dba)_3$ (15 mg, 0.018 mmol), BINAP (12 mg, 0.018 mmol), sodium t-butoxide (35 mg, 0.36 mmol), and anhydrous toluene (10 mL) were successively added, and reacted at 80°C for 6 hours under nitrogen protection. After the reaction was completed, the reaction solution was cooled and filtered. Then the toluene was removed by concentration under reduced pressure. The resulting residue was poured into a saturated salt solution and extracted three times with ethyl acetate. The organic phase was collected and concentrated under reduced pressure to obtain a yellow oily compound **A-102-a** (40 mg, yield of 66%).

Step two:

To the dry sealed tube, compound **A-102-a** (30 mg, 0.09 mmol), compound **Int-1** (25 mg, 0.11 mmol), NHC-Pd (12 mg, 0.018 mmol), BINAP (12 mg, 0.018 mmol), cesium carbonate (120 mg, 0.36 mmol), and dioxane (20 mL) were added and uniformly mixed. The reaction solution was gradually heated to 120°C and reacted for 4 hours under nitrogen protection. After the reaction was completed, the reaction solution was cooled and filtered. The resulting filtrate was poured into a saturated salt solution and extracted three times with ethyl acetate, the organic phase was collected and concentrated under reduced pressure, and the residue was subjected to a reverse-phase preparative purification to obtain a white solid compound **A-102** (6.1 mg, yield of 14%). LCMS (ESI): *m/z* = 491.1 $[M+H]^+$; $^1H$ NMR (400 MHz, $CD_3OD$) δ 8.13 (d, J = 5.5 Hz, 1H), 7.51 (dd, J = 16.3, 7.2 Hz, 3H), 7.13 (dt, J = 109.6, 31.3 Hz, 2H), 6.21 (d, J = 5.6 Hz, 1H), 5.15 (q, J = 6.8 Hz, 1H), 4.07 (s, 3H), 3.83 (t, J = 5.0 Hz, 2H), 3.80-3.72 (m, 2H), 3.29-3.26(m, 2H), 3.23 (dd, J = 9.7, 4.6 Hz, 2H), 2.18 (s, 3H), 1.74 (d, J = 6.8 Hz, 3H).

**Example 25 Synthesis of compound B-1**

Synthetic route:

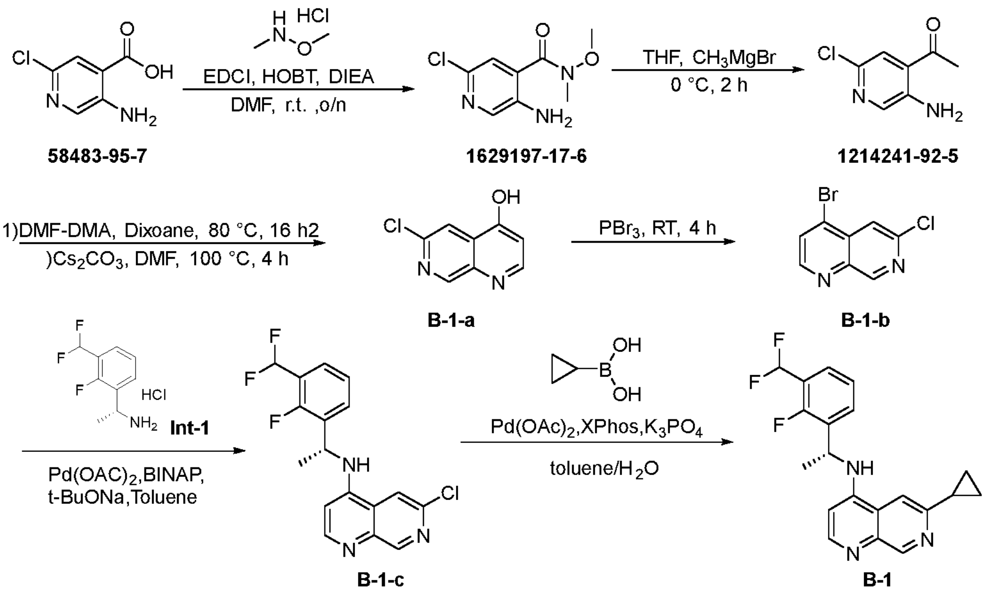


Step one:

To a dry flask, compound **58483-95-7** (30.0 g, 174 mmol), DMF (360 mL), s2 (25.5 g, 261 mmol), EDCI (49.9 g, 261 mmol), HOBT (35.2 g, 261 mmol), and DIEA (67.0 g, 522 mmol) were successively added. The mixture was stirred overnight at room temperature under nitrogen protection. After the reaction was completed, the reaction solution was poured into water and extracted with ethyl acetate. The organic phase was washed successively with an ammonium chloride solution and a saturated salt solution, dried over anhydrous sodium sulfate, filtered, and subjected to rotary evaporation. The residue was beat with a small amount of ethyl acetate to obtain a white solid **1629197-17-6** (19.0 g, yield of 51%). LCMS (ESI): *m/z* =216.0 $[M+H]^+$

Step two:

To a dry three-neck flask, compound **1629197-17-6** (19.0 g, 88 mmol) and tetrahydrofuran (160 mL) were successively added, and methyl magnesium bromide (117 mL, 353 mmol) was slowly dropwise added at 0°C. The reaction was performed at 0°C while stirring for 2 hours. After the reaction was completed, the reaction solution was poured into an icy ammonium chloride solution to be quenched and then extracted with ethyl acetate. The organic phase was dried over anhydrous sodium sulfate, filtered, and subjected to rotary evaporation. The residue was beat with ethyl acetate (10 mL) to obtain a yellow solid **1214241-92-5** (11.0 g, yield of 73%). LCMS (ESI): *m/z* = 171.0 $[M+H]^+$

Step three:

To a dry flask, compound **1214241-92-5** (11.0 g, 64.7 mmol), 1,4-dioxane (80 mL), and DMF-DMA (9.25 g, 77.7 mmol) were successively added. The reaction solution reacted at 80°C for 16 hours under nitrogen protection. After the reaction was completed, the reaction solution was concentrated under reduced pressure. The residue was dissolved in DMF (60 mL), and then cesium carbonate (42.2 g, 129 mmol) was added and stirred at 100°C for 4 hours. After the reaction was completed, the reaction solution was filtered and the filtrate was concentrated under reduced pressure to obtain a brown solid crude product **B-1-a** (11.7 g). LCMS (ESI): m/z =181.0 $[M+H]^+$.

Step four:

Compound **B-1-a** (500 mg, 0.49 mmol) was dissolved in 15 mL of DMF, and phosphorus tribromide (973 mg, 3.59 mmol) was dropwise added to the reaction system at 0°C and reacted at room temperature for 4 hours. After the reaction was completed, the reaction solution was poured into ice water to be quenched, then a saturated sodium bicarbonate solution was used to adjust pH = about 8, and then the reaction solution was extracted with ethyl acetate. Then the organic phase was backwashed with water and a saturated salt solution, dried over sodium sulfate, filtered, and concentrated under reduced pressure to obtain a crude product brown solid compound **B-1-b** (450 mg, yield of 66.7%). LCMS (ESI): *m/z* = 242.9 $[M+H]^+$.

Step five:

Compound **B-1-b** (220 mg, 0.90 mmol) was dissolved in 20 mL of toluene, then compound **Int-1** (245 mg, 1.08 mmol), sodium-t-butoxide (173 mg, 1.80 mmol), BINAP (112 mg, 0.18 mmol), and palladium acetate (20 mg, 0.09 mmol) were added and uniformly stirred. The reaction solution was gradually heated to 100°C and reacted overnight under nitrogen protection. After the reaction (incomplete reaction), the reaction solution was cooled to room temperature, poured into an icy saturated ammonium chloride solution to be quenched, and then extracted with ethyl acetate. Then the organic phase was backwashed with a saturated salt solution and dried over sodium sulfate. The organic phase was filtered and concentrated under reduced pressure, and the residue was purified by a column chromatography to obtain a yellow solid compound **B-1-c** (150 mg, yield of 47.3%). LCMS (ESI): m/z = 352.1 $[M+H]^+$.

Step six:

Compound **B-1-c** (70 mg, 0.20 mmol) and compound cyclopropylboronic acid (137 mg, 1.59 mmol) were dissolved in a 7 mL/0.7 mL toluene/water mixed solvent, then potassium phosphate (84.3 mg, 0.40 mmol), XPhos (19 mg, 0.04 mmol), and $Pd(OAc)_2$ (4.45 mg, 0.02 mmol) were successively added, and heated to 110°C and reacted overnight under nitrogen protection. After the reaction was completed, the reaction solution was cooled and poured into an icy saturated ammonium chloride solution to be quenched and then extracted with ethyl acetate. Then the organic phase was backwashed with a saturated salt solution, dried over sodium sulfate, filtered, and concentrated under reduced pressure. The residue was firstly purified by a thin-layer chromatography and then subjected to a preparative purification to obtain compound **B-1** (11.3 mg, white solid) with the yield of 15.9%. LCMS (ESI): *m/z* =358.1 $[M+H]^+$; $^1H$ NMR (400 MHz, $CD_3OD$): $\delta$ 8.99 (s, 1H), 8.29 (d, *J* = 5.5 Hz, 1H), 8.12 (s, 1H), 7.46-7.60 (m, 2H), 7.23 (t, *J* = 7.7 Hz, 1H), 7.04 (t, *J* = 54.8 Hz, 1H), 6.34 (d, *J* = 5.6 Hz, 1H), 5.16 (q, *J* = 6.7 Hz, 1H), 2.21-2.37 (m, 1H), 1.75 (d, *J* = 6.8 Hz, 3H), 1.09 (d, *J* = 7.6 Hz, 4H).

**Example 26 Synthesis of compound B-2**

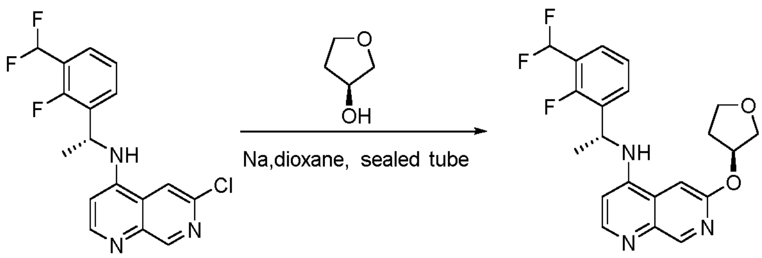


**B-1-c** **B-2**

Sodium metal (19.6 mg, 0.85 mmol) was slowly added to compound (S)-tetrahydrofuran-3-ol (0.5 mL) at room temperature and reacted at room temperature for 2 hours. Compound **B-1-c** (30 mg, 0.09 mmol) was dissolved in dioxane (12 drops), and the reaction mixture of the sodium metal and the (S)-tetrahydrofuran-3-ol was added to the reaction system. The reaction system was gradually heated to 100°C and reacted overnight under nitrogen protection. After the reaction (incomplete reaction), the reaction solution was cooled and poured into an icy saturated ammonium chloride solution to be quenched, and then extracted with ethyl acetate. Then the organic phase was backwashed with a saturated salt solution, dried over sodium sulfate, and concentrated under reduced pressure. The crude product was firstly purified by a preparative TLC. Then the firstly purified crude product was prepared by an acid method (TFA) to obtain a white solid compound **B-2** (4.2 mg, yield of 12.2%). LCMS (ESI): *m/z* = 404.2 $[M+H]^+$; $^1H$ NMR (400 MHz, $CD_3OD$): $\delta$ 8.98 (d, *J* = 0.6 Hz, 1H), 8.37 (d, *J*= 6.9 Hz, 1H), 7.96 (d, *J* = 0.6 Hz, 1H), 7.64-7.55 (m, 2H), 7.31 (t, *J* = 7.7 Hz, 1H), 7.02 (t, *J* = 54.7 Hz, 1H), 6.64 (d, *J* = 7.0 Hz, 1H), 5.70-5.74 (m, 1H), 5.41 (q, *J* = 6.8 Hz, 1H), 3.91-4.10 (m, 4H), 2.31-2.41 (m, 1H), 2.15-2.24 (m, 1H), 1.82 (d, *J* = 6.8 Hz, 3H).

**Example 27 Synthesis of compound B-3**

Synthetic route:

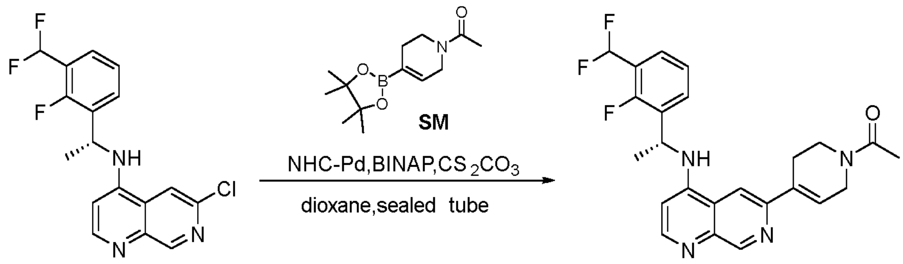


B-1-c B-3

Compound **B-1-c** (30 mg, 0.09 mmol) and compound **SM** (32.1 mg, 0.13 mmol) were dissolved in 2 mL of dioxane, cesium carbonate (55.6 mg, 0.17 mmol), BINAP (5.3 mg, 0.0085 mmol), and NHC-Pd (4.6 mg) were successively added into the reaction system, and then the reaction system was heated to 110°C in a sealed tube and reacted for 4 hours under nitrogen protection. After the reaction was completed, the reaction solution was cooled and poured into an icy saturated ammonium chloride solution to be quenched and then extracted with ethyl acetate. Then the organic phase was backwashed with a saturated salt solution, dried over sodium sulfate, filtered, and concentrated under reduced pressure. The residue was subjected to a preparative purification to obtain compound **B-3** (4.3 mg, white solid) with the yield of 11.4%. LCMS (ESI): $m/z$ = 441.2 $[M+H]^+$; $^1H$ NMR (400 MHz, $CD_3OD$): $\delta$ 9.22 (s, 1H), 8.60 (d, $J$ = 19.4 Hz, 1H), 8.44 (d, $J$ = 7.0 Hz, 1H), 7.63 (dt, $J$ = 23.4, 7.4 Hz, 2H), 7.33 (t, $J$ = 7.8 Hz, 1H), 6.86-7.18 (m, 2H), 6.80 (d, $J$= 7.1 Hz, 1H), 5.47 (q, $J$= 6.8 Hz, 1H), 4.31-4.48 (m, 2H), 3.86 (dt, $J$ = 23.0, 5.8 Hz, 2H), 2.84 (m, 2H), 2.20 (s, 3H), 1.86 (d, $J$ = 6.8 Hz, 3H).

The synthesis of the following compound was the same as that of **B-3**.

| | | | | |
|---|---|---|---|---|
| **B-109** | 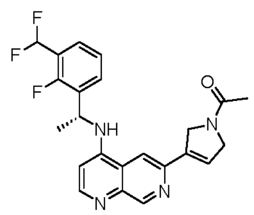 | White solid | 427.0 | $^1H$ NMR (400 MHz, $CD_3OD$); $\delta$ 9.11 (s, 1H), 8.43-8.27 (m, 2H), 7.60-7.48 (m, 2H), 7.24 (t, $J$ = 7.8 Hz, 1H), 7.04 (t, $J$ = 54.8 Hz, 1H), 6.82 (dd, $J$ = 4.7, 2.2 Hz, 1H), 6.41 (d, $J$ = 5.5 Hz, 1H), 5.18 (m, $J$ = 6.8, 3.4 Hz, 1H), 4.92 (d, $J$ = 2.1 Hz, 1H), 4.78 (s, 1H), 4.62 (s, 1H), 4.47 (s, $J$ = 2.7 Hz, 1H), 2.21 (d, $J$ = 29.6 Hz, 3H), 1.77 (dd, $J$ = 6.8, 2.9 Hz, 3H). |

**Example 28 Synthesis of compound B-4**

Synthetic route:

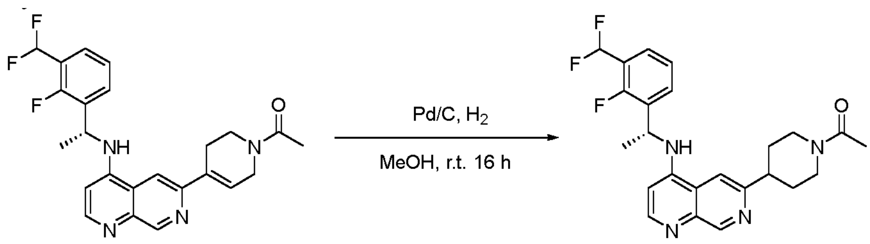


B-3 B-4

Compound **B-3** (12.11 mg, 0.03 mmol) was dissolved in 10 mL of methanol, the air was replaced with a hydrogen balloon, stirring was performed at room temperature for 16 hours, and the reaction was monitored by LCMS. After the reaction was completed, Pd/C was filtered out and the solvent was subjected to rotary evaporation to obtain a white solid **B-4** (6.31 mg) with the yield of 52%. LCMS (ESI): $m/z$ = 443.2 $[M+H]^+$.

The synthesis of the following compound was the same as that of **B-4.**

| | | | | |
|---|---|---|---|---|
| B-110 | 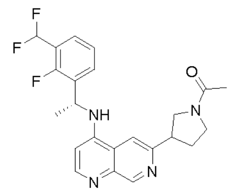 | White solid | 429.3 | $^1$H NMR (400 MHz, $CD_3OD$): δ 9.12 (d, *J* = 1.9 Hz, 1H), 8.36 (dd, *J* = 5.5, 2.4 Hz, 1H), 8.27 (d, *J* = 6.6 Hz, 1H), 7.59-7.46 (m, 2H), 7.23 (t, *J* = 7.7 Hz, 1H), 7.04 (t, *J* = 54.8 Hz, 1H), 6.40 (d, *J* = 5.5 Hz, 1H), 5.17 (q, *J* = 6.9 Hz, 1H), 4.06 (dd, *J* = 11.0, 7.6 Hz, 1H), 3.90-3.63 (m, 4H), 2.55-2.39 (m, 2H), 2.13 (s, 3H), 1.75 (dd, *J* = 6.8, 1.7 Hz, 3H). |

**Example 29 Synthesis of compound B-17**

Synthetic route:

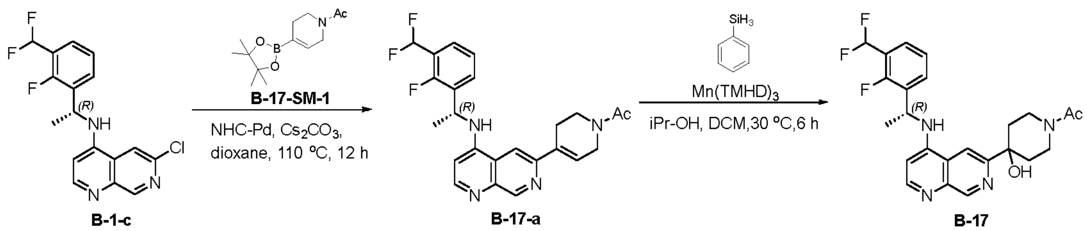


<u>Step one:</u>

Compound **B-1-c** (100 mg, 0.284 mmol), compound **B-17-SM-1** (110 mg, 0.426 mmol), NHC-Pd (19.4 mg, 0.028 mmol), and $Cs_2CO_3$ (185 mg, 0.568 mmol) were dissolved in 6 mL of dioxane and the reaction solution reacted at 110°C for 12 hours in a sealed tube under nitrogen protection. After the reaction was completed, the reaction solution was cooled, poured into an icy ammonium chloride solution to be quenched, and extracted with ethyl acetate. The organic phase was backwashed with a saturated salt solution, dried over sodium sulfate, filtered, and concentrated under reduced pressure. The residue was purified by a column chromatography to obtain compound **B-17-a** (120 mg, yellow oil) with the yield of 96%. LCMS (ESI): *m*/*z* = 441.1 $[M+H]^+$.

<u>Step two:</u>

Compound **B-17-a** (120 mg, 0.273 mmol), phenylsilane (74 mg, 0.682 mmol), and Mn(TMHD)$_3$ (33 mg, 0.055 mmol) were dissolved in 5 mL of IPA and 0.5 mL of a DCM solution and then reacted at 30°C for 6 hours under an oxygen atmosphere. After the reaction was completed, the reaction solution was poured into an ammonium chloride solution to be quenched and extracted with ethyl acetate. The organic phase was backwashed with a saturated salt solution, dried over sodium sulfate, filtered, and concentrated under reduced pressure. The residue was purified by a column chromatography to obtain compound **B-17** (65 mg, yellow solid) with the yield of 52%. LCMS (ESI): *m*/*z* = 459.2 $[M+H]^+$.

**Example 30 Synthesis of compound B-21**

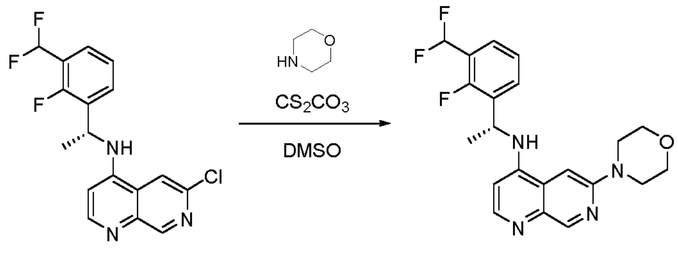


B-1-c B-21

Compound B-1-c (30 mg, 85 mmol), morpholine (0.5 mL), and cesium carbonate (3.0 eq) were added to a sealed tube, uniformly mixed with 5 mL of DMSO, and reacted overnight at 150°C. After the reaction was completed, the solid was filtered out, and the filtrate was subjected to a reverse-phase preparative separation to obtain a target compound B-21 as a yellow solid (7 mg, yield of 18%). LCMS (ESI): m/z = 403.2 $[M+H]^+$; $^1H$ NMR (400 MHz, $CD_3OD$): $\delta$8.93 (s, 1H), 8.20-8.22 (d, $J$= 6.8 Hz, 1H), 7.58-7.61 (m, 3H), 7.31-7.33 (m, 1H), 6.89-7.16 (t, $J$= 54.4 Hz, 1H), 6.56-6.58 (d, $J$= 6.8 Hz, 1H), 5.39-5.41 (m, 1H), 3.85-3.88 (m, 2H), 3.71-3.73 (m, 2H), 1.82-1.84 (d, $J$ = 6.8 Hz, 3H).

**Example 31 Synthesis of compound B-22**

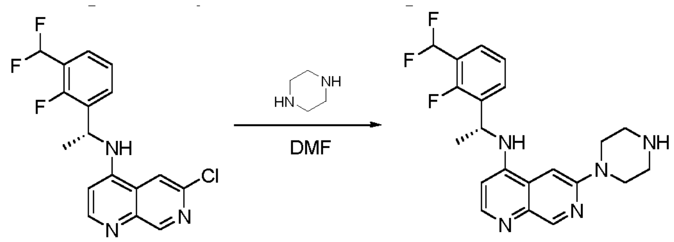


B-1-c B-22

Compound B-1-c (70 mg, 199 mmol) and piperazine (0.5 mL) were added to a sealed tube, uniformly mixed with 5 mL of DMF, and reacted overnight at 150°C. After the reaction was completed, the reaction solution was subjected to a reverse-phase preparative separation to obtain a target compound B-22 as a brown solid (34 mg, yield of 42.5%). LCMS (ESI): m/z = 402.2 $[M+H]^+$; $^1H$ NMR (400 MHz, $CD_3OD$): $\delta$ 8.99 (s, 1H), 8.26-8.28 (d, $J$ = 7.2 Hz, 1H), 7.79 (s, 1H), 7.58-7.65 (m, 2H), 7.30-7.33 (t, $J$ = 7.6 Hz, 1H), 6.88-7.16 (t, $J$ = 54.4 Hz, 1H), 6.61-6.30 (d, $J$ = 7.2 Hz, 1H), 5.41-5.43 (m, 1H), 4.03-4.05 (m, 4H), 3.40-3.43 (m, 4H), 1.83-1.84 (d, $J$ = 6.8 Hz, 3H).

**Example 32 Synthesis of compound B-23**

Synthetic route:

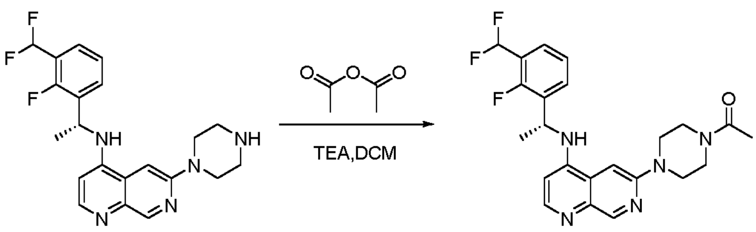


B-22 B-23

Crude product compound **B-22** (80 mg, 0.20 mmol) was dissolved in 6 mL of DCM, and acetic anhydride (20.2 mg, 0.20 mmol) and TEA (60.3 mg, 0.60 mmol) were added at 0°C, followed by reaction at room temperature for 2 hours. After the reaction was completed, the reaction solution was poured into an icy saturated ammonium chloride solution to

be quenched and then extracted with DCM. Then the organic phase was backwashed with a saturated salt solution, dried over sodium sulfate, filtered, and concentrated under reduced pressure. Compound **B-23** (10.3 mg, yellow solid) with the yield of 15.5% was obtained by preparation. LCMS (ESI): *m/z* = 444.2 $[M+H]^+$; $^1H$ NMR (400 MHz, $CD_3OD$): $\delta$ 8.87 (s, 1H), 8.13 (d, *J* = 5.5 Hz, 1H), 7.48-7.61 (m, 2H), 7.45 (d, *J*= 12.5 Hz, 1H), 7.23 (t, *J*= 7.7 Hz, 1H), 7.04 (t, *J* = 54.8 Hz, 1H), 6.24 (d, *J*= 5.5 Hz, 1H), 5.20-5.11 (m, 1H), 3.65-3.81 (m, 8H), 2.19 (s, 3H), 1.75 (d, *J* = 6.8 Hz, 3H).

**Example 33 Synthesis of compound B-24**

Synthetic route:

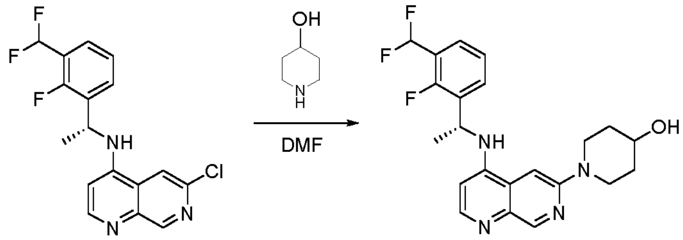


B-1-c B-24

Compound **B-1-c** (100 mg, 0.28 mmol) and 4-hydroxypiperidine (574 mg, 5.68 mmol) were dissolved in 0.4 mL of DMF and then heated to 120°C in a sealed tube and reacted overnight under nitrogen protection. After the reaction (incomplete reaction), the reaction solution was cooled and poured into an icy saturated ammonium chloride solution to be quenched and extracted with ethyl acetate. Then the organic phase was backwashed with a saturated salt solution, dried over sodium sulfate, filtered, and concentrated under reduced pressure. The residue was subjected to a preparative purification to obtain compound **B-24** (3.4 mg, yellow solid) with the yield of 2.9%. LCMS (ESI): *m/z* = 417.2 $[M+H]^+$; $^1H$ NMR (400 MHz, $CD_3OD$) : $\delta$ 8.89 (s, 1H), 8.17 (d, *J* = 6.8 Hz, 1H), 7.55-7.63 (m, 3H), 7.31 (t, *J* = 7.8 Hz, 1H), 7.02 (t, *J* = 54.8 Hz, 1H), 6.53 (d, *J* = 7.0 Hz, 1H), 5.31-5.44 (m, 1H), 4.30 (d, *J* = 12.8 Hz, 2H), 3.94 (dd, *J*= 10.7, 6.8 Hz, 1H), 3.40 (d, *J*= 12.7 Hz, 2H), 2.03-1.95 (m, 2H), 1.83 (d, *J*= 6.8 Hz, 3H), 1.53-1.62 (m, 2H).

**Example 34 Synthesis of compound B-25**

Synthetic route:

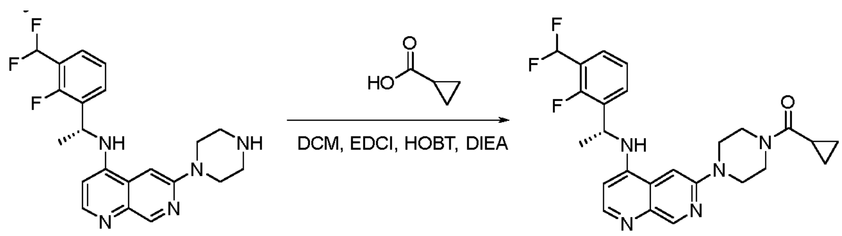


B-22 B-25

To a dry flask, compound **B-22** (30 mg, 0.08 mmol), dichloromethane (5 mL), cyclopropanecarboxylic acid (7.0 mg, 0.08 mmol), EDCI (21 mg, 0.11 mmol), HOBT (15 mg, 0.11 mmol), and DIEA (29 mg, 0.23 mmol) were successively added. The mixture was stirred overnight at room temperature under nitrogen protection. After the reaction was completed, the reaction solution was diluted with water and extracted with dichloromethane. The resulting organic phase was dried over sodium sulfate, filtered, and concentrated under reduced pressure. The residue was purified by Prep-HPLC to obtain a white solid compound **B-25** (9.9 mg, yield of 26%). LCMS (ESI): *m/z* = 470.2 $[M+H]^+$; $^1H$ NMR (400 MHz, $CD_3OD$) $\delta$ 8.87 (s, 1H), 8.12 (d, *J* = 5.6 Hz, 1H), 7.52 (q, *J* = 7.2 Hz, 2H), 7.42 (s, 1H), 7.20 (t, *J* = 8.0 Hz, 1H), 7.04 (t, *J* = 54.8 Hz, 1H), 6.21 (d, *J* = 5.6 Hz, 1H), 5.14 (q, J = 6.8 Hz, 1H), 3.66-3.98 (m, 8H), 2.03-2.07 (m, 1H), 1.74 (d, *J* = 6.8 Hz, 3H), 0.85-0.94 (m, 4H).

**Example 35 Synthesis of compound B-26**

Synthetic route:

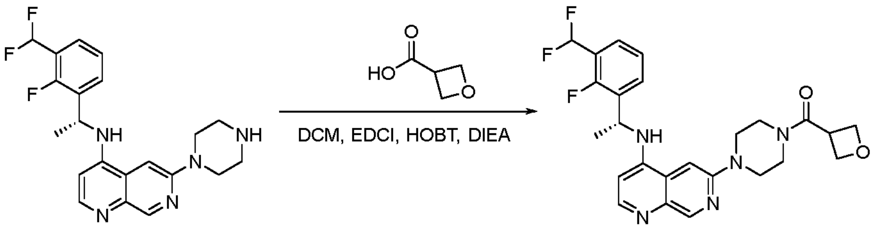


B-22 B-26

To a dry flask, compound **B-22** (30 mg, 0.08 mmol), dichloromethane (5 mL), oxetan-3-formic acid (7.6 mg, 0.08 mmol), EDCI (21 mg, 0.11 mmol), HOBT (15 mg, 0.11 mmol), and DIEA (29 mg, 0.23 mmol) were successively added. The mixture was stirred overnight at room temperature under nitrogen protection. After the reaction was completed, the reaction solution was diluted with water and extracted with dichloromethane. The resulting organic phase was dried over sodium sulfate, filtered, and concentrated under reduced pressure. The residue was purified by Prep-HPLC to obtain a white solid compound **B-26** (10.9 mg, yield of 30%). LCMS (ESI): *m/z* = 486.3 $[M+H]^+$; $^1H$ NMR (400 MHz, $CD_3OD$) $\delta$ 8.86 (s, 1H), 8.12 (d, *J* = 5.6 Hz, 1H), 7.52 (q, *J* = 7.2 Hz, 2H), 7.42 (s, 1H), 7.22 (t, *J* = 8.0 Hz, 1H), 7.04 (t, *J* = 54.8 Hz, 1H), 6.21 (d, *J*= 5.6 Hz, 1H), 5.14 (q, *J*= 6.8 Hz, 1H), 4.86-4.96 (m, 4H), 4.25-4.35 (m, 1H), 3.81-3.85 (m, 2H), 3.63-3.73 (m, 4H), 3.44-3.50 (m, 2H), 1.74 (d, *J*= 6.8 Hz, 3H).

**Example 36 Synthesis of compound B-27**

Synthetic route:

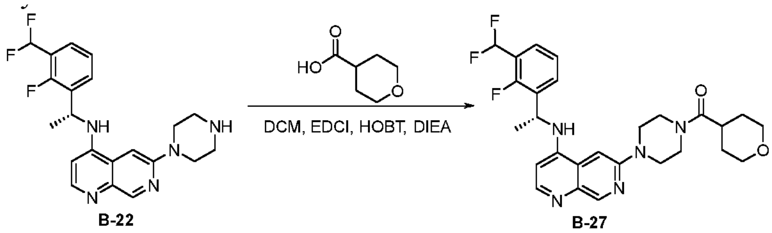


B-22 B-27

To a dry flask, compound **B-22** (30 mg, 0.08 mmol), dichloromethane (5 mL), tetrahydro-2H-pyran-4-formic acid (8.0 mg, 0.08 mmol), EDCI (21 mg, 0.11 mmol), HOBT (15 mg, 0.11 mmol), and DIEA (29 mg, 0.23 mmol) were successively added. The mixture was stirred overnight at room temperature under nitrogen protection. After the reaction was completed, the reaction solution was diluted with water and extracted with dichloromethane. The resulting organic phase was dried over sodium sulfate, filtered, and concentrated under reduced pressure. The residue was purified by Prep-HPLC to obtain a white solid **B-27** (8.9 mg, yield of 20%). LCMS (ESI): *m/z* = 514.3 $[M+H]^+$; $^1H$ NMR (400 MHz, $CD_3OD$) $\delta$ 8.87 (s, 1H), 8.12 (d, *J* = 5.6 Hz, 1H), 7.52 (q, *J* = 7.2 Hz, 2H), 7.42 (s, 1H), 7.20 (t, *J* = 8.0 Hz, 1H), 7.04 (t, *J* = 54.8 Hz, 1H), 6.21 (d, *J* = 5.6 Hz, 1H), 5.14 (q, *J* = 6.8 Hz, 1H), 3.48-3.80 (m, 12H), 3.05-3.13 (m, 1H), 1.66-1.85 (m, 7H).

**Example 37 Synthesis of compound B-30**

Synthetic route:

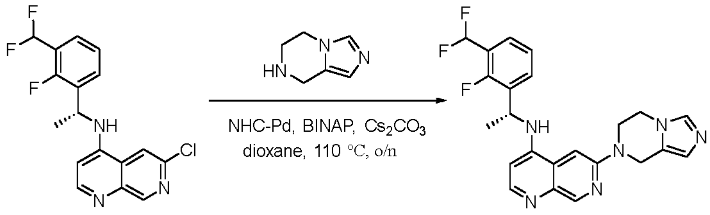


B-1-c B-30

Compound **B-1-c** (50 mg, 0.14 mmol), 5H,6H,7H,8H-imidazo[1,5-a]pyrazine (87 mg, 0.71 mmol), and cesium carbonate (139 mg, 0.43 mmol) were added to a sealed tube containing 1,4-dioxane (10 mL), and then NHC-Pd (10 mg, 0.01 mmol) and BINAP (10 mg, 0.01 mmol) were added, uniformly mixed, and reacted overnight at 110°C under inert gas nitrogen protection. After the reaction was completed, the reaction solution was cooled and extracted with ethyl acetate, the upper layer organic phase was collected, dried, and concentrated under reduced pressure, and the residue was purified by a column chromatography to obtain a white solid compound **B-30** (15.7 mg, yield of 25%). LCMS (ESI): *m*/*z* = 439.1 $[M+H]^+$; $^1H$ NMR (400 MHz, $CDCl_3$) $\delta$ 9.11 (s, 1H), 8.32 (d, J = 5.1 Hz, 1H), 7.53 (m, 2H), 7.44 (t, *J*= 7.3 Hz, 1H), 7.18 (t, *J* = 7.7 Hz, 1H), 6.96 (m, 1H), 6.67 (s, 1H), 6.19 (d, *J*= 5.2 Hz, 2H), 5.98-5.15 (m, 2H), 4.82 (s, 2H), 4.34-4.45 (m, 2H), 4.25-4.34 (m, 2H), 1.77 (d, *J*= 6.5 Hz, 3H).

**Example 38 Synthesis of compound B-31**

Synthetic route:

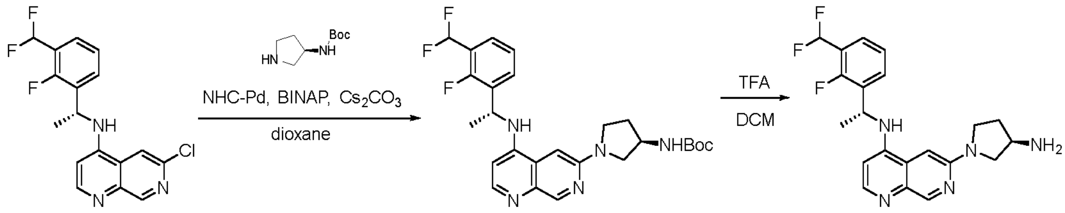


B-1-c B-31-a B-31

Step one:

To a dry sealed tube, compound **B-1-c** (100 mg, 0.28 mmol), tert-butylpyrrolidin-3-yl carbamate (265 mg, 1.42 mmol), BINAP (19 mg, 0.03 mmol), cesium carbonate (279 mg, 0.96 mmol), and 1,4-dioxane (20 mL) were successively added, and after nitrogen replacement for several times, NHC-Pd (14 mg, 0.02 mmol) was added. The reaction was performed at 110°C for 12 hours under nitrogen protection. After the reaction was completed, the reaction solution was cooled and concentrated under reduced pressure. The concentrated product was purified by a glass silica gel column to obtain a yellow oily compound **B-31-a** (76 mg, yield of 53%).

Step two:

Compound **B-31-a** (20 mg, 0.04 mmol) was dissolved in dichloromethane (6 mL), and trifluoroacetic acid (2 mL) was added and reacted at room temperature for 1 hour under nitrogen protection. After the reaction was completed, the reaction solution was concentrated under reduced pressure and diluted with a saturated sodium bicarbonate solution and dichloromethane. The organic phase was dried and concentrated under reduced pressure, and then the firstly purified crude product was prepared by an acid method to obtain a yellow solid compound **B-31**(10 mg, yield of 62.5%). LCMS (ESI): m/z =402.2 $[M+H]^+$; $^1H$ NMR (400 MHz, $CD_3OD$) : $\delta$ 8.94 (s, 1H), 8.21 (d, *J* = 6.8 Hz, 1H), 7.55-7.66 (m, 2H), 7.39 (s, 1H), 7.32 (t, J = 7.8 Hz, 1H), 7.02 (t, *J* = 54.7 Hz, 1H), 6.58 (d, *J* = 6.9 Hz, 1H), 5.42 (dd, *J* = 6.7 Hz, 1H), 4.12 (m, 1H), 3.98 (dd, J = 12.1, 6.1 Hz, 1H), 3.90-3.78 (m, 2H), 3.65-3.76 (m, 1H), 2.59 (m, 1H), 2.17-2.38 (m, 1H), 1.84 (d, *J* = 6.8 Hz, 3H).

The synthesis of the following compound was the same as that of **B-31**.

| No. | Structure | Character | LCMS (ESI): *m/z* [M+H]+ | $^1$H NMR |
|---|---|---|---|---|
| **B-33** | 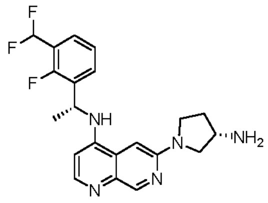 | Yellow solid | 402.2 | $^1$H NMR (400 MHz, $CD_3OD$) $\delta$ 8.79 (s, 1H), 8.03 (d, *J* = 5.3 Hz, 1H), 7.51 (dd, *J* = 16.9, 7.5 Hz, 2H), 7.22 (t, *J* = 7.7 Hz, 1H), 6.88-7.18 (m, 2H), 6.14 (d, *J* = 5.4 Hz, 1H), 5.13 (q, *J* = 6.7 Hz, 1H), 3.78 (ddd, *J* = 26.8, 10.3, 6.1 Hz, 3H), 3.62 (dd, *J* = 15.3, 9.0 Hz, 1H), 3.44-3.35 (m, 1H), 2.33 (dd, *J* = 13.0, 6.7 Hz, 1H), 1.97 (dd, *J* = 12.7, 6.6 Hz, 1H), 1.74 (d, *J* = 6.8 Hz, 3H). |

**Example 39 Synthesis of compound B-32**

Synthetic route:

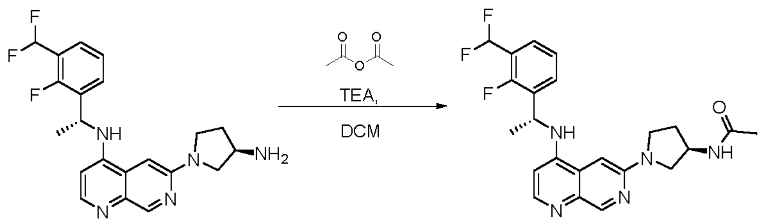


**B-31** **B-32**

Compound **B-31** (50 mg, 0.13 mmol) was dissolved in dichloromethane (10 mL) and triethylamine (0.34 mL, 0.25 mmol) was added. The reaction was performed under an ice bath. Acetic anhydride (0.12 mL, 0.125 mmol) was added. The reaction was performed at room temperature for 2 hours under nitrogen protection. After the reaction was completed, the reaction solution was concentrated under reduced pressure and diluted with a saturated sodium bicarbonate solution and dichloromethane. The organic phase was dried and concentrated under reduced pressure, and then the firstly purified crude product was subjected to a neutral preparation to obtain a white solid compound **B-32**(14 mg, yield of 25.2%). LCMS (ESI): *m/z* = 444.2 [M+H]+; $^1$H NMR (400 MHz, $CD_3OD$): $\delta$ 8.80 (s, 1H), 8.04 (d, *J* = 5.3 Hz, 1H), 7.51 (dd, *J* = 15.9, 7.8 Hz, 2H), 7.22 (t, *J* = 7.7 Hz, 1H), 6.85-67.18 (m, 2H), 6.15 (d, *J* = 5.4 Hz, 1H), 5.13 (m, 1H), 4.42-4.66 (m, 1H), 3.86 (m, 1H), 3.75 (m, 1H), 3.61 -3.70 (m, 1H), 3.47 (dd, *J* = 10.8, 4.3 Hz, 1H), 2.36 (m, 1H), 2.00-2.15 (m, 1H), 1.97 (s, 3H), 1.74 (d, *J* = 6.8 Hz, 3H).

The synthesis of the following compound was the same as that of **B-32**.

| No. | Structure | Character | LCMS (ESI): *m/z* [M+H]+ | $^1$H NMR |
|---|---|---|---|---|
| **B-34** | 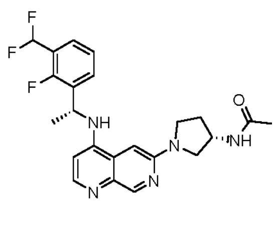 | Yellow solid | 442.2 | $^1$H NMR (400 MHz, $CD_3OD$): $\delta$ 8.80 (s, 1H), 8.04 (d, *J* = 5.3 Hz, 1H), 7.51 (dd, *J* = 15.6, 7.7 Hz, 2H), 7.22 (t, *J* = 7.7 Hz, 1H), 6.84-7.19 (m, 2H), 6.15 (d, *J*= 5.4 Hz, 1H), 5.13 (q, *J* = 6.9 Hz, 1H), 4.51-4.60 (m, 1H), 3.85 (dd, *J*= 10.8, 6.2 Hz, 1H), 3.62-3.77 (m, 2H), 3.49 (dd, *J* = 10.7, 4.3 Hz, 1H), 2.36 (dd, *J* = 13.6, 6.3 Hz, 1H), 2.03-2.14 (m, 1H), 1.97 (s, 3H), 1.74 (d, *J* = 6.8 Hz, 3H). |

**Example 40 Synthesis of compound B-35**

Synthetic route:

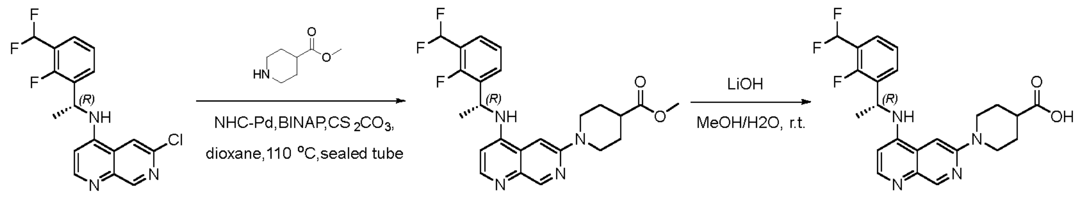


B-1-c B-35-a B-35

Step one:

Compound **B-1-c** (120 mg, 0.14 mmol) and methyl 4-piperidinecarboxylate (244 mg, 0.71 mmol) were dissolved in 12 mL of dioxane, cesium carbonate (333.4 mg, 0.34 mmol), BINAP (21.2 mg, 0.014 mmol), and NHC-Pd (22.5 mg) were successively added into the reaction system, and then the reaction system was heated to 110°C in a sealed tube and reacted overnight under nitrogen protection. After the reaction was completed, the reaction solution was cooled and poured into an icy ammonium chloride solution to be quenched and then extracted with ethyl acetate. Then the organic phase was backwashed with a saturated salt solution, dried over sodium sulfate, filtered, and concentrated under reduced pressure. The residue was purified by a column chromatography to obtain compound **B-35-a** (80 mg, yellow solid) with the yield of 51.1%. LCMS (ESI): *m*/*z* = 459.2 $[M+H]^+$.

Step two:

Compound **B-35-a** (40 mg, 0.087 mmol) and lithium hydroxide (11 mg, 0.26 mmol) were dissolved in 1 mL of methanol and 3 mL of tetrahydrofuran, and reacted at room temperature for 24 hours. After the reaction was completed, the pH of the reaction solution was adjusted to 2-3 with 1 N HCl and then the aqueous phase was concentrated under reduced pressure. The residue was dissolved by acetonitrile, filtered, and subjected to a preparative purification to obtain compound **B-35** (3.7 mg, white solid) with the yield of 9.6%. LCMS (ESI): *m*/*z* = 445.2 $[M+H]^+$; $^1H$ NMR (400 MHz, $CD_3OD$) $\delta$ 8.84 (s, 1H), 8.09 (d, *J* = 5.7 Hz, 1H), 7.53 (dd, *J* = 16.8, 7.9 Hz, 2H), 7.43 (s, 1H), 7.24 (t, *J* = 7.7 Hz, 1H), 7.03 (t, J = 54.8 Hz, 1H), 6.25 (d, *J* = 5.8 Hz, 1H), 5.19 (d, *J* = 6.8 Hz, 1H), 4.43 (d, *J* = 13.1 Hz, 2H), 3.07 (d, *J* = 11.3 Hz, 2H), 2.52-2.59 (m, 1H), 2.05 (d, *J* = 10.1 Hz, 2H), 1.83 (d, *J* = 11.2 Hz, 2H), 1.76 (d, *J* = 6.8 Hz, 3H).

**Example 41 Synthesis of compound B-36**

Synthetic route:

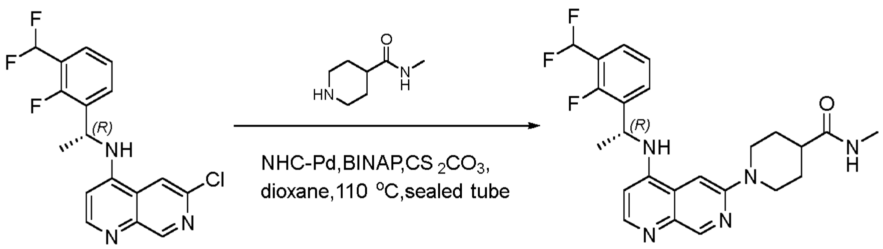


B-1-c B-36

Compound **B-1-c** (50 mg, 0.14 mmol) and N-methyl-4-piperidin-formamide (101 mg, 0.71 mmol) were dissolved in 2 mL of dioxane, cesium carbonate (139 mg, 0.34 mmol), BINAP (8.8 mg, 0.014 mmol), and NHC-Pd (7.5 mg) were successively added into the reaction system, and then the reaction system was heated to 110°C in a sealed tube and reacted overnight under nitrogen protection. After the reaction was completed, the reaction solution was cooled and poured into an icy ammonium chloride solution to be quenched and then extracted with ethyl acetate. Then the organic

phase was backwashed with a saturated salt solution, dried over sodium sulfate, filtered, and concentrated under reduced pressure. The residue was subjected to a preparative purification to obtain compound **B-36** (15.8 mg, white solid) with the yield of 24.3%. LCMS (ESI): *m/z* = 458.2 $[M+H]^+$ ; $^1H$ NMR (400 MHz, $CD_3OD$) : $\delta$ 8.83 (s, 1H), 8.09 (d, *J* = 5.5 Hz, 1H), 7.52 (d, *J* = 7.7 Hz, 2H), 7.41 (s, 1H), 7.23 (t, *J* = 7.7 Hz, 1H), 6.97 (d, *J* = 54.9 Hz, 1H), 6.20 (d, *J* = 5.5 Hz, 1H), 5.15 (d, *J* = 6.6 Hz, 1H), 4.54 (d, *J* = 12.5 Hz, 2H), 2.97 (t, *J* = 11.1 Hz, 2H), 2.74 (s, 3H), 2.45-2.52 (m, 1H), 1.88 (dd, *J* = 22.7, 10.9 Hz, 4H), 1.74 (d, *J* = 6.8 Hz, 3H).

The synthesis of the following compounds was the same as that of **B-36**.

| No. | Structure | Character | LCMS (ESI): *m/z* $[M+H]^+$ | $^1H$ NMR |
|---|---|---|---|---|
| **B-28** | 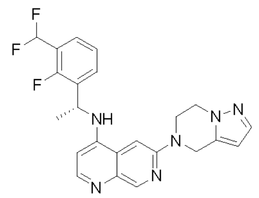 | Yellow solid | 439.1 | $^1H$ NMR (400 MHz, $CDCl_3$) $\delta$ 9.11 (s, 1H), 8.32 (d, *J*= 5.1 Hz, 1H), 7.53 (m, 2H), 7.44 (t, *J* = 7.3 Hz, 1H), 7.18 (t, *J* = 7.7 Hz, 1H), 6.96 (m, 1H), 6.67 (s, 1H), 6.19 (d, *J* = 5.2 Hz, 2H), 5.15-4.98 (m, 2H), 4.82 (s, 2H), 4.45-4.34 (m, 2H), 4.34-4.25 (m, 2H), 1.77 (d, *J* = 6.5 Hz, 3H). |
| **B-37** | 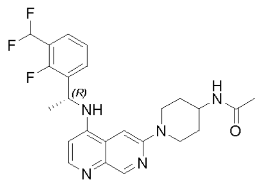 | White solid | 458.5 | $^1H$ NMR (400 MHz, $CD_3OD$) $\delta$ 8.83 (s, 1H), 8.09 (d, *J* = 5.4 Hz, 1H), 7.53-7.49 (m, 2H), 7.40 (s, 1H), 7.22 (t, *J* = 7.7 Hz, 1H), 7.03 (t, *J* = 54.8 Hz, 1H), 6.19 (d, *J* = 5.5 Hz, 1H), 5.14 (q, *J* = 6.8 Hz, 1H), 4.43 (dd, *J* = 13.3, 3.2 Hz, 2H), 3.98-3.90 (m, 1H), 3.08 (t, *J* = 12.6 Hz, 2H), 2.01 (d, *J* = 9.5 Hz, 2H), 1.95 (s, 3H), 1.74 (d, *J* = 6.8 Hz, 3H), 1.56-1.63 (m, 2H). |
| **B-38** | 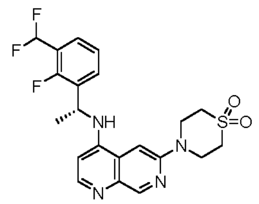 | Light green solid | 451.1 | $^1H$ NMR (400 MHz, DMSO-$d_6$): $\delta$ 8.86 (s, 1H), 8.16-8.17 (d, *J* = 5.2 Hz, 1H), 7.61 (s, 1H), 7.51-7.58 (m, 2H), 7.25-7.39 (m, 3H), 6.13-6.14 (d, *J* = 5.2 Hz, 1H), 5.06-5.09 (m, 1H), 4.22-4.18 (m, 4H), 3.17-3.25 (s, 4H), 1.66-1.68 (d, *J* = 7.2 Hz, 3H). |
| **B-41** | 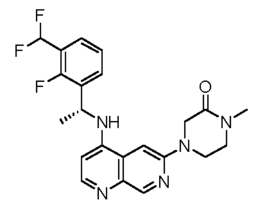 | White solid | 430.1 | $^1H$ NMR (400 MHz, $CD_3OD$) $\delta$ 8.89 (s, 1H), 8.14 (d, *J* = 5.1 Hz, 1H), 7.51-7.57 (m, 2H), 7.40 (s, 1H), 7.22 (t, *J* = 7.7 Hz, 1H), 7.05 (t, *J* = 54.8 Hz, 1H), 6.24 (d, *J* = 5.2 Hz, 1H), 5.15-5.17 (m, 1H), 4.26 (s, 2H), 4.05-4.07 (m, 2H), 3.60-3.63 (m, 2H), 3.08 (s, 3H), 1.76 (d, *J* = 7.2 Hz, 3H). |
| **B-43** | 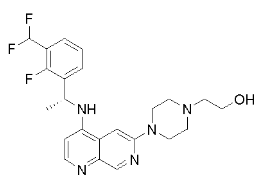 | White solid | 446.1 | $^1H$ NMR (400 MHz, $CD_3OD$): $\delta$ 8.85 (s, 1H), 8.11 (d, *J* = 5.4 Hz, 1H), 7.52 (q, *J* = 7.5 Hz, 2H), 7.40 (s, 1H), 7.22 (t, *J* = 7.7 Hz, 1H), 7.03 (t, *J* = 54.8 Hz, 1H), 6.21 (d, *J* = 5.5 Hz, 1H), 5.14 (q, *J* = 6.6 Hz, 1H), 3.78 (t, *J* = 5.9 Hz, 2H), 3.75-3.67 (m, 4H), 2.83-2.75 (m, 4H), 2.67 (t, *J* = 5.9 Hz, 2H), 1.74 (d, *J* = 6.8 Hz, 3H). |

**Example 42 Synthesis of compound B-44**

Synthetic route:

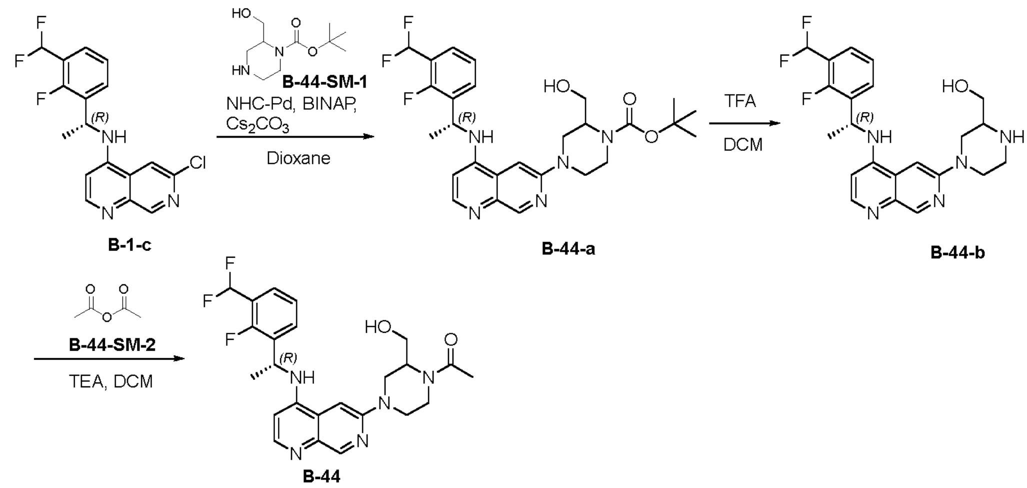


Step one:

Compound **B-1-c** (80 mg, 0.227 mmol), compound **B-44-SM-1** (245 mg, 1.136 mmol), NHC-Pd (15 mg, 0.023 mmol), BINAP (14 mg, 0.023 mmol), and $Cs_2CO_3$ (222 mg, 0.68 mmol) were dissolved in 5 mL of dioxane and reacted while stirring at 110°C for 3 hours in a sealed tube under nitrogen protection. After the reaction was completed, the reaction solution was cooled and poured into an ammonium chloride solution to be quenched and then extracted with ethyl acetate. Then the organic phase was backwashed with a saturated salt solution, dried over sodium sulfate, filtered, and concentrated under reduced pressure. The residue was purified by a column chromatography to obtain compound **B-44-a** (90 mg, yellow oil) with the yield of 74.4%. LCMS (ESI): *m*/*z* = 531.1 $[M+H]^+$.

Step two:

Compound **B-44-a** (90 mg, 0.17 mmol) was dissolved in 3 mL of DCM, and 3 mL of a TFA solution was dropwise added under ice bath and then reacted at room temperature for 2 hours. After the reaction was completed, the reaction solution was poured into a sodium bicarbonate solution to be quenched and then extracted with dichloromethane. The organic phase was backwashed with water and a saturated salt solution, dried over sodium sulfate, filtered, and concentrated under reduced pressure to obtain compound **B-44-b** (70 mg, yellow oil, crude product) with the yield of 100%. LCMS (ESI): *m*/*z* =432.0 $[M+H]^+$.

Step three:

Compound **B-44-b** (60 mg, 0.0557 mmol), compound **B-44-SM-2** (6 mg, 0.0557 mmol), and triethylamine (11 mg, 0.111mmol) were dissolved in 5 mL of dichloromethane and reacted while stirring for 2 hours at room temperature. After the reaction was completed, the reaction solution was poured into an ammonium chloride solution and then extracted with dichloromethane. Then the organic phase was backwashed with a saturated salt solution, dried over sodium sulfate, filtered, and concentrated under reduced pressure. The residue was subjected to a preparative purification to obtain compound **B-44** (3.7 mg, yellow solid) with the yield of 14%. LCMS (ESI): *m*/*z* = 474.1 $[M+H]^+$; $^1H$ NMR (400 MHz, $CD_3OD$): $\delta$ 8.85 (s, 1H), 8.11 (d, *J* = 5.1 Hz, 1H), 7.55-7.48 (m, 2H), 7.43 (s, 1H), 7.22 (t, *J* = 7.6 Hz, 1H), 7.04 (t, *J* = 54.8 Hz, 1H), 6.20 (d, J = 5.4 Hz, 1H), 5.14 (d, *J*= 7.0 Hz, 1H), 4.76 (s, 1H), 4.63-4.31 (m, 3H), 4.21 (s, 1H), 3.99-3.73 (m, 2H), 3.20-2.88 (m, 2H), 2.22 (dd, *J* = 17.8, 2.6 Hz, 3H), 1.74 (dd, *J* = 6.9, 2.4 Hz, 3H).

The synthesis of the following compound was the same as that of **B-44**.

| No. | Structure | Character | LCMS (ESI): *m*/*z* [M+H]+ | 1H NMR |
|---|---|---|---|---|
| **B-45** | 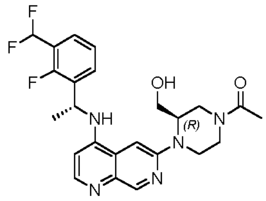 | Yellow solid | 474.1 | 1H NMR (400 MHz, $CD_3OD$) $\delta$ 8.86 (d, *J* = 4.4 Hz, 1H), 8.10 (dd, *J* = 5.3, 3.3 Hz, 1H), 7.57-7.47 (m, 2H), 7.33 (d, *J* = 5.4 Hz, 1H), 7.22 (t, *J* = 7.7 Hz, 1H), 7.04 (t, *J* = 54.8 Hz, 1H), 6.20 (dd, *J* = 5.4, 2.8 Hz, 1H), 5.14 (q, *J* = 6.9 Hz, 1H), 4.75-4.61 (m, 1H), 4.50 (d, *J* = 13.6 Hz, 1H), 4.28 (d, *J* = 13.6 Hz, 1H), 4.03 (dd, *J* = 29.0, 12.4 Hz, 1H), 3.70-3.58 (m, 2H), 3.49 (ddd, *J* = 32.3, 22.1, 6.7 Hz, 2H), 3.15 (ddd, *J* = 28.4, 16.5, 7.6 Hz, 1H), 2.21 (d, *J* = 6.7 Hz, 3H), 1.74 (d, *J* = 6.8 Hz, 3H). |
| **B-47** | 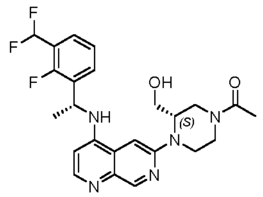 | | 474.1 | 1H NMR (400 MHz, $CD_3OD$) $\delta$ 8.85 (d, *J* = 4.6 Hz, 1H), 8.12-8.05 (m, 1H), 7.56-7.47 (m, 2H), 7.34 (d, *J* = 5.4 Hz, 1H), 7.22 (t, *J* = 7.7 Hz, 1H), 7.04 (t, *J* = 54.8 Hz, 1H), 6.19 (d, *J* = 5.4 Hz, 1H), 5.14 (q, *J* = 6.7 Hz, 1H), 4.82 (s, 1H), 4.75-4.61 (m, 1H), 4.50 (d, *J* = 13.4 Hz, 1H), 4.29 (d, *J* = 13.4 Hz, 1H), 4.03 (dd, *J* = 25.4, 13.3 Hz, 1H), 3.70-3.58 (m, 2H), 3.57-3.46 (m, 1H), 3.22-3.05 (m, 1H), 2.21 (d, *J* = 7.4 Hz, 3H), 1.74 (d, *J* = 6.8 Hz, 3H). |

**Example 43 Synthesis of compound B-46**

Synthetic route:

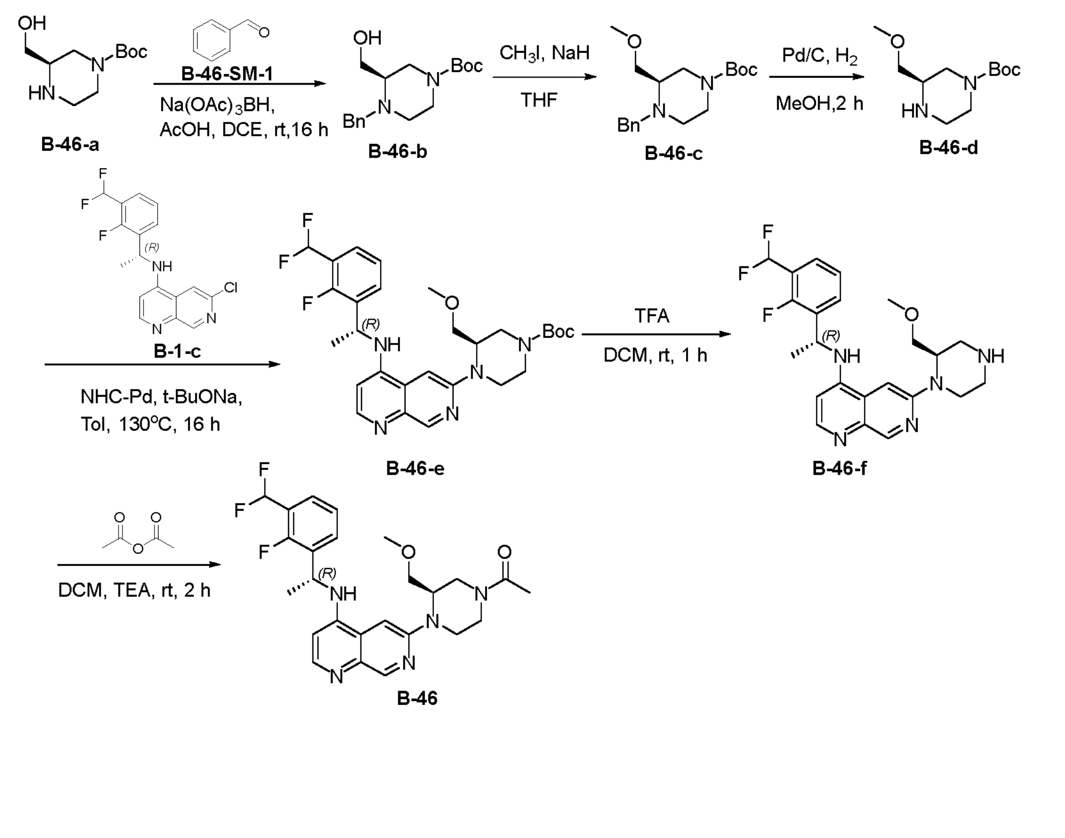

Step one:

Compound **B-46-a** (2.0 g, 9.3 mmol), compound **B-46-SM-1** (1.18 g, 11.1 mmol), and AcOH (556 mg, 9.3 mmol) were dissolved in an eggplant-shaped flask containing DCE (30 mL), and sodium borohydride acetate (2.55 g, 12.0 mmol) was added at 0°C and then reacted at room temperature overnight. After the reaction was completed, the reaction solution was extracted with ethyl acetate. The organic phase was dried and concentrated to obtain a yellow oily compound **B-46-b** (1.6 g, yield of 57%). LCMS (ESI): *m*/*z* = 307.2 $[M+H]^+$.

Step two:

Compound **B-46-b** (600 mg, 1.97 mmol) was dissolved in an eggplant-shaped flask containing tetrahydrofuran (5 mL) and cooled to 0°C, NaH (158 mg, 3.95 mmol) was added at 0°C and stirred for 30 minutes, and methyl iodide (364 mg, 2.56 mmol) was added thereto and reacted at room temperature for 3 hours. After the reaction was completed, the reaction solution was quenched with an ammonium chloride aqueous solution and extracted, and the organic phase was concentrated under reduced pressure to obtain a yellow oily compound **B-46-c** (550 mg, yield of 87%). LCMS (ESI): *m*/*z* =3 21.2 $[M+H]^+$.

Step three:

Compound **B-46-c** (550 mg, 1.7 mmol) and Pd/C (550 mg) were dissolved in an eggplant-shaped flask containing methanol (10 mL) and reacted at room temperature for 2 hours under a hydrogen condition. After the reaction was completed, the reaction solution was filtered and the filtrate was concentrated under reduced pressure. The residue was purified by a silica gel column to obtain a yellow solid compound **B-46-d** (260 mg, yield of 76%). LCMS (ESI): *m*/*z* =231.2 $[M+H]^+$.

Step four:

Compound **B-46-d** (260 mg, 1.1 mmol), compound **B-1-c** (135 mg, 0.38 mmol), NHC-Pd (26 mg, 0.04 mmol), and sodium-t-butoxide (111 mg, 1.1 mmol) were dissolved in an eggplant-shaped flask containing toluene (5 mL), and stirred overnight at 130°C. After the reaction was completed, the reaction solution was cooled and concentrated under reduced pressure. The residue was purified by the silica gel column to obtain a yellow oily compound **B-46-e** (25 mg, yield of 4%). LCMS (ESI): *m*/*z* = 546.3 $[M+H]^+$.

Step five:

Compound **B-46-e** (25 mg, 0.05 mmol) was dissolved in an eggplant-shaped flask containing DCM (2 mL), and trifluoroacetic acid (0.5 mL) was added thereto and stirred at room temperature for 1 hour. After the reaction was completed, the reaction solution was concentrated under reduced pressure to obtain a yellow oily compound **B-46-f** (20 mg, yield of 98%). LCMS (ESI): *m*/*z* = 446.2 $[M+H]^+$.

Step six:

Compound **B-46-f** (20 mg, 0.04 mmol), acetic anhydride (4.6 mg, 0.04 mmol), and triethylamine (14 mg, 0.12 mmol) were dissolved in an eggplant-shaped flask containing DCM (2 mL) and stirred at room temperature for 2 hours. After the reaction was completed, the reaction solution was extracted with ethyl acetate and concentrated. The residue was purified by prep-HPLC to obtain to a white solid compound **B-46** (4.1 mg, yield of 20%). LCMS (ESI): *m*/*z* = 488.2 $[M+H]^+$; $^1H$ NMR (400 MHz, $CD_3OD$): $\delta$ 8.86 (d, *J* = 5.2 Hz, 1H), 8.10 (dd, *J* = 5.4, 3.0 Hz, 1H), 7.52 (q, *J* = 8.0 Hz, 2H), 7.35 (s, 1H), 7.23 (t, *J* = 7.7 Hz, 1H), 7.03 (t, *J* = 54.8 Hz, 1H), 6.20 (dd, *J* = 5.5, 1.7 Hz, 1H), 5.14 (q, *J* = 6.8 Hz, 1H), 5.03-4.68 (m, 1H), 4.61-4.53 (m, 1H), 4.27-4.18 (m, 1H), 4.11-3.97 (m, 1H), 3.58-3.47 (m, 2H), 3.46-3.32 (m, 4H), 3.25 (d, *J* = 3.7 Hz, 1H), 3.13-3.03 (m, 1H), 2.19 (d, *J* = 12.0 Hz, 3H), 1.74 (d, *J* = 6.8 Hz, 3H).

The synthesis of the following compound was the same as that of **B-46**.

| No | Structure | Character | LCMS (ESI): $m/z$ $[M+H]^+$ | $^1$H NMR |
|---|---|---|---|---|
| **B-48** | 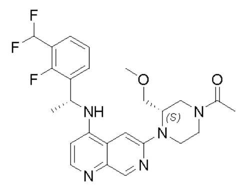 | White solid | 488.2 | $^1$H NMR (400 MHz, $CD_3OD$) $\delta$ 8.86 (d, $J$ = 5.5 Hz, 1H), 8.10 (dd, $J$ = 5.4, 3.7 Hz, 1H), 7.51 (q, $J$ = 7.4 Hz, 2H), 7.35 (d, $J$ = 1.9 Hz, 1H), 7.22 (t, $J$ = 7.7 Hz, 1H), 7.03 (t, $J$ = 54.8 Hz, 1H), 6.19 (dd, $J$ = 5.5, 3.1 Hz, 1H), 5.14 (q, $J$ = 6.7 Hz, 1H), 4.98-4.95 (m, 0.5H), 4.70-4.66 (m, 0.5H), 4.57-4.55 (m, 1H), 4.24-4.21 (m, 1H), 4.08-4.03 (m, 1H), 3.57-3.47 (m, 2H), 3.45-3.38 (m, 1H), 3.37 (s, 3H), 3.28-3.24 (m, 1H), 3.14-3.02 (m, 1H), 2.19 (d, $J$ = 11.2 Hz, 3H), 1.74 (d, $J$ = 6.8 Hz, 3H). |

**Example 44 Synthesis of compound B-49**

Synthetic route:

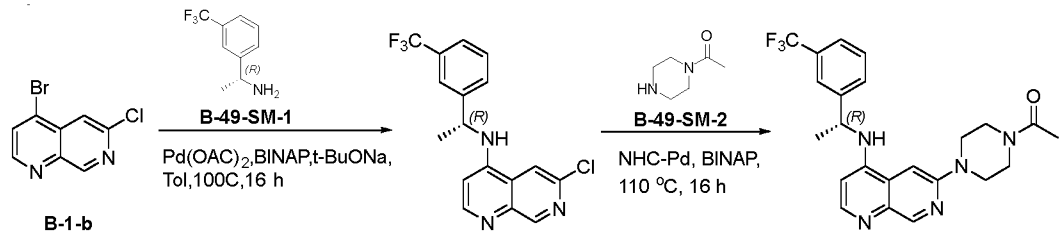

B-1-b B-49-1 B-49

Step one:

Sodium-t-butoxide (55 mg, 0.57 mmol) was added to a toluene (10 mL) solution of compound **B-1-b** (100 mg, 0.28 mmol), compound **B-49-SM-1** (60 mg, 0.31 mmol), $Pd(OAc)_2$ (7.0 mg, 0.028 mmol), and BINAP (35 mg, 0.056 mmol), and uniformly stirred. The reaction solution reacted overnight at 100°C under nitrogen protection. After the reaction was completed, the reaction solution was cooled to room temperature, quenched in a saturated ammonium chloride solution, and extracted with ethyl acetate. The organic phase was dried over anhydrous sodium sulfate. The organic phase was filtered and concentrated under reduced pressure, and the residue was purified by a column chromatography to obtain a yellow solid compound **B-49-1** (80 mg, yield of 55%). LCMS (ESI): $m/z$ = 352.1 $[M+H]^+$.

Step two:

$Cs_2CO_3$ (222 mg, 0.68 mmol) was added to a dioxane solution of compound **B-49-1** (80 mg, 0.23 mmol), **B-49-SM-2** (145 mg, 1.1 mmol), NHC-Pd (15 mg, 0.023 mmol), and BINAP (14 mg, 0.023 mmol), and reacted overnight at 110°C under nitrogen protection. After the reaction was completed, the reaction solution was cooled to room temperature, quenched with a saturated ammonium chloride solution, dried over ethyl acetate, filtered, and concentrated under reduced pressure. The residue was subjected to a column chromatography to obtain the crude product and the crude product was subjected to a reverse-phase preparation to obtain **B-49** (33.7 mg, yield of 34%). LCMS (ESI): $m/z$ = 444.1 $[M+H]^+$; $^1$H NMR (400 MHz, $CD_3OD$): $\delta$ 8.87 (s, 1H), 8.11 (d, $J$ = 5.6 Hz, 1H), 7.72 (s, 1H), 7.68 (d, $J$= 6.8 Hz, 1H), 7.54-7.52 (m, 2H), 7.43 (s, 1H), 6.25 (d, $J$ = 4.2 Hz, 1H), 4.93 (q, $J$ = 6.8 Hz, 1H), 3.80-3.65 (m, 8H), 2.18 (s, 3H), 1.72 (d, $J$ = 6.8 Hz, 3H).

The synthesis of the following compound was the same as that of **B-49.**

| No . | Structure | Character | LCMS (ESI): *m/z* [M+H]+ | 1H NMR |
|---|---|---|---|---|
| **B-50** | 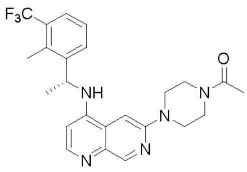 | White solid | 458.0 | 1H NMR (400 MHz, $CD_3OD$): $\delta$ 8.87 (s, 1H), 8.11 (d, *J* = 5.6 Hz, 1H), 7.61 (d, 7 = 8.0 Hz, 1H), 7.57 (d, *J* = 8.0 Hz, 1H), 7.46 (s, 1H), 7.27 (t, *J* = 7.6 Hz, 1H), 6.07 (d, *J* = 5.6 Hz, 1H), 5.13 (q, *J* = 6.8 Hz, 1H), 3.80-3.66 (m, 8H), 2.61 (s, 3H), 2.19 (s, 3H), 1.69 (d, *J* = 6.8 Hz, 3H). |

**Example 45 Synthesis of compound B-51**

Synthetic route:

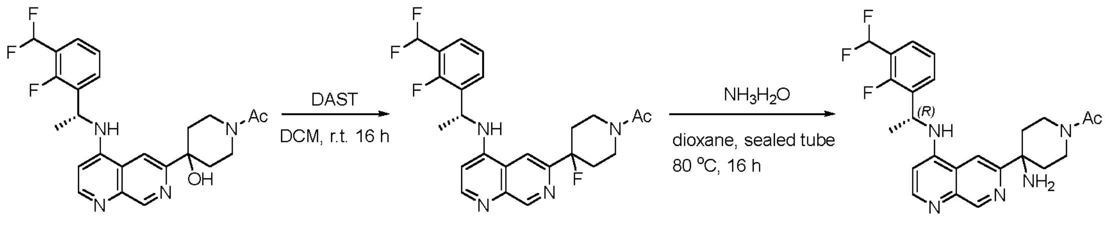


B-17 B-51-a B-51

Step one:

Compound **B-17** (55 mg, 0.12 mmol) and DAST (38.7 mg, 0.24 mmol) were dissolved in 4 mL of dichloromethane. The mixture reacted while stirring overnight at room temperature under nitrogen protection. After the reaction was completed, the reaction solution was poured into an ammonium chloride solution to be quenched and extracted with dichloromethane. The organic phase was backwashed with water and a saturated salt solution, dried over sodium sulfate, filtered, and concentrated under reduced pressure to obtain compound **B-51-a** (70 mg, yellow oil, crude product) with the yield of 100%. LCMS (ESI): *m/z* = 461 [M+H]+.

Step two:

Compound **B-51-a** (70 mg, 0.152 mmol) was dissolved in 2 mL of ammonium hydroxide and 1 mL of a dioxane solution, and then reacted in a sealed tube at 80°C for 16 hours under nitrogen protection. After the reaction was completed, the reaction solution was poured into an ammonium chloride solution to be quenched and extracted with ethyl acetate. The organic phase was backwashed with water and a saturated salt solution, dried over sodium sulfate, filtered, and concentrated under reduced pressure. The residue was subjected to a preparative purification to obtain compound **B-51** (2.3 mg, yellow solid) with the yield of 3.3%. LCMS (ESI): *m/z* = 458.1 [M+H]+; 1H NMR (400 MHz, $CD_3OD$): $\delta$ 9.16 (s, 1H), 8.43-8.33 (m, 2H), 7.54 (dt, *J*= 15.1, 7.1 Hz, 2H), 7.23 (t, *J*= 7.8 Hz, 1H), 7.04 (t, *J*= 54.8 Hz, 1H), 6.40 (d, *J* = 5.5 Hz, 1H), 5.18 (q, *J* = 6.8 Hz, 1H), 4.11 (dt, *J* = 13.5, 4.7 Hz, 1H), 3.75 (d, *J* = 10.8 Hz, 2H), 3.54 (t, *J* = 11.5 Hz, 1H), 2.48-2.36 (m, 2H), 2.16 (s, 3H), 1.96-1.82 (m, 2H), 1.77 (d, *J* = 6.8 Hz, 3H).

**Example 46 Synthesis of compound B-52**

Synthetic route:

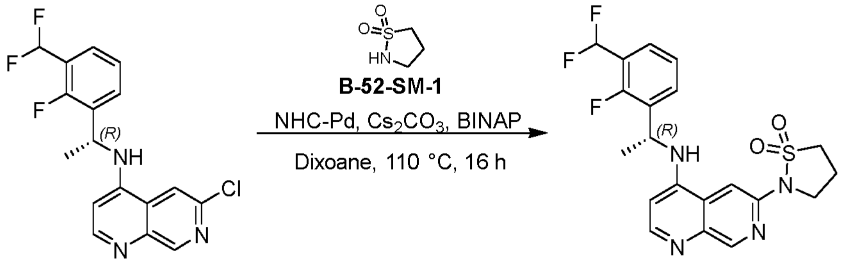


B-1-c B-52

To a dry flask, compound **B-1-c** (48 mg, 0.14 mmol), compound **B-52-SM-1** (33 mg, 0.27 mmol), 1,4-dioxane (5 mL), cesium carbonate (134 mg, 0.41 mmol), BINAP (17 mg, 0.027 mmol), and NHC-Pd (18 mg, 0.027 mmol) were successively added. The reaction was performed while stirring at 110°C for 16 hours under nitrogen protection. After the reaction was completed, the reaction solution was cooled and concentrated under reduced pressure. The residue was firstly purified by a rapid column chromatography and then purified by prep-HPLC to obtain a white solid (20.2 mg, yield of 34%). LCMS (ESI): $m/z$ = 437.0 $[M+H]^+$; $^1H$ NMR (400 MHz, DMSO-$d_6$): $\delta$ 8.96 (s, 1H), 8.32 (d, $J$ = 5.2 Hz, 1H), 7.78 (s, 1H), 7.70-7.48 (m, 3H), 7.43-7.09 (m, 2H), 6.27 (d, $J$ = 5.4 Hz, 1H), 5.11 (m, 1H), 4.07-3.91 (m, 2H), 3.57-3.52 (m, 2H), 2.50-2.44 (m, 2H), 1.68 (d, $J$ = 6.8 Hz, 3H).

The synthesis of the following compound was the same as that of **B-52**.

| No . | Structure | Character | LCMS (ESI): $m/z$ $[M+H]^+$ | $^1H$ NMR |
|---|---|---|---|---|
| **B-54** | 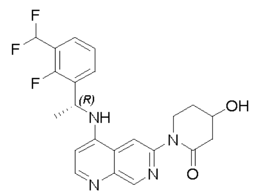 | White solid | 431.0 | $^1H$ NMR (400 MHz, $CD_3OD$): $\delta$ 9.07 (s, 1H), 8.39 (s, 1H), 8.38 (d, $J$ = 5.6 Hz, 1H), 7.57-7.49 (m, 2H), 7.20 (t, $J$ = 7.2 Hz, 1H), 6.89 (t, $J$ = 54.8 Hz, 1H), 6.39 (d, $J$ = 5.6 Hz, 1H), 5.16 (q, $J$ = 6.8 Hz, 1H), 4.33-4.31 (m, 1H), 4.14-4.08 (m, 1H), 3.93-3.87 (m, 1H), 2.91-2.86 (m, 1H), 2.61-2.57 (m, 1H), 2.26-2.22 (m, 1H), 2.08-2.03 (m, 1H), 1.72 (d, $J$ = 6.8 Hz, 3H). |
| **B-55** | 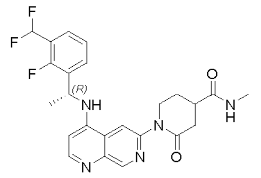 | White solid | 472.1 | $^1H$ NMR (400 MHz, $CD_3OD$): $\delta$ 9.07 (s, 1H), 8.38 (d, $J$ = 7.5 Hz, 2H), 7.63-7.38 (m, 2H), 7.22 (t, $J$ = 7.7 Hz, 1H), 7.03 (t, $J$ = 54.8 Hz, 1H), 6.39 (d, $J$ = 5.6 Hz, 1H), 5.16 (d, $J$ = 6.8 Hz, 1H), 4.04-3.85 (m, 2H), 3.02-2.91 (m, 1H), 2.85-2.74 (m, 4H), 2.72 (d, $J$ = 4.9 Hz, 1H), 2.23 (d, $J$ = 4.4 Hz, 1H), 2.16 (d, $J$ = 5.3 Hz, 1H), 1.73 (d, $J$ = 6.8 Hz, 3H). |
| **B-57** | 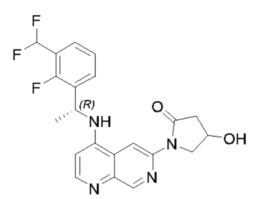 | White solid | 417.0 | $^1H$ NMR (400 MHz, DMSO-$d_6$): $\delta$ 9.01 (t, $J$ = 1.0 Hz, 1H), 8.88 (d, $J$ = 1.0 Hz, 1H), 8.35 (dd, $J$ = 5.2, 3.0 Hz, 1H), 7.71 (dd, $J$ = 15.8, 7.2 Hz, 1H), 7.63 (t, $J$ = 7.6 Hz, 1H), 7.52 (d, $J$ = 5.4 Hz, 1H), 7.43-7.07 (m, 2H), 6.28 (dd, $J$ = 7.4, 5.4 Hz, 1H), 5.34 (dd, $J$ = 12.1, 3.3 Hz, 1H), 5.13-5.06 (m, 1H), 4.45 (s, 1H), 4.23 (ddd, $J$ = 33.4, 11.4, 4.9 Hz, 1H), 4.00 (dd, $J$ = 49.7, 11.3 Hz, 1H), 2.96 (ddd, $J$ = 17.1, 5.9, 2.6 Hz, 1H), 2.47-2.36 (m, 1H), 1.67 (dd, $J$ = 6.8, 2.6 Hz, 3H). |
| **B-58** | 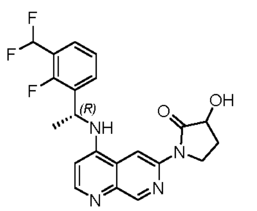 | White solid | 417.0 | $^1H$ NMR (400 MHz, $CD_3OD$): $\delta$ 9.03 (s, 1H), 8.82 (d, $J$ = 5.3 Hz, 1H), 8.32 (d, $J$ = 5.5 Hz, 1H), 7.54 (dt, $J$ = 25.1, 6.9 Hz, 2H), 7.22 (t, $J$ = 7.8 Hz, 1H), 7.04 (t, $J$ = 54.8 Hz, 1H), 6.37 (d, $J$ = 5.5 Hz, 1H), 5.18 (q, $J$ = 6.9 Hz, 1H), 4.60 (dd, $J$ = 9.5, 8.2 Hz, 1H), 4.31 (dd, $J$ = 10.7, 9.1 Hz, 1H), 4.04-3.95 (m, 1H), 2.61 (dt, $J$ = 10.6, 6.5 Hz, 1H), 2.10-1.97 (m, 1H), 1.76 (d, $J$ = 6.8 Hz, 3H). |

(continued)

| No . | Structure | Character | LCMS (ESI): $m/z$ $[M+H]^+$ | $^1H$ NMR |
|---|---|---|---|---|
| **B-59** | 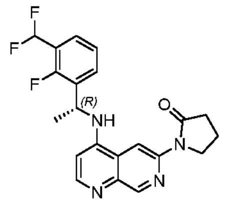 | White solid | 401.2 | $^1H$ NMR (400 MHz, DMSO-$d_6$): $\delta$ 9.01 (s, 1H), 8.86 (s, 1H), 8.35 (d, $J$ = 5.2 Hz, 1H), 7.70 (d, $J$ = 7.2 Hz, 1H), 7.63 (t, $J$ = 7.4 Hz, 1H), 7.52 (t, $J$ = 7.1 Hz, 1H), 7.31-7.22 (m, 2H), 6.28 (d, $J$ = 5.3 Hz, 1H), 5.10 (t, $J$ = 6.9 Hz, 1H), 4.17 (dt, $J$ = 10.5, 7.3 Hz, 1H), 4.06 (dt, $J$ = 10.6, 6.6 Hz, 1H), 2.63 (td, $J$ = 7.7, 7.3, 2.3 Hz, 2H), 2.13 (q, $J$ = 7.2, 6.8 Hz, 2H), 1.67 (d, $J$ = 6.8 Hz, 3H). |
| **B-60** | 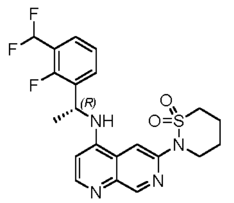 | White solid | 451.0 | $^1H$ NMR (400 MHz, DMSO-$d_6$): $\delta$ 9.04 (s, 1H), 8.42 (d, $J$ = 5.3 Hz, 1H), 8.37 (s, 1H), 7.83 (d, $J$ = 6.9 Hz, 1H), 7.59 (t, $J$ = 7.3 Hz, 1H), 7.53 (t, $J$ = 7.0 Hz, 1H), 7.29-7.12 (m, 2H), 6.33 (d, $J$ = 5.4 Hz, 1H), 5.14-5.07 (m, 1H),, 3.97-3.86 (m, 2H), 3.41-3.33 (m, 2H), 2.29-2.20 (m, 2H), 2.00-1.87 (m, 2H),,1.66 (d, $J$ = 6.8 Hz, 3H). |
| **B-61** | 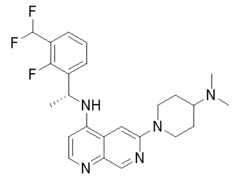 | Yellow solid | 444.2 | $^1H$ NMR (400 MHz, $CD_3OD$): $\delta$ 8.87 (s, 1H), 8.13 (d, $J$ = 5.6 Hz, 1H), 7.58-7.46 (m, 3H), 7.24 (t, $J$ = 7.7 Hz, 1H), 7.03 (t, $J$ = 54.8 Hz, 1H), 6.27 (d, $J$ = 5.7 Hz, 1H), 5.18 (q, $J$ = 6.9 Hz, 1H), 4.73 (d, $J$ = 13.5 Hz, 2H), 3.48-3.39 (m, 1H), 3.02 (t, $J$ = 12.9 Hz, 2H), 2.87 (s, 6H), 2.21 (d, $J$ = 12.4 Hz, 2H), 1.86-1.73 (m, 5H). |

**Example 47 Synthesis of compound B-53**

Synthetic route:

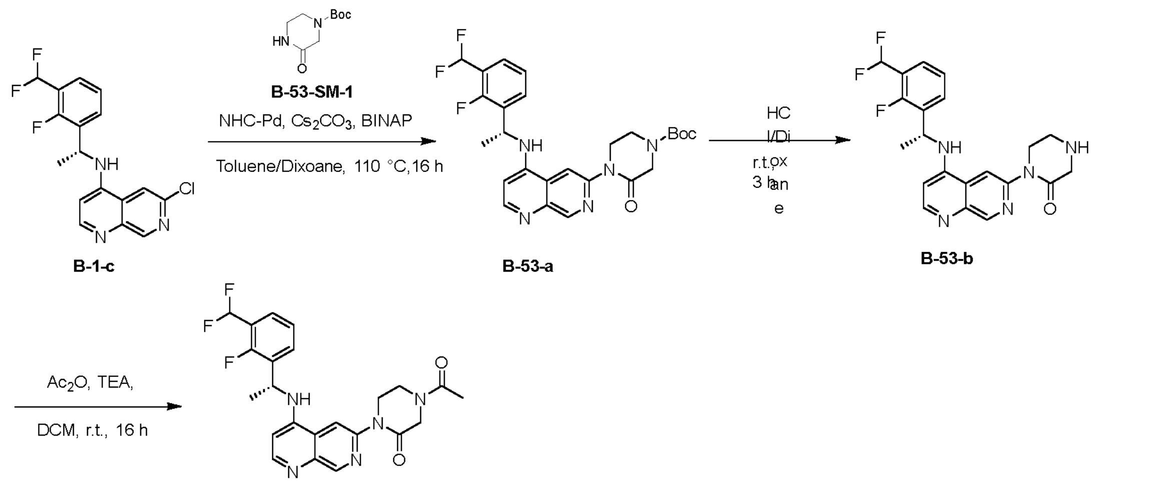


B-1-c B-53-a B-53-b B-53

Step one:

To a dry flask, compound **B-1-c** (100 mg, 0.28 mmol), **B-53-SM-1** (114 mg, 0.57 mmol), toluene/1,4-dioxane (10 mL, v/v = 1:1), cesium carbonate (278 mg, 0.85 mmol), BINAP (35 mg, 0.057 mmol), and NHC-Pd (38 mg, 0.057

mmol) were successively added. The reaction was performed while stirring overnight (16 hours) at 110°C under nitrogen protection. After the reaction was completed, the reaction solution was cooled and concentrated under reduced pressure. The residue was purified by a rapid column chromatography to obtain a brown solid **B-53-a** (100 mg, yield of 68%). LCMS (ESI): *m/z* =5 16.2 [M+H]+.

Step two:

To a dry flask, compound **B-53-a** (100 mg, 0.19 mmol) and HCl/1,4-dioxane (4 mL) were successively added. The reaction was performed while stirring at room temperature for 3 hours. After the reaction was completed, the reaction solution was concentrated under reduced pressure. The residue was adjusted to be alkaline using a saturated sodium bicarbonate solution and extracted with ethyl acetate. The organic phase was dried over sodium sulfate, filtered, and subjected to rotary evaporation to obtain a brown solid **B-53-b** (60 mg, yield of 74%). LCMS (ESI): *m/z* = 416.2 [M+H]+.

Step three:

To a dry flask, compound **B-53-b** (60 mg, 0.14 mmol), dichloromethane (4 mL), triethylamine (44 mg, 0.43 mmol), and acetic anhydride (15 mg, 0.15 mmol) were successively added. The mixture reacted while stirring overnight at room temperature. After the reaction was completed, the reaction solution was diluted with water and extracted with dichloromethane. The organic phase was subjected to rotary evaporation. The residue was purified by Prep-HPLC to obtain a white solid **B-53** (20.6 mg, yield of 31%). LCMS (ESI): *m/z* = 458.1 [M+H]+; $^1$H NMR (400 MHz, DMSO-$d_6$): $\delta$ 9.07 (s, 1H), 8.58 (s, 1H), 8.42 (d, *J* = 5.3 Hz, 1H), 7.78 (d, *J* = 7.0 Hz, 1H), 7.60 (t, *J* = 7.5 Hz, 1H), 7.53 (t, *J* = 7.1 Hz, 1H), 7.42-7.09 (m, 2H), 6.33 (d, *J* = 5.4 Hz, 1H), 5.11 (p, *J* = 6.8 Hz, 1H), 4.47-4.24 (m, 2H), 4.16-3.73 (m, 4H), 2.11 (d, *J* = 7.7 Hz, 3H), 1.65 (d, *J* = 6.7 Hz, 3H).

**Example 48 Synthesis of compound B-56**

Synthetic route:

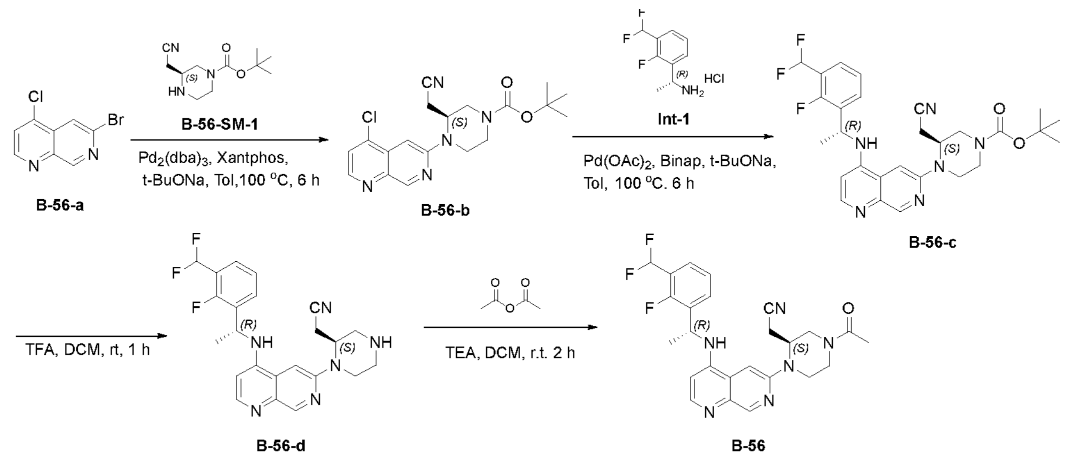


**[0484]**

Step one:

Compound **B-56-a** (227 mg, 0.93 mmol), **B-56-SM-1** (250 mg, 1.1 mmol), Pd$_2$(dba)$_3$ (85 mg, 0.09 mmol), Xantphos (107 mg, 0.18 mmol), and sodium-t-butoxide (267 mg, 2.8 mmol) were dissolved in an eggplant-shaped flask containing toluene (10 mL), and reacted at 100°C for 6 hours under nitrogen protection. After the reaction was completed, the reaction solution was cooled and extracted with ethyl acetate. The organic phase was dried and concentrated. The residue was purified by a silica gel column to obtain a yellow solid compound **B-56-b** (60 mg, yield of 16.8%). LCMS (ESI): *m/z* = 388.1 [M+H]+.

Step two:

Compound **B-56-b** (60 mg, 0.16 mmol), **Int-1** (39 mg, 0.17 mmol), Pd(OAc)$_2$ (3.5 mg, 0.02 mmol), BINAP (19

mg, 0.04 mmol), and sodium-t-butoxide (30 mg, 0.4 mmol) were dissolved in an eggplant-shaped flask containing toluene (10 mL), and reacted at 100°C for 6 hours. After the reaction was completed, the reaction solution was cooled and extracted with ethyl acetate. The organic phase was dried and concentrated. The residue was purified by the silica gel column to obtain a yellow solid compound **B-56-c** (50 mg, yield of 60%). LCMS (ESI): *m/z* = 541.2 $[M+H]^+$.

Step three:

Compound **B-56-c** (50 mg, 0.09 mmol) was dissolved in an eggplant-shaped flask containing dichloromethane (3 mL), and trifluoroacetic acid (0.5 mL) was added thereto and reacted at room temperature for 1 hour. After the reaction was completed, the pH of the reaction solution was adjusted using a sodium bicarbonate aqueous solution to be about 8 and the reaction solution was extracted with ethyl acetate. The organic phase was concentrated under reduced pressure to obtain a yellow oily compound **B-56-d** (30 mg, yield of 75%). LCMS (ESI): *m/z* = 441.2 $[M+H]^+$.

Step four:

Compound **B-56-d** (30 mg, 0.07 mmol), acetic anhydride (7 mg, 0.07 mmol), and triethylamine (21 mg, 0.21 mmol) were dissolved in an eggplant-shaped flask containing DCM (2 mL) and stirred at room temperature for 2 hours. After the reaction was completed, the reaction solution was extracted with ethyl acetate and concentrated. The residue was purified by prep-HPLC to obtain to a white solid compound **B-56** (4.8 mg, yield of 15%). LCMS (ESI): *m/z* = 483.2 $[M+H]^+$; $^1H$ NMR (400 MHz, $CD_3OD$): $\delta$ 8.91 (d, *J* = 2.5 Hz, 1H), 8.14 (dd, *J* = 5.3, 1.5 Hz, 1H), 7.52 (q, *J* = 8.1 Hz, 2H), 7.46 (d, *J* = 2.3 Hz, 1H), 7.22 (t, *J* = 7.7 Hz, 1H), 7.04 (t, *J* = 54.8 Hz, 1H), 6.22 (d, *J* = 5.4 Hz, 1H), 5.24 (d, *J* = 27.4 Hz, 1H), 5.15 (q, *J*= 6.9 Hz, 1H), 4.73-4.51 (m, 1H), 4.26-4.04 (m, 2H), 3.54-3.41 (m, 1H), 3.24 (dd, J = 13.7, 3.8 Hz, 1H), 3.14-3.06 (m, 1H), 2.97-2.72 (m, 2H), 2.23 (s, 3H), 1.75 (d, *J*= 6.8 Hz, 3H).

**Example 49 Synthesis of compound B-62**

Synthetic route:

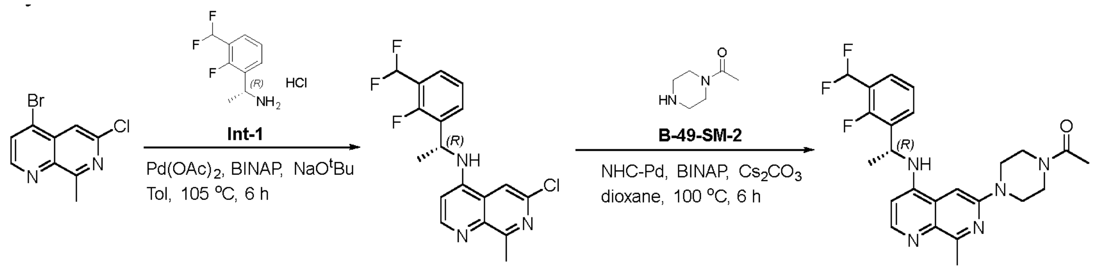


B-62-a B-62-b B-62

Step one:

$Pd(OAc)_2$ (4 mg, 0.018 mmol), BINAP (12 mg, 0.018 mmol), and sodium-t-butoxide (52 mg, 0.54 mmol) were added in a toluene (10 mL) solution dissolved with compound **B-62**-a (45 mg, 0.18 mmol) and compound **Int-1** (40 mg, 0.18 mmol) under nitrogen protection. After uniformly mixed, the reaction solution was gradually heated to 105°C and reacted for 6 hours under nitrogen protection. After the reaction was completed, the reaction solution was poured into a saturated salt solution and extracted three times with ethyl acetate. The organic phase was collected, dried, and concentrated under reduced pressure. The residue was purified by a glass silica gel column to obtain a yellow oily compound **B-62-b** (40 mg, yield of 63%).

Step two:

NHC-Pd (7 mg, 0.01 mmol), BINAP (7 mg, 0.01 mmol), and cesium carbonate (120 mg, 0.4 mmol) were added in a dioxane (10 mL) solution dissolved with compound **B-62-b** (35 mg, 0.1 mmol) and acetylpiperazine (15 mg, 0.12 mmol). After uniformly mixed, the reaction solution was gradually heated to 100°C and reacted for 6 hours under nitrogen protection. After the reaction was completed, the reaction solution was cooled and poured into a saturated salt solution and extracted three times with ethyl acetate, the organic phase was collected, dried, and concentrated under reduced

pressure, and the residue was subjected to a reverse-phase preparative purification to obtain compound **B-62** (4.4 mg, yellow solid) with the yield of 10%. LCMS (ESI): *m/z* = 458.2 $[M+H]^+$; $^1H$ NMR (400 MHz, DMSO-$d_6$): $\delta$ 8.12 (d, *J* = 5.1 Hz, 1H), 7.53 (q, *J* = 7.9 Hz, 2H), 7.42-7.07 (m, 4H), 6.13 (d, *J* = 5.2 Hz, 1H), 5.06 (t, *J* = 6.8 Hz, 1H), 3.68-3.59 (m, 6H), 3.57 (d, *J* = 5.8 Hz, 2H), 2.75 (s, 3H), 2.08 (s, 3H), 1.67 (d, *J* = 6.8 Hz, 3H).

The synthesis of the following compounds was the same as that of **B-62**.

| No. | Structure | Character | LCMS (ESI): *m/z* $[M+H]^+$ | $^1H$ NMR |
|---|---|---|---|---|
| **B-63** | 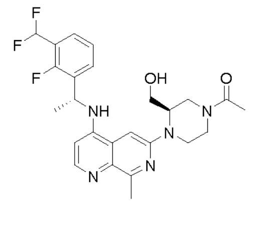 | Yellow solid | 488.0 | $^1H$ NMR (400 MHz, $CD_3OD$): $\delta$ 8.08 (dd, *J* = 5.3, 2.8 Hz, 1H), 7.51 (dd, *J* = 13.3, 6.3 Hz, 2H), 7.13 (ddd, *J* = 101.1, 55.5, 31.3 Hz, 3H), 6.20 (d, *J* = 5.4 Hz, 1H), 5.12 (q, *J* = 6.7 Hz, 1H), 4.85 (d, *J* = 3.7 Hz, 1H), 4.75-4.58 (m, 1H), 4.28 (d, *J* = 13.4 Hz, 1H), 4.02 (dd, *J* = 27.0, 12.7 Hz, 1H), 3.68-3.47 (m, 3H), 3.29-2.99 (m, 2H), 2.81 (d, *J* = 2.1 Hz, 3H), 2.21 (d, *J* = 6.5 Hz, 3H), 1.73 (d, *J* = 6.8 Hz, 3H). |
| **B-64** | 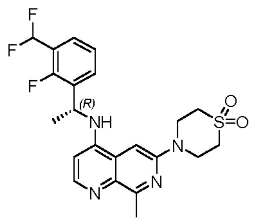 | Yellow solid | 466.0 | $^1H$ NMR (400 MHz, $CD_3OD$): $\delta$ 8.26 (s, 1H), 7.57-7.48 (m, 2H), 7.40 (s, 1H), 7.22 (t, *J* = 8.0 Hz, 1H), 7.00 (t, *J* = 55.2 Hz, 1H), 5.77 (q, *J* = 7.2 Hz, 1H), 4.31-4.28 (m, 4H), 3.18-3.15 (m, 4H), 2.75 (s, 3H), 1.69 (d, *J* = 6.8 Hz, 3H). |
| **B-65** | 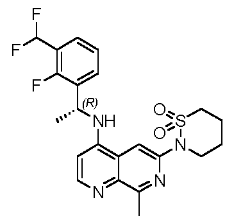 | White solid | 465.0 | $^1H$ NMR (400 MHz, DMSO-$d_6$): $\delta$ 8.92 (d, *J* = 7.3 Hz, 1H), 8.52 (s, 1H), 8.17 (s, 1H), 7.64 (t, *J* = 7.4 Hz, 1H), 7.52 (t, *J* = 7.1 Hz, 1H), 7.40-7.08 (m, 2H), 5.79 (q, *J* = 7.0 Hz, 1H), 3.92 (dt, *J* = 8.2, 4.1 Hz, 2H), 3.33 (d, *J* = 8.4 Hz, 2H), 2.79 (s, 3H), 2.24 (q, *J* = 6.1 Hz, 2H), 2.01-1.90 (m, 3H), 1.62 (d, *J* = 7.0 Hz, 3H). |

**Example 50 Synthesis of compound B-66**

Synthetic route:

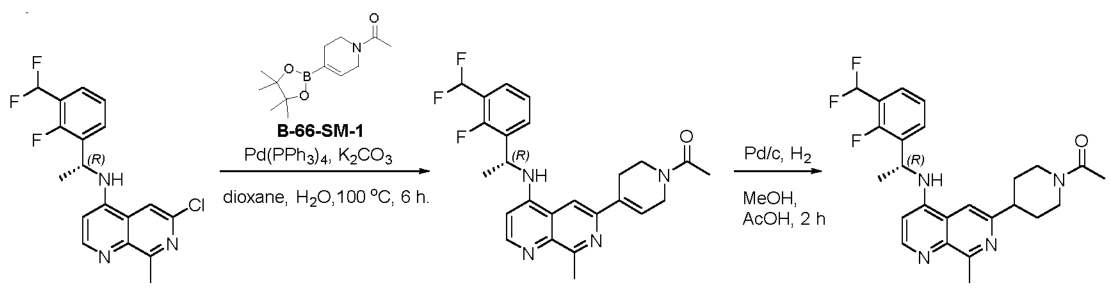


B-62-b B-66-a B-66

Step one:

Compound **B-62-b** (70 mg, 0.19 mmol) and **B-66-SM-1** (96 mg, 0.38 mmol) were dissolved in 5 mL of 1,4-dioxane and 1 mL of water, and then $Pd(PPh_3)_4$ (44 mg, 0.04 mmol) and potassium carbonate (79 mg, 0.57 mmol) were added to the solution, and stirred and reacted at 100°C for 6 hours under nitrogen protection. After the reaction was completed, the reaction solution was cooled and concentrated under reduced pressure, water was added, and the reaction solution was extracted with ethyl acetate. The organic phase was collected, dried over anhydrous sodium sulfate and concentrated, and purified by a column chromatography and concentrated to obtain compound **B-66-a** (85 mg, yellow oil) with the yield of 71%.

Step two:

Compound **B-66-a** (50 mg, 0.11 mmol) was dissolved in 10 mL of methanol, and a small amount of acetic acid (1-2 drops) and then palladium-carbon (25 mg) were added, and stirred and reacted at room temperature for 2 hours under a hydrogen environment. After the reaction was completed, the reaction solution was filtered and concentrated. The organic phase was subjected to a preparative purification to obtain compound **B-66** (14.4 mg, white solid) with the yield of 29%. LCMS (ESI): $m/z$ = 457.2 $[M+H]^+$; $^1H$ NMR (400 MHz, DMSO-$d_6$): $\delta$ 8.34 (d, $J$ = 5.3 Hz, 1H), 8.04 (s, 1H), 7.63-7.47 (m, 3H), 7.43-7.08 (m, 2H), 6.28 (d, $J$ = 5.4 Hz, 1H), 5.07 (q, $J$ = 6.8 Hz, 1H), 4.61 (d, $J$ = 12.7 Hz, 1H), 3.99 (d, $J$ = 13.0 Hz, 1H), 3.21 (t, $J$= 12.9 Hz, 1H), 3.01 (dtd, $J$= 11.9, 8.3, 4.2 Hz, 1H), 2.81 (s, 3H), 2.73-2.60 (m, 1H), 2.06 (s, 3H), 2.03-1.87 (m, 2H), 1.75 (dddd, J = 20.8, 12.4, 8.6, 4.0 Hz, 2H), 1.66 (d, $J$= 6.8 Hz, 3H).

**Example 51 Synthesis of compound B-67**

Synthetic route:

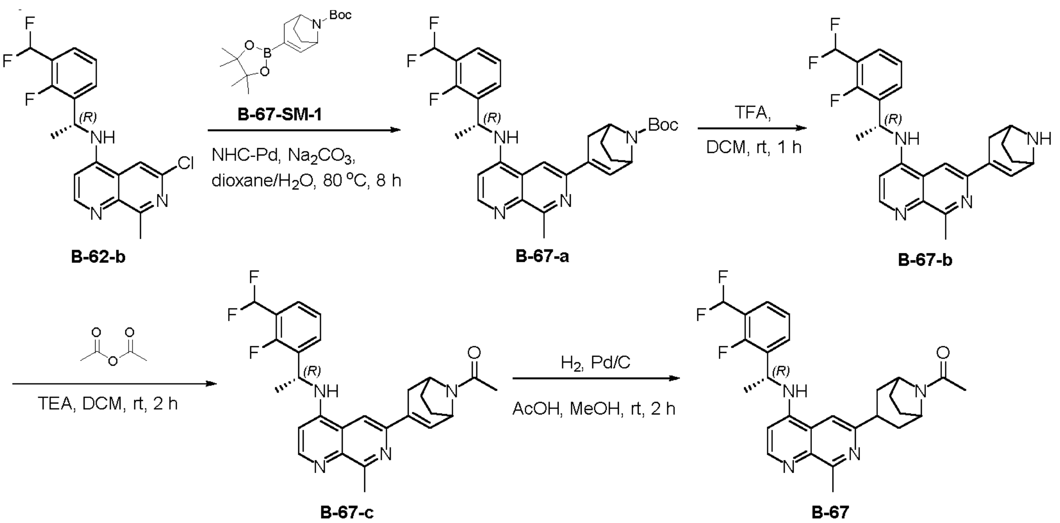


B-62-b B-67-a B-67-b B-67-c B-67

Step one:

NHC-Pd (75 mg, 0.11 mmol) and sodium carbonate (116 mg, 1.10 mmol) were added to a dioxane/water (10 mL/2 mL) solution dissolved with compound **B-62-b** (200 mg, 0.55 mmol) and compound **B-67-SM-1** (220 mg, 0.66 mmol) to be uniformly mixed. The reaction solution was gradually heated to 80°C and reacted for 8 hours under nitrogen protection. After the reaction was completed, the reaction solution was poured into a saturated salt solution and extracted three times with ethyl acetate, the organic phase was collected, dried, and concentrated under reduced pressure, and the residue was purified by a glass silica gel column to obtain a yellow oily compound **B-67-a** (300 mg, yield of 99%).

Step two:

Under nitrogen protection, TFA (25 mL) was added to a DCM (20 mL) solution dissolved with compound **B-67-a** (300 mg, 0.55 mmol), uniformly mixed, and reacted at room temperature for 1 hour. After the reaction was completed, the reaction solution was poured into a saturated salt solution and extracted three times with ethyl acetate, the organic phase was collected, dried, and concentrated under reduced pressure, and the residue was purified by the glass silica gel column to obtain a yellow oily compound **B-67-b** (173 mg, yellow liquid) with the yield of 72%.

Step three:

Triethylamine (69 mg) and acetic anhydride (35 mg, 0.34 mmol) were added to a dichloromethane (10 mL) solution of compound **B-67-b** (173 mg, 0.36 mmol), and the reaction was performed at room temperature for 2 hours. After the reaction was completed, the reaction solution was extracted three times with dichloromethane, the organic phase was collected, dried, and concentrated under reduced pressure, and the residue was purified by the glass silica gel column to obtain a yellow oily compound **B-67-c** (173 mg, yellow oil) with the yield of 93%.

Step four:

Pd/C (25 mg) and acetic acid (20 mg) were added to a methanol (10 mL) solution of compound **B-67-c** (50 mg, 0.10 mmol), and the reaction was performed at room temperature for 2 hours under hydrogen. After the reaction was completed, the reaction solution was poured into a saturated salt solution and extracted three times with ethyl acetate, the organic phase was collected, dried, and concentrated under reduced pressure, and the residue was purified by the glass silica gel column to obtain a white solid **B-67** (18.9 mg, yield of 37.8%). LCMS (ESI): *m/z* = 483.2 $[M+H]^+$; $^1H$ NMR (400 MHz, DMSO-$d_6$): $\delta$ 8.32 (d, J = 5.3 Hz, 1H), 8.00 (d, J = 2.0 Hz, 1H), 7.53 (m, 3H), 7.42-7.02 (m, 2H), 6.26 (dd, J = 5.6, 1.8 Hz, 1H), 5.06 (m, 1H), 4.62 (m, 1H), 4.44-4.20 (m, 1H), 3.46-3.35 (m, 1H), 2.79 (s, 3H), 2.11-2.00 (m, 5H), 1.96- 1.75 (m, 6H), 1.65 (m, 3H).

**Example 52 Synthesis of compound B-68**

Synthetic route:

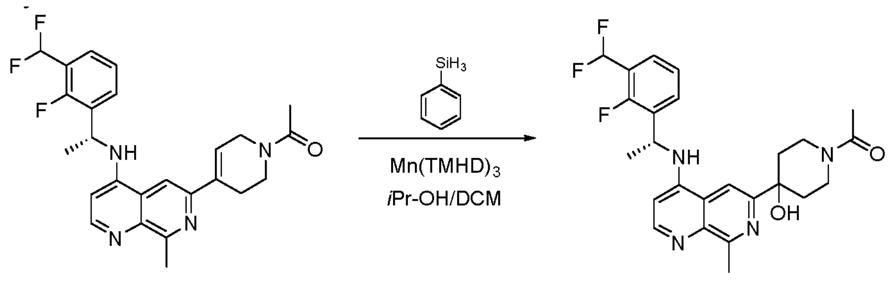


B-66-a B-68

Compound **B-66-a** (40 mg, 0.09 mmol) and Mn(TMHD)$_3$ (11 mg, 0.02 mmol) were successively added to iPr-OH/DCM (5 mL/0.5 mL), and stirred at room temperature for 0.5 hour. Then phenylsilane (24 mg, 0.2 mmol) was added in the reaction and stirred at 30°C for 3 hours. After the reaction was completed, the reaction solution was extracted 3 times with ethyl acetate and washed 3 times with a saturated ammonium chloride aqueous solution. The organic phase was dried over anhydrous sodium sulfate, filtered, concentrated, and purified by Prep-HPLC to obtain compound **B-68** (14.2 mg, yield of 35%). LCMS (ESI): *m/z* = 473.1 $[M+H]^+$; $^1H$ NMR (400 MHz, $CD_3OD$): $\delta$ 8.34 (d, *J* = 5.5 Hz, 1H), 8.28 (s, 1H), 7.59-7.44 (m, 2H), 7.22 (t, *J* = 7.7 Hz, 1H), 7.04 (t, *J* = 54.8 Hz, 1H), 6.39 (d, *J* = 5.5 Hz, 1H), 5.15 (q, *J* = 6.8 Hz, 1H), 4.52 (d, *J* = 12.9 Hz, 1H), 3.91 (d, *J* = 13.6 Hz, 1H), 3.75-3.61 (m, 1H), 3.21 (dd, *J* = 12.9, 10.3 Hz, 1H), 2.90 (s, 3H), 2.43-2.23 (m, 2H), 2.19 (s, 3H), 1.90-1.70 (m, 5H).

The synthesis of the following compounds was the same as that of **B-68**.

| No. | Structure | Character | LCMS (ESI): *m/z* [M+H]+ | $^1$H NMR |
|---|---|---|---|---|
| **B-69** | 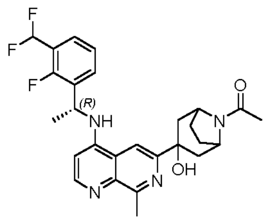 | White solid | 499.2 | $^1$H NMR (400 MHz, DMSO-$d_6$): $\delta$ 8.48 (s, 1H), 8.34 (d, *J* = 5.3 Hz, 1H), 7.73 (d, *J* = 7.0 Hz, 1H), 7.52 (m, 2H), 7.40 -7.00 (m, 2H), 6.39- 6.21 (m, 1H), 5.1-5.02 (m, 1H), 4.98 (s, 1H), 4.46 (s, 1H), 4.22 (s, 1H), 3.24 (m, 1H), 3.04 (m, 1H), 2.82 (s, 3H), 2.01 (m, 5H), 1.65 (m, 4H), 1.43 (m, 3H). |

**Example 53 Synthesis of compound B-70**

Synthetic route:

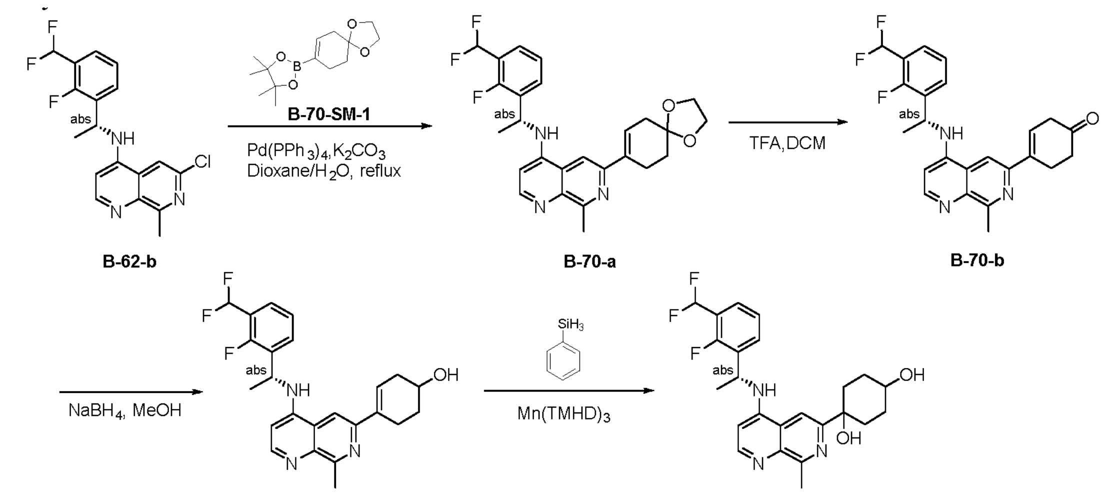


B-62-b B-70-a B-70-b B-70-c B-70

Step one:

Compound **B-62-b** (200 mg, 0.55 mmol), compound **B-70-SM-1** (290 mg, 1.1 mmol), $Pd(PPh_3)_4$ (126 mg, 0.11 mmol), and $K_2CO_3$ (226 mg, 1.6 mmol) were added to dioxane/water (5:1, 12 mL), and stirred under reflux for 12 hours under nitrogen protection. After the reaction was completed, the reaction solution was cooled and extracted 3 times with ethyl acetate and washed 3 times with a saturated ammonium chloride aqueous solution. The organic phase was dried over anhydrous sodium sulfate, filtered, concentrated, and purified by a silica gel chromatographic column (PE:EA = 3:1) to obtain compound **B-70-a** (200 mg, yield of 78%). LCMS (ESI): *m/z* = 470.1 [M+H]+.

Step two:

Compound **B-70-a** (200 mg, 0.43 mmol) was added to DCM (15 mL), and TFA (1.5 mL) was dropwise added at 0°C. After the reaction was completed, the organic phase was concentrated and purified by the silica gel chromatographic column (DCM:MeOH = 20:1) to obtain compound **B-70-b** (400 mg, yield of 98%). LCMS (ESI): *m/z* = 426.1 [M+H]+.

Step three:

To a dry flask, compound **B-70-b** (140 mg, 0.33 mmol) was added to MeOH (10 mL), and sodium borohydride (50 mg, 1.3 mmol) was dropwise added at low temperature and stirred at room temperature for 2 hours. After the reaction was completed, the reaction solution was extracted 3 times with dichloromethane and washed 3 times with a saturated ammonium chloride aqueous solution. The organic phase was dried over anhydrous sodium sulfate, filtered, concentrated, and purified by the silica gel chromatographic column (DCM:MeOH = 20:1) to obtain compound **B-70-c** (50 mg, yield of 36%). LCMS (ESI): *m/z* = 428.1 $[M+H]^+$.

Step four:

Compound **B-70-c** (40 mg, 0.09 mmol) and $Mn(TMHD)_3$ (11 mg, 0.02 mmol) were successively added to iPrOH/DCM (5 mL/0.5 mL), and stirred at room temperature for 0.5 hour. Then phenylsilane (24 mg, 0.2 mmol) was added in the reaction and stirred at 30°C for 3 hours. After the reaction was completed, the reaction solution was extracted 3 times with ethyl acetate and washed 3 times with a saturated ammonium chloride aqueous solution. The organic phase was dried over anhydrous sodium sulfate, filtered, concentrated, and purified by Prep-HPLC to obtain compound **B-70** (12.5 mg, yield of 30%). LCMS (ESI): *m/z* = 446.1 $[M+H]^+$; $^1H$ NMR (400 MHz, $CD_3OD$): $\delta$ 8.33 (dd, *J* = 5.5, 1.8 Hz, 1H), 8.23 (d, *J* = 5.7 Hz, 1H), 7.57-7.47 (m, 2H), 7.22 (t, *J* = 7.8 Hz, 1H), 7.04 (t, *J* = 54.8 Hz, 1H), 6.38 (d, *J* = 5.6 Hz, 1H), 5.20-5.04 (m, 1H), 3.83 (dd, *J* = 69.2, 62.1 Hz, 1H), 2.91 (d, *J* = 4.8 Hz, 3H), 2.56 (t, *J* = 11.1 Hz, 1H), 2.38-2.01 (m, 2H), 1.96-1.59 (m, 8H).

**Example 54 Synthesis of compound B-71**

Synthetic route:

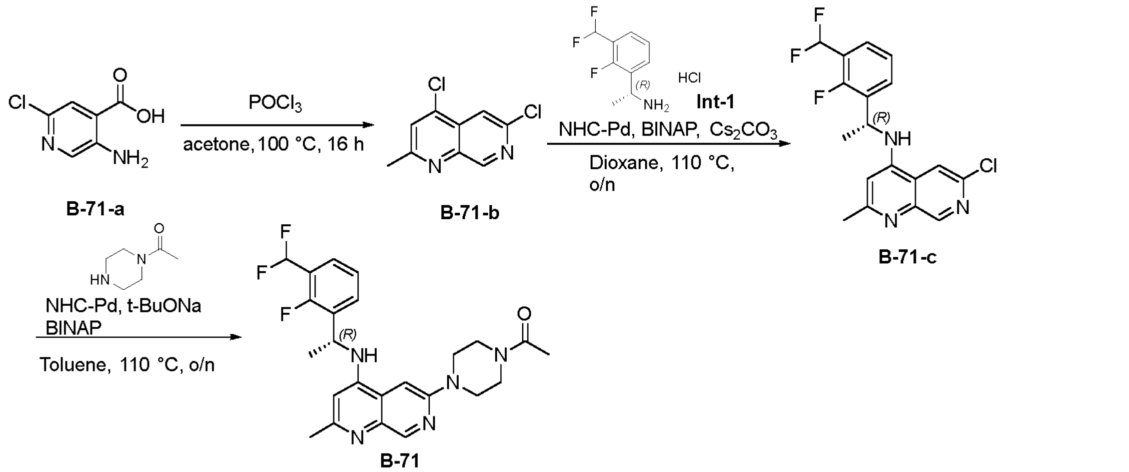


B-71-a

B-71-b

B-71-c

B-71

Step one:

To a dry flask, compound **B-71-a** (500 mg, 2.9 mmol), phosphorus oxychloride (10 mL), and acetone (840 mg, 14.5 mmol) were successively added, and reacted while stirring overnight at 100°C. After the reaction was completed, the reaction solution was cooled, concentrated under reduced pressure, 2 mol/L of a sodium hydroxide solution was used for quench and adjusting the pH to 10-14, and the reaction solution was extracted with ethyl acetate (20 mL×3). The organic phase was dried over sodium sulfate, filtered, and concentrated under reduced pressure to obtain a brown solid **B-71-b** (480 mg, yield of 78%). LCMS (ESI): *m/z* = 213.0 $[M+H]^+$

Step two:

To a dry flask, compound **B-71-b** (100 mg, 0.47 mmol), (R)-1-(3-(difluoromethyl)-2-fluorophenyl)ethyl-1-amine hydrochloride (106 mg, 0.47 mmol), dioxane (8 mL), cesium carbonate (459 mg, 1.41 mmol), BINAP (29 mg, 0.047 mmol), and NHC-Pd (32 mg, 0.047 mmol) were successively added. The reaction was performed while stirring overnight at 110°C under nitrogen protection. After the reaction was completed, the reaction solution was cooled and concentrated under reduced pressure. The residue was purified by a rapid column chromatography to obtain a yellow solid **B-71-c** (50 mg, yield of 29%). LCMS (ESI): *m/z* = 366.2 $[M+H]^+$.

Step three:

To a sealed tube, compound **B-71-c** (25 mg, 0.068 mmol), 1-(piperazin-1-yl)ethan-1-one (18 mg, 0.14 mmol),

toluene (2 mL), sodium t-butoxide (20 mg, 0.20 mmol), BINAP (4 mg, 0.007 mmol), and NHC-Pd (4 mg, 0.007 mmol) were successively added. The reaction was performed while stirring overnight at 110°C under nitrogen protection. After the reaction was completed, the reaction solution was cooled and concentrated under reduced pressure. The residue was firstly purified by the rapid column chromatography and then further purified by prep-HPLC to obtain a yellow solid **B-71**(24.5 mg, yield of 79%). LCMS (ESI): *m*/*z* = 458.1 $[M+H]^+$; $^1H$ NMR (400 MHz, DMSO-$d_6$) $\delta$ 8.75 (s, 1H), 7.55 (dt, *J* = 13.0, 6.9 Hz, 2H), 7.43-7.12 (m, 4H), 6.11 (s, 1H), 5.19-4.95 (m, 1H), 3.64-3.55 (m, 8H), 2.29 (s, 3H), 2.08 (s, 3H), 1.66 (d, *J* = 6.7 Hz, 3H).

The synthesis of the following compounds was the same as that of **B-71**.

| No. | Structure | Character | LCMS (ESI): *m*/*z* $[M+H]^+$ | $^1H$ NMR |
|---|---|---|---|---|
| **B-72** | 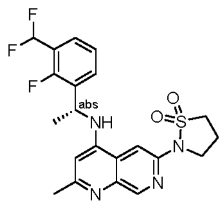 | White solid | 451.0 | $^1H$ NMR (400 MHz, DMSO-$d_6$): $\delta$ 8.87 (s, 1H), 7.74 (s, 1H), 7.61 (t, *J* = 7.5 Hz, 1H), 7.55-7.52 (m, 2H), 7.40-7.12 (m, 2H), 6.22 (s, 1H), 5.12 (m, 1H), 4.04-3.91 (m, 2H), 3.57-3.49 (m, 2H), 2.50-2.43 (m, 2H), 2.36 (s, 3H), 1.67 (d, *J* = 6.8 Hz, 3H). |

**Example 55 Synthesis of compound B-73**

Synthetic route:

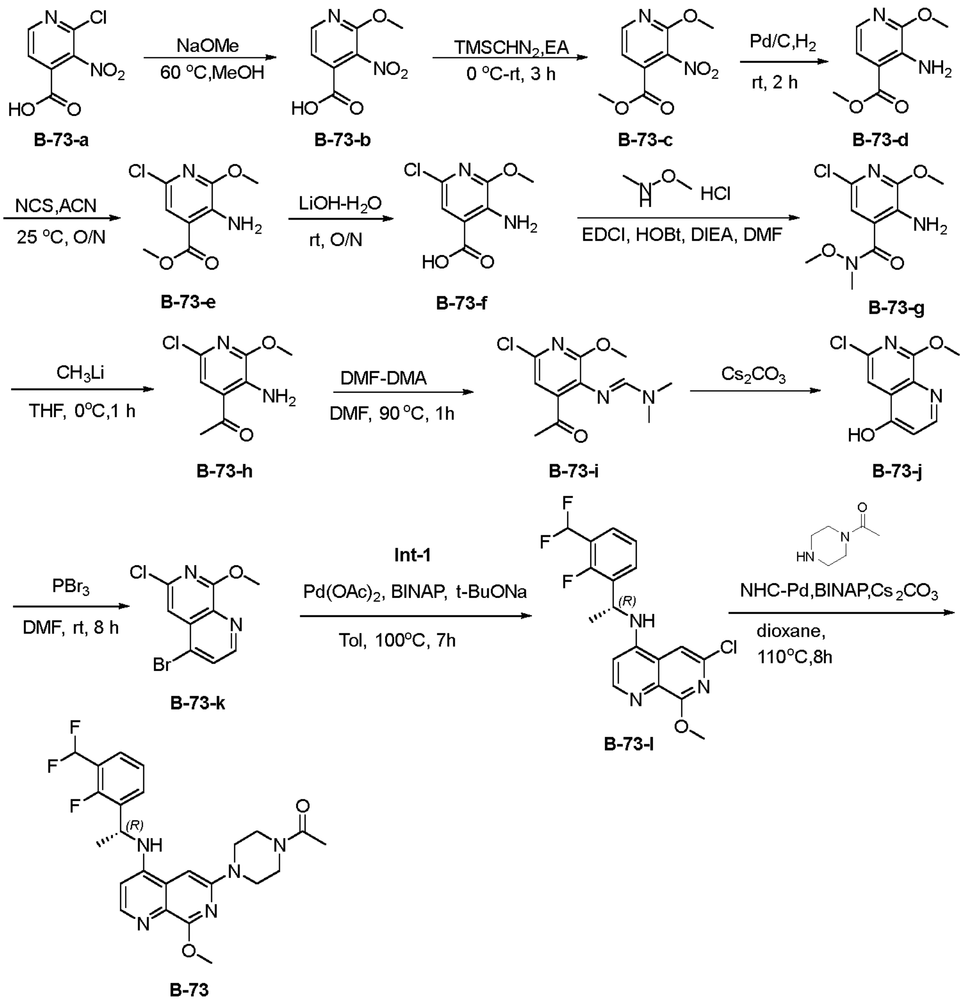


Step one:

Compound **B-73-a** (5 g, 24.8 mmol) and sodium methoxide (13.3 g, 248 mmol) were dissolved in an eggplant-shaped flask containing methanol (100 mL), and stirred overnight at 60°C. After the reaction was completed, the reaction solution was extracted with ethyl acetate. The organic phase was concentrated under reduced pressure to obtain a yellow solid compound **B-73-b** (4.4 g, yield of 89%). LCMS (ESI): m/z =199.0 $[M+H]^+$.

Step two:

Compound **B-73-b** (4.4 mg, 22 mmol) was dissolved in a three neck flask containing ethyl acetate (50 mL) and cooled to 0°C, and TMSCHN2 (22 mL) was dropwise added under 0°C and reacted at room temperature for 3 hours. After the reaction was completed, the reaction solution was quenched with an ammonium chloride aqueous solution and extracted. The organic phase was concentrated under reduced pressure. The residue was purified by a silica gel column to obtain a yellow solid compound **B-73-c** (3 g, yield of 64%). LCMS (ESI): m/z =213.0$[M+H]^+$.

Step three:

Compound **B-73-c** (3.0 g, 14 mmol) was dissolved in an eggplant-shaped flask containing methanol (50 mL), and Pd/C (3.0 g) was added thereto. After hydrogen replacement, the reaction was performed at room temperature for 2 hours. After the reaction was completed, the reaction solution was filtered. The organic phase was concentrated under reduced pressure to obtain a white solid compound **B-73-d** (2.5 g, yield of 83%). LCMS (ESI): *m/z* = 183.1 $[M+H]^+$.

Step four:

Compound **B-73-d** (2.5 g, 13.7 mmol) and NCS(1.84 g, 13.7 mmol) were dissolved in an eggplant-shaped flask

containing ACN(50 mL) and stirred overnight at 25°C. After the reaction was completed, the reaction solution was extracted with ethyl acetate. The organic phase was concentrated under reduced pressure to obtain a yellow solid compound **B-73-e** (2.9 g, yield of 97%). LCMS (ESI): *m/z* = 217.0 $[M+H]^+$.

Step five:

Compound **B-73-e** (2.9 g, 13.4 mmol) and LiOH-$H_2O$(1.74 g, 40.2 mmol) were dissolved in an eggplant-shaped flask containing MeOH/$H_2O$/THF(10 mL/10 mL/30 mL) and stirred overnight at room temperature. After the reaction was completed, the pH of the reaction solution was adjusted to be 2 using 1 mmol of dilute hydrochloric acid, a solid was separated out, and the solid was filtered out to be dried to obtain a yellow solid compound **B-73-f** (2.2 g, yield of 91%). LCMS (ESI): *m/z* = 203.0 $[M+H]^+$.

Step six:

Compound **B-73-f** (2.2 g, 10.8 mmol), dimethylhydroxylamine hydrochloride (1.6 g, 16.3 mmol), EDCI (3.14 mg, 16.3 mmol), HOBT (2.2 g, 16.3 mmol), and DIEA (4.2 g, 32.6 mmol) were dissolved in an eggplant-shaped flask containing DMF (40 mL) and stirred at overnight at 25°C. After the reaction was completed, the reaction solution was extracted with ethyl acetate and concentrated. The residue was purified by the silica gel column to obtain a yellow solid compound **B-73-g** (1.2 g, yield of 46%). LCMS (ESI): *m/z* = 246.1 $[M+H]^+$.

Step seven:

Compound **B-73-g** (1.2 g, 4.9 mmol) was dissolved in a three neck flask containing THF (20 mL), and $CH_3Li$ (15.3 mL) was added at 0°C and stirred at 0°C for 1 hour. After the reaction was completed, the reaction solution quenched with ammonium chloride and extracted with ethyl acetate, the organic phase was concentrated under reduced pressure, and the residue was purified by the silica gel column to obtain a yellow oily compound **B-73-h** (840 mg, yield of 86%). LCMS (ESI): *m/z* = 201.0 $[M+H]^+$.

Step eight:

Compound **B-73-h** (840 mg, 4 mmol) and DMF-DMA (480 mg, 4 mmol) were dissolved in an eggplant-shaped flask containing DMF (20 mL), and stirred at 90°C for 1 hour. After the reaction was completed, the reaction solution crude product **B-73-i** was directly used for next step. LCMS (ESI): *m/z* = 256.1 $[M+H]^+$.

Step nine:

Cesium carbonate (4 g, 12 mmol) was added to the reaction solution crude product **B-73-i** from the previous step and stirred overnight at 100°C. After the reaction was completed, the reaction solution was extracted with ethyl acetate. The organic phase was concentrated under reduced pressure. The residue was purified by the silica gel column to obtain a yellow solid compound **B-73-j** (320 mg, yield of 36%). LCMS (ESI): *m/z* = 211.0 $[M+H]^+$.

Step ten:

Compound **B-73-j** (320 mg, 1.5 mmol) was dissolved in an eggplant-shaped flask containing DMF (5 mL), and $PBr_3$ (1.03 g, 3.8 mmol) was added thereto at 0°C and stirred at room temperature for 8 hours. After the reaction was completed, the reaction solution was extracted with ethyl acetate and water. The extract was concentrated to obtain a yellow oily compound **B-73-k** (300 mg, yield of 72%). LCMS (ESI): *m/z* = 272.9 $[M+H]^+$.

Step eleven:

Compound **B-73-k** (200 mg, 0.73 mmol), **Int-1** (181 mg, 0.8 mmol), Pd(OAc)2 (16.4 mg, 0.073 mmol), BINAP (90 mg, 0.14 mmol), and t-BuONa (21 mg, 2.2 mmol) were dissolved in an eggplant-shaped flask containing toluene (5 mL), and stirred at 100°C for 7 hours after nitrogen replacement. After the reaction was completed, the reaction solution was extracted with ethyl acetate and concentrated. The residue was purified by the silica gel column to obtain a yellow oily compound **B-73-l** (100 mg, yield of 36%). LCMS (ESI): *m/z* = 382.1 $[M+H]^+$.

Step twelve:

Compound **B-73-I** (10 mg, 0.03 mmol), acetylpiperazine (10 mg, 0.09 mmol), NHC-Pd (1.0 mg, 0.015 mmol), BINAP (1.6 mg, 0.015 mmol), and $Cs_2CO_3$ (26 mg, 0.09 mmol) were dissolved in an eggplant-shaped flask containing dioxane (2 mL), and stirred at 110°C for 8 hours after nitrogen replacement. The operation was repeated five times or more. After the reaction was completed, the reaction solution was extracted with ethyl acetate and concentrated. The residue was purified by prep-HPLC to obtain to a white solid compound **B-73** (3.3 mg, yield of 5%). LCMS (ESI): *m/z* = 474.2 $[M+H]^+$; $^1$H NMR (400 MHz, DMSO-$d_6$): δ 8.02 (d, *J* = 5.1 Hz, 1H), 7.53 (q, *J* = 7.2 Hz, 2H), 7.42-7.05 (m, 3H), 6.93 (s, 1H), 6.15 (d, *J* = 5.3 Hz, 1H), 5.12-4.96 (m, 1H), 3.96 (s, 3H), 3.63 (s, 6H), 3.58-3.50 (m, 2H), 2.08 (s, 3H), 1.66 (d, *J* = 6.8 Hz, 3H).

**Example 56 Synthesis of compound B-74**

Synthetic route:

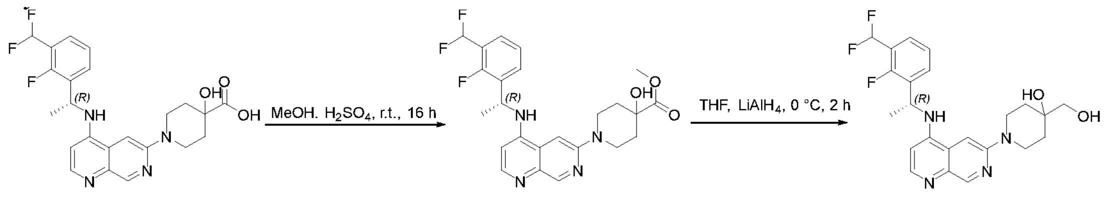


B-74-a B-74-b B-74

Step one:

To a dry flask, compound **B-74-a** (80 mg, 0.17 mmol), methanol (10 mL), and concentrated sulfuric acid (0.1 mL) were successively added. The mixture reacted while stirring overnight at room temperature. After the reaction was completed, the reaction solution was concentrated under reduced pressure. The concentrated product was adjusted to be alkaline using a saturated sodium bicarbonate solution and extracted with ethyl acetate. The organic phase was washed with a saturated salt solution, dried over anhydrous sodium sulfate, filtered, and concentrated under reduced pressure to obtain a yellow solid **B-74-b** (55 mg, yield of 67%). LCMS (ESI): *m/z* = 475.2 $[M+H]^+$.

Step two:

To a dry flask, compound **B-74-b** (55 mg, 0.12 mmol) and tetrahydrofuran (5 mL) were successively added, and LiAlH4 (0.46 mL, 1 mol/L in THF) was dropwise added at 0°C. The reaction was performed while stirring at 0°C for 2 hours. After the reaction was completed, the reaction solution was quenched with 1 mL of water, dried over sodium sulfate, filtered, and concentrated under reduced pressure, and the residue was purified by prep-HPLC to obtain a yellow solid **B-74** (4.3 mg, yield of 8.3%). LCMS (ESI): *m/z* = 447.0 $[M+H]^+$; $^1$H NMR (400 MHz, $CD_3OD$): δ 8.82 (s, 1H), 8.07 (d, *J*= 5.3 Hz, 1H), 7.51 (q, *J* = 8.0 Hz, 2H), 7.40 (s, 1H), 7.22 (t, *J* = 7.7 Hz, 1H), 7.04 (t, *J* = 54.9 Hz, 1H), 6.17 (d, *J* = 5.4 Hz, 1H), 5.13 (q, *J* = 6.8 Hz, 1H), 4.20 (d, *J*= 13.1 Hz, 2H), 3.44-3.31 (m, 4H), 1.85-1.66 (m, 7H).

**Example 57 Synthesis of compound B-75**

Synthetic route:

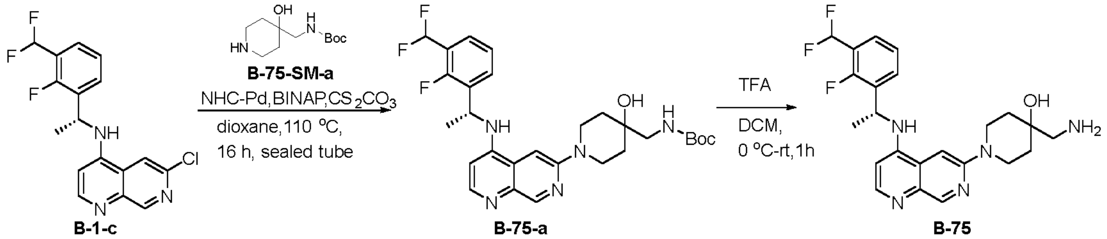


B-1-c B-75-a B-75

Step one:

To a sealed tube, compound **B-1-c** (50 mg, 0.14 mmol) and compound **B-75-SM-a** (163.3 mg, 0.71 mmol) were dissolved in dioxane (3 mL), then NHC-Pd (7.5 mg), BINAP (8.8 mg, 0.014 mmol), and $Cs_2CO_3$ (138.9 mg, 0.43 mmol) were added, nitrogen replacement was performed, and the mixture reacted overnight at 110°C. After the reaction was completed, the reaction solution was extracted with a saturated ammonium chloride solution and EA. The organic phase was backwashed with a saturated salt solution, dried over sodium sulfate, filtered, and concentrated under reduced pressure. The residue was purified by a column chromatography to obtain compound **B-75-a** (45 mg, yellow solid) with the yield of 58.0%. LCMS (ESI): *m*/*z* = 546.0 $[M+H]^+$.

Step two:

Compound **B-75-a** (60 mg, 0.14 mmol) was dissolved in DCM (8 mL), and then TFA (2 mL) was added at 0°C and reacted at room temperature for 1 hour. After the reaction was completed, the reaction solution was concentrated under reduced pressure and then extracted with a saturated sodium bicarbonate solution and DCM. The organic phase was backwashed with water and a saturated salt solution, dried over sodium sulfate, filtered, and concentrated under reduced pressure. The residue was subjected to a formic acid reverse-phase preparative purification to obtain compound **B-75** (22.2 mg, yellow solid) with the yield of 45.3%. LCMS (ESI): *m*/*z* = 446.1 $[M+H]^+$; $^1H$ NMR (400 MHz, $CD_3OD$): $\delta$ 8.85 (s, 1H), 8.11 (d, *J* = 5.6 Hz, 1H), 7.53 (dd, *J* = 13.9, 7.0 Hz, 2H), 7.46 (d, *J* = 2.2 Hz, 1H), 7.24 (t, *J* = 7.7 Hz, 1H), 7.03 (t, *J* = 54.8 Hz, 1H), 6.25 (dd, *J* = 5.4, 3.2 Hz, 1H), 5.21-5.14 (m, 1H), 4.26-4.18 (m, 2H), 3.52-3.44 (m, 2H), 2.96 (s, 2H), 1.82-1.73 (m, 7H).

**Example 58 Synthesis of compound B-76**

Synthetic route:

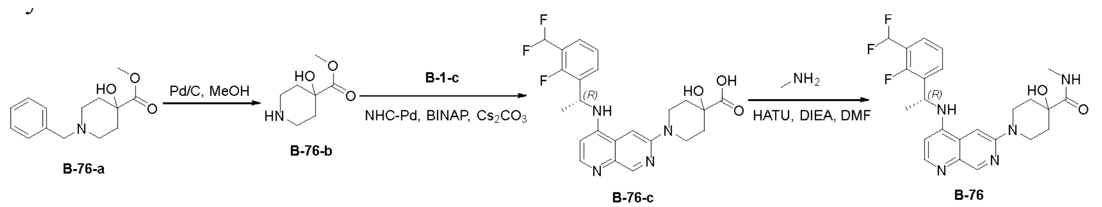

B-76-a B-76-b B-76-c B-76

Step one:

Compound **B-76-a** (900 mg, 3.61 mmol) and Pd/C (100 mg) were dissolved in 10 mL of methanol. The reaction solution reacted at room temperature for 10 hours under a hydrogen environment. After the reaction was completed, Pd/C was filtered out. The reaction solution was concentrated under reduced pressure and a methanol solvent was removed to obtain compound **B-76-b** (620 mg, yellow oil) with the yield of 100%. LCMS (ESI): *m*/*z* =160 $[M+H]^+$.

Step two:

Compound **B-76-b** (620 mg, 3.924 mmol), compound **B-1-c** (690 mg, 1.96 mmol), NHC-Pd (134 mg, 0.196 mmol), BINAP (122 mg, 0.196 mmol), and $Cs_2CO_3$ (1.9 g, 5.89 mmol) were dissolved in 15 mL of dioxane and reacted while stirring at 110°C for 12 hours in a sealed tube under nitrogen environment. After the reaction was completed, the reaction solution was cooled and poured into ice water to be quenched. A large amount of a yellow solid was separated out. The reaction solution was filtered, and the filter residue was collected, washed with water, and dried to obtain compound **B-76-c** (140 mg, yellow solid) with the yield of 15%. LCMS (ESI): *m*/*z* = 461 $[M+H]^+$.

Step three:

Compound **B-76-c** (40 mg, 0.087 mmol), methylamine 2 M (0.2 mL, 0.174 mmol), HATU (40 mg, 0.1 mmol), and DIEA (28 mg, 0.217 mmol) were dissolved in 5 mL of DMF, and reacted at room temperature for 2 hours. After the reaction was completed, the reaction solution was poured into an ammonium chloride solution to be quenched and extracted with ethyl acetate. The organic phase was backwashed with water and a saturated salt solution, dried over

sodium sulfate, filtered, and concentrated under reduced pressure. The residue was subjected to a preparative purification to obtain compound **B-76** (solid) with the yield of 76.5%. LCMS (ESI): *m/z* =474 [M+H]$^+$; $^1$H NMR (400 MHz, DMSO-$d_6$): $\delta$8.81 (s, 1H), 8.10 (d, *J* = 5.1 Hz, 1H), 7.86 (q, *J* = 4.7 Hz, 1H), 7.54 (dt, *J* = 14.1, 7.3 Hz, 2H), 7.41 (s, 1H), 7.40-7.10 (m, 3H), 6.09 (d, *J* = 5.2 Hz, 1H), 5.46 (s, 1H), 5.07 (p, *J* = 6.8 Hz, 1H), 4.34-4.27 (m, 2H), 3.28-3.17 (m, 2H), 2.62 (d, *J* = 4.6 Hz, 3H), 2.01 (ddt, *J* = 13.1, 8.9, 4.3 Hz, 2H), 1.67 (d, *J* = 6.7 Hz, 3H), 1.56 (d, *J* = 13.3 Hz, 2H).

**Example 59 Synthesis of compound B-77**

Synthetic route:

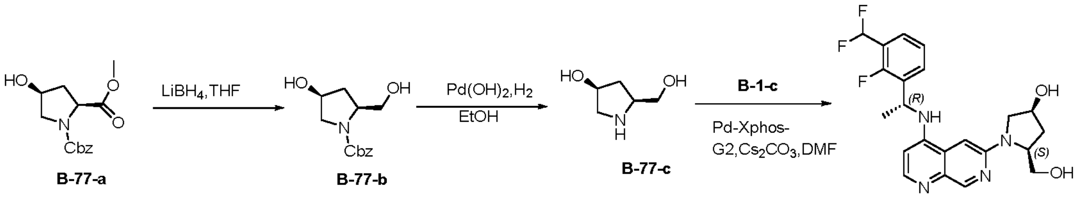


B-77-a B-77-b B-77-c B-77

Step one:

At 0°C, lithium borohydride (470 mg, 21.6 mmol) was added to a tetrahydrofuran (20 mL) solution containing **B-77-a** (2.0 g, 7.2 mmol) and reacted while stirring at room temperature for 16 hours. After the reaction was completed, the reaction solution was quenched with water, diluted with hydrochloric acid (2 M, 5 mL), and extracted with ethyl acetate. The extract was washed with a saturated salt solution and concentrated under reduced pressure. The residue was purified by a silica gel column chromatography (dichloromethane:methanol = 1:0 to 20:1) to obtain compound **B-77-b** (532 mg, white solid) with the yield of 29%. LCMS(ESI): *m/z* = 251.9 [M+H]$^+$.

Step two:

Palladium hydroxide/carbon (29 mg, 10%, 0.21 mmol) was added to an ethanol (5 mL) solution of **B-77-b** (532 mg, 2.1 mmol), hydrogen replacement was performed 3 times, and reaction was performed while stirring at 25°C for 12 hours under a hydrogen (15 psi) atmosphere. After the reaction was completed, a catalyst was filtered out. The filtrate was concentrated under reduced pressure to obtain crude product **B-77-c** (282 mg, brown liquid). LCMS(ESI): *m/z* = 118.0 [M+H]$^+$.

Step three:

**B-1-c** (100 mg, 0.28 mmol), cesium carbonate (182 mg, 0.56 mmol), chlorine(2-dicyclohexylphosphino-2',4',6'-triisopropyl-1,1'-biphenyl)[2-(2'-amino-1,1'-biphenyl)]palladium(II) (22 mg, 0.028 mmol) were added to a N,N-dimethylformamide (2 mL) of **B-77-c** (36 mg, 0.31 mmol). Argon replacement was performed 3 times and the reaction was performed while stirring at 100°C for 12 hours. After the reaction was completed, the reaction solution was concentrated under reduced pressure, diluted with water, and extracted with ethyl acetate. The extract was washed with a saturated salt solution (20 mL) and concentrated under reduced pressure. The residue was purified by a silica gel column chromatography (dichloromethane:methanol = 1:0 to 10:1) to obtain the crude product and the crude product was further purified by a reverse-phase column chromatography (water:acetonitrile = 1:0 to 1:1) to obtain **B-77** (2.5 mg, yellow solid) with the yield of 2%. LCMS(ESI): *m/z* = 433.3 [M+H]$^+$. $^1$H NMR (600 MHz, $CD_3OD$) : $\delta$8.82 (s, 1H), 8.07 (d, *J* = 4.1 Hz, 1H), 7.57-7.48 (m, 2H), 7.23 (d, *J* = 7.7 Hz, 1H), 7.17-6.95 (m, 2H), 6.17 (d, *J* = 5.3 Hz, 1H), 5.16 (q, *J* = 6.7 Hz, 1H), 4.58 (s, 1H), 4.46-4.41 (m, 1H), 4.06 (dd, *J* = 10.8, 4.4 Hz, 1H), 3.86 (dd, *J* = 10.8, 4.2 Hz, 1H), 3.69 (d, *J* = 4.3 Hz, 2H), 2.49-2.43 (m, 1H), 2.10 (d, J = 13.7 Hz, 1H), 1.76 (d, *J* = 6.8 Hz, 3H).

The synthesis of the following compounds was the same as that of **B-77**.

| No. | Structure | Character | LCMS (ESI): *m/z* $[M+H]^+$ | $^1H$ NMR |
|---|---|---|---|---|
| **B-78** | 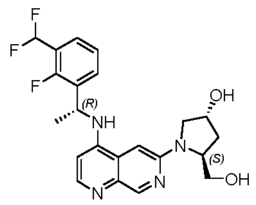 | Yellow solid | 433.3 | $^1H$ NMR (400 MHz, DMSO-$d_6$) : $\delta$ 8.77 (s, 1H), 8.05 (d, *J* = 4.9 Hz, 1H), 7.58-7.48 (m, 2H), 7.40-7.09 (m, 4H), 7.03 (s, 1H), 6.06 (d, *J* = 5.0 Hz, 1H), 5.10-5.04 (m, 1H), 5.03 (d, *J* = 4.5 Hz, 1H), 4.82 (t, *J* = 5.1 Hz, 1H), 4.57 (d, *J* = 5.3 Hz, 1H), 4.33 (s, 1H), 3.73 (dd, *J* = 10.1, 5.9 Hz, 1H), 3.68-3.62 (m, 1H), 3.50-3.43 (m, 1H), 2.27-2.19 (m, 1H), 1.98-1.90 (m, 1H), 1.67 (d, *J* = 6.6 Hz, 3H). |
| **B-79** | 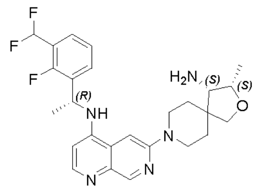 | White solid | 485.9 | $^1H$ NMR (600 MHz, $CD_3OD$): $\delta$ 8.73 (s, 1H), 7.99 (d, *J* = 5.3 Hz, 1H), 7.47-7.36 (m, 2H), 7.30 (s, 1H), 7.12 (t, *J* = 7.7 Hz, 1H), 6.94 (t, *J* = 54.9 Hz, 1H), 6.08 (d, *J* = 5.4 Hz, 1H), 5.03 (q, *J* = 6.7 Hz, 1H), 4.20-4.13 (m, 1H), 4.05-3.95 (m, 2H), 3.80 (d, *J* = 8.7 Hz, 1H), 3.67 (d, J = 8.7 Hz, 1H), 3.29-3.22 (m, 1H), 3.18-3.11 (m, 1H), 2.94 (d, *J* = 4.9 Hz, 1H), 1.89-1.73 (m, 1H), 1.69 (s, 1H), 1.64 (d, J = 6.8 Hz, 3H), 1.14 (d, *J* = 6.5 Hz, 3H). |

**Example 60 Synthesis of compound B-80** (mixture of cis-trans-isomers compound **B-80-P1** and compound **B-80-P2**)

Synthetic route:

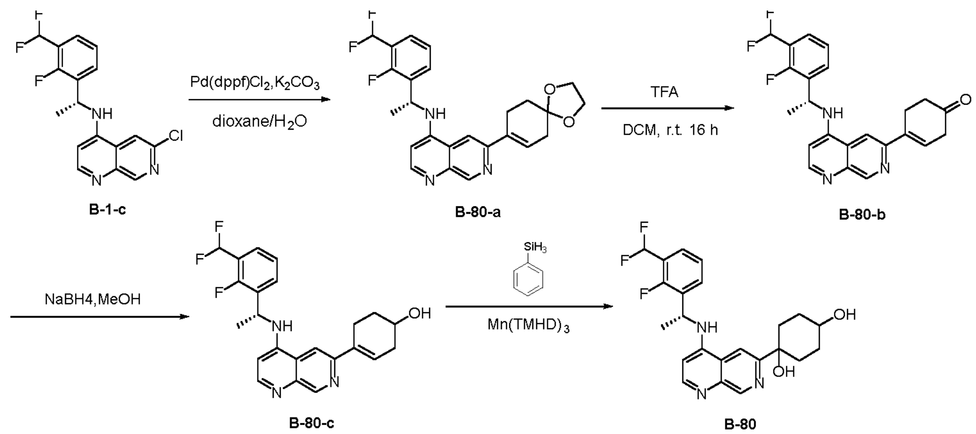


Step one:

Compound **B-1-c** (1.5 g, 3.4 mmol) and compound **B-80-SM-a** (1.6 g, 6.8 mmol) were dissolved in a mixed solvent of 20 mL of 1,4-dioxane and 4 mL of water, and then Pd(dppf)$Cl_2$ (750 mg, 1.02 mmol) and $K_2CO_3$ (1.4 g, 10.2 mmol) were added, and the mixture was heated to 105°C and reacted for 12 hours under a $N_2$ atmosphere. After the reaction was completed, the reaction solution was cooled and quenched with an ammonium chloride solution and then extracted with ethyl acetate. Then the organic phase was washed with a saturated salt solution, dried over anhydrous sodium sulfate, filtered, and concentrated under reduced pressure. The residue was purified by a column chromatography to obtain a brown oily compound **B-80-a** (1.4 g, yield of 91%). LCMS (ESI): *m/z* = 442.1 $[M+H]^+$.

Step two:

Compound **B-80-a** (1.4 g, 3.0 mmol) was dissolved in 20 mL of dichloromethane, and TFA (18 mL) was added and reacted at room temperature for 12 hours. After the reaction was completed, the reaction solution was lowered to

0°C and the pH of the reaction solution was adjusted using a sodium bicarbonate solution to be about 9, extracted with dichloromethane, and dried over anhydrous sodium sulfate. Then the organic phase was filtered and concentrated under reduced pressure to obtain a brown oily compound **B-80-b** (1.2 g, yield of 93%). LCMS (ESI): m/z = 412.1 $[M+H]^+$.

Step three:

Compound **B-80-b** (1.2 g, 2.9 mmol) was dissolved in 20 mL of absolute methanol, sodium borohydride (460 mg, 12.1 mmol) was slowly added in an ice bath, then the reaction temperature was gradually recovered to room temperature, and the reaction was performed for 4 hours. After the reaction was completed, the reaction solution was extracted with an ammonium chloride solution and dichloromethane, and dried over sodium sulfate. Then the organic phase was filtered and concentrated under reduced pressure. The residue was purified by a column chromatography to obtain a brown oily compound **B-80-c** (900 mg, yield of 75%). LCMS (ESI): *m/z* = 414.1 $[M+H]^+$.

Step four:

Compound **B-80-c** (900 mg, 2.18 mmol) and compound $Mn(THMD)_3$ (cas: 14324-99-3, 340 mg, 0.44 mmol) were dissolved in a mixed solvent of 20 mL of isopropanol and 4 mL of dichloromethane, stirred at room temperature for 5 minutes, further phenylsilane (746 mg, 5.5 mmol) was added, and then the mixture was heated to 35°C under an $O_2$ atmosphere and reacted for 4 hours. After the reaction was completed, the reaction solution was cooled and quenched with an ammonium chloride solution, extracted with dichloromethane, and then dried over sodium sulfate. The organic phase was filtered and concentrated under reduced pressure. The residue was purified by prep-HPLC ($NH_4HCO_3$ system) to obtain cis-trans-isomers compound **B-80-P1** and compound **B-80-P2** (P1: 102.4 mg, P2: 123.5 mg, yield of 12%) as white solids. LCMS (ESI): *m/z* = 432.1 $[M+H]^+$; **B-80-P1:** $^1$H NMR (400 MHz, $CD_3OD$): $\delta$ 9.10 (s, 1H), 8.47 (s, 1H), 8.35 (d, *J* = 5.5 Hz, 1H), 7.53 (dd, *J* = 17.0, 8.7 Hz, 2H), 7.23 (t, *J* = 7.7 Hz, 1H), 7.04 (t, *J* = 54.9 Hz, 1H), 6.39 (d, *J* = 5.6 Hz, 1H), 5.17 (d, *J* = 6.9 Hz, 1H), 3.74 (s, 1H), 2.24 (s, 2H), 1.87 (t, *J* = 11.4 Hz, 6H), 1.76 (d, J = 6.8 Hz, 3H). **B-80-P2:** $^1$H NMR (400 MHz, $CD_3OD$): $\delta$ 9.13 (s, 1H), 8.45 (s, 1H), 8.35 (d, *J* = 5.5 Hz, 1H), 7.60-7.44 (m, 2H), 7.23 (t, *J* = 7.8 Hz, 1H), 7.04 (t, *J* = 54.8 Hz, 1H), 6.39 (d, *J* = 5.6 Hz, 1H), 5.18 (d, *J* = 6.6 Hz, 1H), 4.05 (s, 1H), 2.59 (t, *J* = 10.9 Hz, 2H), 2.10 (t, *J* = 11.4 Hz, 2H), 1.79-1.61 (m, 7H).

Referring to the synthesis of compound **B-1-c**, the following R-configuration chiral amine hydrochlorides: (R)-1-(2-methyl-3-(trifluoromethyl)phenyl)ethan-1-amine hydrochloride (cas: 2230840-58-9, purchased), (R)-1-(2-fluoro-3-(trifluoromethyl)phenyl)ethan-1-amine hydrochloride (**Int-3**), (R)-1-(2-methyl-3-(difluoromethyl)phenyl)ethan-1-amine hydrochloride (cas: 2230840-57-8, purchased), and (R)-1-(2-fluoro-3-(difluoroethyl)phenyl)ethan-1-amine hydrochloride (**Int-4**) were respectively used to replace **Int-1** in the original route. After the corresponding intermediates were synthesized, referring to the synthesis of compound **B-80**, the following compounds **B-84**, **B-85, B-86,** and **B-87** were synthesized.

Referring to the synthesis of **B-80**, the following compound **B-88** was synthesized by replacing **B-1-c** with intermediate **B-71-c**.

Referring to the synthesis of **B-80**, the following compound **B-178** was synthesized by replacing **B-1-c** with intermediate **B-73-l**.

| No. | Structure | Character | LCMS (ESI): *m/z* [M+H]+ | $^1$H NMR |
|---|---|---|---|---|
| **B-84** (mixture of cis-trans-isomers **B-84-P1** and **B-84-P2**) | 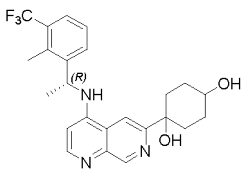 | White solid | 446.1 | **B-84-P1:** $^1$H NMR (400 MHz, DMSO-$d_6$) $\delta$ 9.07 (s, 1H), 8.60 (s, 1H), 8.35 (d, *J* = 5.3 Hz, 1H), 7.91 (d, *J* = 6.6 Hz, 1H), 7.68 (d, *J* = 7.9 Hz, 1H), 7.58 (d, *J* = 7.7 Hz, 1H), 7.33 (t, *J* = 7.8 Hz, 1H), 6.13 (d, *J* = 5.4 Hz, 1H), 5.08 (q, *J* = 6.7 Hz, 1H), 4.96 (s, 1H), 4.32 (s, 1H), 3.90 (s, 1H), 2.58 (s, 3H), 2.55-2.45 (m, 2H), 1.97-1.90 (m, 2H),1.63-1.44 (m, 7H). **B-84-P2**: $^1$H NMR (400 MHz, DMSO-$d_6$) $\delta$ 9.06 (s, 1H), 8.65 (s, 1H), 8.36 (d, *J* = 5.2 Hz, 1H), 7.93 (s, 1H), 7.69 (d, *J* = 7.9 Hz, 1H), 7.59 (d, *J* = 7.9 Hz, 1H), 7.34 (t, *J* = 8.0 Hz, 1H), 6.14 (d, *J* = 5.2 Hz, 1H), 5.10-5.06 (m, 2H), 4.54 (d, *J* = 4.6 Hz, 1H), 3.55-3.52 (m, 1H), 2.58 (s, 3H), 2.17-2.05 (m, 2H), 1.79-1.67 (m, 6H), 1.63-1.61 (m, 3H). |
| **B-85** (mixture of cis-trans-isomers **B-85-P1** and **B-85-P2**) | 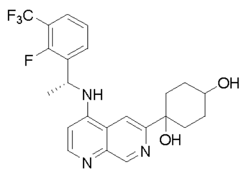 | Pale brown solid | 450.1 | **B-85-P1:** $^1$H NMR (400 MHz, DMSO-$d_6$): $\delta$ 9.09 (s, 1H), 8.57 (s, 1H), 8.39 (d, *J* = 5.3 Hz, 1H), 7.88 (d, *J* = 6.8 Hz, 1H), 7.76 (t, *J* = 7.2 Hz, 1H), 7.69 (t, *J* = 7.1 Hz, 1H), 7.37 (t, *J* = 7.8 Hz, 1H), 6.31 (d, *J* = 5.4 Hz, 1H), 5.15 (p, *J* = 6.7 Hz, 1H), 4.98 (s, 1H), 4.32 (d, *J* = 2.7 Hz, 1H), 3.90 (s, 1H), 2.49-2.40 (m, 2H), 1.94 (t, *J* = 12.6 Hz, 2H), 1.69 (d, *J* = 6.8 Hz, 3H), 1.55 (d, *J* = 10.2 Hz, 2H), 1.47 (d, *J* = 13.1 Hz, 2H). **B-85-P2**: $^1$H NMR (400 MHz, DMSO-$d_6$): $\delta$ 9.07 (s, 1H), 8.62 (s, 1H), 8.39 (d, *J* = 5.3 Hz, 1H), 7.90 (d, *J* = 6.9 Hz, 1H), 7.76 (t, *J* = 7.0 Hz, 1H), 7.69 (t, *J* = 7.3 Hz, 1H), 7.37 (t, *J* = 7.8 Hz, 1H), 6.32 (d, *J* = 5.4 Hz, 1H), 5.19-5.12 (m, 1H), 5.09 (s, 1H), 4.52 (d, *J* = 4.6 Hz, 1H), 3.60-3.47 (m, 1H), 2.20-2.07 (m, 2H), 1.71 (dd, *J* = 13.9, 6.0 Hz, 9H). |
| **B-86** (mixture of cis-trans-isomers **B-86-P1** and **B-**86-P2) | 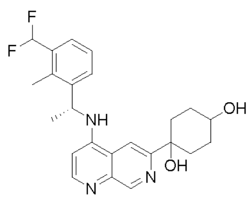 | White solid | 428.0 | **B-86-P1:** $^1$H NMR (400 MHz, $CD_3OD$): $\delta$ 9.11 (s, 1H), 8.45 (s, 1H), 8.31 (d, *J* = 5.5 Hz, 1H), 7.53 (d, *J* = 8.0 Hz, 1H), 7.42 (d, *J* = 8.0 Hz, 1H), 7.22 (t, *J* = 7.7 Hz, 1H), 6.98 (t, *J* = 55.3 Hz, 1H), 6.22 (d, *J* = 5.7 Hz, 1H), 5.10 (q, *J* = 6.8 Hz, 1H), 4.05 (s, 1H), 2.58 (d, *J* = 11.3 Hz, 5H), 2.10 (t, *J* = 11.0 Hz, 2H), 1.74-1.66 (m, 7H). **B-86-P2:** $^1$H NMR (400 MHz, $CD_3OD$): $\delta$ 9.08 (s, 1H), 8.48 (s, 1H), 8.31 (d, *J* = 5.5 Hz, 1H), 7.52 (d, *J* = 8.2 Hz, 1H), 7.42 (d, *J* = 7.8 Hz, 1H), 7.22 (t, *J* = 7.8 Hz, 1H), 6.98 (t, *J* = 55.2 Hz, 1H), 6.22 (d, *J* = 5.6 Hz, 1H), 5.09 (q, *J* = 6.7 Hz, 1H), 3.74 (dd, *J* = 14.2, 7.4 Hz, 1H), 2.56 (s, 3H), 2.24 (dt, *J* = 12.0, 5.7 Hz, 2H), 1.88 (dd, *J* = 18.6, 11.3 Hz, 6H), 1.69 (d, *J* = 6.7 Hz, 3H). |

(continued)

| No. | Structure | Character | LCMS (ESI): *m/z* [M+H]+ | $^1$H NMR |
|---|---|---|---|---|
| **B-87** (mixture of cis-trans-isomers **B-87-P1** and **B-87-P2**) | 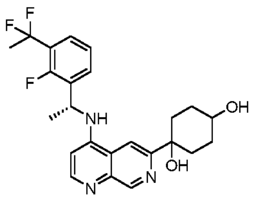 | White solid | 446.0 | **B-87-P1:** $^1$H NMR (400 MHz, $CD_3OD$): δ 9.13 (s, 1H), 8.45 (s, 1H), 8.36 (d, *J* = 5.6 Hz, 1H), 7.55-7.43 (m, 2H), 7.18 (t, *J* = 7.8 Hz, 1H), 6.40 (d, *J* = 5.6 Hz, 1H), 5.19 (q, *J* = 6.8 Hz, 1H), 4.05 (s, 1H), 2.59 (t, *J* = 11.2 Hz, 2H), 2.13-1.96 (m, 5H), 1.78-1.66 (m, 7H). **B-87-P2:** $^1$H NMR (400 MHz, $CD_3OD$): δ 9.10 (s, 1H), 8.48 (s, 1H), 8.36 (d, *J* = 5.6 Hz, 1H), 7.49 (dt, *J* = 14.7, 7.5 Hz, 2H), 7.18 (t, *J* = 7.7 Hz, 1H), 6.39 (d, *J* = 5.6 Hz, 1H), 5.20-5.15 (m, 1H), 3.74 (t, *J* = 8.0 Hz, 1H), 2.28-2.19 (m, 2H), 2.02 (t, *J* = 18.6 Hz, 3H), 1.88 (dd, *J* = 18.6, 11.1 Hz, 6H), 1.75 (d, *J* = 6.8 Hz, 3H). |
| **B-88** (mixture of cis-trans-isomers **B-88-P1** and **B-88-P2)** | 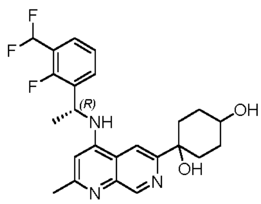 | White solid | 446.2 | **B-88-P1:** $^1$H NMR (400 MHz, DMSO-$d_6$) δ 8.98 (s, 1H), 8.49 (s, 1H), 7.72 (d, *J* = 7.3 Hz, 1H), 7.64 (t, *J* = 7.4 Hz, 1H), 7.53 (t, *J* = 7.1 Hz, 1H), 7.44-7.06 (m, 2H), 6.23 (s, 1H), 5.10 (p, *J* = 6.9 Hz, 1H), 4.92 (s, 1H), 4.30 (d, *J* = 2.9 Hz, 1H), 3.89 (s, 1H), 2.50-2.40 (m, 2H), 2.37 (s, 3H), 1.92 (t, *J* = 12.8 Hz, 2H), 1.67 (d, *J* = 6.8 Hz, 3H), 1.55-1.44 (m, 4H). **B-88-P2**:$^1$H NMR (400 MHz, DMSO-$d_6$) δ 8.96 (s, 1H), 8.52 (s, 1H), 7.72-7.60 (m, 2H), 7.53 (t, *J* = 7.1 Hz, 1H), 7.43-7.09 (m, 2H), 6.23 (s, 1H), 5.10 (p, *J* = 6.8 Hz, 1H), 5.03 (s, 1H), 4.50 (d, *J* = 4.6 Hz, 1H), 3.52 (dt, *J* = 9.5, 5.0 Hz, 1H), 2.36 (s, 3H), 2.08 (td, *J* = 13.8, 8.6 Hz, 2H), 1.83-1.56 (m, 9H). |
| **B-178** (mixture of cis-trans-isomers **B-178-P1** and **B-178-P2**) | 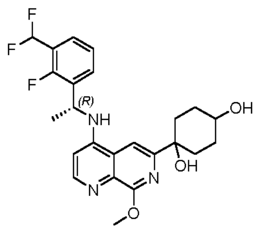 | White solid | 462.0 | **B-178-P1:** $^1$H NMR (400 MHz, $CD_3OD$): δ 8.23-8.24 (d, *J* = 5.6 Hz, 1H), 7.92 (s, 1H), 7.49-7.55 (m, 2H), 7.21- 7.24 (t, *J* = 8.0 Hz, 1H), 6.90-7.17 (t, *J* = 54.4 Hz, 1H), 6.39-6.40 (d, *J* = 5.6 Hz, 1H), 5.16 (m, 1H), 4.15 (s, 3H), 2.59 (m, 2H), 2.07 (m, 2H), 1.66-1.75 (m, 7H). **B-178-P2:** $^1$H NMR (400 MHz, $CD_3OD$): δ 8.22-8.24 (d, *J* = 5.6 Hz, 1H), 7.91 (s, 1H), 7.49-7.54 (m, 2H), 7.17- 7.24 (t, *J* = 8.0 Hz, 1H), 6.90-7.17 (t, *J* = 54.4 Hz, 1H), 6.38-6.39 (d, *J* = 5.6 Hz, 1H), 5.13-5.15 (m, 1H), 4.12 (s, 3H), 3.72 (m, 1H), 2.24 (m, 2H), 1.85-1.90 (m, 6H), 1.73-1.74 (d, *J* = 5.6 Hz, 2H). |

**Example 61 Synthesis of compound B-81** (mixture of cis-trans-isomers compound **B-81-P1** and compound **B-81-P2**)

Synthetic route:

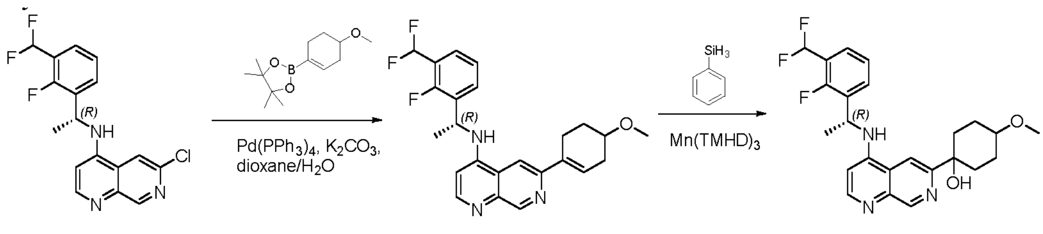


B-1-c B-81-a B-81

Step one:

Compound **B-1-c** (50 mg, 0.14 mmol), borate (170 mg, 0.71 mmol), $Pd(PPh_3)_4$ (32 mg, 0.07 mmol), and $K_2CO_3$ (60 mg, 0.43 mmol) were added to dioxane/water (5:1, 6 mL), and stirred at 105°C for 12 hours under nitrogen protection. After the reaction was completed, the reaction solution was cooled and extracted 3 times with ethyl acetate and washed 3 times with a saturated ammonium chloride aqueous solution. The organic phase was dried over anhydrous sodium sulfate, filtered, concentrated, and purified by a silica gel chromatographic column (DCM:MeOH = 20:1) to obtain compound **B-81-a** (50 mg, yield of 83%). LCMS (ESI): *m/z* = 428.1 $[M+H]^+$.

Step two:

Compound **B-81-a** (50 mg, 0.12 mmol) and $Mn(TMHD)_3$ (21 mg, 0.04 mmol) were successively added to i-PrOH/DCM (5 mL/0.5 mL), and stirred at room temperature for 0.5 hour. Then phenylsilane (32 mg, 0.3 mmol) was added in the reaction and stirred at 30°C for 3 hours. After the reaction was completed, the reaction solution was extracted 3 times with ethyl acetate and washed 3 times with a saturated ammonium chloride aqueous solution. The organic phase was dried over anhydrous sodium sulfate, filtered, concentrated, and purified by Prep-HPLC to obtain cis-trans-isomers compound **B-81-P1** (5.2 mg, yield of 10%) and compound **B-81-P2** (7.0 mg, yield of 13%). LCMS (ESI): *m/z* = 446.1 $[M+H]^+$ .**B-81**-P1: $^1H$ NMR (400 MHz, $CD_3OD$): $\delta$ 9.10 (s, 1H), 8.48 (s, 1H), 8.35 (d, *J* = 5.5 Hz, 1H), 7.63-7.37 (m, 2H), 7.23 (t, *J* = 7.7 Hz, 1H), 7.08 (dd, *J* = 89.3, 34.5 Hz, 1H), 6.39 (d, *J* = 5.6 Hz, 1H), 5.18 (q, J = 6.9 Hz, 1H), 3.45-3.37 (m, 4H), 2.28-2.15 (m, 2H), 2.02 (d, *J* = 10.1 Hz, 2H), 1.87 (t, *J*= 12.0 Hz, 3H), 1.77 (t, *J* = 11.4 Hz, 4H). **B-81-P2:** $^1H$ NMR (400 MHz, $CD_3OD$) : $\delta$ 9.11 (s, 1H), 8.43 (s, 1H), 8.35 (d, *J*= 5.5 Hz, 1H), 7.62-7.47 (m, 2H), 7.23 (t, *J* = 7.7 Hz, 1H), 7.04 (t, *J* = 54.8 Hz, 1H), 6.38 (d, *J*= 5.6 Hz, 1H), 5.17 (q, *J* = 6.9 Hz, 1H), 3.59 (s, 1H), 3.39 (s, 3H), 2.46 (t, *J* = 13.1 Hz, 2H), 2.05 (t, *J*= 13.2 Hz, 2H), 1.90 (d, *J* = 9.9 Hz, 2H), 1.76 (d, *J* = 6.8 Hz, 3H), 1.65 (d, *J* = 13.0 Hz, 2H).

**Example 62 Synthesis of compound B-82**

Synthetic route:

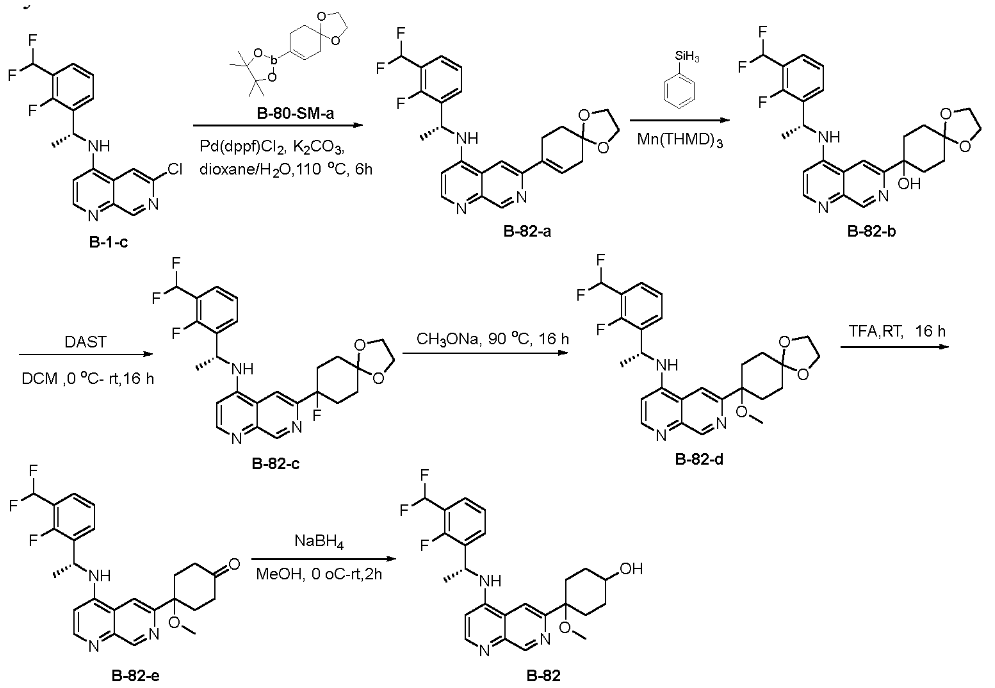


Step one:

After compound **B-1-c** (230 mg, 0.65 mmol) and compound **B-80-SM-a** (348 mg, 1.3 mmol) were added in dioxane/$H_2O$ (5 mL/1 mL) to be uniformly mixed, Pd(dppf)$Cl_2$(348 mg, 0.20 mmol) and $K_2CO_3$(348 mg, 1.96 mmol) were added. Nitrogen replacement was performed. The reaction was performed at 110°C for 6 hours. After the reaction was completed, the reaction solution was extracted with a saturated ammonium chloride solution and EA. The organic phase was backwashed with a saturated salt solution, dried over sodium sulfate, filtered, and concentrated under reduced pressure. The residue was purified by a column chromatography to obtain compound **B-82-a** (250 mg, brown solid) with the yield of 77.2%. LCMS (ESI): $m/z$ = 456 $[M+H]^+$.

Step two:

Compound **B-82-a** (166 mg, 0.36 mmol) and Mn(THMD)$_3$ (44.0 mg, 0.073 mmol) were added to a mixed solution of IPA/DCM (5 mL/0.5 mL) and stirred for 5 min, and then phenylsilane (98.3 mg, 0.91 mmol) was added. Oxygen replacement was performed. The reaction was performed at 30°C for 4 hours. After the reaction was completed, the reaction solution was concentrated under reduced pressure. The residue was subjected to a column chromatography to obtain compound **B-82-b** (120 mg, yellow solid) with the yield of 69.6%. LCMS (ESI): $m/z$ = 474 $[M+H]^+$.

Step three:

DAST (68 mg, 0.42 mmol) was added to a dichloromethane (6 mL) solution of compound **B-82-b** (100 mg, 0.21 mmol) at 0°C and reacted overnight at room temperature under nitrogen protection. After the reaction was completed, the reaction solution was extracted with a saturated ammonium chloride solution and DCM. The organic phase was backwashed with a saturated salt solution, dried over sodium sulfate, filtered, and concentrated under reduced pressure to obtain crude product compound **B-82-c** (105 mg, yellow solid). LCMS (ESI): $m/z$ = 476 $[M+H]^+$.

Step four:

To a sealed tube, sodium methoxide (113.4 mg, 2.1 mmol) was added to a methanol (5 mL) solution dissolved with compound **B-82-c** (100 mg, 0.21 mmol) and reacted overnight at 90°C. After the reaction was completed, the reaction solution was cooled and extracted with a saturated ammonium chloride solution and DCM. The organic phase was backwashed with a saturated salt solution, dried over sodium sulfate, filtered, and concentrated under reduced pressure. The residue was purified by a column chromatography to obtain compound **B-82-d** (65 mg, light yellow solid) with the yield of 63.4%. LCMS (ESI): $m/z$ = 488 $[M+H]^+$.

Step five:

TFA (1 mL) was added to a DCM (2 mL) solution dissolved with compound **B-82-d** (65 mg, 0.13 mmol) and reacted overnight at room temperature. After the reaction was completed, the reaction solution was extracted with a saturated sodium bicarbonate solution and DCM (pH = 8). The organic phase was backwashed with a saturated salt solution, dried over sodium sulfate, filtered, and concentrated under reduced pressure to obtain crude product compound **B-82-e** (70 mg, yellow solid). LCMS (ESI): *m*/*z* = 444 $[M+H]^+$.

Step six:

$NaBH_4$ (24 mg, 0.13 mmol) was added to a methanol (2 mL) solution dissolved with compound **B-82-e** (70 mg, 0.13 mmol) at 0°C and reacted at room temperature for 2 hours under nitrogen protection. After the reaction was completed, the reaction solution was extracted with a saturated ammonium chloride solution and EA. The organic phase was backwashed with a saturated salt solution, dried over sodium sulfate, filtered, and concentrated under reduced pressure. The residue was subjected to a reverse-phase preparative purification to obtain compound **B-82** (2.8 mg, white solid) with the yield of 4.4%. LCMS (ESI): *m*/*z* = 446 $[M+H]^+$. $^1H$ NMR (400 MHz, $CD_3OD$): $\delta$ 9.12 (s, 1H), 8.41 (s, 1H), 8.36 (d, *J* = 5.5 Hz, 1H), 7.59-7.47 (m, 2H), 7.23 (t, *J* = 7.7 Hz, 1H), 7.04 (t, *J* = 54.8 Hz, 1H), 6.40 (d, *J* = 5.6 Hz, 1H), 5.18 (q, *J* = 6.8 Hz, 1H), 3.75-3.64 (m, 1H), 3.10 (s, 3H), 2.32 (d, *J* = 14.1 Hz, 2H), 2.01 (ddd, *J* = 13.8, 10.5, 6.8 Hz, 2H), 1.86 (dd, *J* = 28.6, 10.7 Hz, 3H), 1.76 (d, *J* = 6.8 Hz, 4H).

Referring to the synthesis of **B-82**, the following compound **B-104** was synthesized by replacing **B-82-b** in the original route with a target compound **B-103.**

| No. | Structure | Character | LCMS (ESI): m/z $[M+H]^+$ | $^1H$ NMR |
|---|---|---|---|---|
| **B-104** | 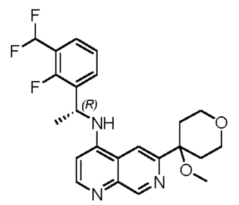 | White solid | 432.2 | $^1H$ NMR (400 MHz, $CD_3OD$) $\delta$ 9.16 (d, *J* = 0.6 Hz, 1H), 8.43 (s, 1H), 8.38 (d, *J* = 5.5 Hz, 1H), 7.62-7.47 (m, 2H), 7.24 (t, *J* = 7.8 Hz, 1H), 7.04 (t, *J* = 54.8 Hz, 1H), 6.41 (d, *J* = 5.6 Hz, 1H), 5.18 (q, *J* = 6.7 Hz, 1H), 3.89 (dd, *J* = 31.8, 10.6 Hz, 4H), 3.08 (s, 3H), 2.32-2.17 (m, 4H), 1.77 (d, *J* = 6.8 Hz, 3H). |

**Example 63 Synthesis of compound B-83** (mixture of cis-trans-isomers compound **B-83-P1** and compound **B-83-P2**)

Synthetic route:

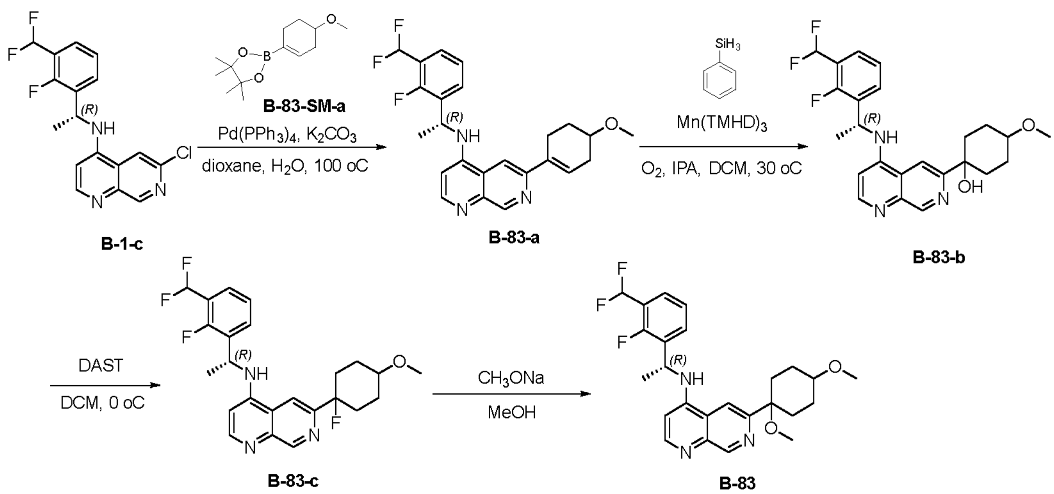

B-1-c B-83-a B-83-b B-83-c B-83

Step one:

To a dry flask, compound **B-1-c** (80 mg, 0.227 mmol), **B-83-SM-a** (162 mg, 0.682 mmol), $Pd(PPh_3)_4$ (53 mg, 0.045 mmol), $K_2CO_3$(94 mg, 0.682 mmol), dioxane (20 mL), and water (4 mL) were successively added. The reaction was performed while stirring at 100°C for 16 hours under nitrogen protection. The reaction solution was cooled, filtered, extracted with ethyl acetate, washed with a saturated salt solution, and dried over anhydrous sodium sulfate. The organic phase was concentrated under reduced pressure and the residue was purified by a column chromatography (dichloromethane:methanol = 10:1) to obtain a yellow solid compound **B-83-a** (96 mg, yield of 98%). LCMS (ESI): m/z = 428.2 $[M+H]^+$

Step two:

To a dry flask, compound **B-83-a** (80 mg, 0.187 mmol), $Mn(TMHD)_3$ (23 mg, 0.037 mmol), IPA (8 mL), and DCM (1.6 mL) were successively added, and stirred at room temperature for 5 minutes. Then phenylsilane (37 mg, 0.480 mmol) was added. The reaction was performed while stirring at 30°C for 2 hours under an oxygen environment. After the reaction was completed, the reaction solution was poured water, filtered, extracted with ethyl acetate, and concentrated. The residue was purified by the column chromatography (dichloromethane:methanol = 10:1) to obtain a yellow oily compound **B-83-b** (70 mg, yield of 74%). LCMS (ESI): m/z = 446.3 $[M+H]^+$

Step three:

To a dry flask, compound **B-83-b** (60 mg, 0.135 mmol) and DCM (10 mL) were added, and stirred at 0°C for 10 minutes. Then DAST (65 mg, 0.404 mmol) was slowly dropwise added. The reaction was performed while stirring overnight at room temperature under a nitrogen environment. After the reaction was completed, the system was subjected to rotary evaporation, washed with a saturated ammonium chloride solution, extracted with dichloromethane, and concentrated. The residue was purified by the column chromatography (dichloromethane:methanol = 10:1) to obtain a yellow oily compound **B-83-c** (52 mg, yield of 74%). LCMS (ESI): m/z = 448.4 $[M+H]^+$

Step four:

To a dry sealed tube, compound **B-83-c** (30 mg, 0.067 mmol) and $CH_3ONa$ (11 mg, 0.201 mmol) were added, and dissolved in MeOH (5 mL). The reaction was performed while stirring overnight at 110°C. After the reaction was completed, the system was cooled, subjected to rotary evaporation, washed with a saturated ammonium chloride solution, extracted with ethyl acetate, and concentrated. The residue was subjected to a prep-HPLC preparative purification to obtain cis-trans-isomers **B-83-P1:** 5.2 mg and compound **B-83-P2:** 5.9 mg as white solids with the total yield of 36%. **B-83-P1:** LCMS (ESI): *m/z* =460.1 $[M+H]^+$; $^1$H NMR (400 MHz, $CD_3OD$) $\delta$ 9.12 (s, 1H), 8.42 (s, 1H), 8.35 (t, *J*= 8.0 Hz, 1H), 7.53 (dt, *J* = 14.2, 7.1 Hz, 2H), 7.23 (t, *J* = 7.7 Hz, 1H), 7.04 (t, *J* = 54.8 Hz, 1H), 6.39 (t, *J* = 5.6 Hz, 1H), 5.17 (q, *J* = 6.8 Hz, 1H), 3.40 (d, *J* = 11.0 Hz, 3H), 3.34 (dd, *J* = 9.7, 5.5 Hz, 1H), 3.10 (s, 3H), 2.33 (d, *J* = 14.3 Hz, 2H), 2.06-1.93 (m, 4H), 1.80-1.69 (m, 5H). **B-83-P2:** LCMS (ESI): *m/z* =460.1 $[M+H]^+$; $^1$H NMR (400 MHz, $CD_3OD$) $\delta$ 9.14 (s, 1H), 8.40 (s, 1H), 8.37 (d, *J* = 5.6 Hz, 1H), 7.60-7.48 (m, 2H), 7.24 (t, *J* = 7.7 Hz, 1H), 7.04 (t, *J* = 54.8 Hz, 1H), 6.40 (d, *J* = 5.6 Hz, 1H), 5.18 (q, *J* = 6.8 Hz, 1H), 3.57 (s, 1H), 3.37 (s, 3H), 3.05 (s, 3H), 2.30 (dd, *J*= 17.9, 7.9 Hz, 2H), 2.07-2.00 (m, 2H), 1.94 (d, *J* = 12.9 Hz, 2H), 1.89-1.81 (m, 2H), 1.76 (d, *J* = 6.8 Hz, 3H).

**Example 64 Synthesis of compound B-89** (mixture of cis-trans-isomers compound **B-89-P1** and compound **B-89-P2**)

Synthetic route:

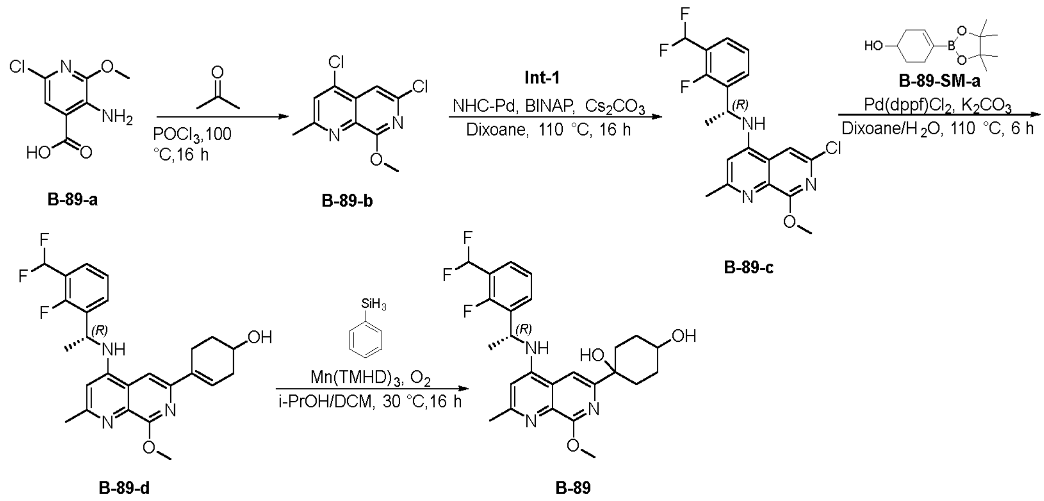


B-89-a B-89-b B-89-c B-89-d B-89

Step one:

To a dry flask, compound **B-89-a** (1.0 g, 4.94 mmol), $POCl_3$(20 mL), and acetone (1.43 g, 24.7 mmol) were successively added. The reaction was performed while stirring overnight at 100°C under nitrogen protection. After the reaction was completed, the reaction solution was cooled and concentrated under reduced pressure. The residue was quenched with ice and extracted with ethyl acetate. The organic phase was dried over sodium sulfate and filtered. The residue was purified by a rapid column chromatography to obtain a yellow solid **B-89-b** (460 mg, yield of 38%). LCMS (ESI): *m/z* = 243.0 $[M+H]^+$

Step two:

To a dry flask, compound **B-89-b** (460 mg, 1.9 mmol), **Int-1** (430 mg, 1.9 mmol), 1,4-dioxane (40 mL), cesium carbonate (1.86 g, 5.7 mmol), BINAP (238 mg, 0.38 mmol), and NHC-Pd (246 mg, 0.38 mmol) were successively added. The reaction was performed while stirring overnight at 110°C under nitrogen protection. After the reaction was completed, the reaction solution was cooled and concentrated under reduced pressure. The residue was purified by the rapid column chromatography to obtain a yellow solid **B-89-c** (470 mg, yield of 62%). LCMS (ESI): *m/z* = 396.1 $[M+H]^+$

Step three:

To a dry flask, compound **B-89-c** (470 mg, 1.2 mmol), compound **B-89-SM-a** (400 mg, 1.8 mmol), 1,4-dioxane/water (20 mL, v/v = 8:1), potassium carbonate (497 mg, 3.6 mmol), and Pd(dppf)$Cl_2$ (263 mg, 0.36 mmol) were successively added. The reaction was performed while stirring at 110°C for 6 hours under nitrogen protection. After the reaction was completed, the reaction solution was cooled and concentrated under reduced pressure. The residue was purified by the rapid column chromatography to obtain a brown solid **B-89-d** (500 mg, yield of 89%). LCMS (ESI): *m/z* = 458.2 $[M+H]^+$

Step four:

To a dry flask, compound **B-89-d** (450 mg, 0.98 mmol), isopropanol/dichloromethane (30 mL, v/v = 10:1), and $Mn(TMHD)_3$ (119 mg, 0.2 mmol) were successively added, and stirred for 5 minutes. Then phenylsilane (266 mg, 2.5 mmol) was added. The reaction was performed while stirring overnight at 30°C under oxygen protection. After the reaction was completed, the reaction solution was concentrated under reduced pressure and purified by prep-HPLC to obtain white solid cis-trans-isomers **B-89-P1** (33.0 mg) and compound **B-89-P2** (24.5 mg) with the total yield of 12%. **B-89-P1:** LCMS (ESI): *m/z* = 476.1 $[M+H]^+$; $^1H$ NMR (400 MHz, $CD_3OD$): $\delta$ 7.99 (s, 1H), 7.61-7.52 (m, 2H), 7.27 (t, *J* = 7.7 Hz, 1H), 7.03 (t, *J* = 54.7 Hz, 1H), 6.45 (s, 1H), 5.26 (d, *J* = 6.8 Hz, 1H), 4.19 (s, 3H), 4.03 (s, 1H), 2.62-2.55 (m, 2H), 2.50 (s, 3H), 2.11-2.03 (m, 2H), 1.86-1.52 (m, 7H). **B-89-P2:** LCMS (ESI): *m/z* = 476.1 $[M+H]^+$; $^1H$ NMR (400 MHz, $CD_3OD$): $\delta$ 7.98 (s, 1H), 7.63-7.49 (m, 2H), 7.26 (t, *J* = 7.7 Hz, 1H), 7.03 (t, *J* = 54.8 Hz, 1H), 6.44 (s, 1H), 5.28-5.22 (m, 1H), 4.16 (s, 3H), 3.77-3.62 (m, 1H), 2.50 (s, 3H), 2.26-2.17 (m, 2H), 1.95-1.80 (m, 6H), 1.76 (d, *J* = 6.8 Hz, 3H).

**Example 65 Synthesis of compound B-90** (mixture of cis-trans-isomers compound **B-90-P1** and compound **B-90-P2**)

Synthetic route:

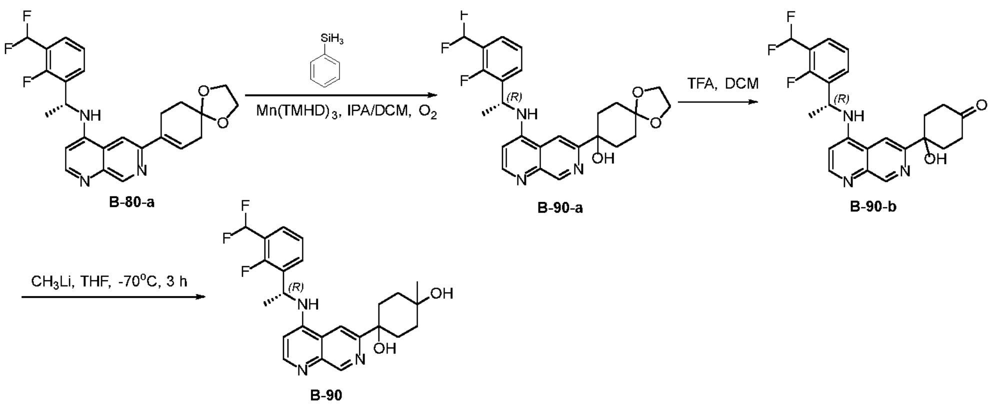


B-80-a B-90-a B-90-b B-90

Step one:

Compound **B-80-a** (290 mg, 0.64 mmol) and Mn(TMHD)$_3$ (80 mg, 0.13 mmol) were dissolved in an eggplant-shaped flask containing IPA/DCM (5 mL/0.5 mL) and stirred at room temperature for 10 minutes, and then phenylsilane (178 mg, 1.6 mmol) was added. Oxygen replacement was performed. The reaction was performed at 30°C for 4 hours. After the reaction was completed, the reaction solution was concentrated under reduced pressure. The residue was purified by a silica gel column to obtain a yellow solid compound **B-90-a** (177 mg, yield of 59%). LCMS (ESI): *m*/*z* = 474.2 $[M+H]^+$

Step two:

Compound **B-90-a** (177 mg, 0.37 mmol) was dissolved in a three neck flask containing dichloromethane (3 mL), and TFA (1 mL) was dropwise added and reacted at room temperature for 1 hour. After the reaction was completed, the pH of the reaction solution was adjusted using a sodium bicarbonate aqueous solution to be 8 and the reaction solution was extracted with ethyl acetate. The organic phase was concentrated under reduced pressure to obtain a yellow oily compound **B-90-b** (130 mg, yield of 86%). LCMS (ESI): *m*/*z* = 430.2 $[M+H]^+$

Step three:

Compound **B-90-b** (50 mg, 0.12 mmol) was dissolved in a three neck flask containing THF (3 mL), and after nitrogen displacement, $CH_3Li$ (0.15 mL, 0.24 mmol) was added at -78°C and reacted at -78°C for 3 hours. After the reaction was completed, the reaction solution was extracted with dichloromethane and concentrated. The residue was purified by prep-HPLC to obtain to cis-trans-isomers compound **B-90-P1** and compound **B-90-P2** (P1: 8.2 mg, P2: 5.0 mg, total yield of 25%) as white solids. LCMS (ESI): *m*/*z* = 446.2 $[M+H]^+$; **B-90-P1:** $^1$H NMR (400 MHz, DMSO-$d_6$): $\delta$ 9.08 (s, 1H), 8.60 (s, 1H), 8.37 (d, *J* = 5.3 Hz, 1H), 7.84 (d, *J* = 7.0 Hz, 1H), 7.62 (t, *J* = 7.4 Hz, 1H), 7.53 (t, *J* = 7.1 Hz, 1H), 7.42-7.12 (m, 2H), 6.29 (d, *J* = 5.4 Hz, 1H), 5.16-5.06 (m, 1H), 5.04 (s, 1H), 4.28 (s, 1H), 2.19 (q, *J* = 13.3 Hz, 2H), 1.92 (t, *J* = 11.7 Hz, 2H), 1.68 (d, *J* = 6.7 Hz, 5H), 1.42 (d, *J* = 12.5 Hz, 2H), 1.21 (s, 3H). **B-90-P2:** $^1$H NMR (400 MHz, DMSO-$d_6$): $\delta$ 9.07 (s, 1H), 8.57 (s, 1H), 8.37 (d, *J* = 5.3 Hz, 1H), 7.87 (d, *J* = 7.1 Hz, 1H), 7.62 (t, *J* = 7.4 Hz, 1H), 7.53 (t, *J* = 7.1 Hz, 1H), 7.41-7.12 (m, 2H), 6.29 (d, *J* = 5.4 Hz, 1H), 5.10 (t, *J* = 6.9 Hz, 1H), 4.92 (s, 1H), 3.98 (s, 1H), 2.44-2.38 (m, 2H), 1.90-1.77 (m, 2H), 1.68 (d, *J* = 6.8 Hz, 3H), 1.48 (d, *J* = 11.7 Hz, 4H), 1.19 (s, 3H).

**Example 66 Synthesis of compound B-91**

Synthetic route:

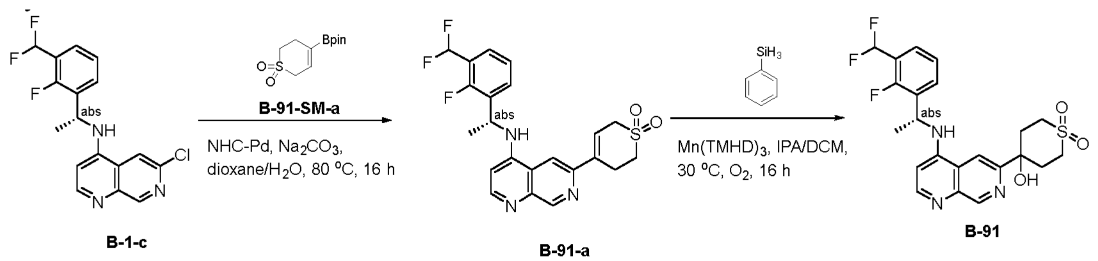


Step one:

To a dry flask, compound **B-1-c** (91 mg, 0.26 mmol), 1,4-dioxane/water (10 mL, v/v = 5: 1), compound **B-91-SM-a** (80 mg, 0.31 mmol), cesium carbonate (55 mg, 0.51 mmol), and NHC-Pd (77 mg, 0.10 mmol) were successively added. The reaction was performed while stirring overnight at 80°C under nitrogen protection. After the reaction was completed, the reaction solution was cooled and concentrated under reduced pressure. The residue was purified by a rapid column chromatography to obtain a yellow solid **B-91-a** (166 mg, yield of 95%). LCMS (ESI): *m/z* = 448.1 $[M+H]^+$

Step two:

To a dry flask, compound **B-91-a** (140 mg, 0.32 mmol), isopropanol/dichloromethane (10 mL, v/v = 10:1), and Mn(TMHD)$_3$ (38 mg, 0.05 mmol) were successively added and stirred for 5 minutes. Phenylsilane (85 mg, 0.81 mmol) was added. The reaction was performed while stirring overnight at 30°C under oxygen protection. After the reaction was completed, the reaction solution was concentrated under reduced pressure. The residue was firstly purified by the rapid column chromatography to obtain **B-91** (18.2 mg, yield of 18%). LCMS (ESI): *m/z* = 466.1 $[M+H]^+$; $^1H$ NMR (400 MHz, $CD_3OD$): $\delta$ 9.11 (s, 1H), 8.55 (d, *J* = 0.8 Hz, 1H), 8.35 (d, *J* = 5.5 Hz, 1H), 7.52 (m, 2H), 7.21 (t, *J* = 7.7 Hz, 1H), 7.02 (t, *J* = 54.7 Hz, 1H), 6.39 (d, *J* = 5.6 Hz, 1H), 5.13-5.16 (m, 1H), 3.60 (t, *J* = 14.5 Hz, 2H), 3.16-3.00 (m, 2H), 2.89-2.93 (m, 2H), 2.35-2.10 (m, 2H), 1.74 (d, *J* = 6.8 Hz, 3H).

The synthesis of the following compounds was the same as that of **B-91**.

| No. | Structure | Character | LCMS (ESI): *m/z* $[M+H]^+$ | $^1H$ NMR |
|---|---|---|---|---|
| **B-99** | 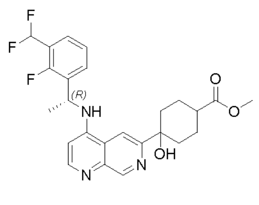 | White solid | 474.0 | $^1H$ NMR (400 MHz, DMSO-$d_6$): $\delta$ 9.08 (d, *J* = 1.5 Hz, 1H), 8.61 (d, *J* = 17.0 Hz, 1H), 8.39 (d, *J* = 5.3 Hz, 1H), 7.88 (t, *J* = 6.1 Hz, 1H), 7.63 (s, 1H), 7.54 (t, *J* = 6.8 Hz, 1H), 7.29 (dt, *J* = 58.2, 44.7 Hz, 2H), 6.30 (dd, *J* = 5.5, 2.6 Hz, 1H), 5.19 (s, 1H), 5.12 (d, *J* = 9.3 Hz, 1H), 3.65 (s, 3H), 2.22-1.87 (m, 4H), 1.87-1.75 (m, 3H), 1.66 (t, *J* = 15.7 Hz, 5H). |
| **B-103** | 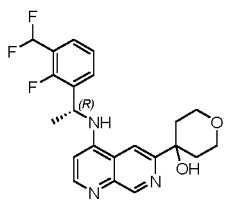 | White solid | 418.2 | $^1H$ NMR (400 MHz, $CD_3OD$) $\delta$ 9.13 (s, 1H), 8.48 (s, 1H), 8.36 (d, *J* = 5.5 Hz, 1H), 7.60-7.42 (m, 2H), 7.23 (t, *J* = 7.7 Hz, 1H), 7.04 (t, *J* = 54.8 Hz, 1H), 6.40 (d, *J* = 5.5 Hz, 1H), 5.18 (q, *J* = 6.7 Hz, 1H), 4.09-3.87 (m, 4H), 2.48 (td, *J* = 13.0, 5.0 Hz, 2H), 1.79-1.70 (m, 5H). |

**Example 67 Synthesis of compound B-92** (mixture of cis-trans-isomers compound **B-92-P1** and compound **B-92-P2**)

Synthetic route:

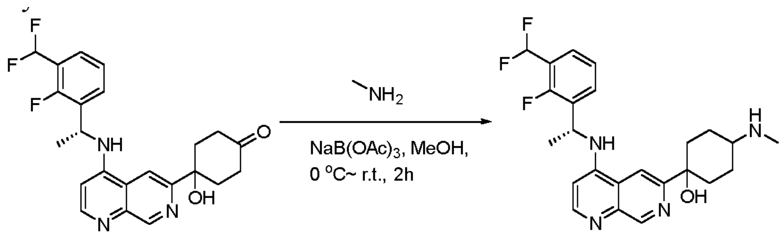


B-90-b B-92

Step one:

Compound **B-90-b** (70 mg, 0.16 mmol) was dissolved in MeOH (3 mL), a methylamine solution (0.81 mL, 1.6 mmol, 2.0 mol) was added at 0°C and stirred for 30 min under nitrogen protection, and then $NaB(OAc)_3$ (69.0 mg, 0.32 mmol) was added and reacted at room temperature for 2 hours under nitrogen protection. After the reaction was completed, the reaction solution was extracted with a saturated ammonium chloride solution and EA. The organic phase was backwashed with a saturated salt solution, dried over sodium sulfate, filtered, and concentrated under reduced pressure. The residue was subjected to a formic acid reverse-phase preparative purification to obtain cis-trans-isomers compound B-92-P1 and compound B-92-P2 (P1: 3.5 mg, P2: 14.1 mg, white solid) with the yield of P1: 4.8% and P2: 19.5%. LCMS (ESI): *m/z* = 445 $[M+H]^+$; **B-92-P1:** $^1H$ NMR (400 MHz, $CD_3OD$) : $\delta$ 9.14 (s, 1H), 8.57 (s, 1H), 8.39 (d, *J* = 5.4 Hz, 1H), 7.59-7.49 (m, 2H), 7.23 (t, *J* = 7.7 Hz, 1H), 7.03 (t, *J* = 54.8 Hz, 1H), 6.43 (d, *J* = 5.6 Hz, 1H), 5.18 (q, *J* = 6.8 Hz, 1H), 3.29-3.26 (m, 1H), 2.71 (s, 3H), 2.60 (t, *J* = 9.7 Hz, 2H), 2.25 (dd, J = 13.0, 6.7 Hz, 2H), 1.86-1.73 (m, 7H). **B-92-P2**: $^1H$ NMR (400 MHz, $CD_3OD$) : $\delta$ 9.11 (s, 1H), 8.51 (s, 1H), 8.37 (d, *J* = 5.5 Hz, 1H), 7.60-7.48 (m, 2H), 7.23 (t, *J* = 7.7 Hz, 1H), 7.03 (t, J = 54.8 Hz, 1H), 6.42 (d, *J* = 5.6 Hz, 1H), 5.18 (q, *J* = 6.7 Hz, 1H), 3.20 (dd, *J* = 13.0, 9.8 Hz, 1H), 2.77 (s, 3H), 2.30 (t, *J* = 12.6 Hz, 2H), 2.05 (dt, *J* = 24.7, 11.1 Hz, 6H), 1.76 (d, *J* = 6.8 Hz, 3H).

Referring to the synthesis of compound **B-92**, isopropylamine, dimethylamine, piperazine, N-methylpiperazine were used respectively to replace methylamine in the original route to synthesize the following compounds: **B-93**, **B-94**, **B-97**, and **B-98.**

| No. | Structure | Character | LCMS (ESI): *m/z* $[M+H]^+$ | $^1H$ NMR |
|---|---|---|---|---|
| **B-93** | 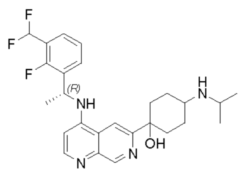 | Yellow solid | 473.2 | $^1H$ NMR (400 MHz, $CD_3OD$): $\delta$ 9.13 (d, *J* = 17.0 Hz, 1H), 8.55 (d, *J* = 36.5 Hz, 1H), 8.42-8.28 (m, 1H), 7.60-7.44 (m, 2H), 7.23 (t, *J* = 7.7 Hz, 1H), 7.03 (t, *J* = 54.8 Hz, 1H), 6.43 (t, *J* = 5.9 Hz, 1H), 5.19 (t, *J* = 6.8 Hz, 1H), 3.67-3.33 (m, 2H), 2.78-2.15 (m, 3H), 2.13-1.92 (m, 5H), 1.76 (d, *J* = 6.8 Hz, 3H), 1.36 (dd, *J* = 11.3, 6.5 Hz, 6H). |

(continued)

| No. | Structure | Character | LCMS (ESI): *m/z* $[M+H]^+$ | $^1$H NMR |
|---|---|---|---|---|
| B-94 (mixture of cis-trans-isomers B-94-P1 and B-94-P2) | 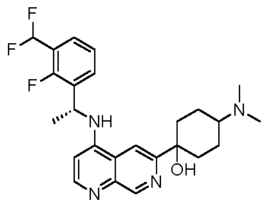 | White solid | 459.0 | **B-94-P1:** $^1$H NMR (400 MHz, $CD_3OD$) : $\delta$ 9.15 (s, 1H), 8.59 (s, 1H), 8.38 (d, *J* = 5.5 Hz, 1H), 7.55 (dt, *J* = 18.9, 7.1 Hz, 2H), 7.23 (t, *J* = 7.7 Hz, 1H), 7.04 (t, *J* = 54.8 Hz, 1H), 6.42 (d, *J* = 5.6 Hz, 1H), 5.19 (q, *J* = 6.7 Hz, 1H), 4.59 (s, 1H), 2.78 (d, *J* = 16.0 Hz, 8H), 2.19 (t, *J* = 11.0 Hz, 2H), 1.88 (d, *J* = 9.9 Hz, 4H), 1.77 (d, *J* = 6.8 Hz, 3H).<br>**B-94-P2:** $^1$H NMR (400 MHz, $CD_3OD$): $\delta$ 9.11 (s, 1H), 8.51 (s, 1H), 8.37 (d, *J* = 5.5 Hz, 1H), 7.58-7.48 (m, 2H), 7.23 (t,J = 7.9 Hz, 1H), 7.04 (t, *J* = 54.7 Hz, 1H), 6.41 (d,, *J* = 5.7 Hz, 1H), 5.18 (q, *J* = 7.2 Hz, 1H), 4.58 (s, 1H), 2.84 (s, 6H), 2.36-2.26 (m, 2H), 2.05 (dt, *J* = 22.5, 10.7 Hz, 6H), 1.76 (d, *J* = 6.8 Hz, 3H). |
| B-97 (mixture of cis-trans-isomers B-97-P1 and B-97-P2) | 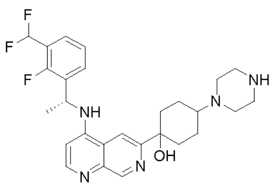 | White solid | 500.0 | **B-97-P1:** $^1$H NMR (400 MHz, $CD_3OD$): $\delta$ 9.15 (s, 1H), 8.55 (s, 1H), 8.38 (d, *J* = 5.2 Hz, 1H), 7.55 (dt, *J* = 18.9, 7.0 Hz, 2H), 7.24 (t, *J* = 7.7 Hz, 1H), 7.03 (t, *J* = 54.8 Hz, 1H), 6.45 (d, *J* = 5.5 Hz, 1H), 5.21 (dd, *J* = 13.0, 6.2 Hz, 1H), 3.24 (s, 4H), 2.79 (s, 4H), 2.75-2.66 (m, 2H), 2.54 (s, 1H), 2.03 (t, *J* = 9.7 Hz, 2H), 1.74 (dd, *J* = 28.8, 8.8 Hz, 7H).<br>**B-97-P2**: $^1$H NMR (400 MHz, $CD_3OD$): $\delta$ 9.10 (s, 1H), 8.49 (d, *J* = 3.7 Hz, 1H), 8.37 (d, *J* = 5.5 Hz, 1H), 7.60-7.49 (m, 2H), 7.23 (t, *J* = 7.7 Hz, 1H), 7.03 (t, *J* = 54.8 Hz, 1H), 6.42 (d, *J* = 5.6 Hz, 1H), 5.19 (q, *J* = 6.7 Hz, 1H), 3.23 (s, 4H), 2.96 (s, 4H), 2.71 (t, *J* = 11.2 Hz, 1H), 2.24 (t, *J* = 11.6 Hz, 2H), 1.96 (dd, *J* = 23.4, 13.1 Hz, 6H), 1.76 (d, *J* = 6.8 Hz, 3H). |
| **B-98** | 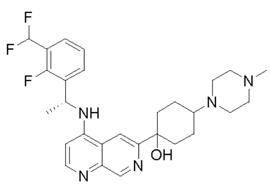 | White solid | 514.0 | $^1$H NMR (400 MHz, $CD_3OD$): $\delta$ 9.13 (d, *J* = 21.0 Hz, 1H), 8.48 (d, *J* = 7.6 Hz, 1H), 8.35 (d, *J* = 5.5 Hz, 1H), 7.60-7.47 (m, 2H), 7.23 (t, *J* = 7.7 Hz, 1H), 7.04 (t, *J* = 54.8 Hz, 1H), 6.39 (d, *J* = 5.5 Hz, 1H), 5.17 (q, *J* = 6.7 Hz, 1H), 2.64 (ddd, *J* = 26.6, 20.9, 11.4 Hz, 9H), 2.34-2.17 (m, 5H), 1.97-1.71 (m, 9H). |

**Example 68 Synthesis of compound B-95** (mixture of cis-trans-isomers compound **B-95-P1** and compound **B-95-P2**)

Synthetic route:

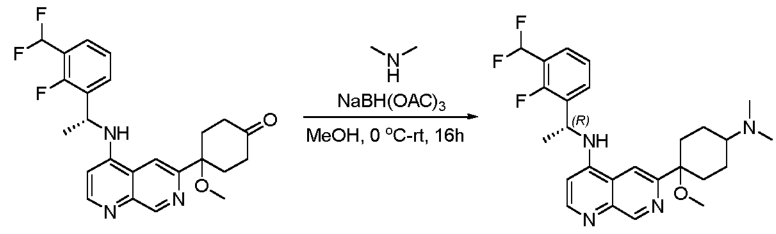


B-82-e B-95

Step one:

Dimethylamine (0.7 mL, 1.35 mmol, 2.0 M in THF) was added to a methanol (6 mL) solution dissolved with compound **B-82-e** (60 mg, 0.13 mmol) at 0°C and reacted at 0°C for 30 minutes under nitrogen protection. $NaBH(OAc)_3$(57 mg, 0.27 mmol) was then added and reacted overnight at room temperature. After the reaction was completed, the reaction solution was concentrated under reduced pressure to remove the solvent. The residue was dissolved with ethyl acetate. The organic phase was backwashed with a saturated salt solution, dried over sodium sulfate, filtered, and concentrated under reduced pressure. The residue was subjected to a reverse-phase preparative purification to obtain cis-trans-isomers compound **B-95-P1** (4.7 mg, white solid, yield of 7.5%) and compound **B-95-P2** (18.5 mg, white solid, yield of 28%). **B-95-P1:** LCMS (ESI): *m/z* = 473.1 $[M+H]^+$; $^1H$ NMR (400 MHz, CD3OD): $\delta$ 9.18 (s, 1H), 8.55 (d, *J* = 11.3 Hz, 2H), 8.39 (d, *J* = 5.5 Hz, 1H), 7.55 (dt, *J* = 16.1, 7.3 Hz, 2H), 7.24 (t, J = 7.7 Hz, 1H), 7.04 (t, *J* = 54.8 Hz, 1H), 6.43 (d, J = 5.6 Hz, 1H), 5.19 (q, *J* = 6.8 Hz, 1H), 3.06-3.01 (m, 4H), 2.99-2.91 (m, 2H), 2.63 (s, 6H), 2.18-2.08 (m, 2H), 1.96-1.85 (m, 2H), 1.77 (d, *J*= 6.8 Hz, 3H), 1.61 (d, J = 9.7 Hz, 2H). **B-95-P1**: LCMS (ESI): *m/z* = 473.1 $[M+H]^+$; $^1H$ NMR (400 MHz, $CD_3OD$): $\delta$ 9.13 (s, 1H), 8.41 (s, 1H), 8.37 (d, *J* = 5.5 Hz, 1H), 7.54 (dt, *J* = 14.7, 7.2 Hz, 2H), 7.24 (t, *J* = 7.7 Hz, 1H), 7.04 (t, *J* = 54.8 Hz, 1H), 6.40 (d, *J* = 5.6 Hz, 1H), 5.18 (q, *J* = 6.8 Hz, 1H), 3.11 (s, 3H), 2.50-2.39 (m, 9H), 2.05-1.89 (m, 4H), 1.86-1.79 (m, 2H), 1.77 (d, *J* = 6.8 Hz, 3H).

**Example 69 Synthesis of compound B-96** (mixture of cis-trans-isomers compound **B-96-P1** and compound **B-96-P2**)

Synthetic route:

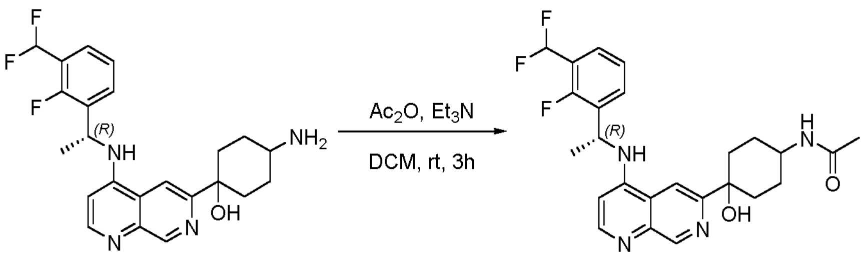


B-96-a B-96

Step one:

Compound **B-96-a** (60 mg, 0.139 mmol) and triethylamine (28 mg, 0.277 mmol) were dissolved in 3 mL of dichloromethane, and acetic anhydride (14 mg, 0.137mmol) was added and reacted at room temperature for 3 hours. After the reaction was completed, water was added to the reaction solution. Then the reaction solution was extracted with dichloromethane. The organic phase was backwashed with a saturated salt solution, dried over sodium sulfate, filtered, and concentrated under reduced pressure. The residue was subjected to a preparative purification for chiral resolution of a mixture of cis-trans-isomers, B-96, to obtain compound **B-96-P1** (12.4 mg, white solid) and compound **B-96-P2** (3.7 mg, white solid) with the total yield of 24.4%. LCMS (ESI): m/z = 473.0 $[M+H]^+$; **B-96-P1:** $^1H$ NMR (400 MHz, $CD_3OD$): $\delta$ 9.11 (s, 1H), 8.46 (s, 1H), 8.35 (d, *J* = 5.5 Hz, 1H), 7.62-7.41 (m, 2H), 7.23 (t, *J*= 7.7 Hz, 1H), 7.04 (t, *J* = 54.8 Hz, 1H), 6.40 (d, *J* = 5.7 Hz, 1H), 5.18 (q, *J* = 6.8 Hz, 1H), 3.83 (s, 1H), 2.31-2.20 (m, 2H), 1.96 (s, 3H), 1.89 (d, *J* = 5.7 Hz, 6H), 1.76 (d, *J* = 6.8 Hz, 3H). **B-96-P2:** $^1H$ NMR (400 MHz, $CD_3OD$): $\delta$ 9.14 (s, 1H), 8.53 (s, 1H), 8.37 (d, *J* = 5.5 Hz, 1H), 7.69-7.39 (m, 2H), 7.23 (t, *J* = 7.7 Hz, 1H), 7.04 (t, *J* = 54.8 Hz, 1H), 6.42 (d, *J* = 5.6 Hz, 1H), 5.18 (q, *J* = 6.8 Hz, 1H), 4.09 (s, 1H), 2.54 (t, *J* = 10.5 Hz, 2H), 2.14-2.04 (m, 2H), 1.99 (s, 3H), 1.79-1.61 (m, 7H).

**Example 70 Synthesis of compound B-100**

Synthetic route:

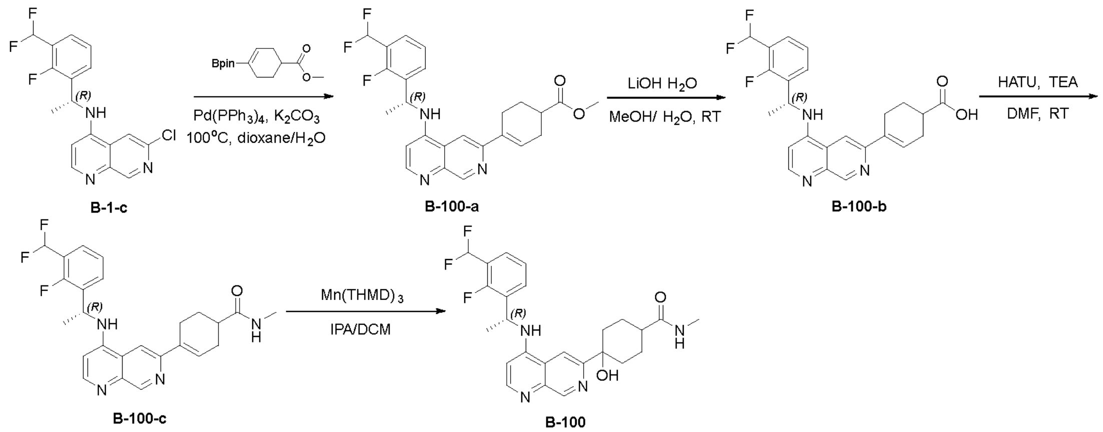


B-1-c B-100-a B-100-b B-100-c B-100

Step one:

To a dry flask, compound **B-1-c** (200 mg, 0.62 mmol), 4-(4,4,5,5-tetramethyl-1,3,2-dioxaboran-2-yl)cyclohexyl-3-olefine acid (296 mg, 1.24 mmol), $Pd(PPh_3)_4$ (136 mg, 0.13 mmol), and $K_2CO_3$ (232 mg, 1.86 mmol) were added, dissolved in 1,4-dioxane/water (10 mL/2 mL), reacted overnight at 100°C under nitrogen protection, cooled, concentrated under reduced pressure, and eluted with PE/EA= 1/8 for purification to obtain a white solid **B-100-a** (220 mg, yield of 81%). LCMS (ESI): *m*/*z* = 456.0 $[M+H]^+$

Step two:

To a dry flask, compound **B-100-a** (210 mg, 0.47 mmol) and lithium hydroxide (76 mg, 1.88 mmol) were added, dissolved in methanol/water (10 mL/5 mL), reacted overnight at room temperature, concentrated under reduced pressure, and eluted by EA = 100% for purification to obtain a yellow oily compound **B-100-b** (200 mg, yield of 97%). LCMS (ESI): *m*/*z* = 442.0 $[M+H]^+$.

Step three:

To a dry flask, compound **B-100-b** (200 mg, 0.45 mmol), methylamine hydrochloride (30 mg, 0.45 mmol), HATU (256 mg, 0.68 mmol), and TEA (182 mg, 1.8mmol) were added, dissolved in 10 mL of DMF, reacted overnight at room temperature, washed with 50 mL of a saturated aqueous ammonium chloride solution three times, and extracted with ethyl acetate. The organic phase was concentrated under reduced pressure to obtain a white solid **B-100-c** (200 mg, yield: 98%). LCMS (ESI): *m*/*z* = 455.0 $[M+H]^+$.

Step three:

To a dry flask, compound **B-100-c** (200 mg, 0.44 mmol) and $Mn(THMD)_3$ (55 mg, 0.09 mmol) were added, dissolved in isopropanol/dichloromethane 5 mL/0.5 mL, and stirred at room temperature for 5 min, phenylsilane (119 mg, 0.11mmol) was added and reacted at 30°C for 1 hour. The reaction solution was concentrated under reduced pressure and purified to obtain cis-trans-isomers B-100-P1 and B-100-P2 as white solids (10 mg, yield of 5%). LCMS (ESI): *m*/*z* = 473.0 $[M+H]^+$; **B-100-P1:** $^1H$ NMR (400 MHz, $CD_3OD$) $\delta$ 9.15 (s, 1H), 8.48 (s, 1H), 8.36 (d, *J* = 5.6 Hz, 1H), 7.61-7.47 (m, 2H), 7.23 (t, *J* = 7.7 Hz, 1H), 7.04 (t, *J* = 54.8 Hz, 1H), 6.41 (d, *J* = 5.7 Hz, 1H), 5.19 (d, *J* = 6.8 Hz, 1H), 2.84-2.74 (m, 2H), 2.70 (d, *J* = 4.0 Hz, 3H), 2.48-2.41 (m, 1H), 1.96 (dd, *J* = 11.3, 4.8 Hz, 2H), 1.79 (dd, J = 16.2, 9.1 Hz, 7H); **B-100-P2:** $^1H$ NMR(400 MHz, $CD_3OD$) $\delta$ 9.10 (s, 1H), 8.49 (s, 1H), 8.36 (d, *J* = 5.5 Hz, 1H), 7.60-7.50 (m, 2H), 7.23 (t, *J* = 7.8 Hz, 1H), 7.04 (t, *J* = 54.8 Hz, 1H), 6.40 (d, *J* = 5.7 Hz, 1H), 5.23-5.14 (m, 1H), 2.76 (d, *J* = 3.9 Hz, 3H), 2.36 (t, *J* = 11.9 Hz, 1H), 2.25-2.06 (m, 4H), 1.89-1.75 (m, 7H).

**Example 71 Synthesis of compound B-101** (mixture of cis-trans-isomers compound **B-101-P1** and compound **B-101-P2)**

Synthetic route:

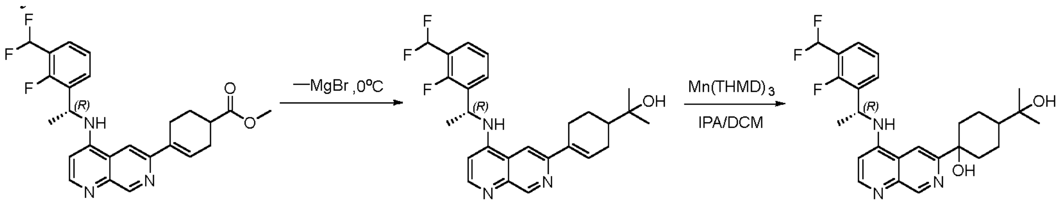


B-100-a B-101-a B-101

Step one:

To a dry flask, compound **B-100-a** (100 mg, 0.22 mmol) was added, 10 mL of methylmagnesium bromide was added under nitrogen protection, and the reaction was performed at 0°C for 3 hours and quenched with a saturated ammonium chloride aqueous solution. The reaction solution was extracted with 50 mL of ethyl acetate three times and eluted by EA = 100% for purification to obtain compound a yellow oily compound **B-101-a** (53 mg, yield of 53%). LCMS (ESI): *m*/*z* = 456.0 $[M+H]^+$

Step two:

To a dry flask, compound **B-101-a** (53 mg, 0.12 mmol) and $Mn(THMD)_3$ (15 mg, 0.02 mmol) were dissolved in isopropanol/dichloromethane 3 mL/0.3 mL and stirred at room temperature for 5 min, and phenylsilane (32 mg, 0.30 mmol) was added and reacted at 30°C for 1 hour. The reaction solution was concentrated under reduced pressure and purified to obtain cis-trans-isomers **B-101-P1** and **B-101-P2: B-101-P1**, white solid, 8.1 mg; and **B-101-P2**, white solid, 5.0 mg. The total yield was 23%. LCMS (ESI): *m*/*z* = 474.0 $[M+H]^+$; **B-101-P1:** $^1H$ NMR (400 MHz, $CD_3OD$) $\delta$ 9.16 (s, 1H), 8.50 (s, 1H), 8.36 (d, J = 5.5 Hz, 1H), 7.60-7.47 (m, 2H), 7.22 (t, *J* = 7.8 Hz, 1H), 7.03 (t, *J* = 54.8 Hz, 1H), 6.39 (d, *J* = 5.6 Hz, 1H), 5.19 (q, *J* = 6.9 Hz, 1H), 2.99 (t, *J* = 9.5 Hz, 2H), 1.91-1.74 (m, 7H), 1.51 (t, *J* = 12.3 Hz, 1H), 1.21 (t, *J* = 12.8 Hz, 2H), 1.06 (s, 6H); **B-101-P2:** $^1H$ NMR (400 MHz, $CD_3OD$) $\delta$ 9.10 (s, 1H), 8.47 (s, 1H), 8.35 (d, *J* = 5.5 Hz, 1H), 7.54 (dt, *J* = 14.3, 7.5 Hz, 2H), 7.23 (t, *J* = 7.7 Hz, 1H), 7.04 (t, *J* = 54.8 Hz, 1H), 6.39 (d, *J* = 5.6 Hz, 1H), 5.18 (q, J = 6.7 Hz, 1H), 2.17 (dt, *J*= 13.3, 6.6 Hz, 2H), 1.91-1.70 (m, 9H), 1.52 (t, *J* = 12.0 Hz, 1H), 1.23 (s, 6H).

Referring to the synthesis of **B-101**, the following compound **B-115** was synthesized by replacing **Int-1** in the original route with R-chiral amine **Int-3.**

| No. | Structure | Character | LCMS (ESI): *m/z* $[M+H]^+$ | $^1$H NMR |
|---|---|---|---|---|
| B-115 (mixture of cis-trans-isomers B-115-P1 and B-115-P2) | 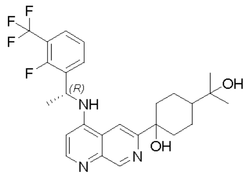 | P1, P2 Both white solid | 492.2 | **B-115-P1:** $^1$H NMR (400 MHz, $CD_3OD$) $\delta$ 9.16 (s, 1H), 8.51 (s, 1H), 8.36-8.38 (d, *J* = 5.6 Hz, 1H), 7.66-7.70 (t, *J* = 7.2 Hz, 1H), 7.59-7.62 (t, *J* = 7.2 Hz, 1H), 7.26-7.29 (t, *J* = 8.0 Hz, 1H), 6.38-6.39 (t, *J* = 5.6 Hz, 1H), 5.20-5.25 (m, 1H), 2.96- 3.02 (m, 2H), 1.83 -1.89 (m, 2H), 1.77-1.82 (m, 5H), 1.48-1.52 (m, 1H), 1.15-1.25 (m, 2H), 1.06 (s, 6H). **B-115-P1:** $^1$H NMR (400 MHz, $CD_3OD$) $\delta$ 9.11 (s, 1H), 8.47 (s, 1H), 8.36-8.37 (d, *J* = 5.6 Hz, 1H), 7.67-7.68 (t, *J* = 7.2 Hz, 1H), 7.58-7.61 (t, *J* = 7.2 Hz, 1H), 7.26-7.29 (t, *J* = 8.0 Hz, 1H), 5.20-5.22 (m, 1H), 2.15-2.20 (m, 2H), 1.83-1.91 (m, |

**Example 72 Synthesis of compound B-102** (mixture of cis-trans-isomers compound **B-102-P1** and compound **B-102-P2**)

Synthetic route:

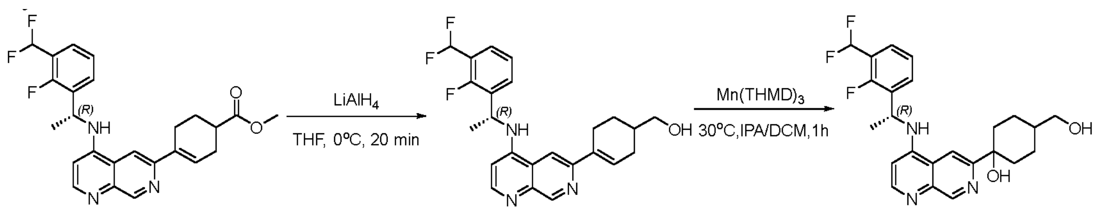


B-100-a B-102-a B-102

Step one:

To a dry flask, compound **B-100-a** (100 mg, 0.22 mmol) was added and dissolved in 5 mL of tetrahydrofuran, and lithium aluminum hydride (17 mg, 0.44 mmol) was added under nitrogen protection and reacted at 0°C for 20 min. The reaction solution was slowly added into water to be quenched, extracted with ethyl acetate, concentrated under reduced pressure, and eluted by EA = 100% for purification to obtain a yellow oily compound **B-102-a** (40 mg, yield of 43%). LCMS (ESI): *m/z* = 428.0 $[M+H]^+$.

Step two:

To a dry flask, compound **B-102-a** (35 mg, 0.08 mmol) and $Mn(THMD)_3$ (11 mg, 0.02 mmol) were added, dissolved in isopropanol/dichloromethane 2 mL/0.2 mL, and stirred at room temperature for 5 min, and phenylsilane (22 mg, 0.20 mmol) was added and reacted at 30°C for 1 hour. The reaction solution was concentrated under reduced pressure and purified to obtain cis-trans-isomers B-102-P1 and B-102-P2 as white solids (10.7 mg, yield of 31%). LCMS (ESI): *m/z* = 446.0 $[M+H]^+$. **B-102-P1:** $^1$H NMR (400 MHz, $CD_3OD$) $\delta$9.18 (s, 1H), 8.53 (s, 1H), 8.40 (d, *J* = 5.5 Hz, 1H), 7.65-7.52 (m, 2H), 7.28 (t, *J* = 7.7 Hz, 1H), 7.00 (d, *J* = 54.7 Hz, 1H), 6.44 (d, *J* = 5.5 Hz, 1H), 5.23 (d, *J* = 7.0 Hz, 1H), 3.58 (d, *J* = 7.0 Hz, 2H), 2.70 (s, 2H), 2.01 (s, 2H), 1.81 (d, *J* = 6.8 Hz, 6H), 1.44 (s, 2H). **B-102-P2:** $^1$H NMR (400 MHz, $CD_3OD$) $\delta$ 9.15 (s, 1H), 8.52 (s, 1H), 8.40 (d, *J*= 5.7 Hz, 1H), 7.66-7.54 (m, 2H), 7.29 (d, *J* = 8.0 Hz, 1H), 6.99 (d, *J* = 54.7 Hz, 1H), 6.44 (d, *J* = 5.6 Hz, 1H), 5.22 (d, *J* = 6.6 Hz, 1H), 3.53 (d, *J* = 5.5 Hz, 2H), 2.22 (d, *J* = 12.2 Hz, 2H), 1.86 (dd, *J* = 30.2, 11.3 Hz, 8H), 1.66 (d, *J* = 10.2 Hz, 2H).

**Example 73 Synthesis of compound B-105**

Synthetic route:

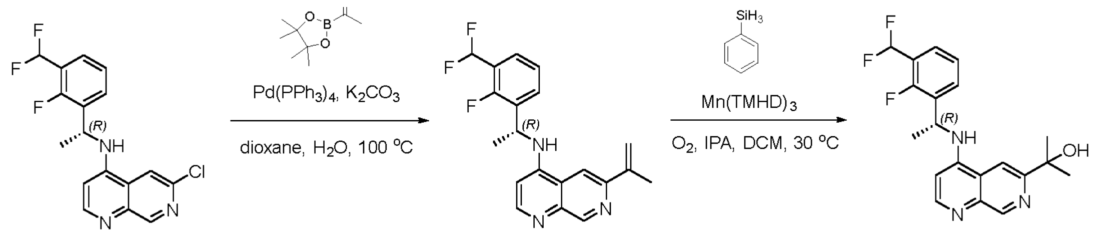


B-1-c B-105-a B-105

Step one:

To a dry sealed tube, compound **B-1-c** (100 mg, 0.28 mmol), propen-2-borate (95.5 mg, 0.57 mmol), Pd(PPh3)4 (66 mg, 0.06 mmol), $K_2CO_3$(118 mg, 0.85 mmol), dioxane (20 mL), and water (4 mL) were successively added. The reaction was performed while stirring at 100°C for 16 hours. The reaction solution was cooled, extracted with ethyl acetate, washed with a saturated salt solution, and dried over anhydrous sodium sulfate. The organic phase was concentrated under reduced pressure and the residue was purified by a column chromatography (petroleum ether:ethyl acetate = 1:1) to obtain a yellow oily compound **B-105-a** (114.8 mg, crude product). LCMS (ESI): *m/z* = 358.1 $[M+H]^+$.

Step two:

To a dry flask, compound **B-105-a** (90 mg, 0.252 mmol), Mn(TMHD)$_3$ (31 mg, 0.050 mmol), IPA (6 mL), and DCM (1.2 mL) were successively added, and stirred at room temperature for 5 minutes. Then phenylsilane (68.1 mg, 0.630 mmol) was added. The reaction was performed while stirring at 30°C for 2 hours under an oxygen environment. After the reaction was completed, the reaction solution was poured into water, filtered, extracted with ethyl acetate, and concentrated. The residue was subjected to a prep-HPLC preparation and freeze-dried to obtain to a white solid product **B-105** (6.5 mg, yield of 7%). LCMS (ESI): *m/z* = 376.2 $[M+H]^+$. $^1H$ NMR (400 MHz, DMSO-$d_6$) $\delta$ 9.06 (s, 1H), 8.57 (s, 1H), 8.38 (s, 1H), 7.85 (d, *J* = 6.8 Hz, 1H), 7.63 (t, *J* = 7.2 Hz, 1H), 7.53 (t, *J* = 6.9 Hz, 1H), 7.42-7.08 (m, 2H), 6.29 (d, *J* = 3.8 Hz, 1H), 5.34 (s, 1H), 5.18-5.01 (m, 1H), 1.67 (d, *J* = 6.6 Hz, 3H), 1.57 (s, 6H).

**Example 74 Synthesis of compound B-106**

Synthetic route:

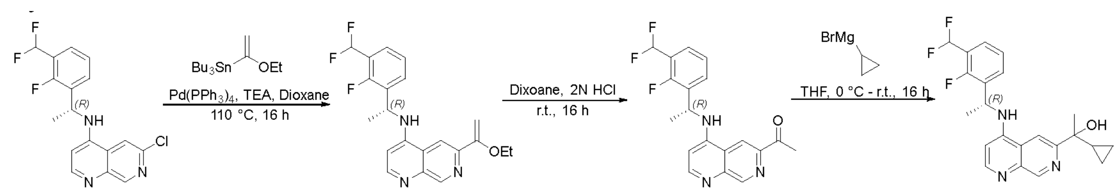


B-1-c B-106-a B-106-b B-106

Step one:

To a dry flask, compound **B-1-c** (200 mg, 0.57 mmol), 1,4-dioxane (16 mL), a compound tin reagent (410 mg, 1.14 mmol), tetrtriphenylphosphine palladium (132 mg, 0.11 mmol), and triethylamine (144 mg, 1.43 mmol) were successively added. The reaction was performed while stirring overnight at 110°C under nitrogen protection. After the reaction was completed, the reaction solution was cooled and concentrated under reduced pressure. The residue was purified by a rapid column chromatography to obtain a brown solid **B-106-a** (130 mg, yield of 59%). LCMS (ESI): *m/z* = 388.2 $[M+H]^+$.

Step two:

To a dry flask, compound **B-106-a** (130 mg, 0.34 mmol), 1,4-dioxane (3 mL), and hydrochloric acid (3 mL, 2 mol/L in $H_2O$) were successively added. The mixture reacted while stirring overnight at room temperature. After the reaction was completed, the reaction solution was concentrated under reduced pressure. The residue was adjusted to be alkaline using a saturated sodium bicarbonate solution and extracted with ethyl acetate. The organic phase was dried over sodium sulfate, filtered, and subjected to rotary evaporation to obtain a brown solid **B-106-b** (100 mg, yield of 92%). LCMS (ESI): *m/z* = 360.1 $[M+H]^+$.

Step three:

To a dry flask, compound **B-106-b** (90 mg, 0.20 mmol) and tetrahydrofuran (4 mL) were successively added, and cyclopropyl magnesium bromide (0.6 mL, 3 mol/L in THF) was dropwise added at 0°C and reacted while stirring overnight at room temperature. After the reaction was completed, the reaction solution was quenched with a saturated ammonium chloride solution and extracted with ethyl acetate. The organic phase was dried over sodium sulfate, filtered, and subjected to rotary evaporation. The residue was purified by prep-HPLC to obtain a white solid **B-106** (4.2 mg, yield of 5.3%). LCMS (ESI): *m/z* = 402.2 $[M+H]^+$. $^1H$ NMR(400 MHz, $CD_3OD$) $\delta$ 9.10 (s, 1H), 8.46 (s, 1H), 8.36 (d, *J*= 5.5 Hz, 1H), 7.60-7.48 (m, 2H), 7.26-7.21 (m, 1H), 7.04 (t, *J* = 54.8 Hz, 1H), 6.41- 6.38 (m, 1H), 5.21-5.15 (m, 1H), 1.76 (d, *J* = 6.8 Hz, 3H), 1.69 (m, 3H), 1.51-1.46 (m, 1H), 0.60-0.57 (m, 1H), 0.51-0.43 (m, 2H), 0.31-0.26 (m, 1H).

**Example 75 Synthesis of compound B-107**

Synthetic route:

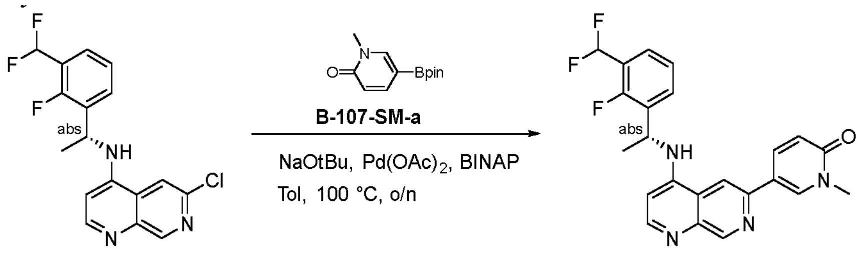


B-1-c B-107

Step one:

Compound **B-1-c** (150 mg, 0.43 mmol) and **B-107-SM-a** (301 mg, 1.38 mmol) were dissolved in toluene (5 mL), and sodium t-butoxide (123 mg, 1.28 mmol), $Pd(OAc)_2$ (9 mg, 0.04 mmol), and BINAP (25 mg, 0.04 mmol) were successively added to the reaction system, then heated to 100°C in a sealed tube under nitrogen protection, and reacted overnight. After the reaction was completed, the reaction solution was cooled and quenched with an ammonium chloride solution and then extracted with ethyl acetate. Then the organic phase was backwashed with a saturated salt solution, dried over sodium sulfate, filtered, and concentrated under reduced pressure. The residue was subjected to a preparative purification to obtain compound **B-107** (5.5 mg, white solid) with the yield of 19%. LCMS (ESI): *m/z* = 425.2 $[M+H]^+$. $^1H$ NMR (400 MHz, $CD_3OD$): $\delta$ 9.19 (d, *J* = 0.7 Hz, 1H), 8.66 (dd, *J* = 10.2, 1.6 Hz, 2H), 8.46 (dd, *J* = 9.5, 2.6 Hz, 1H), 8.39 (d, *J* = 5.5 Hz, 1H), 7.59 (dt, *J* = 13.5, 7.2 Hz, 2H), 7.28 (t, *J* = 7.7 Hz, 1H), 7.07 (t, *J*= 54.8 Hz, 1H), 6.75 (d, *J*= 9.4 Hz, 1H), 6.44 (d, *J*= 5.7 Hz, 1H), 5.23 (m, 1H), 3.76 (s, 3H), 1.82 (d, *J*= 6.8 Hz, 3H).

Referring to the synthesis of **B-107,** the following borates were used: 1-methyl-4-(4,4,5,5-tetramethyl-1,3,2-dioxopentan-2-yl)pyridin-2(1H)-one (cas: 1160790-84-0) and tert-butyl-3-(4,4,5,5-tetramethyl-1,3,2-dioxaborolan-2-yl)-2,5-dihydro-1H-pyrrol-1-formate (cas: 212127-83-8) to replace **B-107-SM-a** in the original route. The following compound **B-108** was synthesized.

| No. | Structure | Character | LCMS (ESI): *m/z* [M+H]+ | $^1$H NMR |
|---|---|---|---|---|
| **B-108** | 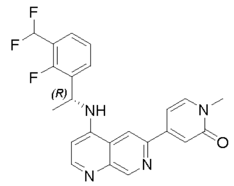 | Yellow solid | 425.1 | $^1$H NMR (400 MHz, $CD_3OD$) δ 9.20 (s, 1H), 8.93 (s, 1H), 8.41 (d, *J* = 5.7 Hz, 1H), 7.79 (d, *J* = 7.1 Hz, 1H), 7.59 (t, *J* = 7.5 Hz, 1H), 7.53 (t, *J* = 7.2 Hz, 1H), 7.43 (d, *J* = 2.0 Hz, 1H), 7.34-7.21 (m, 2H), 7.05 (t, *J* = 54.8 Hz, 1H), 6.47 (d, *J* = 5.7 Hz, 1H), 5.22 (q, *J* = 6.8 Hz, 1H), 3.64 (s, 3H), 1.80 (d, *J* = 6.8 Hz, 3H). |

**Example 76 Synthesis of compound B-111**

Synthetic route:

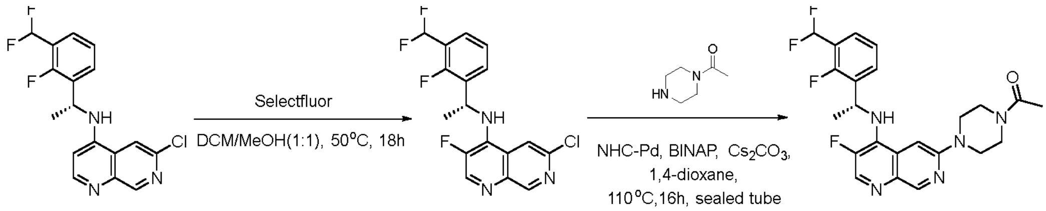


B-1-c B-111-a B-111

Step one:

Compound **B-1-c** (100 mg, 0.285 mmol) and Selectflour (131 mg, 0.370 mmol) were dissolved in a mixed solvent of 2 mL of dichloromethane and 2 mL of methanol, then heated to 50°C, and reacted overnight. After the reaction was cooled, water was added to the reaction solution and then the reaction solution was extracted with dichloromethane. Then the organic phase was backwashed with a saturated salt solution, dried over sodium sulfate, filtered, and concentrated under reduced pressure. The residue was purified by a silica gel plate to obtain compound **B-111-a** (17 mg, green oil) with the yield of 16.1%. LCMS (ESI): *m/z* = 370.0 [M+H]+.

Step two:

Compound **B-111-a** (10 mg, 0.027 mmol) and acetylpiperazine (10.4 mg, 0.081 mmol) were dissolved in 1 mL of a 1,4-dioxane solvent, BINAP (3 mg, 0.005 mmol), cesium carbonate (27 mg, 0.083 mmol), and NHC-Pd (2 mg, 0.003 mmol) were added to the reaction system, and then the reaction system was charged with nitrogen, heated to 110°C, and reacted overnight. After the reaction was completed, the reaction solution was cooled, water was added to the reaction solution to be quenched, and the reaction solution was extracted with ethyl acetate. Then the organic phase was backwashed with a saturated salt solution, dried over sodium sulfate, filtered, and concentrated under reduced pressure. The residue was subjected to a preparative purification to obtain compound **B-111** (1.8 mg, yellow solid) with the yield of 14.4%. LCMS (ESI): *m/z* = 462.1 [M+H]+. 1H NMR (400 MHz, $CD_3OD$) δ 8.86 (s, 1H), 8.14 (d, *J* = 6.0 Hz, 1H), 7.49 (m, 2H), 7.35 (s, 1H), 7.20 (t, *J* = 7.7 Hz, 1H), 6.99 (t, *J* = 54.8 Hz, 1H), 5.62 (d, *J* = 6.7 Hz, 1H), 4.06-3.42 (m, 8H), 2.19 (s, 3H), 1.71 (d, *J* = 6.7 Hz, 3H).

**Example 77 Synthesis of compound B-112**

Synthetic route:

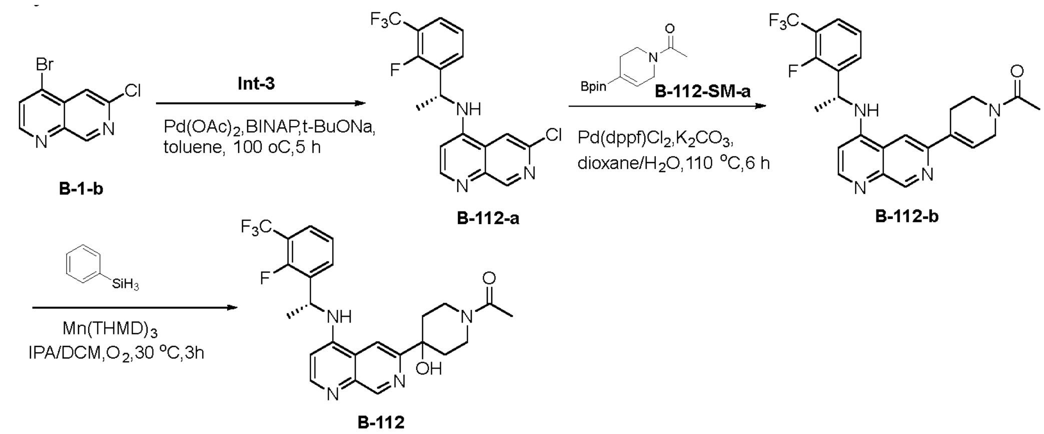


B-1-b B-112-a B-112-b B-112

Step one:

After compound **B-1-b** (200 mg, 0.82 mmol) and **Int-3** (187.4 mg, 0.91 mmol) were dissolved in toluene (10 mL), sodium-t-butoxide (158 mg, 1.65 mmol), BINAP (102.6 mg, 0.16 mmol), and palladium acetate (18.4 mg, 0.082 mmol) were added, nitrogen replacement was performed, and the mixture reacted at 100°C for 5 hours. After the reaction was completed, the reaction solution was cooled, quenched with a saturated ammonium chloride solution, and extracted with EA. The organic phase was backwashed with a saturated salt solution, dried over sodium sulfate, filtered, and concentrated under reduced pressure. The residue was purified by a column chromatography to obtain compound **B-112-a** (259 mg, yellow oil) with the yield of 85.1%. LCMS (ESI): *m/z* = 370 $[M+H]^+$.

Step two:

Compound **B-112-a** (125 mg, 0.34 mmol) and **B-112-SM-a** (169.6 mg, 0.68 mmol) were dissolved in a mixed solution of dioxane/$H_2O$ (2.5 mL/0.5 mL), then potassium carbonate (139.9 mg, 1.02 mmol) and Pd(dppf)$Cl_2$ (73.6 mg, 0.102 mmol) were added, nitrogen replacement was performed, and the reaction was performed at 110°C for 6 hours. After the reaction was completed, the reaction solution was cooled and extracted with a saturated ammonium chloride solution and EA. The organic phase was backwashed with a saturated salt solution, dried over sodium sulfate, filtered, and concentrated under reduced pressure. The residue was purified by a column chromatography to obtain compound **B-112-b** (135 mg, pale brown solid) with the yield of 87.1%. LCMS (ESI): *m/z* =459 $[M+H]^+$.

Step three:

Compound **B-112-b** (85 mg, 0.19 mmol) was dissolved in IPA/DCM (4 mL/0.4 mL), compound Mn$(THMD)_3$ (22.4 mg, 0.038 mmol) was added and stirred at room temperature for 5 minutes, then phenylsilane (50.0 mg, 0.475 mmol) was added, oxygen replacement was performed, and the reaction was performed at 30°C for 3 hours. After the reaction was completed, the reaction solution was extracted with a saturated ammonium chloride solution and DCM. The organic phase was backwashed with a saturated salt solution, dried over sodium sulfate, filtered, and concentrated under reduced pressure. The residue was subjected to a neutral reverse-phase preparative purification to obtain compound **B-112** (25.3 mg, white solid) with the yield of 28.7%. LCMS (ESI): *m/z* = 477 $[M+H]^+$. $^1$H NMR (400 MHz, $CD_3OD$): $\delta$ 9.12 (s, 1H), 8.51 (s, 1H), 8.38 (d, *J* = 5.5 Hz, 1H), 7.68 (t, *J* = 7.2 Hz, 1H), 7.61 (t, *J* = 7.1 Hz, 1H), 7.28 (t, *J* = 7.8 Hz, 1H), 6.39 (d, *J* = 5.6 Hz, 1H), 5.21 (q, *J* = 6.8 Hz, 1H), 4.51 (d, *J* = 13.1 Hz, 1H), 3.91 (d, *J* = 13.7 Hz, 1H), 3.69 (td, J = 13.0, 2.3 Hz, 1H), 3.22 (td, *J* = 12.8, 2.5 Hz, 1H), 2.33 (dtd, *J* = 31.1, 13.2, 4.7 Hz, 2H), 2.18 (s, 3H), 1.85 (td, *J* = 15.1 Hz, 2H), 1.77 (d, *J* = 6.8 Hz, 3H).

**Example 78 Synthesis of compound B-113**

Synthetic route:

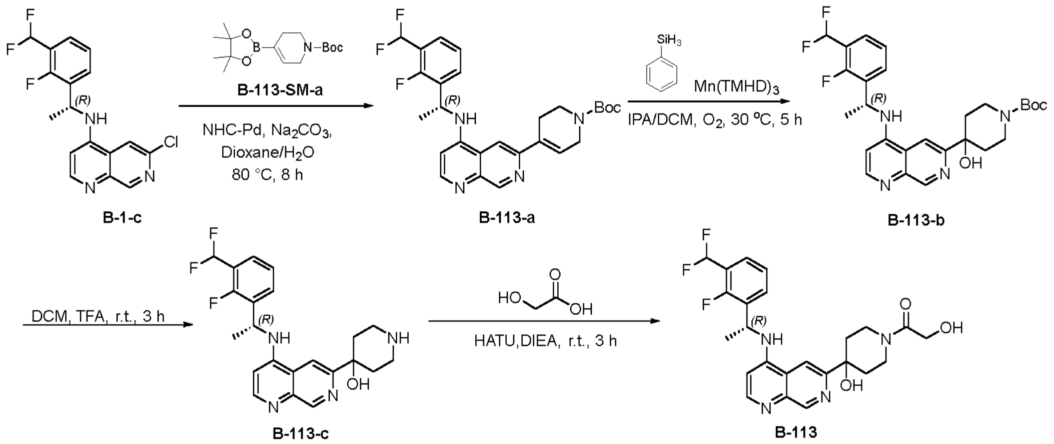


B-1-c B-113-a B-113-b B-113-c B-113

Step one:

To a dry round-bottom flask, compound **B-1-c** (200 mg, 0.57 mmol), compound **B-113-SM-a** (211 mg, 0.68 mmol), sodium carbonate (121 mg, 1.14 mmol), NHC-Pd (78 mg, 0.11 mmol), and dioxane/$H_2O$ (10:1mL) were successively added. The reaction was performed while stirring at 80°C for 8 hours under nitrogen protection. After the reaction was completed, the reaction solution was cooled and concentrated under reduced pressure. The residue was purified by a rapid column chromatography to obtain a yellow oily compound **B-113-a** (260 mg, yield of 80%). LCMS (ESI): *m/z* = 499.2 $[M+H]^+$.

Step two:

To a dry flask, compound **B-113-a** (260 mg, 0.44 mmol) and $Mn(TMHD)_3$ (53 mg, 0.08 mmol) were successively added and reacted in IPA/DCM (5 mL/1 mL) for 5-10 minutes, and then 120 mg of phenylsilane was added to the reaction system and reacted at 30°C for 5 hours under an oxygen atmosphere. After the reaction was completed, the reaction solution was concentrated under reduced pressure to obtain a yellow solid **B-113-b** (136 mg, yield of 60%). LCMS (ESI): *m/z* = 517.2 $[M+H]^+$.

Step three:

To a dry flask, compound **B-113-b** (136 mg, 0.26 mmol) and TFA (1.0 mL) were successively added, placed in DCM (4.0 mL), and stirred for three hours. After the reaction was completed, the reaction solution was concentrated under reduced pressure and purified to obtain a yellow oily compound **B-113-c** (100 mg, yield of 91%). LCMS (ESI): *m/z* = 417.2 $[M+H]^+$.

Step four:

To a dry round-bottom flask, compound **B-113-c** (40 mg, 0.11 mmol), hydroxyacetic acid (11 mg, 0.15 mmol), HATU (53 mg, 0.14 mmol), DIEA (38 mg, 0.28 mmol), and DMF (2.0 mL) were successively added. The reaction was performed while stirring at room temperature for 3 hours. After the reaction was completed, the reaction solution was cooled and concentrated under reduced pressure. The residue was purified by the rapid column chromatography to obtain a yellow oily compound **B-113** (5.6 mg, yield of 12%). LCMS (ESI): *m/z* = 475.2 $[M+H]^+$. $^1H$ NMR (400 MHz, DMSO-$d_6$) $\delta$ 9.04 (s, 1H), 8.58 (s, 1H), 8.35 (d, *J* = 5.3 Hz, 1H), 7.84 (d, *J* = 7.1 Hz, 1H), 7.56-7.58 (m, 1H), 7.50-7.54 (s, 1H), 7.27-7.22 (m, 2H), 6.28 (d, *J* = 5.4 Hz, 1H), 5.46 (s, 1H), 5.05-5.09 (m, 1H), 4.48-4.45 (m, 1H), 4.29-4.32 (m, 1H), 4.10-4.14 (m, 2H), 3.59-3.62 (m, 1H), 3.09-3.11 (m, 1H), 2.24-2.06 (m, 2H), 2.02-1.87 (m, 1H), 1.67-1.70 (m, 2H), 1.63-1.65 (m, 3H).

Referring to the synthesis of **B-113,** the following compounds were used: 2-methoxyacetic acid, 2-cyanoacetic acid, L-lactic acid, D-lactic acid, 2-hydroxyisobutyric acid, N-methylglycine, cyclopropanecarboxylic acid, 2-picolinic acid, tripicolinic acid, 4-picolinic acid, oxetan-3-carboxylic acid, (R)-tetrahydro-3-furoic acid, (S)-tetrahydro-3-furoic acid, tetrahydropyran-4-formic acid, (S)-4-BOC-morpholin-2-carboxylic acid, (R)-4-BOC-morpholin-2-carboxylic acid, 3-pyridineacetic acid, 4-pyridineacetic acid, (S)-tetrahydro-3-furanacetic acid, 2-difluoromethoxyacetic acid, (R)-2-(4-(tert-butoxycarbonyl)morpholin-2-yl)acetic acid, (S)-2-(4-(tert-butoxycarbonyl)morpholin-2-yl)acetic acid, etc. to replace hydroxyacetic acid in the original route. The following compounds were synthesized.

| No . | Structure | Character | LCMS (ESI): *m/z* [M+H]+ | $^1$H NMR |
|---|---|---|---|---|
| **B-114** | 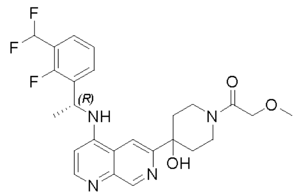 | White solid | 489.2 | $^1$H NMR (400 MHz, $CD_3OD$) $\delta$ 9.12 (s, 1H), 8.50 (s, 1H), 8.36 (d, *J* = 5.5 Hz, 1H), 7.61-7.46 (m, 2H), 7.23 (t, *J* = 7.7 Hz, 1H), 7.04 (t, *J* = 54.8 Hz, 1H), 6.40 (d, *J* = 5.6 Hz, 1H), 5.18 (q, *J* = 6.8 Hz, 1H), 4.49 (d,J= 13.1 Hz, 1H), 4.30-4.16 (m, 2H), 3.86 (d, *J* = 13.4 Hz, 1H), 3.63 (t, *J* = 12.0 Hz, 1H), 3.46 (d, *J* = 13.8 Hz, 3H), 3.25 (d, *J* = 12.9 Hz, 1H), 2.34 (qd, *J* = 13.4, 4.6 Hz, 2H), 1.86 (d, *J* = 13.8 Hz, 2H), 1.76 (d, *J* = 6.8 Hz, 3H). |
| **B-116** | 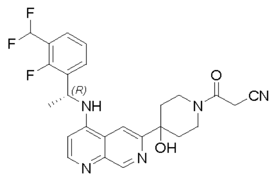 | White solid | 484.1 | $^1$H NMR (400 MHz, CD3OD) $\delta$ 9.08 (s, 1H), 8.63 (s, 1H), 8.39-8.40 (d, *J* = 5.2 Hz, 1H), 7.87-7.89 (d, *J* = 6.8 Hz, 1H), 7.60-7.63 (t, *J* = 7.2 Hz, 1H), 7.51-7.55 (t, *J* = 7.2 Hz, 1H), 7.12-7.39 (m, 2H), 6.31-6.32 (d, *J* = 3.6 Hz, 1H), 5.51 (s, 1H), 5.09-5.12 (m, 1H), 4.31-4.34 (m, 1H), 4.04-4.19 (q, J = 18.4 Hz, 2H), 3.49-3.61 (m, 2H), 3.07-3.13 (t, J = 12.8 Hz, 1H), 2.27-2.33 (m, 1H), 2.15-2.19 (m, 1H), 1.70-1.74 (m, 2H), 1.67- 1.68 (d, J = 6.8, 3H). |
| **B-119** | 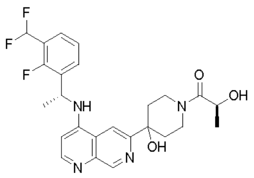 | White solid | 489.2 | $^1$H NMR (400 MHz, $CD_3OD$): $\delta$ 9.12 (s, 1H), 8.50 (d, *J* = 4.0 Hz, 1H), 8.36 (d, *J* = 5.5 Hz, 1H), 7.61-7.39 (m, 2H), 7.23 (t, *J* = 7.7 Hz, 1H), 7.03 (t, *J* = 54.8 Hz, 1H), 6.41 (d, *J* = 5.6 Hz, 1H), 5.18 (q, *J* = 6.8 Hz, 1H), 4.66 (dd, *J* = 6.5, 3.4 Hz, 1H), 4.50 (s, 1H), 4.01 (d, *J* = 13.7 Hz, 1H), 3.66 (d, *J* = 7.0 Hz, 1H), 3.27-3.14 (m, 1H), 2.44-2.26 (m, 2H), 1.88 (s, 2H), 1.76 (d, *J* = 6.8 Hz, 3H), 1.38 (dd, *J* = 13.1, 6.6 Hz, 3H). |
| **B-120** | 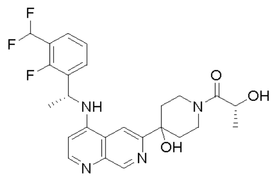 | White solid | 489.2 | $^1$H NMR (400 MHz, DMSO-$d_6$): $\delta$ 9.08 (s, 1H), 8.62 (s, 1H), 8.39 (d, *J* = 5.3 Hz, 1H), 7.88 (d, *J* = 7.0 Hz, 1H), 7.61 (t, *J* = 7.4 Hz, 1H), 7.53 (t, *J* = 6.8 Hz, 1H), 7.40-7.28 (m, 1H), 7.21 (t, *J* = 29.8 Hz, 1H), 6.31 (d, *J* = 5.4 Hz, 1H), 5.48 (s, 1H), 5.10 (p, *J* = 6.7 Hz, 1H), 4.83 (dd, *J* = 14.5, 7.0 Hz, 1H), 4.56-4.44 (m, 1H), 4.42-4.28 (m, 1H), 4.01-3.86 (m, 1H), 3.52-3.41 (m, 1H), 3.09 (t, *J* = 12.5 Hz, 1H), 2.28-2.10 (m, 2H), 1.79-1.69 (m, 2H), 1.68 (d, *J* = 6.8 Hz, 3H), 1.23 (t, *J* = 6.4 Hz, 3H). |
| **B-121** | 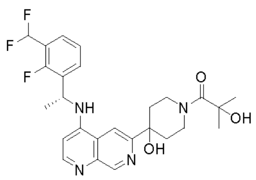 | White solid | 503.2 | $^1$H NMR (400 MHz, $CD_3OD$): $\delta$ 9.11 (s, 1H), 8.50 (d, *J* = 0.9 Hz, 1H), 8.36 (d, *J* = 5.5 Hz, 1H), 7.54 (dt, *J* = 16.5, 7.1 Hz, 2H), 7.24 (d, *J* = 7.8 Hz, 1H), 7.04 (t, *J* = 54.8 Hz, 1H), 6.40 (d, *J* = 5.7 Hz, 1H), 5.18 (br s, 1H), 4.56 (br s, 1H), 3.67 (br s, 1H), 3.63 (br s, 2H), 3.56 (br s, 1H), 2.37 (br s, 2H), 1.85 (d, *J* = 13.6 Hz, 2H), 1.76 (d, *J* = 6.8 Hz, 3H), 1.49 (s, 6H). |

(continued)

| No . | Structure | Chara cter | LCMS (ESI): *m/z* $[M+H]^+$ | $^1$H NMR |
|---|---|---|---|---|
| **B-122** | 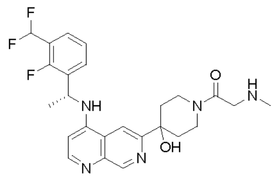 | White solid | 488.2 | $^1$H NMR (400 MHz, $CD_3OD$): $\delta$ 9.11 (d, *J* = 0.8 Hz, 1H), 8.50 (d, *J* = 0.9 Hz, 1H), 8.36 (d, *J* = 5.5 Hz, 1H), 7.54 (dt, *J* = 15.3, 7.2 Hz, 2H), 7.23 (t, *J* = 7.7 Hz, 1H), 7.03 (t, *J* = 54.8 Hz, 1H), 6.41 (d, *J* = 5.6 Hz, 1H), 5.18 (d, *J* = 6.8 Hz, 1H), 4.50 (d, *J* = 13.3 Hz, 1H), 3.82 (d, *J* = 13.6 Hz, 1H), 3.65 (d, *J* = 12.9 Hz, 1H), 3.59 (d, *J* = 6.6 Hz, 2H), 3.24 (s, 1H), 2.47 (d, *J* = 3.3 Hz, 3H), 2.41-2.26 (m, 2H), 1.92-1.81 (m, 2H), 1.76 (d, *J* = 6.8 Hz, 3H). |
| **B-127** | 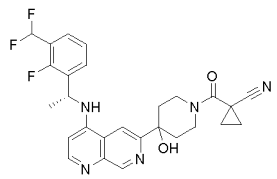 | White solid | 510.0 | $^1$H NMR (400 MHz, $CD_3OD$) $\delta$ 9.13 (s, 1H), 8.49 (s, 1H), 8.36 (d, *J* = 5.5 Hz, 1H), 7.54 (dt, *J* = 18.0, 7.1 Hz, 2H), 7.22 (d, *J* = 7.7 Hz, 1H), 7.04 (t, *J* = 54.8 Hz, 1H), 6.41 (d, *J* = 5.6 Hz, 1H), 5.18 (m, 1H), 3.18-3.05 (m, 2H), 2.82 (s, 3H), 2.80-2.69 (m, 4H), 2.52 (m, 2H), 1.78 (dd, *J* = 17.2, 9.9 Hz, 5H). |
| **B-128** | 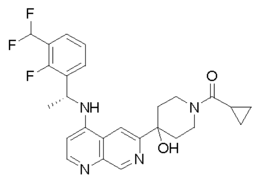 | White solid | 485.1 | $^1$H NMR (400 MHz, DMSO-$d_6$) $\delta$ 9.08 (s, 1H), 8.62 (s, 1H), 8.39 (d, *J* = 5.3 Hz, 1H), 7.88 (d, *J* = 6.9 Hz, 1H), 7.61 (t, *J* = 7.3 Hz, 1H), 7.53 (t, *J* = 7.1 Hz, 1H), 7.42-7.28 (m, 1H), 7.22 (t, *J* = 29.4 Hz, 1H), 6.31 (d, *J* = 5.4 Hz, 1H), 5.49 (s, 1H), 5.20-5.02 (m, 1H), 4.36 (d, *J* = 11.9 Hz, 1H), 4.22 (d, *J* = 12.1 Hz, 1H), 3.57 (t, *J* = 13.1 Hz, 1H), 3.07 (t, *J* = 12.1 Hz, 1H), 2.25 (d, *J* = 13.2 Hz, 1H), 2.16-1.99 (m, 2H), 1.77 (d, *J* = 12.8 Hz, 1H), 1.68 (d, *J* = 6.8 Hz, 4H), 0.79-0.67 (m, 4H). |
| **B-129** | 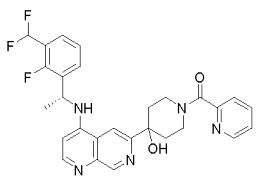 | White solid | 522.2 | $^1$H NMR (400 MHz, $CD_3OD$): $\delta$ 9.13 (d, *J* = 0.9 Hz, 1H), 8.64-8.60 (m, 1H), 8.51 (s, 1H), 8.37 (d, *J* = 5.5 Hz, 1H), 7.99 (td, *J* = 7.8, 1.7 Hz, 1H), 7.66 (dt, *J* = 7.8, 1.1 Hz, 1H), 7.60-7.48 (m, 3H), 7.24 (d, *J* = 7.1 Hz, 1H), 7.04 (t, *J* = 54.8 Hz, 1H), 6.41 (d, *J* = 5.5 Hz, 1H), 5.18 (d, *J* = 6.8 Hz, 1H), 4.66 (d, *J* = 13.2 Hz, 1H), 3.70 (d, *J* = 8.2 Hz, 2H), 3.50-3.44 (m, 1H), 2.53-2.33 (m, 2H), 1.98 (d, *J* = 13.5 Hz, 1H), 1.76 (d, *J* = 6.8 Hz, 4H). |
| B-130 | 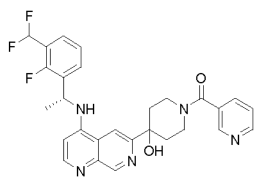 | White solid | 522.0 | $^1$H NMR (400 MHz, $CD_3OD$) : $\delta$ 9.13 (s, 1H), 8.71 (s, 1H), 8.66 (d, *J* = 3.9 Hz, 1H), 8.53 (s, 1H), 8.37 (d, *J* = 5.5 Hz, 1H), 7.99 (d, *J* = 7.9 Hz, 1H), 7.60-7.48 (m, 3H), 7.23 (t, *J*= 7.7 Hz, 1H), 7.04 (t, *J* = 54.8 Hz, 1H), 6.41 (d, *J* = 5.6 Hz, 1H), 5.18 (q, *J* = 6.8 Hz, 1H), 4.66 (d, *J* = 12.6 Hz, 1H), 3.81-3.63 (m, 2H), 3.49 (t, *J* = 12.4 Hz, 1H), 2.52-2.33 (m, 2H), 2.05-1.79 (m, 2H), 1.76 *(d, J* = 6.8 Hz, 3H). |

(continued)

| No . | Structure | Chara cter | LCMS (ESI): *m/z* $[M+H]^+$ | $^1H$ NMR |
|---|---|---|---|---|
| **B-131** | 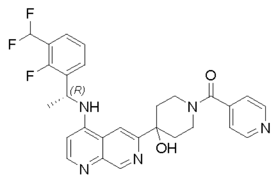 | White solid | 522.0 | $^1H$ NMR (400 MHz, DMSO-$d_6$) δ 9.10 (s, 1H), 8.68 (d, *J* = 5.6 Hz, 2H), 8.63 (s, 1H), 8.39 (d, *J* = 5.3 Hz, 1H), 7.89 (d, *J* = 7.0 Hz, 1H), 7.61 (br s, 1H), 7.53 (br s, 1H), 7.45 (d, *J* = 5.7 Hz, 2H), 7.28 (br s, 1H), 7.27 (d, *J* = 54.4 Hz, 1H), 6.32 (d, *J* = 5.4 Hz, 1H), 5.57 (s, 1H), 5.16-5.05 (m, 1H), 4.49 (d, *J* = 11.6 Hz, 1H), 3.61-3.59 (m, 1H), 3.28-3.26 (m, 2H), 2.21-2.17 (m, 2H), 1.87 (s, 1H), 1.68 (d, *J* = 6.6 Hz, 4H). |
| **B-132** | 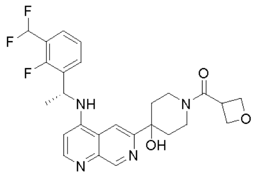 | White solid | 501.2 | 1H NMR (400 MHz, $CD_3OD$) δ 9.13 (d, J = 0.8 Hz, 1H), 8.52 (d, J = 1.0 Hz, 1H), 8.38 (d, J = 5.5 Hz, 1H), 7.76-7.62 (m, 1H), 7.61-7.51 (m, 2H), 7.25 (t, J = 7.7 Hz, 1H), 7.06 (t, J = 54.8 Hz, 1H), 6.42 (d, J = 5.6 Hz, 1H), 5.20 (d, J = 6.8 Hz, 1H), 4.99-4.90 (m, 3H), 4.62-4.49 (m, 2H), 4.30 (ddd, J = 15.5, 8.4, 7.1 Hz, 1H), 3.61 (td, J = 13.0, 2.7 Hz, 1H), 3.56-3.41 (m, 1H), 3.28 (dd, J = 13.0, 2.9 Hz, 1H), 2.39-2.24 (m, 2H), 1.87 (dq, J = 13.7, 2.7 Hz, 2H), 1.78 (d, J = 6.8 Hz, 3H). |
| **B-133** | 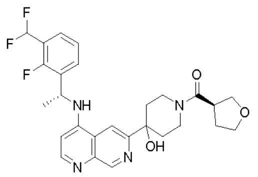 | White solid | 515.1 | $^1H$ NMR (400 MHz, DMSO-$d_6$) δ 9.08 (s, 1H), 8.62 (s, 1H), 8.39 (d, *J* = 5.3 Hz, 1H), 7.87 (d, *J* = 7.1 Hz, 1H), 7.61 (t, *J* = 7.1 Hz, 1H), 7.53 (t, *J* = 7.0 Hz, 1H), 7.41-7.28 (m, 1H), 7.21 (t, *J* = 29.9 Hz, 1H), 6.31 (d, *J* = 5.3 Hz, 1H), 5.48 (s, 1H), 5.17-4.99 (m, 1H), 4.38 (d, *J* = 13.3 Hz, 1H), 3.91 (t, *J* = 8.1 Hz, 2H), 3.80-3.62 (m, 3H), 3.55-3.37 (m, 2H), 3.07 (t, *J* = 11.8 Hz, 1H), 2.24-2.09 (m, 2H), 2.09-2.01 (m, 2H), 1.78-1.69 (m, 2H), 1.67 (d, *J* = 6.7 Hz, 3H). |
| **B-134** | 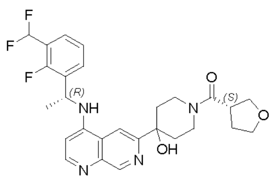 | White solid | 515.1 | $^1H$ NMR (400 MHz, $CD_3OD$) δ 9.11 (d, *J* = 0.9 Hz, 1H), 8.52-8.47 (m, 1H), 8.36 (d, *J* = 5.5 Hz, 1H), 7.54 (dt, *J* = 15.5, 7.3 Hz, 2H), 7.23 (t, *J* = 7.7 Hz, 1H), 7.03 (t, *J* = 54.8 Hz, 1H), 6.41 (d, *J* = 5.6 Hz, 1H), 5.18 (q, *J* = 6.8 Hz, 1H), 4.53 (d, *J* = 13.6 Hz, 1H), 4.10-3.98 (m, 2H), 3.97-3.86 (m, 2H), 3.87-3.79 (m, 1H), 3.71 (d, *J* = 13.3 Hz, 1H), 3.59-3.49 (m, 1H), 3.24 (d, *J* = 12.9 Hz, 1H), 2.41-2.26 (m, 2H), 2.24-2.12 (m, 2H), 1.94-1.81 (m, 2H), 1.76 (d, *J* = 6.8 Hz, 3H). |
| **B-135** | 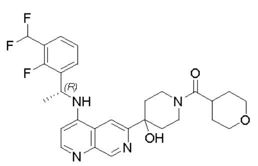 | White solid | 529.1 | $^1H$ NMR (400 MHz, DMSO-$d_6$) δ 9.08 (s, 1H), 8.63 (s, 1H), 8.39 (d, *J* = 5.3 Hz, 1H), 7.90 (d, *J* = 7.1 Hz, 1H), 7.62 (t, *J* = 6.8 Hz, 1H), 7.53 (t, *J* = 7.2 Hz, 1H), 7.42-7.10 (m, 2H), 6.32 (d, *J* = 5.4 Hz, 1H), 5.48 (s, 1H), 5.15-5.06 (m, 1H), 4.41-4.37 (m, 1H), 3.97-3.84 (m, 3H), 3.54-3.31 (m, 3H), 3.07-2.92 (m, 2H), 2.22-2.10 (m, 2H), 1.78-1.54 (m, 9H). |

(continued)

| No . | Structure | Chara cter | LCMS (ESI): *m/z* [M+H]+ | 1H NMR |
|---|---|---|---|---|
| **B-136** | 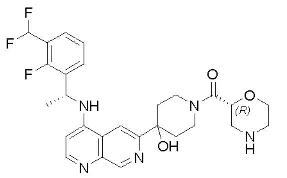 | White solid | 530.2 | 1H NMR (400 MHz, DMSO-$d_6$) δ 9.07 (s, 1H), 8.61 (s, 1H), 8.39 (d, *J* = 5.3 Hz, 1H), 7.88 *(d, J* = 6.4 Hz, 1H), 7.61 (t, *J* = 7.2 Hz, 1H), 7.53 (t, *J* = 6.9 Hz, 1H), 7.41-7.28 (m, 1H), 7.21 (t, *J* = 29.8 Hz, 1H), 6.30 (d, J = 5.3 Hz, 1H), 5.47 (s, 1H), 5.17-5.01 (m, 1H), 4.32 (t, *J* = 11.7 Hz, 1H), 4.23-4.13 (m, 1H), 3.95 (t, *J* = 13.2 Hz, 1H), 3.74 (d, *J* = 10.2 Hz, 1H), 3.56-3.41 (m, 2H), 3.05 (dd, *J* = 19.0, 10.5 Hz, 1H), 2.83-2.74 (m, 2H), 2.71-2.64 (m, 2H), 2.31-1.83 (m, 3H), 1.73 (d, *J* = 11.1 Hz, 2H), 1.67 (d, *J* = 6.8 Hz, 3H). |
| **B-137** | 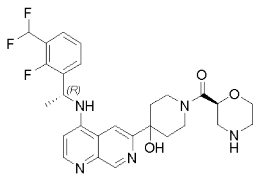 | Yellow oil | 530.0 | 1H NMR (400 MHz, DMSO-$d_6$) δ 9.07 (s, 1H), 8.62 (s, 1H), 8.39 (d, *J* = 5.3 Hz, 1H), 7.88 *(d, J* = 6.9 Hz, 1H), 7.61 (t, *J* = 7.5 Hz, 1H), 7.53 (t, *J* = 7.0 Hz, 1H), 7.43-7.09 (m, 2H), 6.31 (d, *J* = 5.4 Hz, 1H), 5.49 (s, 1H), 5. 09-5.12 (m, 1H), 4.23-4.32 (m, 2H), 3.93-3.95 (m, 1H), 3.75-3.76 (m, 1H), 3.46-3.55 (m, 2H), 3.06-3.08 (m, 1H), 2.83-2.84 (m, 2H), 2.78-2.65 (m, 2H), 2.13-2.18 (m, 3H), 1.72-1.75 (m, 2H), 1.67-1.68 (m, 3H). |
| **B-138** | 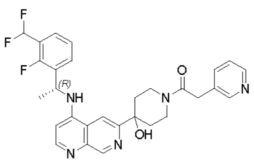 | White solid | 536.2 | 1H NMR (400 MHz, $CD_3OD$) *δ* 9.11 (s, 1H), 8.49 (s, 2H), 8.44 (dd, *J* = 4.9, 1.6 Hz, 1H), 8.36 (d, *J* = 5.5 Hz, 1H), 7.80 (dt, *J* = 8.0, 1.9 Hz, 1H), 7.54 (dt, *J* = 15.6, 7.2 Hz, 2H), 7.43 (dd, *J* = 7.8, 4.9 Hz, 1H), 7.23 (t, *J* = 7.7 Hz, 1H), 7.04 (t, *J* = 54.8 Hz, 1H), 6.41 (d, *J* = 5.5 Hz, 1H), 5.18 (q, *J* = 6.8 Hz, 1H), 4.53 (d, *J* = 14.0 Hz, 1H), 4.06 (d, *J* = 13.5 Hz, 1H), 3.94 (d, *J* = 1.6 Hz, 2H), 3.71 (t, *J* = 12.5 Hz, 1H), 3.26 (dd, *J* = 12.8, 2.8 Hz, 1H), 2.31 (dt, *J* = 17.6, 10.9 Hz, 2H), 1.86 (d, *J* = 13.8 Hz, 2H), 1.76 (d, *J* = 6.8 Hz, 3H). |
| **B-139** | 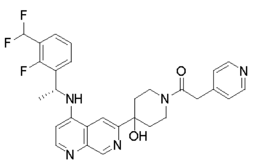 | Light yellow solid | 536.0 | 1H NMR (400 MHz, $CD_3OD$) *δ* 9.11 (s, 1H), 8.50 (d, *J* = 5.8 Hz, 3H), 8.36 (d, *J* = 5.5 Hz, 1H), 7.59-7.47 (m, 2H), 7.40 (d, *J* = 6.0 Hz, 2H), 7.23 (t, *J* = 7.7 Hz, 1H), 7.03 (t, *J* = 54.8 Hz, 1H), 6.41 (d, *J* = 5.6 Hz, 1H), 5.18 (q, *J* = 6.8 Hz, 1H), 4.54 (d, *J* = 13.4 Hz, 1H), 4.05-3.89 (m, 3H), 3.68 (t, *J* = 11.9 Hz, 1H), 3.26 (s, 1H), 2.30 (ddd, *J* = 25.6, 12.9, 5.7 Hz, 2H), 1.90-1.80 (m, 2H), 1.76 (d, *J* = 6.8 Hz, 3H). |
| **B-140** | 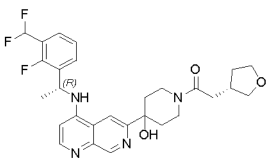 | White solid | 529.2 | 1H NMR (400 MHz, DMSO-$d_6$) δ 8.26 (s, 1H), 7.80 (s, 1H), 7.57 (d, *J* = 5.3 Hz, 1H), 7.05 (d, *J* = 7.1 Hz, 1H), 6.80 (t, *J* = 7.5 Hz, 1H), 6.72 (t, *J* = 7.1 Hz, 1H), 6.58-6.29 (m, 2H), 5.49 (d, *J* = 5.4 Hz, 1H), 4.69-4.61 (m, 1H), 4.36-4.21 (m, 1H), 3.56 (d, *J* = 12.7 Hz, 1H), 3.09-2.98 (m, 2H), 2.79-2.84 (m, 1H), 2.79-2.82 (m, 1H), 2.66 (t, *J* = 12.7 Hz, 1H), 2.48-2.42 (m, 1H), 2.22 (t, *J* = 12.6 Hz, 1H), 1.81-1.70 (m, 2H), 1.67-1.60 (m, 1H), 1.48-1.27 (m, 2H), 1.27-1.16 (m, 1H), 0.91 (d, *J* = 13.5 Hz, 2H), 0.86 (d, *J* = 6.7 Hz, 3H), 0.67-0.73 (m, 1H). |

(continued)

| No . | Structure | Character | LCMS (ESI): *m/z* $[M+H]^+$ | $^1H$ NMR |
|---|---|---|---|---|
| **B-141** | 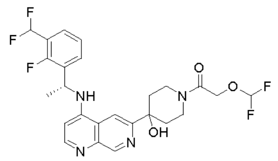 | White solid | 525.2 | $^1H$ NMR (400 MHz, DMSO-$d_6$) δ 9.08 (s, 1H), 8.63 (s, 1H), 8.39 (d, *J* = 5.3 Hz, 1H), 7.88 (d, *J* = 7.1 Hz, 1H), 7.61 (t, *J* = 7.4 Hz, 1H), 7.53 (t, *J* = 6.9 Hz, 1H), 7.43-7.28 (m, 1H), 7.22 (t, *J* = 29.7 Hz, 1H), 6.73 (t, *J* = 76.0 Hz, 1H), 6.31 (d, *J* = 5.4 Hz, 1H), 5.51 (s, 1H), 5.11 (p, *J* = 6.5 Hz, 1H), 4.70 (q, *J* = 14.6 Hz, 2H), 4.33 (d, *J* 12.6 Hz, 1H), 3.63 (d, *J* = 12.8 Hz, 1H), 3.46 (t, *J* = 12.5 Hz, 1H), 3.09 (t, *J* = 12.5 Hz, 1H), 2.34-2.07 (m, 2H), 1.72 (d, *J* = 13.3 Hz, 2H), 1.67 (d, *J* = 6.8 Hz, 3H). |
| **B-142** | 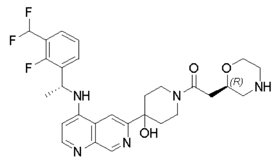 | Light yellow solid | 544.2 | $^1H$ NMR (600 MHz, MeOD) δ 9.352 (s, 1H), 9.15-9.09 (m, 1H), 8.57 (d, J = 6.3 Hz, 1H), 7.77 (s, 1H), 7.62 -7.60 (m, 1H),7.37-7.35 (m, 1H), 7.14-6.96 (m, 1H), 6.91 (d, J = 6.3 Hz, 1H),, 5.531 (s, 1H), 4.57 -4.56 (m, 1H), 4.26 (s, 1H,, 4.11 -4.02(m, 2H), 3.93-3.87 (m, 1H), 3.70 (m, 1H), 3.621 (s, 1H), 3.52-3.48 (m, 1H), 3.26-3.18 (m, 1H), 3.09-3.02 (m, 1H), 2.97-2.88 (m, 1H), 2.77-2.66 (m, 1H),, 2.48-2.23 (m, 1H),, 1.91 (s, 5H). |
| **B-143** | 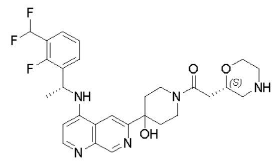 | Light yellow solid | 544.2 | 1H NMR (600 MHz, $CD_3OD$) δ 9.12 (d, J = 3 Hz, 1H), 8.52 (d, J = 10.8 Hz, 1H), 8.38 (d, J = 5.4Hz, 1H), 7.59-7.56 (m, 1H), 7.26 -7.24 (m, 1H), 7.37-7.35 (m, 1H) 6.47 -6.42 (m, 1H), 6.42 (d, J = 5.4 Hz, 1H),, 5.21-5.180 (m, 1H), 4.56 - 4.53 (m, 1H), 4.03-3.69 (m, 2H), 3.90-3.87 (m,1H), 3.71-3.65 (m, 2H), 2.87-2.84 (m, 2H), 2.86-2.76 (m, 1H),, 2.68 -2.63 (m, 1H),, 2.54-2.48 (m, 1H),, 1.90-1.84 (m, 2H),, 1.78 (d, J = 6.6 Hz, 3H). |
| **B-167** | 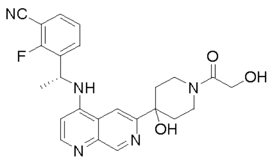 | White solid | 450.2 | 1H NMR (600 MHz, $CD_3OD$): δ 9.14 (s,,1H), 8.53 (s, 1H), 8.40 (d, J = 5.5 Hz, 1H), 7.74 (td, J = 7.6, 1.7 Hz, 1H), 7.70 (ddd, J = 7.8, 6.0, 1.7 Hz, 1H), 7.31 (t, J = 7.8 Hz, 1H), 6.43 (d, J = 5.5 Hz, 1H), 5.21 (q, J = 6.4 Hz, 1H), 4.61 (s,2H) 4.54-4.48 (m, 1H), 4.34 (d, J = 6.4 Hz, 2H), 3.86-3.70 (m, 1H), 3.64 (d, J = 12.8 Hz, 1H), 2.43-2.28 (m, 1H), 1.88 (d, J = 13.6 Hz, 2H), 1.79 (d, J = 6.8 Hz, 3H). |

Similarly, referring to the synthesis of **B-113**, compound **B-123** was synthesized by replacing **B-1-c** in the original route with intermediate **B-111-a**; and compound **B-124** was synthesized by replacing hydroxyacetic acid in the original route with (S)-2-hydroxypropionic acid.

| No. | Structure | Character | LCMS (ESI): *m/z* [M+H]+ | $^1$H NMR |
|---|---|---|---|---|
| **B-123** | 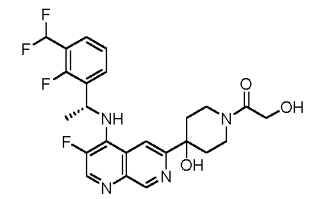 | White solid | 493.2 | 1H NMR (400 MHz, $CD_3OD$): $\delta$ 9.13 (s,,1H), 8.50 (s,,1H), 8.42-8.44 (d,J= 5.2 Hz, 1H), 7.54-7.57 (m, 1H), 7.46-7.49 (m, 1H), 7.19-7.23 (t, *J* = 7.6 Hz, 1H), 6.85-7.12 (t, *J* = 55.0 Hz, 1H), 5.65-5.67 (m, 1H), 4.47-4.56 (m, 1H), 4.30-4.31 (d, *J* = 3.6 Hz, 1H), 3.71-3.74 (m, 1H), 3.56-3.63 (m, 1H), 3.25 (m, 1H), 2.32 (m, 2H), 1.82-1.85 (m, 2H), 1.72 -1.74 (d, *J* = 6.8 Hz, 1H). |
| **B-124** | 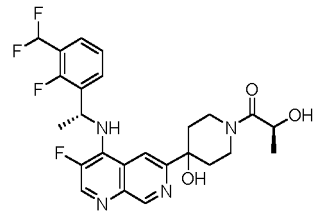 | White solid | 507.2 | 1H NMR (400 MHz, $CD_3OD$): $\delta$ 9.15 (s,1H), 8.49 (s,1H), 8.40-8.44 (d, *J* = 5.2 Hz, 1H), 7.56-7.58 (m, 1H), 7.46-7.49 (m, 1H), 7.20-7.23 (t, *J* = 7.6 Hz, 1H), 6.69-7.13 (t, *J* = 55.0 Hz, 1H), 5.55-5.66 (m, 1H), 4.47-4.56 (m, 1H), 4.30-4.31 (d, *J* = 3.6 Hz, 1H), 3.71-3.74 (m, 1H), 3.56-3.63 (m, 1H), 3.25 (m, 1H), 1.82-1.85 (m, 2H), 1.72 -1.74 (d, *J* = 6.8 Hz, 1H), 1.25 (t, *J* = 6.6 Hz, 3H). |

Similarly, referring to the synthesis of **B-113**, compounds **B-173** and **B-175** were synthesized by replacing **B-1-c** in the original route with intermediate **B-62-b** and **B-73-1**; and compounds **B-174** and **B-176** were synthesized by replacing hydroxyacetic acid in the original route with (S)-2-hydroxypropionic acid.

| No. | Structure | Character | LCMS (ESI): *m/z* [M+H]+ |
|---|---|---|---|
| **B-173** | 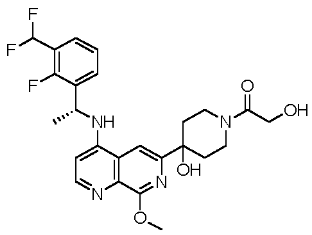 | White solid | 505.0 |
| **B-175** | 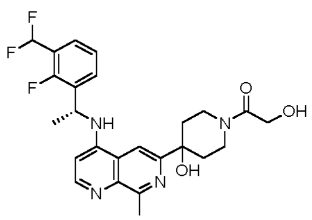 | White solid | 489.0 |
| **B-174** | 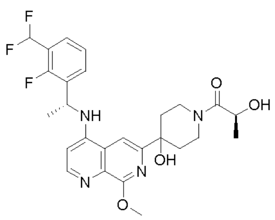 | White solid | 519.2 |

(continued)

| No. | Structure | Character | LCMS (ESI): *m/z* [M+H]$^+$ |
|---|---|---|---|
| **B-176** | 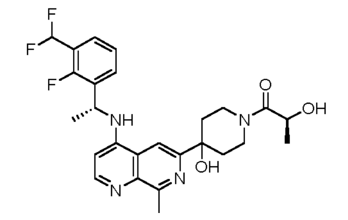 | White solid | 503.1 |

**Example 79 Synthesis of compound B-118**

Synthetic route:

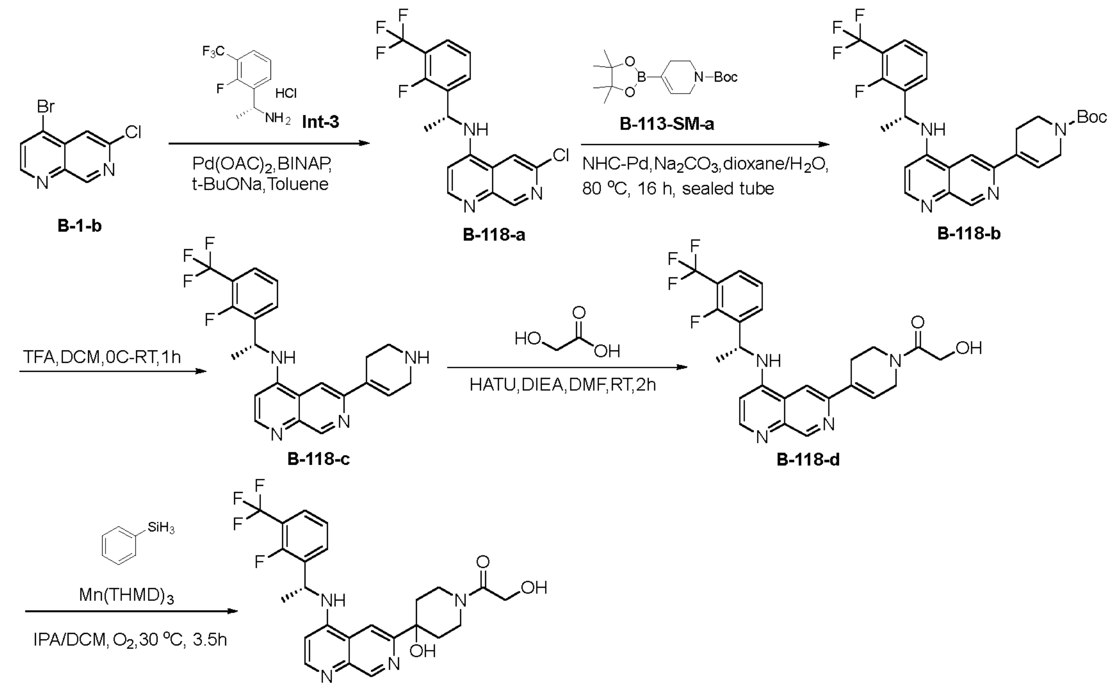


Step one:

Compound **B-1-b** (220 mg, 0.90 mmol) was dissolved in 20 mL of toluene, then compound **Int-3** (262 mg, 1.08 mmol), sodium-t-butoxide (173 mg, 1.80 mmol), BINAP (112 mg, 0.18 mmol), and palladium acetate (20 mg, 0.09 mmol) were added and uniformly stirred. The reaction solution was gradually heated to 100°C and reacted overnight under nitrogen protection. After the reaction (incomplete reaction), the reaction solution was cooled to room temperature, poured into an icy saturated ammonium chloride solution to be quenched, and then extracted with ethyl acetate. Then the organic phase was backwashed with a saturated salt solution and dried over sodium sulfate. The organic phase was filtered and concentrated under reduced pressure, and the residue was purified by a column chromatography to obtain a yellow solid compound **B-118-a** (189 mg, yield of 57.0%). LCMS (ESI): *m/z* = 370.1 [M+H]$^+$.

Step two:

To a sealed tube, compound **B-118-a** (250 mg, 0.68 mmol) and compound **B-113-SM-a** (300.6 mg, 1.02 mmol) were dissolved in dioxane/$H_2O$ (7.5 mL/1.5 mL), then sodium carbonate (143.2 mg, 1.36 mmol) and NHC-Pd (46.2 mg, 0.068 mmol) were added, nitrogen replacement was performed, and the mixture reacted overnight at 80°C. After the

reaction was completed, the reaction solution was cooled, quenched with a saturated ammonium chloride solution, and extracted with EA. The organic phase was backwashed with a saturated salt solution, dried over sodium sulfate, filtered, and concentrated under reduced pressure. The residue was purified by the column chromatography to obtain compound **B-118-b** (236 mg, yellow solid) with the yield of 66.5%. LCMS (ESI): *m/z* =517 [M+H]+

Step three:

Compound **B-118-b** (236 mg, 0.46 mmol) was dissolved in DCM (2 mL), and then TFA (0.5 mL) was added at 0°C and reacted at room temperature for 1 hour. After the reaction was completed, the reaction solution was concentrated under reduced pressure. A saturated sodium bicarbonate solution was added to the residue and adjust to residue to be alkaline. The organic phase was backwashed with a saturated salt solution, dried over sodium sulfate, filtered, and concentrated under reduced pressure to obtain compound **B-118-c** (167 mg, brown solid) with the yield of 87.7%. LCMS (ESI): m/z =417 [M+H]+

Step four:

After compound **B-118-c** (80 mg, 0.19 mmol) and hydroxyacetic acid (21.9 mg, 0.29 mmol) were dissolved in DMF (3.5 mL), and then HATU (87.5 mg, 0.23 mmol) and DIEA (61.9 mg, 0.48 mmol) were added and reacted at room temperature for 2 hours. After the reaction was completed, the reaction solution was quenched with a saturated ammonium chloride solution and extracted with EA. The organic phase was backwashed with a saturated ammonium chloride solution and a saturated salt solution, dried over sodium sulfate, filtered, and concentrated under reduced pressure. The residue was purified by a thin-layer chromatography to obtain compound **B-118-d** (47 mg, yellow oil) with the yield of 51.6%. LCMS (ESI): m/z =475 [M+H]+

Step five:

Compound **B-118-d** (47 mg, 0.10 mmol) was dissolved in IPA/DCM (4 mL/0.4 mL), Mn(THMD)$_3$ (12.0 mg, 0.02 mmol) was added and stirred at room temperature for 5 minutes, then phenylsilane (26.7 mg, 0.25 mmol) was added, oxygen replacement was performed, and the reaction was performed at 30°C for 3.5 hours. After the reaction was completed, the reaction solution was extracted with a saturated ammonium chloride solution and DCM. The organic phase was backwashed with a saturated salt solution, dried over sodium sulfate, filtered, and concentrated under reduced pressure. The residue was subjected to a neutral reverse-phase preparative purification to obtain compound **B-118** (21.7 mg, light yellow solid) with the yield of 44.5%. LCMS (ESI): *m/z* = 493 [M+H]+; $^1$H NMR (400 MHz, $CD_3OD$): $\delta$ 9.12 (s, 1H), 8.50 (s, 1H), 8.38 (d, *J* = 5.5 Hz, 1H), 7.68 (t, *J* = 7.1 Hz, 1H), 7.61 (t, *J* = 7.2 Hz, 1H), 7.28 (t, *J* = 7.8 Hz, 1H), 6.39 (d, *J* = 5.6 Hz, 1H), 5.21 (q, *J* = 6.8 Hz, 1H), 4.49 (d, *J* = 12.8 Hz, 1H), 4.354.26 (m, 2H), 3.73 (d, *J* = 13.2 Hz, 1H), 3.60 (t, *J* = 12.1 Hz, 1H), 3.26 (s, 1H), 2.34 (qd, *J* = 13.2, 4.5 Hz, 2H), 1.86 (d, *J* = 13.6 Hz, 2H), 1.77 (d, *J* = 6.8 Hz, 3H).

**Example 80 Synthesis of compound B-125**

Synthetic route:

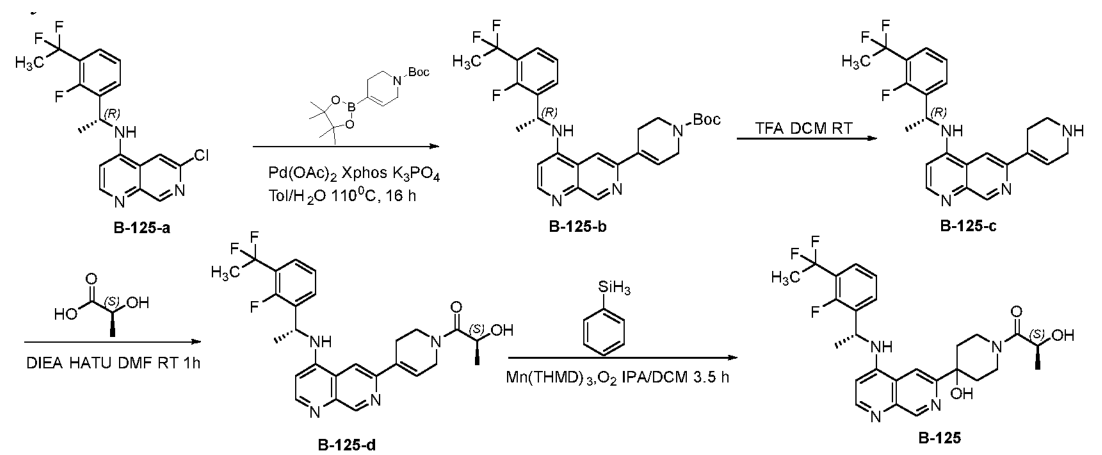


B-125-a B-125-b B-125-c B-125-d B-125

Step one:

To a reaction flask, **B-125-a** (340 mg, 0.93 mmol), 4-(4,4,5,5-tetramethyl-1,3,2-dioxaborolan-2-yl)-3,6-dihydropyridin-1(2H)-tert-butyl formate (578 mg, 1.86 mmol), $Pd(OAc)_2$ (21 mg, 0.093 mmol), Xphos (108 mg, 0.187 mmol), and $K_3PO_4$ (592 mg, 2.81 mg) were successively added, dissolved by adding Tol/$H_2O$ (10:1, 20 mL), and stirred overnight at 110°C under nitrogen protection. After the reaction was completed, the reaction solution was cooled, quenched with a saturated ammonium chloride solution, and extracted with ethyl acetate. The organic phase was washed with a saturated salt solution, dried over anhydrous sodium sulfate, filtered, concentrated, and subjected to column chromatography to obtain 375 mg of yellow oil **B-125-b** with the yield of 78%. LCMS (ESI): m/z =513 $[M+H]^+$

Step two:

**B-125-b** (370 mg, 0.315 mmol) was dissolved in 3 mL DCM, and 1 mL of TFA was added and stirred at room temperature for 1 hour. After the reaction was completed, the reaction solution was subjected to rotary evaporation to obtain 260 mg of **B-125-c** which was directly used in the next reaction. LCMS (ESI): m/z =413 $[M+H]^+$

Step three:

To a reaction flask, **B-125-c** (130 mg, 0.315 mmol), (S)-2-hydroxypropionic acid (34 mg, 0.378 mmol), DIEA (102 mg, 0.79 mmol), and HATU (144 mg, 0.379 mmol) were added, dissolved in 5 mL of DMF, and stirred at room temperature for 1 hour. After the reaction was completed, the reaction solution was quenched with a saturated ammonium chloride solution and extracted with EA. The organic phase was washed with a saturated salt solution, dried over anhydrous sodium sulfate, filtered, concentrated, and subjected to column chromatography to obtain 70 mg of a yellow solid **B-125-d** with the yield of 46%. LCMS (ESI): m/z =485$[M+H]^+$

Step four:

To a reaction flask, **B-125-d** (70 mg, 0.144 mmol), phenylsilane (39 mg, 0.36 mmol), and $Mn(THMD)_3$ (18 mg, 0.029 mmol) were added, and 5 mL of isopropanol/dichloromethane (10:1) was added, and the reacted at 30°C for 3.5 hours under oxygen. After the reaction was completed, the reaction solution was quenched with a saturated ammonium chloride solution and extracted with EA. The organic phase was washed with a saturated salt solution, dried over anhydrous sodium sulfate, filtered, concentrated, and subjected to column chromatography to obtain **B-125** (12 mg) as a white solid with the yield of 16%. LCMS (ESI): m/z =503$[M+H]^+$; $^1H$ NMR (400 MHz, DMSO-*d6*) $\delta$ 9.08 (s, 1H), 8.63 (s, 1H), 8.39 (d, *J* = 5.3 Hz, 1H), 7.88 (d, *J* = 7.1 Hz, 1H), 7.56 (t, *J* = 7.2 Hz, 1H), 7.47 (t, *J* = 7.2 Hz, 1H), 7.24 (t, *J* = 7.8 Hz, 1H), 6.30 (d, *J* = 5.4 Hz, 1H), 5.50 (s, 1H), 5.19-5.03 (m, 1H), 4.83 (dd, J = 11.7, 6.9 Hz, 1H), 4.50 (d, J = 6.4 Hz, 1H), 4.36 (s, 1H), 3.93 (s, 1H), 3.47 (s, 1H), 3.08 (s, 1H), 2.24 (s, 2H), 2.06 (t, *J* = 19.1 Hz, 3H), 1.73 (s, 2H), 1.67 (d, *J* = 6.8 Hz, 3H), 1.23 (t, *J* = 6.6 Hz, 3H).

The synthesis of the following compound was the same as that of **B-125.**

| No. | Structure | Character | LCMS (ESI): *m/z* $[M+H]^+$ | $^1H$ NMR |
|---|---|---|---|---|
| **B-177** | 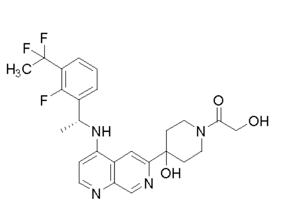 | White solid | 489.0 | $^1H$ NMR (400 MHz, DMSO-$d_6$) $\delta$ 9.07 (s, 1H), 8.62 (s, 1H), 8.39 (d, *J* = 5.3 Hz, 1H), 7.88 (d, *J* = 6.8 Hz, 1H), 7.56 (t, *J* = 7.2 Hz, 1H), 7.47 (t, *J* = 7.2 Hz, 1H), 7.24 (t, *J* = 7.7 Hz, 1H), 6.30 (d, *J* = 5.5 Hz, 1H), 5.50 (s, 1H), 5.16-5.04 (m, 1H), 4.50 (t, *J* = 5.3 Hz, 1H), 4.33 (s, 1H), 4.16 (dd, *J* = 9.5, 5.6 Hz,1H), 3.63 (s, 1H), 3.43 (s, 1H), 3.11 (s, 1H), 2.20 (d, *J* = 13.7 Hz, 2H), 2.06 (t, *J* = 18.9 Hz, 3H), 1.72 (d, *J* = 13.0 Hz, 2H), 1.67 (d, *J* = 6.7 Hz, 3H). |

**Example 81 Synthesis of compound B-126**

Synthetic route:

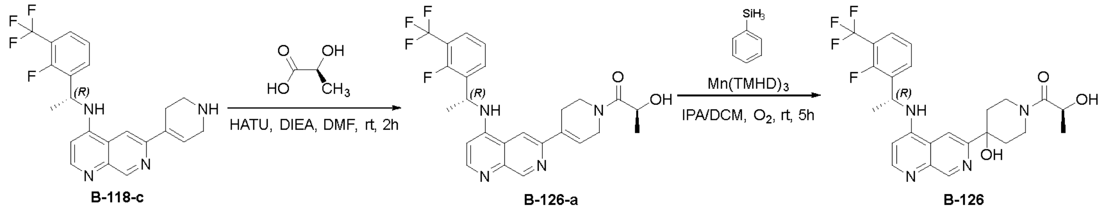


Step one:

Compound **B-118-c** (400 mg, 0.96 mmol), (S)-2-hydroxypropionic acid (136 mg, 1.44 mmol), HATU (548 mg, 1.44 mmol), and DIEA (372 mg, 1.44 mmol) were added in 10 mL of DMF to be uniformly mixed and reacted at room temperature for two hours. After the reaction was completed, the reaction solution was extracted with ethyl acetate, the upper layer organic phase was collected, dried, and concentrated under reduced pressure, and the residue was purified by a column chromatography to obtain a yellow oily compound **B-126-a** (320 mg, yield of 68 %). LCMS (ESI): m/z =489 [M+H]+

Step two:

Compound **B-126-a** (320 mg, 0.65 mmol) and Mn(TMHD)$_3$ (79 mg, 0.12 mmol) were added to IPA/DCM (10 mL/1 mL) to be reacted for 10 minutes, and then phenylsilane (177 mg, 1.51 mmol) was added, uniformly mixed, and reacted at room temperature for 5 hours under oxygen. After the reaction was completed, the reaction solution was filtered and concentrated under reduced pressure. The residue was purified by the column chromatography to obtain a white solid compound **B-126** (32.3 mg, yield of 10%). LCMS (ESI): $m/z$ =507.2 $[M+H]^+$; $^1$H NMR (400 MHz, DMSO-$d_6$) $\delta$ 9.08 (s, 1H), 8.63 (s, 1H), 8.40 (d, $J$ = 5.3 Hz, 1H), 7.90 (d, $J$ = 7.0 Hz, 1H), 7.75 (t, $J$ = 7.4 Hz, 1H), 7.69 (t, $J$ = 7.2 Hz, 1H), 7.37 (t, $J$ = 7.8 Hz, 1H), 6.32 (d, $J$ = 5.4 Hz, 1H), 5.50 (s, 1H), 5.13-5.16 (m, 1H), 4.81-4.86 (m, 1H), 4.48-4.53 (m, 1H), 4.35-4.37 (m, 1H), 3.91-3.93 (m, 1H), 3.47 (t, $J$ = 12.8 Hz, 1H), 3.07-3.09 (m, 1H), 2.12-2.27 (m, 2H), 1.73-1.74 (m, 2H), 1.68 (d, $J$ = 6.8 Hz, 3H), 1.22 (t, $J$ = 6.7 Hz, 3H).

**Example 82 Synthesis of compound B-145**

Synthetic route:

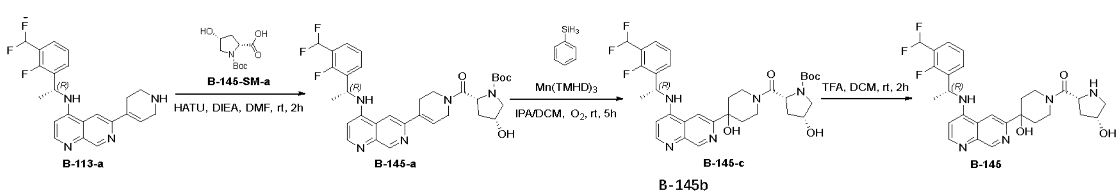


Step one:

To a dry round-bottom flask, compound **B-113-a** (100 mg, 0.25 mmol), **B-145-SM-a** (88 mg, 0.38 mmol), HATU (143 mg, 0.38 mmol), DIEA (98 mg, 0.75 mmol), and DMF (5 mL) were successively added. The reaction was performed at room temperature for 2 hours under nitrogen protection. After the reaction was completed, the reaction was quenched with $NH_4Cl$. The organic phase was extracted with EA and concentrated under reduced pressure. The residue was purified by a rapid column chromatography to obtain a yellow oily compound **B-145-a** (100 mg, yield of 83%). LCMS (ESI): m/z =612.2 $[M+H]^+$

Step two:

To a dry flask, compound **B-145-a** (100 mg, 0.28 mmol) and $Mn(TMHD)_3$ (24 mg, 0.04 mmol) were successively added and reacted in IPA/DCM (5 mL/0.5 mL) for 5-10 minutes, and then phenylsilane (54 mg, 0.5 mmol) was added to the reaction system and reacted at room temperature for 5 hours under an oxygen atmosphere. After the reaction was completed, the reaction solution was concentrated under reduced pressure and purified to obtain yellow oil **B-145-b** (60 mg, yield of 48%). LCMS (ESI): m/z =630.2 $[M+H]^+$

Step three:

To a dry flask, compound **B-145-b** (60 mg, 0.28 mmol) and TFA (0.5 mL) were successively added, and reacted in DCM (2 mL) at room temperature for 2 hours. After the reaction was completed, the reaction solution was concentrated under reduced pressure and purified by a preparative chromatography to obtain a yellow solid **B-145** (12.4 mg, yield of 18%). LCMS (ESI): m/z = 530.2 $[M+H]^+$; 1H NMR (400 MHz, DMSO-d6) δ 9.09 (d, J = 3.9 Hz, 1H), 8.64 (d, J = 7.8 Hz, 1H), 8.40 (d, J = 5.3 Hz, 1H), 7.88-7.90 (m, 1H), 7.63 (t, J = 7.5 Hz, 1H), 7.54 (t, J = 7.0 Hz, 1H), 7.43-7.11 (m, 2H), 6.32 (d, J = 5.4 Hz, 1H), 5.53 (d, J = 3.3 Hz, 1H), 5.12 (t, J = 6.9 Hz, 1H), 4.53-4.60 (m, 1H), 4.39 (d, J = 12.8 Hz, 1H), 4.17 (s, 1H), 3.78-3.86 (m, 2H), 3.45-3.49 (m, 1H), 3.11 (t, J = 12.4 Hz, 1H), 2.86 (d, J = 11.6 Hz, 1H), 2.58-2.62 (m, 1H), 2.34-2.03 (m, 4H), 1.76 (d, J = 16.8 Hz, 2H), 1.69 (d, J = 6.8 Hz, 3H), 1.45-1.49 (m, 1H).

The synthesis of the following compounds was the same as that of **B-145**.

| No. | Structure | Character | LCMS (ESI): m/z $[M+H]^+$ | $^1H$ NMR |
|---|---|---|---|---|
| **B-144** | 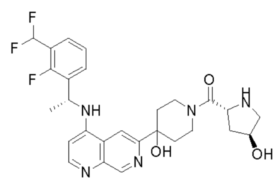 | White solid | 530.3 | $^1H$ NMR (400 MHz, DMSO-$d_6$) δ 9.08 (s, 1H), 8.63 (d, *J* = 5.1 Hz, 1H), 8.39 (d, *J* = 5.3 Hz, 1H), 7.88 (d, *J* = 6.9 Hz, 1H), 7.57 (dt, *J* = 33.9, 7.0 Hz, 2H), 7.27 (dt, J = 59.6, 44.0 Hz, 2H), 6.31 (d, *J* = 5.3 Hz, 1H), 5.50 (d, *J* = 7.7 Hz, 1H), 5.18-4.99 (m, 1H), 4.69 (s, 1H), 4.43-3.75 (m, 4H), 3.49 (dd, *J* = 27.2, 13.7 Hz, 1H), 3.10 (dt, *J* = 18.0, 9.1 Hz, 2H), 2.54 (d, *J* = 9.4 Hz, 1H), 2.26-2.08 (m, 3H), 1.87 (d, *J* = 7.8 Hz, 1H), 1.76 (d, *J* = 18.6 Hz, 3H), 1.68 (d, *J* = 6.8 Hz, 3H). |

**Example 83 Synthesis of compound B-147**

Synthetic route:

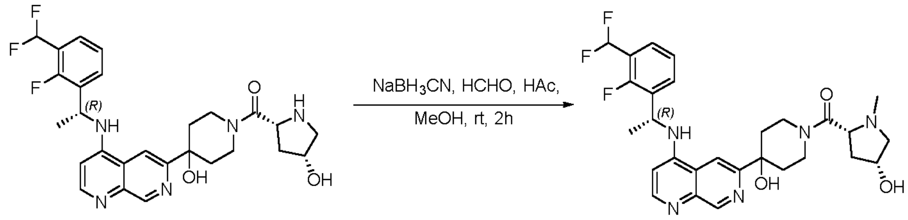


B-145 B-147

Step one:

To a dry round-bottom flask, compound **B-145** (30 mg, 0.05 mmol), $NaBH_3CN$ (4.6 mg, 0.10 mmol), HCHO (0.1 mL), Hac (1 drop), and MeOH (2 mL) were successively added. The reaction was performed at room temperature for 2 hours. After the reaction was completed, the reaction solution was concentrated under reduced pressure. The residue was purified by a preparative chromatography to obtain a yellow solid compound **B-147** (7.3 mg, yield of 24%).

LCMS (ESI): m/z = 544.2 [M+H]$^+$; $^1$H NMR (400 MHz, DMSO-$d_6$) $\delta$ 9.06 (s, 1H), 8.61 (d, *J* = 2.3 Hz, 1H), 8.36 (d, J = 5.2 Hz, 1H), 7.87 (d, *J* = 7.1 Hz, 1H), 7.60 (t, *J* = 7.5 Hz, 1H), 7.51 (t, *J* = 7.1 Hz, 1H), 7.40-7.04 (m, 2H), 6.28 (d, *J* = 5.4 Hz, 1H), 5.46 (d, J = 3.9 Hz, 1H), 5.08 (t, *J* = 6.9 Hz, 1H), 4.86 (d, *J* = 5.2 Hz, 1H), 4.35 (d, *J*= 12.6 Hz, 2H), 4.16 (s, 1H), 3.43-3.45 (m, 1H), 3.04-3.14 (m, 2H), 2.86 (d, *J* = 9.7 Hz, 1H), 2.45-2.34 (m, 1H), 2.24-2.28 (m, 1H), 2.21 (s, 3H), 2.19-2.00 (m, 2H), 1.66-1.71 (m, 2H), 1.65 (d, *J* = 6.7 Hz, 3H).

The synthesis of the following compounds was the same as that of **B-147**.

| No . | Structure | Character | LCMS (ESI): *m/z* [M+H]$^+$ | $^1$H NMR |
|---|---|---|---|---|
| **B-146** | 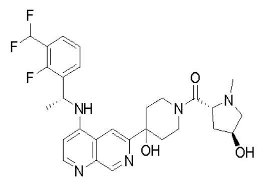 | White solid | 544.2 | $^1$H NMR (400 MHz, DMSO-$d_6$) $\delta$ 9.05 (s, 1H), 8.60 (d, *J* = 2.3 Hz, 1H), 8.36 (d, J = 5.2 Hz, 1H), 7.87 (d, *J* = 7.1 Hz, 1H), 7.61 (t, *J* = 7.5 Hz, 1H), 7.51 (t, *J* = 7.1 Hz, 1H), 7.40-7.04 (m, 2H), 6.28 (d, *J* = 5.4 Hz, 1H), 5.46 (d, J = 3.9 Hz, 1H), 5.08 (t, *J* = 6.9 Hz, 1H), 4.87 (d, *J* = 5.2 Hz, 1H), 4.35 (d, *J* = 12.6 Hz, 2H), 4.16 (s, 1H), 3.44 -3.45 (m, 1H), 3.04-3.14 (m, 2H), 2.86 (d, *J* = 9.7 Hz, 1H), 2.45-2.34 (m, 1H), 2.24-2.28 (m, 1H), 2.23 (s, 3H), 2.19-2.00 (m, 2H), 1.66-1.71 (m, 2H), 1.66 (d, *J* = 6.7 Hz, 3H). |

**Example 84 Synthesis of compound B-149**

Synthetic route:

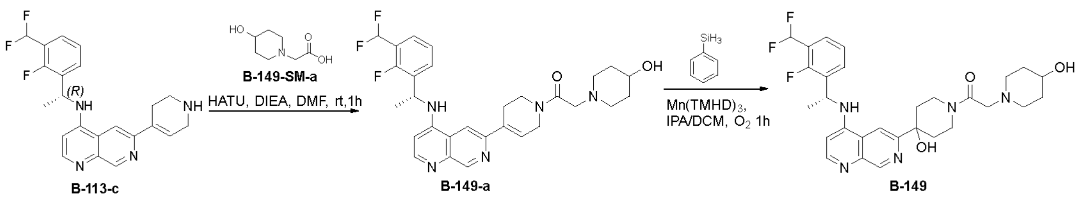


Step one:

Compound **B-113-c** (160 mg, 0.402 mmol), **B-149-SM-a** (100 mg, 0.629 mmol), HATU (229 mg, 0.603 mmol), and DIEA(155 mg, 1.2 mmol) were dissolved in 2 mL of DMF, and reacted while stirring at room temperature for 1 hour. After the reaction was completed, water was added and the reaction solution was extracted with ethyl acetate. The organic phase was collected, dried over anhydrous sodium sulfate, and concentrated. The residue was purified to obtain compound **B-149-a** (150 mg, yellow oil) with the yield of 69.1%. LCMS : m/z =540.5 [M +H]$^+$

Step two:

To a dry flask, compound **B-149-a** (150 mg, 0.278 mmol) was added and dissolved in dichloromethane/isopropanol (10/1 mL), then Mn(TMHD)$_3$ (34 mg, 0.056 mmol) was added and stirred at room temperature for 10 minutes, and phenylsilane (75 mg, 0.694 mmol) was added and reacted at 35°C for 1 hour under an oxygen environment. After the reaction was completed, the solvent was subjected to rotary evaporation. Ethyl acetate was added and the reaction solution was extracted with a salt solution. The organic phase was collected, dried over anhydrous sodium sulfate, concentrated, and subjected to a reverse-phase preparative purification to obtain a white solid compound **B-149** (20 mg, yield of 13.16%). LCMS : m/z = 558.3 [M+H]$^+$; $^1$H NMR (400 MHz, DMSO-d6) $\delta$ 9.07 (s, 1H), 8.62 (s, 1H), 8.39 (d, J = 5.2 Hz, 1H), 7.88 (d, J = 7.3 Hz, 1H), 7.61 (t, J = 7.5 Hz, 1H), 7.53 (t, J = 7.2 Hz, 1H), 7.43-7.28 (m, 1H), 7.28-7.07 (m, 1H), 6.30 (d, J = 5.4 Hz, 1H), 5.46 (s, 1H), 5.10 (t, J = 6.8 Hz, 1H), 4.59-4.48 (m, 1H), 4.31 (d, J = 12.7 Hz, 1H), 4.02 (d, J = 12.9 Hz, 1H), 3.45 (t, J = 12.1 Hz, 2H), 3.25 (d, J = 13.2 Hz, 1H), 3.05 (t, J = 12.3 Hz, 2H), 2.79-2.64 (m, 2H), 2.35-2.21 (m, 1H), 2.20-1.98 (m, 3H), 1.78-1.61 (m, 7H), 1.47-1.31 (m, 2H).

**Example 85 Synthesis of compound B-151**

Synthetic route:

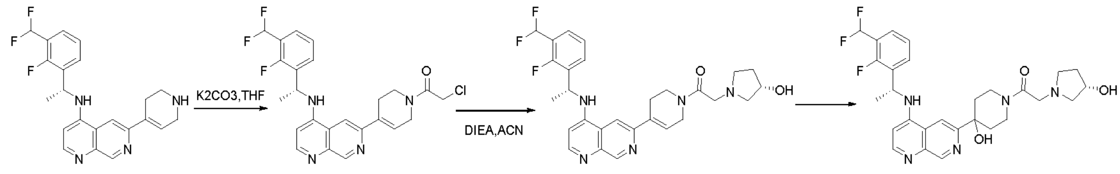


B-113-a B-151-a B-151-b B-151

Step one:

Compound **B-113-a** (200 mg, 0.5 mmol) and potassium carbonate (104 mg, 0.6 mmol) were dissolved in THF (5 mL),and chloroacetyl chloride (62 mg, 0.55 mmol) was dropwise added at 0°C and stirred at room temperature for 2 hours. After the reaction was completed, the reaction solution was poured into a saturated ammonium chloride solution to be quenched and then extracted with DCM. Then the organic phase was washed with a saturated salt solution, dried over sodium sulfate, filtered, and concentrated under reduced pressure. The residue was purified by a silica gel chromatographic column to obtain compound **B-151-a** (160 mg, yield of 67%). LCMS (ESI): m/z = 475 $[M+H]^+$

Step two:

Compound **B-151-a** (150 mg, 0.3 mmol) and DIEA(61 mg, 0.5 mmol) were dissolved in ACN (5 mL), and (S)-pyrrolidin-3-ol (33 mg, 0.4 mmol) was dropwise added at 0°C and stirred at room temperature for 12 hours. After the reaction was completed, the reaction solution was poured into an icy ammonium chloride solution to be quenched and then extracted with DCM. Then the organic phase was washed with a saturated salt solution, dried over sodium sulfate, filtered, and concentrated under reduced pressure. The residue was purified by the silica gel chromatographic column to obtain compound **B-151-b** (110 mg, yield of 68%). LCMS (ESI): m/z = 526 [M+H]+

Step three:

Compound **B-151-b** (110 mg, 0.21 mmol) was dissolved in IPA/DCM (5 mL/0.5 mL), compound $Mn(THMD)_3$ (25 mg, 0.04 mmol) was added and stirred at room temperature for 5 minutes, then phenylsilane (56 mg, 0.51 mmol) was added, oxygen replacement was performed, and the reaction was performed at 30°C for 3 hours. After the reaction was completed, the reaction solution was extracted with a saturated ammonium chloride solution and DCM. The organic phase was backwashed with a saturated salt solution, dried over sodium sulfate, filtered, and concentrated under reduced pressure. The residue was purified by PreP-HPLC to obtain compound **B-151** (14 mg, yield of 12%). LCMS (ESI): m/z = 544 $[M+H]^+$, 1H NMR (400 MHz, $CD_3OD$) $\delta$ 9.11 (d, J = 0.9 Hz, 1H), 8.49 (s, 1H), 8.36 (d, J = 5.5 Hz, 1H), 7.54 (dt, J = 16.2, 7.2 Hz, 2H), 7.23 (t, J = 7.7 Hz, 1H), 7.03 (t, J = 54.8 Hz, 1H), 6.40 (d, J = 5.6 Hz, 1H), 5.18 (q, J = 6.8 Hz, 1H), 4.49 (d, J = 13.1 Hz, 1H), 4.40-4.33 (m, 1H), 4.03 (d, J = 13.4 Hz, 1H), 3.67-3.42 (m, 3H), 3.27-3.20 (m, 1H), 2.92 (ddd, J = 23.5, 11.5, 6.4 Hz, 2H), 2.65 (tt, J = 10.6, 6.1 Hz, 2H), 2.45-2.26 (m, 2H), 2.22-2.12 (m, 1H), 1.86 (d, J = 13.5 Hz, 2H), 1.76 (d, J = 6.8 Hz, 4H).

The synthesis of the following compounds was the same as that of **B-151.**

| No. | Structure | Character | LCMS (ESI): *m/z* $[M+H]^+$ | $^1H$ NMR |
|---|---|---|---|---|
| **B-148** | 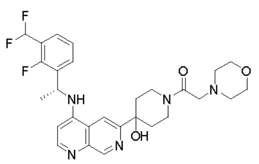 | White solid | 544.1 | |

(continued)

| No. | Structure | Character | LCMS (ESI): *m/z* [M+H]+ | 1H NMR |
|---|---|---|---|---|
| **B-150** | 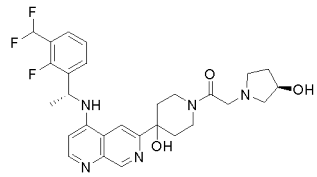 | White solid | 544.0 | 1H NMR (400 MHz, $CD_3OD$) δ 9.12 (s, 1H), 8.50 (s, 1H), 8.36 (d, *J* = 5.5 Hz, 1H), 7.53 (dd, *J* = 16.4, 8.0 Hz, 2H), 7.23 (t, *J* = 7.7 Hz, 1H), 7.04 (t, *J* = 54.8 Hz, 1H), 6.40 (d, J = 5.6 Hz, 1H), 5.17(q, *J* = 6.4 Hz, 1H), 4.48 (d, *J* = 11.6 Hz, 1H) 4.37-4.33 (m, 1H), 4.04-4.01 (m, 1H), 3.66-3.42 (m, 3H), 3.24-3.22 (m, 1H), 2.97-2.87 (m, 2H), 2.67-2.62 (m, 2H), 2.41-2.13 (m, 3H), 1.86 (d, *J* = 14.0 Hz, 2H), 1.75 (d, *J* = 6.8 Hz, 4H). |

**Example 86 Synthesis of compound B-152**

Synthetic route:

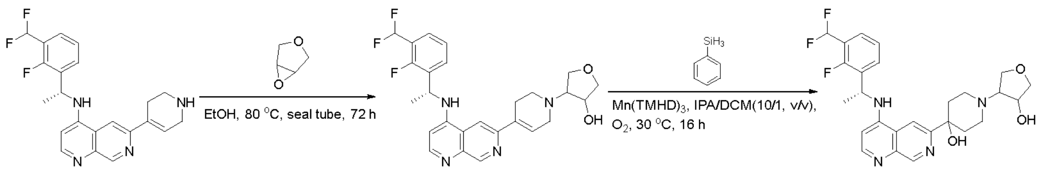


Step one:

To a 75-mL sealed tube, compound **B-113-a** (150 mg, 0.38 mmol) and compound 3,6-dioxabicyclo[3.1.0]hexane (320 mg, 3.8 mmol) were dissolved in ethanol (10 mL), heated to 80°C, and reacted for 72 hours. After the reaction was completed, the solvent was subjected to rotary evaporation. The residue was purified by a glass silica gel column (ethyl acetate/methanol/water = 20/2/1) to obtain a yellow oily compound **B-152-a** (60 mg, yield of 33%).

Step two:

$Mn(TMHD)_3$ (14 mg, 0.024 mmol) was added to an isopropanol/dichloromethane (10 mL/1 mL) solution of compound **B-152-a** (60 mg, 0.12 mmol) and reacted while stirring at 30°C for 10 minutes, and then phenylsilane (33 mg, 0.3 mmol) was added, reacted continuously under an oxygen balloon atmosphere, and reacted while stirring at 30°C for 16 hours. After the reaction was completed, the reaction solution was filtered. The resulting filtrate was concentrated under reduced pressure to obtain the residue. The residue was purified by a reverse-phase preparative chromatography to obtain compound **B-152** (11.9 mg, white solid) with the yield of 26%. LCMS (ESI): m/z =503.0 [M+H]+; 1H NMR (400 MHz, DMSO-*d6*) δ 9.07 (s, 1H), 8.60 (s, 1H), 8.38 (d, *J* = 5.3 Hz, 1H), 7.85 (d, *J* = 7.1 Hz, 1H), 7.61 (t, J = 7.4 Hz, 1H), 7.53 (t, *J* = 7.1 Hz, 1H), 7.42-7.10 (m, 2H), 6.29 (d, *J* = 5.4 Hz, 1H), 5.15 (s, 1H), 5.10 (p, *J* = 6.8 Hz, 1H), 5.01 (d, *J* = 5.0 Hz, 1H), 4.19 (dt, *J* = 8.8, 4.3 Hz, 1H), 3.96 (dd, *J* = 9.1, 6.8 Hz, 1H), 3.79 (dd, *J*= 9.4, 5.8 Hz, 1H), 3.60-3.46 (m, 2H), 2.95 (d, *J* = 11.0 Hz, 1H), 2.74 (td, *J* = 6.5, 3.0 Hz, 1H), 2.55 (d, *J* = 10.2 Hz, 3H), 2.29 (dt, *J* = 13.5, 4.8 Hz, 2H), 1.73-1.60 (m, 5H).

**Example 87 Synthesis of compound B-153**

Synthetic route:

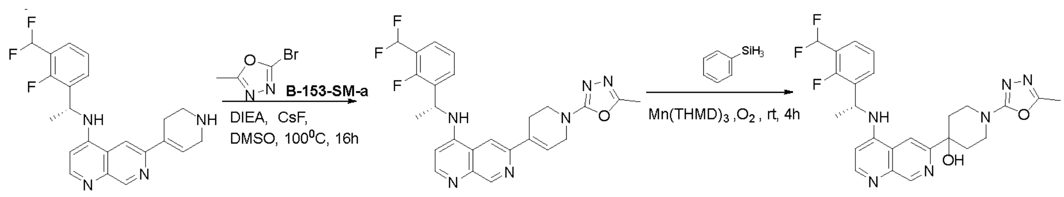


B-113-a B-153-a B-153

Step one:

Compound **B-113-a** (100 mg, 0.251 mmol) and **B-153-SM-a** (82 mg, 0.503 mmol) were dissolved in 2 mL of DMSO, supplemented with DIEA(49 mg, 0.379 mmol) and cesium fluoride (2 mg, 0.013 mmol), heated to 100°C, and reacted for 16 hours. After the reaction was completed, water was added to the reaction solution. Then the reaction solution was extracted with ethyl acetate. The organic phase was backwashed with a saturated salt solution, dried over sodium sulfate, filtered, and concentrated under reduced pressure. The residue was purified by TLC to obtain compound **B-153-a** (60 mg, yellow solid) with the yield of 49.7%. LCMS (ESI): m/z = 481.1 $[M+H]^+$

Step two:

Compound **B-153-a** (60 mg, 0.125 mmol) and $Mn(THMD)_3$ (15 mg, 0.024 mmol) were dissolved in 2 mL of isopropanol and 0.2 mL of dichloromethane, reacted at room temperature for 10 minutes, supplemented with phenylsilane (34 mg, 0.314 mmol), and then reacted at room temperature for 4 hours under an oxygen condition. After the reaction was completed, the reaction solution was quenched with saturated ammonium chloride and extracted with dichloromethane. The organic phase was backwashed with a saturated salt solution, dried over sodium sulfate, filtered, and concentrated under reduced pressure. The residue was subjected to a preparative purification to obtain compound **B-153** (11.4 mg, white solid) with the yield of 18.3%. LCMS (ESI): *m/z* = 499.2 $[M+H]^+$, $^1H$ NMR (400 MHz, CD3OD) δ 9.12 (s, 1H), 8.51 (s, 1H), 8.36 (d, J = 5.5 Hz, 1H), 7.65-7.45 (m, 2H), 7.23 (t, J = 7.7 Hz, 1H), 7.03 (t, J = 54.8 Hz, 1H), 6.41 (d, J = 5.5 Hz, 1H), 5.18 (d, J = 6.7 Hz, 1H), 3.92 (d, J = 13.2 Hz, 2H), 3.64 (t, J = 12.4 Hz, 2H), 2.68-2.27 (m, 5H), 1.89 (d, J = 13.4 Hz, 2H), 1.76 (d, J = 6.8 Hz, 3H).

The synthesis of the following compounds was the same as that of **B-153**.

| No. | Structure | Character | LCMS (ESI): *m/z* $[M+H]^+$ | $^1H$ NMR |
|---|---|---|---|---|
| **B-154** | 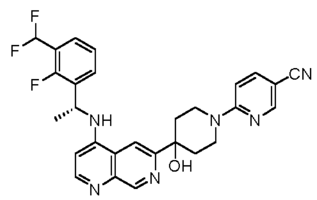 | White solid | 519.0 | |
| **B-155** | 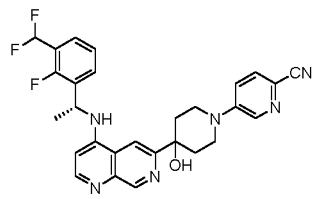 | White solid | 519.0 | |
| **B-156** | 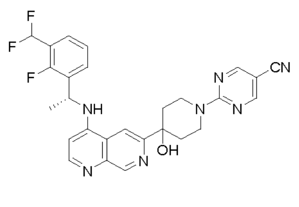 | White solid | 520.2 | $^1H$ NMR (400 MHz, $CD_3OD$) δ 9.11 (d, *J* = 10.8 Hz, 1H), 8.64 (s, 2H), 8.53 (s, 1H), 8.38 (d, *J* = 5.5 Hz, 1H), 7.55 (dt, *J* = 14.4, 7.1 Hz, 2H), 7.25 (t, *J* = 7.7 Hz, 1H), 7.06 (t, *J* = 54.8 Hz, 1H), 6.42 (d, *J* = 5.6 Hz, 1H), 5.19 (q, *J* = 6.8 Hz, 1H), 4.90 (d, *J* = 2.5 Hz, 2H), 3.60 (dt, *J* = 46.6, 23.3 Hz, 2H), 2.39 (td, *J* = 13.3, 4.6 Hz, 2H), 1.92 (d, *J* = 13.4 Hz, 2H), 1.77 (d, *J* = 6.8 Hz, 3H). |

**Example 88 Synthesis of compound B-158**

Synthetic route:

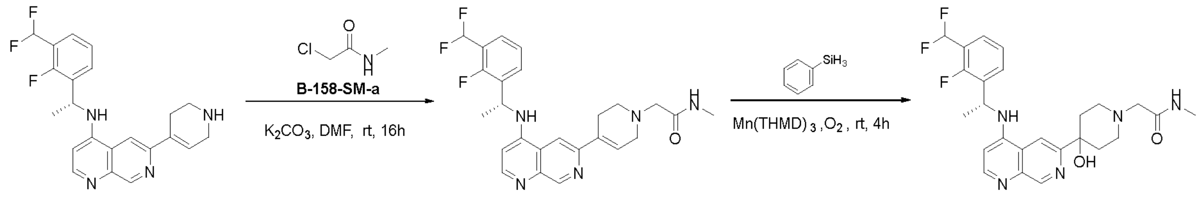


B-113-a B-158-a B-158

Step one:

Compound **B-113-a** (100 mg, 0.251 mmol) and **B-158-SM-a** (55 mg, 0.514 mmol) were dissolved in 3 mL of DMF, supplemented with potassium carbonate (104 mg, 0.753 mmol), and reacted at room temperature for 16 hours. After the reaction was completed, water was added to the reaction solution. Then the reaction solution was extracted with ethyl acetate. The organic phase was backwashed with a saturated salt solution, dried over sodium sulfate, filtered, and concentrated under reduced pressure. The residue was purified by a normal-phase column to obtain compound **B-158-a** (60 mg, red oil) with the yield of 50.9%. LCMS (ESI): m/z = 470.4 $[M+H]^+$

Step two:

Compound **B-158-a** (60 mg, 0.128 mmol) and Mn(THMD)$_3$ (15 mg, 0.024 mmol) were dissolved in 2 mL of isopropanol and 0.2 mL of dichloromethane, reacted at room temperature for 10 minutes, supplemented with phenylsilane (25 mg, 0.231 mmol), and then reacted at room temperature for 4 hours under an oxygen condition. After the reaction was completed, the reaction solution was quenched with saturated ammonium chloride and extracted with dichloromethane. The organic phase was backwashed with a saturated salt solution, dried over sodium sulfate, filtered, and concentrated under reduced pressure. The residue was subjected to a preparative purification to obtain compound **B-158** (10.0 mg, white solid) with the yield of 16.0%. LCMS (ESI): m/z = 488.1 $[M+H]^+$, $^1H$ NMR (400 MHz, $CD_3OD$) $\delta$ 9.13 (s, 1H), 8.49 (s, 1H), 8.36 (d, J = 5.5 Hz, 1H), 7.54 (dt, J = 18.0, 7.1 Hz, 2H), 7.22 (d, J = 7.7 Hz, 1H), 7.04 (t, J = 54.8 Hz, 1H), 6.41 (d, J = 5.6 Hz, 1H), 5.18 (m, 1H), 3.18-3.05 (m, 2H), 2.82 (s, 3H), 2.80-2.69 (m, 4H), 2.52 (m, 2H), 1.78 (dd, J = 17.2, 9.9 Hz, 5H).

The synthesis of the following compounds was the same as that of **B-158**.

| No. | Structure | Character | LCMS (ESI): *m/z* $[M+H]^+$ | $^1H$ NMR |
|---|---|---|---|---|
| **B-117** | 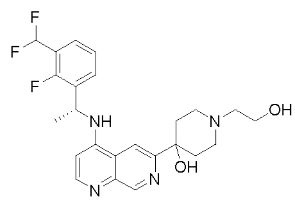 | Light yellow solid | 461.0 | |

(continued)

| No. | Structure | Character | LCMS (ESI): *m/z* [M+H]+ | 1H NMR |
|---|---|---|---|---|
| **B-159** | 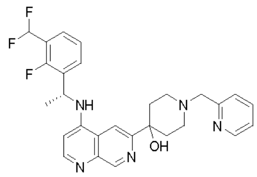 | White solid | 508.0 | 1H NMR (400 MHz, $CD_3OD$) s 9.12 (s, 1H), 8.51 (d, *J* = 4.8 Hz, 1H), 8.46 (s, 1H), 8.35 (d, *J* = 5.6 Hz, 1H), 7.86 (t, *J* = 7.7 Hz, 1H), 7.65 (d, *J* = 7.9 Hz, 1H), 7.60-7.47 (m, 2H), 7.36-7.32 (m, 1H), 7.23 (t, *J* = 7.7 Hz, 1H), 7.04 (t, *J* = 54.8 Hz, 1H), 6.39 (d, *J* = 5.5 Hz, 1H), 5.18 (q, J = 6.8 Hz, 1H), 3.79 (s, 2H), 2.86-2.84 (m, 2H), 2.75 (t, *J* = 11.6 Hz, 2H), 2.50 (td, *J* = 13.0, 4.6 Hz, 2H), 1.85 (d, *J* = 13.2 Hz, 2H), 1.76 (d, *J* = 6.8 Hz, 3H). |
| **B-168** | 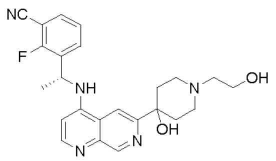 | White solid | 436.2 | 1H NMR (600 MHz, $CD_3OD$) $\delta$ 9.13 (d, J = 0.8 Hz, 1H), 8.55 (s, 1H), 8.39 (d, J = 5.5 Hz, 1H), 7.75 (td, J = 7.6, 1.7 Hz, 1H), 7.67 (ddd, J = 7.7, 6.0, 1.7 Hz, 1H), 7.29 (t, J = 7.8 Hz, 1H), 6.41 (d, J = 5.6 Hz, 1H), 5.19 (q, J = 6.7 Hz, 1H), 3.88 (t, J = 5.6 Hz, 2H), 3.37-3.32 (m, 2H), 3.20 (d, J = 14.2 Hz, 2H), 3.07 (t, J = 5.5 Hz, 2H), 2.65 (d, J = 4.4 Hz, 2H), 1.98 (dt, J = 14.6, 2.8 Hz, 2H), 1.77 (d, J = 6.8 Hz, 3H). |

**Example 89 Synthesis of compound B-160**

Synthetic route:

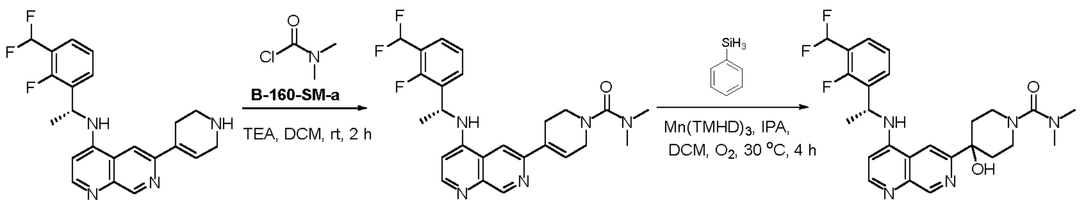


B-113-a B-160-a B-160

Step one:

Compound **B-113-a** (100 mg, 0.25 mmol) and TEA (51 mg, 0.5 mmol) were dissolved in an eggplant-shaped flask containing dichloromethane (3 mL), and **B-160-SM-a** (35 mg, 0.32 mmol) was dropwise added and reacted at room temperature for 2 hours. After the reaction was completed, the reaction solution was extracted with dichloromethane. The organic phase was concentrated under reduced pressure. The residue was purified by a silica gel column to obtain compound **B-160-a** (70 mg, yield of 59%). LCMS (ESI): m/z = 470.2 [M+H]+

Step two:

Compound **B-160-a** (70 mg, 0.15 mmol) and $Mn(TMHD)_3$ (18 mg, 0.03 mmol) were dissolved in an eggplant-shaped flask containing IPA/DCM (3 mL/0.3 mL) and stirred at room temperature for 10 minutes, and then phenylsilane (40.3 mg, 0.37 mmol) was added. Oxygen replacement was performed. The reaction was performed at 30°C for 4 hours. After the reaction was completed, the reaction solution was concentrated under reduced pressure. The residue was purified by the silica gel column to obtain a white solid compound **B-160** (29.5 mg, yield of 41%). LCMS (ESI): m/z = 488.2 [M+H]+, 1H NMR (400 MHz, CD3OD) $\delta$ 9.12 (d, J = 0.9 Hz, 1H), 8.48 (d, J = 0.9 Hz, 1H), 8.36 (d, J = 5.5 Hz, 1H), 7.60-7.47 (m, 2H), 7.23 (t, J = 7.7 Hz, 1H), 7.04 (t, J = 54.8 Hz, 1H), 6.40 (d, J = 5.6 Hz, 1H), 5.18 (q, J = 6.8 Hz, 1H),

3.70-3.60 (m, 2H), 3.45-3.33 (m, 2H), 2.90 (s, 6H), 2.40 (td, J = 13.1, 4.6 Hz, 2H), 1.79 (dd, J = 21.9, 10.1 Hz, 5H).

**Example 90 Synthesis of compound B-162**

Synthetic route:

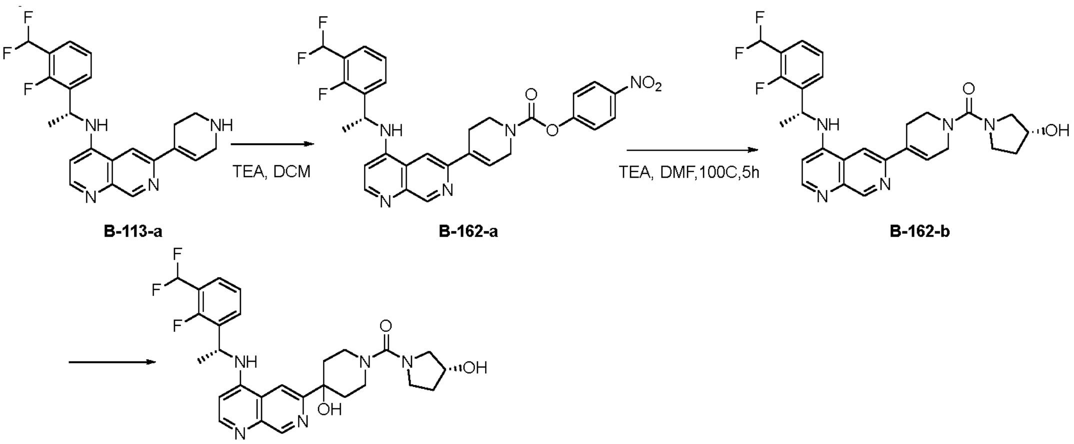


B-113-a B-162-a B-162-b

B-162

Step one:

Compound **B-113-a** (500 mg, 1.3 mmol) and TEA (140 mg, 1.4 mmol) were dissolved in DCM (30 mL), and p-nitrophenyl chloroformate (260 mg, 1.3 mmol) was dropwise added at 0°C and stirred at room temperature for 2 hours. After the reaction was completed, the reaction solution was poured into a saturated ammonium chloride solution to be quenched and then extracted with DCM. Then the organic phase was washed with a saturated salt solution, dried over sodium sulfate, filtered, and concentrated under reduced pressure. The residue was purified by a silica gel chromatographic column to obtain compound **B-162-a** (640 mg, yield of 90%). LCMS (ESI): m/z = 564 [M+H]+

Step two:

Compound **B-162-a** (200 mg, 0.36 mmol) and TEA (167 mg, 1.65 mmol) were dissolved in DMF (5 mL), and (R)-pyrrolidin-3-ol (92 mg, 1.1 mmol) was dropwise added at 0°C and stirred at 100°C for 5 hours. After the reaction was completed, the reaction solution was poured into an icy ammonium chloride solution to be quenched and then extracted with DCM. Then the organic phase was washed with a saturated salt solution, dried over sodium sulfate, filtered, and concentrated under reduced pressure. The residue was purified by the silica gel chromatographic column to obtain compound **B-162-b** (120 mg, yield of 67%). LCMS (ESI): m/z =512 $[M+H]^+$

Step three:

Compound **B-162-b** (120 mg, 0.23 mmol) was dissolved in IPA/DCM (10 mL/1.0 mL), $Mn(TMHD)_3$ (28 mg, 0.05 mmol) was added and stirred at room temperature for 5 minutes, then phenylsilane (56 mg, 0.58 mmol) was added, oxygen replacement was performed, and the reaction was performed at 30°C for 3 hours. After the reaction was completed, the reaction solution was extracted with a saturated ammonium chloride solution and DCM. The organic phase was backwashed with a saturated salt solution, dried over sodium sulfate, filtered, and concentrated under reduced pressure. The residue was purified by PreP-HPLC to obtain compound **B-162** (41.9 mg, white solid, yield of 36%). LCMS (ESI): m/z =530 $[M+H]^+$; $^1H$ NMR (400 MHz, $CD_3OD$) δ 9.12 (s, 1H), 8.49 (s, 1H), 8.36 (d, J = 5.5 Hz, 1H), 7.64-7.45 (m, 2H), 7.23 (t, J = 7.7 Hz, 1H), 7.04 (t, J = 54.8 Hz, 1H), 6.40 (d, J = 5.6 Hz, 1H), 5.18 (q, J = 6.9 Hz, 1H), 4.39 (s, 1H), 3.83-3.60 (m, 4H), 3.52-3.32 (m, 4H), 2.40 (dtd, J = 38.1, 13.2, 4.6 Hz, 2H), 2.03-1.78 (m, 4H), 1.76 (d, J = 6.8 Hz, 3H).

Referring to the synthesis of **B-162**, compound **B-161**, a white solid, with a molecular weight of 530.0, was prepared.

**Example 91 Synthesis of compound B-163**

Synthetic route:

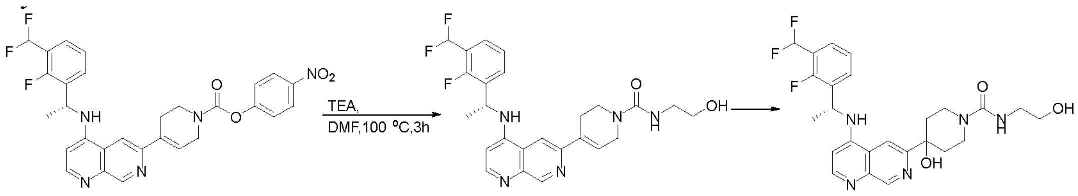

B-162-a B-163-a B-163

Step one:

Compound **B-162-a** (200 mg, 0.36 mmol) and TEA (143 mg, 1.4 mmol) were dissolved in DMF (5 mL), and ethanolamine (65 mg, 1.1 mmol) was dropwise added at 0°C and stirred at 100°C for 3 hours. After the reaction was completed, the reaction solution was poured into an icy ammonium chloride solution to be quenched and then extracted with DCM. Then the organic phase was washed with a saturated salt solution, dried over sodium sulfate, filtered, and concentrated under reduced pressure. The residue was purified by a silica gel chromatographic column to obtain compound **B-163-a** (80 mg, yield of 47%). LCMS (ESI): m/z = 486[M+H]$^+$

Step two:

Compound **B-163-a** (120 mg, 0.25 mmol) was dissolved in IPA/DCM (10 mL/1.0 mL), Mn(THMD)$_3$ (30mg, 0.05 mmol) was added and stirred at room temperature for 5 minutes, then phenylsilane (67 mg, 0.62 mmol) was added, oxygen replacement was performed, and the reaction was performed at 30°C for 3 hours. After the reaction was completed, the reaction solution was extracted with a saturated ammonium chloride solution and DCM. The organic phase was backwashed with a saturated salt solution, dried over sodium sulfate, filtered, and concentrated under reduced pressure. The residue was purified by PreP-HPLC to obtain compound **B-163** (17 mg, white solid, yield of 14%). LCMS (ESI): m/z =504 [M+H]$^+$; $^1$H NMR (400 MHz, CD$_3$OD) δ 9.11 (s, 1H), 8.49 (s, 1H), 8.36 (d, J = 5.5 Hz, 1H), 7.59-7.47 (m, 2H), 7.23 (t, J = 7.8 Hz, 1H), 7.04 (t, J = 54.8 Hz, 1H), 6.40 (d, J = 5.6 Hz, 1H), 5.18 (q, J = 6.9 Hz, 1H), 4.00 (d, J = 13.7 Hz, 2H), 3.63 (t, J = 5.8 Hz, 2H), 3.37 (dd, J = 26.5, 9.2 Hz, 4H), 2.33 (td, J = 13.1, 4.4 Hz, 2H), 1.78 (dd, J = 16.6, 10.0 Hz, 5H).

The synthesis of the following compounds was the same as that of **B-163**.

| No. | Structure | Character | LCMS (ESI): *m/z* [M+H]$^+$ | $^1$H NMR |
|---|---|---|---|---|
| **B-164** | 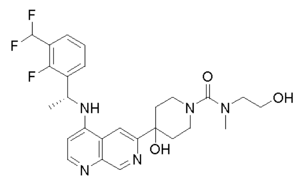 | White solid | 518.0 | $^1$H NMR (400 MHz, CD$_3$OD) δ 9.12 (s, 1H), 8.49 (s, 1H), 8.36 (d, *J* = 5.5 Hz, 1H), 7.61-7.48 (m, 2H), 7.23 (t, *J* = 7.7 Hz, 1H), 7.04 (t, *J* = 54.8 Hz, 1H), 6.40 (d, *J* = 5.6 Hz, 1H), 5.18 (q, *J* = 6.8 Hz, 1H), 3.76-3.64 (m, 4H), 3.47-3.35 (m, 4H), 2.99 (s, 3H), 2.41 (td, *J* = 13.2, 4.4 Hz, 2H), 1.82 (d, *J* = 13.1 Hz, 2H), 1.76 (d, *J* = 6.8 Hz, 3H). |

**Example 92 Synthesis of compound B-165**

Synthetic route:

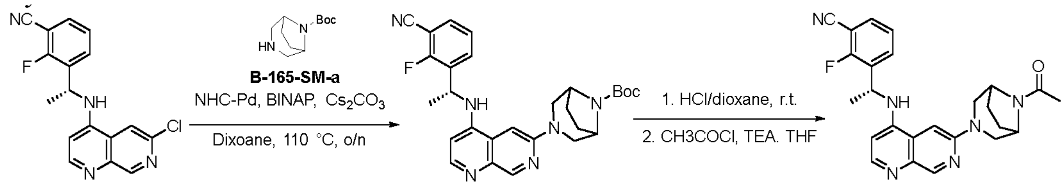


B-165-a B-165-b B-165

Step one:

Compound **B-165-a** (100 mg, 0.31 mmol), compound **B-165-SM-a** (130 mg, 0.61 mmol), NHC-Pd (60 mg, 0.09 mmol), BINAP (86 mg, 0.14 mmol), and cesium carbonate (300 mg, 0.92 mmol) were added in a flask containing 10 mL of 1,4-dioxane, and reacted while stirring at 100°C for 12 hours under argon protection. After the reaction was completed, the reaction solution was subjected to rotary evaporation and water was added. The solution was extracted with ethyl acetate, dried over sodium sulfate, concentrated, and subjected to column chromatography to obtain compound **B-165-b** (15 mg) with the yield of 64.9%. LCMS (ESI): m/z = 503.3 $[M+H]^+$

Step two:

Compound **B-165-b** (100 mg, 0.2 mmol) was added to a flask containing 10 mL of 1,4-dioxane, then 1 mL of (4N) HCl/dioxane was added and stirred at room temperature for one hour, the solvent was subjected to rotary evaporation, then 10 mL of tetrahydrofuran and triethylamine (61 mg, 0.6 mmol) were added, and acetyl chloride was added under ice bath (19 mg, 0.24 mmol) and reacted while stirring at room temperature for 12 hours under argon protection. After the reaction was completed, the reaction solution was subjected to rotary evaporation and water was added. The solution was extracted with ethyl acetate, dried over sodium sulfate, concentrated, and subjected to column chromatography to obtain compound **B-165** (15 mg) with the yield of 23.7%. LCMS (ESI): m/z = 444.9 $[M+H]^+$; $^1$H NMR (600 MHz, $CD_3OD$) $\delta$8.848 (s, 1H), 8.125 (d, *J* = 5.4 Hz, 1H), 7.705-7.653 (m, 2H), 7.308-7.271 (m,, 2H), 6.213 (d, *J* = 5.4 Hz, 1H), 5.181-5.146 (m, 1H), 4.825 (d, J =6.6 Hz, 1H), 4.506 (d, J =7.2 Hz, 1H), 4.366 -4.346 (m, 1H), 4.132 (d, *J* =12 Hz, 1H),3.129-3.104 (m, 2H), 2.188 (s, 3H), 2.120-2.079(m, 1H), 2.007-1.899 (m, 3H), 1.52 (d, *J* = 6.6Hz, 3H).

The synthesis of the following compounds was the same as that of **B-165**.

| No. | Structure | Character | LCMS (ESI): *m/z* $[M+H]^+$ | $^1$H NMR |
|---|---|---|---|---|
| **B-166** | 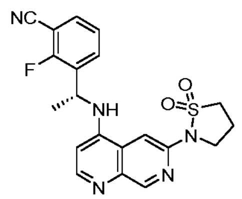 | Light yellow solid | 412.1 | $^1$H NMR (400 MHz, $CD_3OD$): $\delta$ 8.96 (s, 1H), 8.31 (d, J = 5.2 Hz, 1H), 7.82 (s, 1H) 7.73 -7.68 (m, 2H), 7.31-7.28 (m,, 1H), 6.37 (d, J = 6.0 Hz, 1H), 5.22-5.20 (m, 1H), 4.13 -4.10 (m, 2H), 3.57-3.53 (m, 2H), 2.62-2.58 (m, 2H), 2.54-2.43 (m, 2H), 1.76 (d, J = 7.2 Hz, 3H). |

**Example 93 Synthesis of compound B-169**

Synthetic route:

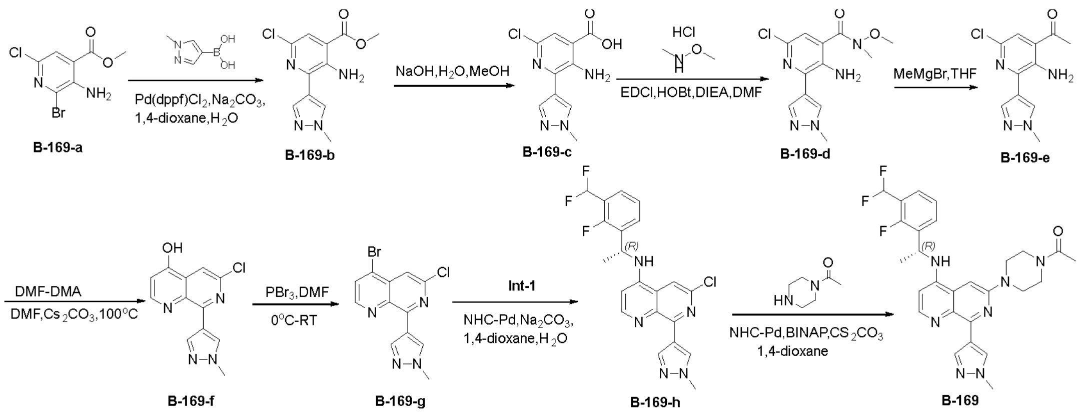


B-169-a B-169-b B-169-c B-169-d B-169-e B-169-f B-169-g B-169-h B-169

Step one:

1,4-dioxane (34 mL)/$H_2O$ (6 mL) was added to compound **B-169-a** (2 g, 7.55 mmol), methylpyrazole boric acid (1.42 g, 11.32 mmol), Pd(dppf)$Cl_2$ (0.54 g, 0.755 mmol), and $Na_2CO_3$ (2 g, 15.1 mmol), $N_2$ replacement was performed, and the reaction was heated to 90°C while stirring for 16 hours. The reaction solution was cooled, poured into an icy ammonium chloride solution to be quenched, and then extracted with EA. Then the organic phase was washed with a saturated salt solution, dried over sodium sulfate, filtered, and concentrated under reduced pressure. The residue was purified by a silica gel chromatographic column to obtain compound **B-169-b** (1.2 g, yellow solid, yield of 60%). LCMS (ESI): m/z = 267 $[M+H]^+$

Step two:

Compound **B-169-b** (1.2 g, 4.49 mmol) was dissolved in MeOH (15 mL) and 2 M NaOH (15 mL) was added, nitrogen replacement was performed, and reaction was performed at 75°C for 3 hours. After the reaction was completed, the pH was adjusted to be about 1-3 using dilute hydrochloric acid. The reaction solution was filtered to obtain compound **B-169-c** (1.2 mg, yellow solid crude product, yield of 100%). LCMS (ESI): m/z = 253 $[M+H]^+$

Step three:

DMF (25 mL) was added to compound **B-169-c** (1.2 g, 4.74 mmol), hydroxylamine hydrochloride (0.7 g, 3.32 mmol), EDCI (1.4 g, 7.11 mmol), HOBt (0.96 g, 7.11 mmol), and DIEA (1.83 g, 14.23 mmol), $N_2$ replacement was performed, and stirring was performed at room temperature for 16 hours. The reaction solution was poured into an icy ammonium chloride solution to be quenched and then extracted with EA. Then the organic phase was washed with a saturated salt solution, dried over sodium sulfate, filtered, and concentrated under reduced pressure. The residue was purified by the silica gel chromatographic column to obtain compound **B-169-d** (700 mg, yellow solid, yield of 50%). LCMS (ESI): m/z =296 $[M+H]^+$

Step four:

Compound **B-169-d** (690 mg, 2.33 mmol) was added to THF (14 mL), $N_2$ replacement was performed, the temperature was lowered to 0°C, 3 M MeMgBr (14 mL) was dropwise added, and the temperature was raised to room temperature for reaction. After the reaction was completed, the reaction solution was poured into an icy ammonium chloride solution to be quenched and then extracted with ethyl acetate. Then the organic phase was washed with a saturated salt solution, dried over sodium sulfate, filtered, and concentrated under reduced pressure. The residue was purified by the silica gel chromatographic column to obtain compound **B-169-e** (280 mg, yellow solid, yield of 48%). LCMS (ESI): m/z =251 $[M+H]^+$

Step five:

Compound **B-169-e** (280 mg, 1.12 mmol) and DMF-DMA (200 mg, 1.69 mmol) were added to DMF (6 mL), $N_2$ replacement was performed, the temperature was raised to 90°C, the reaction was performed for 4 hours, $Cs_2CO_3$ (1.82g, 5.57 mmol) was added, $N_2$ replacement was performed, the temperature was raised to 110°C, and the stirring was performed for 8 hours. After the reaction was completed, the reaction solution was cooled, a small amount of DMF

was added, the solution was filtered and then extracted by EA, and the aqueous phase was retained and concentrated under reduced pressure. The residue was purified by the silica gel chromatographic column to obtain compound **B-169-f** (147 mg, yellow solid, yield of 50.5%). LCMS (ESI): m/z = 261 $[M+H]^+$

Step six:

Compound **B-169-f** (147 mg, 0.56 mmol) was added to DMF (8 mL), $PBr_3$ (1.67 g, 6.18 mmol) was added under ice bath, $N_2$ replacement was performed, and the reaction was performed at room temperature for 1 hour. After the reaction was completed, a small amount of water was added, the pH was adjusted to be 7-8 by $NaHCO_3$, and then the solution was extracted with EA. Then the organic phase was washed with a saturated salt solution, dried over sodium sulfate, filtered, and concentrated under reduced pressure. The residue was purified by the silica gel chromatographic column to obtain compound **B-169-g** (84 mg, yellow solid, yield of 46.4%). LCMS (ESI): m/z = 324 $[M+H]^+$

Step seven:

Compound **B-169-g** (53 mg, 0.164 mmol), **Int-1** (39.1 mg, 0.173 mmol), $Pd(OAc)_2$ (7.35 mg, 0.033 mmol), BINAP (21 mg, 0.033 mmol), and t-BuONa (31 mg, 0.328 mmol) were added to toluene (10 mL), $N_2$ replacement was performed, the temperature was raised to 100°C, and the reaction was performed for 6 hours. After the reaction was completed, the reaction solution was cooled, poured into an icy ammonium chloride solution to be quenched, and then extracted with ethyl acetate. Then the organic phase was washed with a saturated salt solution, dried over sodium sulfate, filtered, and concentrated under reduced pressure. The residue was purified by the silica gel chromatographic column to compound **B-169-h** (45 mg, yellow solid, yield of 63%). LCMS (ESI): m/z = 432 $[M+H]^+$

Step eight:

Compound **B-169-h** (42.3 mg, 0.1 mmol), acetylpiperazine (62.7 mg, 0.5 mmol), NHC-Pd (9 mg, 0.013 mmol), BINAP (6 mg, 0.01 mmol), and $CS_2CO_3$ (100 mg, 0.3 mmol) were added to 1,4-dioxane (14 mL), $N_2$ replacement was performed, the temperature was raised to 110°C, and the reaction was performed in a sealed tube for 16 hours. After the reaction was completed, the reaction solution was poured into an icy ammonium chloride solution to be quenched and then extracted with EA. Then the organic phase was washed with a saturated salt solution, dried over sodium sulfate, filtered, and concentrated under reduced pressure. The residue was purified by PreP-HPLC to obtain compound **B-169** (13.8 mg, yellow solid, yield of 27.4%). LCMS (ESI): *m/z* = 524 $[M+H]^+$; $^1H$ NMR (400 MHz, DMSO-$d_6$) $\delta$ 8.76 (s, 1H), 8.35 (s, 1H), 8.19 (d, *J* = 4.8 Hz, 1H), 7.60-7.49 (m, 2H), 7.34-7.20 (m, 4H), 6.15 (d, *J* = 5.1 Hz, 1H), 5.08 (s, 1H), 3.91 (s, 3H), 3.70 (d, *J* = 25.2 Hz, 8H), 2.10 (s, 3H), 1.68 (d, *J* = 6.5 Hz, 3H).

Referring to the synthesis method of **B-113**, the following compounds were synthesized by replacing **B-1-c** in the original route with intermediate **B-169-h** or replacing hydroxyacetic acid with corresponding carboxylic acid.

| No. | Structure | Character | LCMS (ESI): *m/z* $[M+H]^+$ |
|---|---|---|---|
| **B-170** | 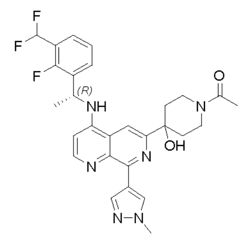 | White solid | 539.2 |
| **B-171** | 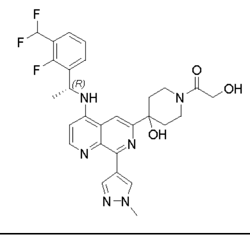 | White solid | 555.0 |

(continued)

| No. | Structure | Character | LCMS (ESI): $m/z$ $[M+H]^+$ |
|---|---|---|---|
| **B-172** | 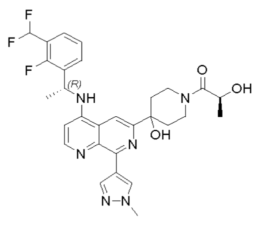 | White solid | 569.2 |

**Example 94 Synthesis of compound C-13**

Synthetic route:

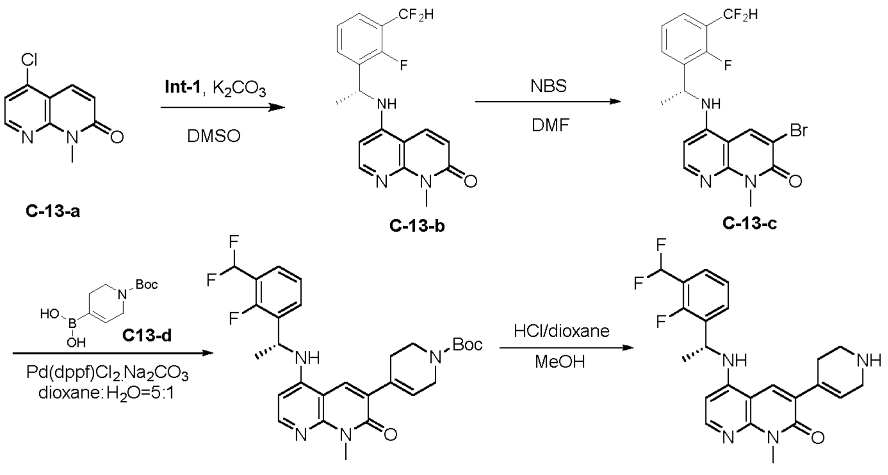


C-13-a, C-13-b, C-13-c, C13-d, C-13-e, C-13

Step one:

To a 100-mL flask, compound **C-13-a** (450 mg, 2.31 mmol, 1.0 eq), compound **Int-1** (573 mg, 2.54 mmol, 1.1 eq), and dimethyl sulfoxide (5 mL) were added, and dissolved while stirring, and potassium carbonate (956 mg, 6,93 mmol, 3.0 eq) was added and stirred at 130°C for 8 hours. LC-MS showed the reaction was completed and the reaction solution was poured into 50 mL of water. The reaction solution was extracted with 50 mL of ethyl acetate three times and the organic phases were combined. The organic phase was dried over anhydrous sodium sulfate for half an hour, and subjected to rotary evaporation and column chromatography to obtain compound **C-13-b.**

Step two:

To a 100-mL flask, compound **C-13-b** (1.0 g, 2.88 mmol, 1.0 eq), compound NBS (512 mg, 2.88 mmol, 1.0 eq), and N,N-dimethylformamide (10 mL) were added. After stirring at 50°C for 8 hours, LC-MS showed that the reaction was completed. 100 mL of water was added, the reaction solution was extracted with 20 mL of ethyl acetate three times, and the organic phases were combined. The organic phase was dried over anhydrous sodium sulfate for half an hour, and subjected to rotary evaporation and column chromatography to obtain compound **C-13-c.**

Step three:

To a solution of dioxane (1 mL) and water (0.2 mL) containing **C-13-c** (50 mg, 0.12 mmol), C13-d (41 mg, 0.18 mmol), sodium carbonate (38 mg, 0.18 mmol), and 1,1'-bisdiphenylphosphino ferrocene dichloropalladium (9 mg, 0.012 mmol) were added, argon replacement was performed for 3 times, and the reaction was performed while stirring at

100°C for 12 hours. After the reaction was completed, the reaction solution was diluted with water (30 mL) and extracted with ethyl acetate (3×30 mL). The extract was washed with a saturated salt solution (20 mL) and concentrated under reduced pressure. The residue was purified by a silica gel column chromatography (dichloromethane:methanol = 20/1) to obtain compound **C-13-e** (60 mg, white solid) with the yield of 95%. LCMS (ESI): m/z = 529.2 $[M+H]^+$.

Step four:

To methanol (1 mL) containing **C-13-e** (20 mg, 0.04 mmol), a hydrochloric acid/1,4-dioxane solution (1 mL, 4 M) was added and stirred at room temperature for 30 minutes. After the reaction was completed, the reaction solution was concentrated under reduced pressure. The residue was subjected to a reversed-phase chromatography (water/acetonitrile = 1/1) to obtain compound **C-13** (10.9 mg, white solid) with the yield of 64%. LCMS (ESI): m/z = 429.2 $[M+H]^+$; $^1$H NMR (400 MHz, $CDCl_3$): $\delta$ 10.15 (s, 2H), 8.04 (s, 1H), 7.69 (d, *J* = 9.8 Hz, 2H), 7.48 (t, *J* = 7.0 Hz, 1H), 7.21 (t, *J* = 7.7 Hz, 1H), 6.84 (t, *J* = 54.9 Hz, 1H), 6.61 (d, J = 9.8 Hz, 1H), 5.64 (s, 1H), 5.23 (s, 1H), 5.13-5.00 (m, 1H), 3.86 (s, 2H), 3.78 (s, 3H), 3.48 (s, 2H), 2.71 (s, 2H), 1.70 (d, *J* = 6.5 Hz, 3H).

**Example 95 Synthesis of compound C-14**

Synthetic route:

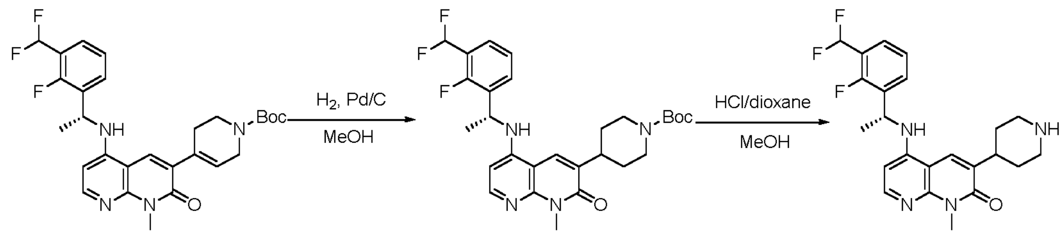


C-13-e C-14-a C-14

Step one:

**C-13-e** (40 mg, 0.076 mmol) was dissolved in methanol (2 mL), palladium/carbon (4 mg, 10%) was added, hydrogen replacement was performed 3 times, and reaction was performed while stirring at 65°C for 12 hours under a hydrogen (15 psi) atmosphere. After the reaction was completed, a catalyst was filtered and the filtrate was concentrated under reduced pressure. The residue was purified by a silica gel column chromatography (dichloromethane:methanol = 20:1) to obtain compound **C-14-a** (30 mg, yellow solid) with the yield of 75%. LCMS (ESI): m/z = 531.3 $[M+H]^+$.

Step two:

**C-14-a** (30 mg, 0.056 mmol) was dissolved in methanol (1 mL), and a hydrochloric acid/1,4-dioxane solution (1 mL, 4 M) was added and stirred at room temperature for 1 hour. After the reaction was completed, the reaction solution was concentrated under reduced pressure. The residue was subjected to a reversed-phase chromatography (water/acetonitrile = 1/3) to obtain compound **C-14** (8.01 mg, brown solid) with the yield of 31%. LCMS (ESI): m/z = 431.2 $[M+H]^+$; $^1$H NMR (600 MHz, $CD_3OD$): $\delta$ 8.06 (s, 1H), 7.95 (d, *J* = 10.0 Hz, 1H), 7.92 (t, *J* = 7.4 Hz, 1H), 7.63 (t, *J* = 7.0 Hz, 1H), 7.42 (t, *J* = 7.8 Hz, 1H), 7.02 (t, *J* = 54.7 Hz, 1H), 6.62 (d, J = 10.0 Hz, 1H), 5.40 (q, *J* = 6.7 Hz, 1H), 3.77 (s, 3H), 3.54 (t, *J* = 12.6 Hz, 2H), 3.24-3.15 (m, 2H), 1.90-2.19 (m, 5H), 1.86 (d, *J* = 6.7 Hz, 3H).

**Example 96 Synthesis of compound C-28**

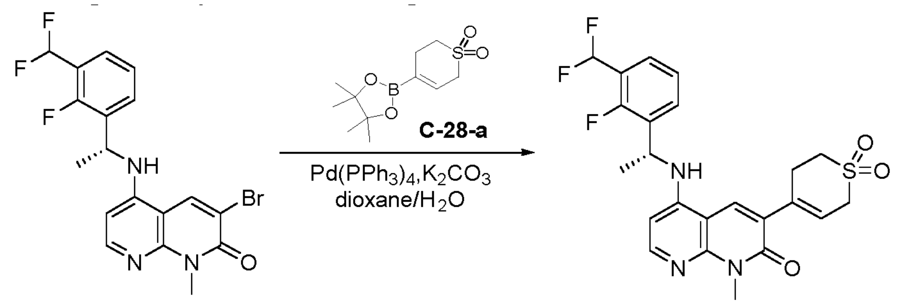


C-13-c C-28

To a single neck flask, **C-13-c** (128 mg, 0.3 mmol), **C-28-a** (155 mg, 0.6 mmol), and potassium carbonate (124 mg, 0.9 mmol) were successively added, and then a mixed solvent of 1,4-dioxane (5 mL) and water (2 mL) was added. Tetratriphenylphosphine palladium (52 mg, 0.045 mmol) was added to the above system, argon replacement was performed for 3 times, and the reaction was performed while stirring at 100°C for 12 hours. After the reaction was completed, the reaction solution was concentrated under reduced pressure. The residue was purified by a silica gel column chromatography (dichloromethane:methanol = 10/1) to obtain the crude product and the crude product was further purified by a reverse-phase column chromatography (water:acetonitrile = 1/0 to 1/3) to obtain compound **C-28** (102 mg, white solid) with the yield of 71%. LCMS (ESI): *m/z* = 478.1 $[M+H]^+$; $^1H$ NMR (600 MHz, $CDCl_3$): $\delta$ 8.08 (s, 1H), 7.78 (d, *J* = 9.9 Hz, 1H), 7.51 (t, *J* = 7.0 Hz, 1H), 7.38 (t, *J* = 7.2 Hz, 1H), 7.22 (t, *J* = 7.7 Hz, 1H), 6.85 (t, *J* = 54.9 Hz, 1H), 6.69 (d, *J* = 9.8 Hz, 1H), 5.61 (s, 1H), 5.09 (dt, *J* = 13.5, 6.7 Hz, 1H), 4.91 (s, 1H), 3.82-3.73 (m, 5H), 3.15-3.27 (m, 2H), 2.85-2.96 (m, 2H), 1.65 (d, *J* = 6.7 Hz, 3H).

**Detection of inhibitory effects of compounds on KRAS-G12C/SOS1**

Experimental objective: The inhibitory effects of the tested compounds on KRAS-G12C/SOS1 were detected. $IC_{50}$ was used to characterize the inhibitory ability of the compounds on the KRAS-G12C/SOS1. The lower $IC_{50}$ value indicated the stronger inhibitory ability. BI-3406 was used as a positive control compound.

Experimental reagents: KRASG12C/SOS binding kit (Cisbio, cat. 63ADK000CB16PEG); DMSO (Sigma, cat. D8418-1L); and 384-well white plate (PerkinElmer, cat. 6007290)

Experimental method:

1. Preparation of compounds: the test compounds were dissolved in 100% DMSO to obtain 10 mM stock solutions and the stock solutions were stored in a refrigerator in the dark.
2. Kinase reaction process:
Preparation of compounds: the test compounds had the concentration of 5,000 nM and diluted in a 384-well plate into a 100% DMSO solution at the 200-fold final concentration, and the compounds were diluted 3-fold with 10 concentrations. 50 nL of the compounds at the 200-fold final concentration were transferred to the plate of interest, the 384-well plate, using a dispenser Echo 550. 50 nL of 100% DMSO was added to each negative control well and positive control well.

A Tag1-SOS1 solution at the 4-fold final concentration was prepared using a diluent buffer.
2.5 µL of the Tag1-SOS1 solution at the 4-fold final concentration was added to the 384-well plate.
ATag2-KRAS-G12C solution at the 4-fold final concentration was prepared using the diluent buffer.
2.5 µL of the Tag2-KRAS-G12C solution at the 4-fold final concentration was respectively added into compound wells and the positive control wells; and 2.5 µL of the diluent buffer was added to the negative control wells.
The 384-well plate was centrifuged at 1,000 rpm for 30 seconds, shaken and mixed uniformly, and incubated at room temperature for 15 minutes.
An Anti-Tag1-TB3+ solution at the 1-fold final concentration and an Anti-Tag2-XL665 solution at the 1-fold final concentration were prepared using a detection buffer, the two solutions were mixed uniformly to obtain a Mix solution, and 5 µL of the Mix solution was added into each well.
The 384-well plate was centrifuged at 1,000 rpm for 30 seconds, shaken and mixed uniformly, and incubated at 4°C for 120 minutes.

Em665/620 was read using an Envision microplate reader.

Data Analysis

$$\text{Calculation formula}$$
$$\text{Inhibition\%} = (\text{Max signal} - \text{Compound signal})/(\text{Max signal} - \text{Min signal}) \times 100$$

wherein the Min signal was the mean value of the negative control wells, and the Max signal was the mean value of the positive control wells.

Dose-response curve fitting

A dose-response curve was fitted using the log value of the concentration as an X axis and the percentage of the inhibitory rate as a Y axis by using the log(inhibitor)vs.response-variable slope of an analytical software GraphPadPrism5, thereby obtaining the $IC_{50}$ value of each compound on an enzyme activity.

$$\text{Fitted formula: } Y = \text{Bottom} + (\text{Top} - \text{Bottom})/(1+10\text{\^{}}((\text{LogIC50} - X)*\text{HillSlope}))$$

Table 1 Inhibitory activity of compounds of examples on interaction of KRAS-G12C/SOS1

| No. | IC50 | No. | IC50 |
|---|---|---|---|
| A-1 | A | A-2 | A |
| A-3 | A | A-4 | B |
| A-5 | A | A-6 | A |
| A-7 | B | A-16 | A |
| A-17 | A | A-18 | A |
| A-39 | A | A-40 | A |
| A-41 | A | A-45 | A |
| A-46 | A | A-58 | A |
| A-59 | A | | |
| A-99 | A | A-100 | A |
| A-101 | A | A-102 | A |
| B-1 | A | B-3 | A |
| B-4 | A | B-17 | A |
| B-21 | A | B-22 | A |
| B-23 | A | B-24 | A |
| B-31 | A | B-32 | A |
| B-33 | A | B-34 | A |
| B-35 | A | B-36 | A |
| B-37 | A | B-38 | A |
| B-41 | A | B-43 | A |
| B-44 | A | B-45 | A |
| B-47 | A | B-48 | A |

(continued)

| No. | IC50 | No. | IC50 |
|---|---|---|---|
| B-49 | A | B-50 | A |
| B-51 | A | B-52 | A |
| B-53 | A | B-54 | A |
| B-55 | A | B-56 | A |
| B-57 | B | B-58 | A |
| B-59 | A | B-60 | A |
| B-61 | A | B-62 | A |
| B-63 | A | B-64 | A |
| B-65 | A | B-66 | A |
| B-67 | A | B-68 | A |
| B-69 | A | B-70 | A |
| B-71 | A | B-72 | A |
| B-73 | A | B-74 | A |
| B-75 | A | B-76 | A |
| B-77 | A | B-78 | A |
| B-79 | A | B-80 | P1: A; P2: A |
| B-81 | P1: A; P2: A | B-82 | A |
| B-83 | P1: A; P2: A | B-84 | P1: B; P2: B |
| B-85 | P1: A; P2: A | B-86 | P1: B; P2: B |
| B-87 | P1: B; P2: B | B-88 | P1: A; P2: A |
| B-89 | P1: A; P2: A | B-90 | P1: A; P2: A |
| B-91 | A | B-94 | P1: A; P2: A |
| B-95 | P1: A; P2: A | B-96 | P1: A; P2: A |
| B-97 | P1: A; P2: A | B-99 | A |
| B-100 | P1: A; P2: A | B-101 | P1: B; P2: A |
| B-102 | P1: A; P2: A | B-103 | A |
| B-104 | A | B-105 | A |
| B-106 | A | B-107 | A |
| B-108 | A | B-109 | A |
| B-110 | A | B-111 | A |
| B-112 | A | B-113 | A |
| B-114 | A | B-115 | P1: B; P2: A |
| B-116 | A | B-117 | A |
| B-118 | A | B-119 | A |
| B-121 | A | B-122 | A |
| B-123 | A | B-124 | A |
| B-125 | A | B-126 | A |
| B-127 | A | B-128 | A |

(continued)

| No. | IC50 | No. | IC50 |
|---|---|---|---|
| B-129 | A | B-130 | A |
| B-131 | A | B-132 | A |
| B-133 | A | B-134 | A |
| B-135 | A | B-136 | A |
| B-137 | A | B-138 | A |
| B-139 | A | B-140 | A |
| B-141 | A | B-144 | A |
| B-145 | A | B-147 | A |
| B-148 | A | B-149 | A |
| B-150 | A | B-151 | A |
| B-152 | A | B-153 | A |
| B-156 | A | B-159 | A |
| B-160 | A | B-161 | A |
| B-162 | A | B-163 | A |
| B-164 | A | B-165 | A |
| B-167 | A | B-168 | B |
| B-169 | A | B-170 | A |
| B-171 | A | B-172 | B |
| B-173 | A | B-174 | B |
| B-175 | A | B-176 | A |
| B-177 | A | B-178 | P1: A; P2: A |
| Note: in $IC_{50}$, "A" indicates $IC_{50} \leq 100$ nM, "B" indicates 100 nM < $IC_{50} \leq 500$ nM, "C" indicates 500 nM < $IC_{50} \leq$ 5,000 nM, "D" indicates $IC_{50}$ > 5,000 nM, and "-" indicates not detected. | | | |

**IC50 values of compounds determined by p-ERK HTRF assay in PC-9 cells**

Experimental objective: an inhibitory effect of the test compounds on the phosphorylation of PC-9ERK of lung cancer cells at a cellular level was evaluated by detecting the phosphorylation of ERK.

**Materials and reagents:**

PRMI 1640 medium (Invitrogen-11875093)
0.25% pancreatin (Invitrogen-25200056)
Phosphoric acid buffer salt solution (PBS, pH 7.4) (Invitrogen-10010023)
Double-antibody (PS) (Invitrogen-15140122)
Fetal bovine serum (FBS) (Gibco-10091148)
Trypan blue (Gibco-15250061)
10-cm culture dish (Corning-430167)
10-mL pipette (Costar-4488)
Pipettor (Sartorius-17009031)
15-mL centrifuge tube (Corning-430052)
50-mL centrifuge tube (Corning-430829)

Cryotube (Thermo-377267)
1.5-mL EP tube (Axygen-MCT-150-C-S)
Cell incubator (Thermo-371)
Microscope (Olympus-CKX41)
Centrifuge (Flying Pigeon-TDL-80-2B)
Cell counter (Countstar-IC-1000)
Envision (Perkin Elmer)
Spark 10M (TECAN)
Phospho-ERK1/2 (Thr202/Tyr204) Experimental kit (Cisbio, Cat. No. 64AERPEG)
PC-9 cells: RPMI1640+10%FBS+1%PS

**Experimental steps:**

1. Plating (Day1)

1) PBS, 0.25% pancreatin, and a cell culture medium were placed in a water-bath kettle at 37°C for preheating;
2) cells were observed under a microscope, a fusion degree was evaluated, and no bacterial and fungi pollution was confirmed;
3) a culture solution was removed, the cells were washed with 5 mL of PBS and digested with 2 mL of the 0.25% trypsin, the digestion was terminated with 10 mL of the fresh cell culture medium, a culture dish was rinsed, and the cells were transferred to a 15-mL centrifuge tube;
4) the collected cells were centrifuged at 1,000 rpm for 5 minutes;
5) after the centrifugation, the supernatant was discarded, the cells were resuspended in 5 mL of the cell culture medium, 20 μL of the resuspended cells were aspirated, and the number and viability of viable cells were recorded;
6) the cell culture medium was used and the cell density was adjusted (PC-9:10,000 cells/well, and 45 μL per well);
7) in a 384-well plate, 45 μL of a cell suspension per well was transferred according to an experimental design figure; and
8) the cells were incubated overnight at 37°C and 5%CO2.

2. Preparation of compound dose gradient solutions (Day2)

1) The compounds were diluted from the stock concentration to 10 mM with 100% DMSO (Trametinib was diluted to 1 mM), then 3-fold diluted into 10 points (5 μL of the diluted compounds with the concentration of 10 mM were transferred to 10 μL of the DMSO in an LDV plate), and centrifuged at 1,000 rpm/min for 1 minute;
2) 5 μL of the diluted compounds of step 1) were transferred to an inter plate 1 containing 45 μL of the cell culture medium (the compounds were diluted from 1,000 X to 100 X);
3) 5 μL of the diluted compounds of step 2) were transferred to an inter plate 2 containing 45 μL of the cell culture medium (the compounds were diluted from 100 X to 10 X);
4) 5 μL of the compounds were pipetted from the inter plate 2 into a cell culture plate (the compounds were diluted from 10 folds to 1 fold), the final concentrations of the test compounds were 10,000 nM, 3333.33 nM, 1111.11 nM, 370.37 nM, 123.46 nM, 41.15 nM, 13.72 nM, 4.57 nM, 1.52 nM, and 0.21 nM, the solution was centrifuged at 1,000 rpm/min for 1 minute, and the final content of the DMSO was 0.1%; and
5) the cells were treated in an incubator at 37°C for 1 hour.

3. Detection (Day2)

1) The cell plate was removed from the incubator at 37°C;
2) the cell plate was centrifuged upside down in a centrifuge at a rotation speed of 450 revolutions for 25 seconds;
3) the pre-heated PBS (50 nL/well) was immediately added, and the solution was centrifuged half a minute at 1,000 rpm and then centrifuged upside down according to step 2);
4) 30 μL of a lysate prepared in advance (if prepared in advance, it should be placed at 4°C) was added and the solution was centrifuged at 1,000 rpm for 1 minute;

5) the cells were lysed at room temperature for 1 hour;
6) a mixed solution of Eu3+-Cryptate antibody (donor) and d2 antibody (receptor) was prepared;
7) 4 μL of the mixed solution of step 6) was added in a 384-well plate (grenier-784075);
8) 16 μL of the cell lysate was transfered to the 784075 containing the mixed solution using bravo after one hour of the lysis; and
9) the plate was sealed and reading (665nm/615nm) was performed using the Envision overnight at room temperature.

**Data Analysis**

Calculation formula

$$\text{Inhibition\%} = (\text{Max signal} - \text{Compound signal})/(\text{Max signal} - \text{Min signal}) \times 100$$

wherein the Min signal was the mean value of the negative control wells, and the Max signal was the mean value of the positive control wells.

Dose-response curve fitting

A dose-response curve was fitted using the log value of the concentration as an X axis and the percentage of the inhibitory rate as a Y axis by using the log(inhibitor)vs.response-variable slope of an analytical software GraphPadPrism5, thereby obtaining the $IC_{50}$ value of each compound on an inhibitory activity on p-ERK of PC-9 cells.

Fitted formula: Y = Bottom+(Top-Bottom)/(1+10^((LogIC50-X)*HillSlope))

Table 2 Inhibitory activity of compounds of examples on p-ERK of PC-9 cells

| No. | IC50 | No. | IC50 |
|---|---|---|---|
| A-99 | A | A-100 | A |
| A-101 | B | A-102 | A |
| B-1 | B | B-3 | A |
| B-4 | A | B-17 | A |
| B-21 | B | B-22 | B |
| B-23 | A | B-24 | A |
| B-25 | A | B-26 | A |
| B-27 | A | B-28 | B |
| B-29 | B | B-30 | A |
| B-31 | B | B-32 | A |
| B-33 | B | B-34 | A |
| B-35 | C | B-36 | A |
| B-37 | C | B-38 | A |
| B-41 | A | B-44 | A |
| B-45 | A | B-47 | A |
| B-48 | A | B-49 | A |
| B-50 | A | B-51 | A |
| B-52 | A | B-53 | B |
| B-54 | B | B-56 | A |
| B-60 | A | B-62 | A |

(continued)

| No. | IC50 | No. | IC50 |
|---|---|---|---|
| B-63 | A | B-64 | B |
| B-65 | B | B-67 | B |
| B-68 | C | B-69 | C |
| B-70 | B | B-71 | A |
| B-72 | B | B-73 | A |
| B-74 | A | B-75 | B |
| B-76 | A | B-78 | A |
| B-79 | A | B-80 | P1: A; P2: B |
| B-81 | P1: A; P2: - | B-82 | A |
| B-83 | P1: B; P2: - | B-85 | P1: B; P2: A |
| B-87 | P1: C; P2: C | B-88 | P1: -; P2: B |
| B-89 | P1: -; P2: B | B-90 | P1: A; P2: - |
| B-91 | B | B-94 | P1: -; P2: A |
| B-96 | P1: A; P2: - | B-97 | P1: -; P2: B |
| B-100 | P1: -; P2: A | B-101 | P1: -; P2: A |
| B-102 | P1: B; P2: A | B-103 | B |
| B-104 | B | B-107 | A |
| B-108 | A | B-109 | A |
| B-110 | A | B-111 | A |
| B-112 | A | B-113 | A |
| B-114 | A | B-115 | P1: C; P2: A |
| B-116 | A | B-117 | B |
| B-118 | A | B-119 | A |
| B-120 | A | B-121 | A |
| B-122 | C | B-123 | A |
| B-124 | A | B-125 | A |
| B-126 | A | B-127 | A |
| B-128 | A | B-129 | B |
| B-130 | B | B-131 | A |
| B-132 | A | B-133 | A |
| B-134 | A | B-135 | B |
| B-136 | C | B-137 | C |
| B-138 | A | B-139 | A |
| B-140 | A | B-141 | A |
| B-142 | C | B-143 | C |
| B-144 | C | B-145 | C |
| B-147 | C | B-148 | A |
| B-149 | C | B-150 | C |

(continued)

| No. | IC50 | No. | IC50 |
|---|---|---|---|
| B-151 | C | B-152 | B |
| B-153 | B | B-156 | A |
| B-159 | B | B-160 | A |
| B-161 | B | B-162 | B |
| B-163 | A | B-164 | A |
| B-165 | B | B-166 | A |
| B-167 | C | B-168 | C |
| B-169 | A | B-170 | A |
| B-171 | A | B-172 | B |
| B-173 | A | B-174 | B |
| B-175 | A | B-176 | B |
| B-177 | A | B-178 | P1: C; P2: A |
| Note: in $IC_{50}$, "A" indicates $IC_{50} \leq 300$ nM, "B" indicates 300 nM < $IC_{50} \leq$ 1,000 nM, "C" indicates $IC_{50}$ > 1,000 nM, and "-" indicates not detected. | | | |

## Claims

1. A compound of formula (I), and an enantiomer, a diastereoisomer, a racemate, a prodrug, a hydrate, a solvate, or a pharmaceutically acceptable salt thereof,

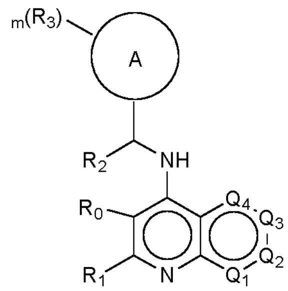

(I)

wherein,

ring A is $C_{6-10}$ aryl, 5-10 membered heteroaryl, or 4-10 membered saturated or unsaturated heterocyclyl;
$m(R_3)$ represents m identical or different $R_3$ substituents at any position of ring A;
m is 0-5; preferably, m is 1, 2, or 3; and more preferably, m is 1 or 2;
each $R_3$ substituent is independently selected from: hydrogen, unsubstituted or substituted $C_{1-4}$ alkyl, unsubstituted or substituted $C_{1-4}$ alkoxy, unsubstituted or substituted $C_{2-4}$ alkynyl, unsubstituted or substituted $C_{3-6}$ cycloalkyl, unsubstituted or substituted 4-6 membered saturated or unsaturated heterocyclyl, halogen, cyano, amino, and a divalent substituent =O; the substitution refers to being substituted by one or more substituents selected from halogen, hydroxy, cyano, and amino; and when ring A is $C_{6-10}$ aryl or 5-10 membered heteroaryl, $R_3$ is not a divalent substituent =O;
$R_0$ is hydrogen, halogen, $C_{1-4}$ alkyl, cyano, or cyclopropyl;
$R_1$ is hydrogen, halogen, unsubstituted or substituted $C_{1-6}$ alkyl, unsubstituted or substituted $C_{3-6}$ cycloalkyl, unsubstituted or substituted 4-10 membered saturated or unsaturated heterocyclyl, unsubstituted or substituted $C_{1-6}$ alkoxy, -CN, -COOH, $-CONH_2$, $-CONH-C_{1-6}$ alkyl, amino, or $-NH-C_{1-6}$ alkyl;

$R_2$ is hydrogen, unsubstituted or substituted $C_{1-6}$ alkyl, or unsubstituted or substituted $C_{3-6}$ cycloalkyl;
$Q_1$ is N, $NR_4$, -C(O)-, or $CR_4$, $Q_2$ is N, $NR_5$, -C(O)-, or $CR_5$, $Q_3$ is N, $NR_6$, -C(O)-, or $CR_6$, $Q_4$ is N or CH; at most two N atoms are contained in $Q_1$, $Q_2$, $Q_3$, and $Q_4$; and a ring where $Q_1$, $Q_2$, $Q_3$, and $Q_4$ located and a fused ring thereof both remain aromaticity;
$R_4$ is selected from hydrogen, unsubstituted or substituted $C_{1-4}$ alkyl, unsubstituted or substituted $C_{1-4}$ alkoxy, unsubstituted or substituted $C_{2-4}$ alkynyl, unsubstituted or substituted $C_{3-6}$ cycloalkyl, unsubstituted or substituted 4-6 membered saturated or unsaturated heterocyclyl, halogen, cyano, and amino;
$R_5$ and $R_6$ are each independently hydrogen, unsubstituted or substituted $C_{1-10}$ alkyl, unsubstituted or substituted $C_{6-10}$ aryl, unsubstituted or substituted 5-10 membered heteroaryl, unsubstituted or substituted $C_{3-6}$ cycloalkyl, unsubstituted or substituted 4-10 membered saturated or unsaturated heterocyclyl, unsubstituted or substituted 5-10 membered heteroarylo-fused 4-10 membered heterocyclyl, halogen, -CN, -COOH, $-OR_7$, $-NH-R_7$, $-CONH-R_7$, $-NHCO-R_7$, $-SO_2-R_7$, or $-SO_2NH-R_7$;
$R_7$ is selected from hydrogen, unsubstituted or substituted $C_{1-10}$ alkyl, unsubstituted or substituted $C_{6-10}$ aryl, unsubstituted or substituted 5-10 membered heteroaryl, unsubstituted or substituted $C_{3-6}$ cycloalkyl, and unsubstituted or substituted 4-10 membered saturated or unsaturated heterocyclyl; and
the substitution in $R_1$, $R_2$, $R_4$, $R_5$, $R_6$, and $R_7$ refers to being substituted by one or more group A substituents, which include: $C_{1-6}$ alkyl, $C_{1-6}$ halogenated alkyl, hydroxy-substituted $C_{1-6}$ alkyl, amino-substituted $C_{1-6}$ alkyl, cyano-substituted $C_{1-6}$ alkyl, $C_{1-6}$ alkoxy-substituted $C_{1-6}$ alkyl, $C_{1-6}$ alkoxy, hydroxy, halogen, cyano, amino, carboxy, $-NH-C_{1-6}$ alkyl, $-NH-C_{3-6}$ cycloalkyl, unsubstituted or group B substituent-substituted $C_{6-10}$ aryl, unsubstituted or group B substituent-substituted 5-10 membered heteroaryl, unsubstituted or group B-substituted $C_{3-6}$ cycloalkyl, unsubstituted or group B substituent-substituted 4-10 membered saturated or unsaturated heterocyclyl, -C(O)-Rs, -C(O)O-Rs, $-C(O)-CH_2-R_8$, -C(O)-NH-Rs, -NH-C(O)-Rs, $-SO_2-R_8$, and $-SO_2NH-R_8$; $R_8$ is selected from hydrogen, unsubstituted or group B substituent-substituted $C_{1-10}$ alkyl, unsubstituted or group B substituent-substituted $C_{1-10}$ alkoxy, unsubstituted or group B substituent-substituted $C_{6-10}$ aryl, unsubstituted or group B substituent-substituted 5-10 membered heteroaryl, unsubstituted or group B substituent-substituted $C_{3-6}$ cycloalkyl, and unsubstituted or group B substituent-substituted 4-10 membered saturated or unsaturated heterocyclyl; and the group B substituent includes: unsubstituted or halogenated $C_{1-6}$ alkyl, unsubstituted or halogenated $C_{1-6}$ alkoxy, hydroxy, halogen, cyano, amino, carboxy, and $-NH-C_{1-6}$ alkyl,
for example, the substitution in $R_1$, $R_2$, $R_4$, $R_5$, $R_6$, and $R_7$ refers to being substituted by one or more group A substituents, which include: $C_{1-6}$ alkyl, $C_{1-6}$ halogenated alkyl, $C_{1-6}$ alkoxy, hydroxy, halogen, cyano, amino, carboxy, $-NH-C_{1-6}$ alkyl, $-NH-C_{3-6}$ cycloalkyl, unsubstituted or group B substituent-substituted $C_{6-10}$ aryl, unsubstituted or group B substituent-substituted 5-10 membered heteroaryl, unsubstituted or group B-substituted $C_{3-6}$ cycloalkyl, unsubstituted or group B substituent-substituted 4-10 membered saturated or unsaturated heterocyclyl, $-C(O)-R_8$, $-C(O)-NH-R_8$, $-NH-C(O)-R_8$, $-SO_2$-R8, and $-SO_2NH-R_8$; $R_8$ is selected from hydrogen, unsubstituted or group B substituent-substituted $C_{1-10}$ alkyl, unsubstituted or group B substituent-substituted $C_{6-10}$ aryl, unsubstituted or group B substituent-substituted 5-10 membered heteroaryl, unsubstituted or group B substituent-substituted $C_{3-6}$ cycloalkyl, and unsubstituted or group B substituent-substituted 4-10 membered saturated or unsaturated heterocyclyl; and the group B substituent includes: $C_{1-6}$ alkyl, $C_{1-6}$ alkoxy, hydroxy, halogen, cyano, amino, and carboxy.

**2.** The compound of formula (I), and the enantiomer, the diastereoisomer, the racemate, the prodrug, the hydrate, the solvate, or the pharmaceutically acceptable salt thereof according to claim 1,
wherein $R_2$ is methyl or ethyl.

**3.** The compound of formula (I), and the enantiomer, the diastereoisomer, the racemate, the prodrug, the hydrate, the solvate, or the pharmaceutically acceptable salt thereof according to claim 1,
wherein ring A is phenyl.

**4.** The compound of formula (I), and the enantiomer, the diastereoisomer, the racemate, the prodrug, the hydrate, the solvate, or the pharmaceutically acceptable salt thereof according to claim 1,

wherein m is 1 or 2; and
each $R_3$ substituent is independently selected from: substituted or unsubstituted $C_{1-4}$ alkyl, substituted or unsubstituted $C_{2-4}$ alkynyl, substituted or unsubstituted 4-6 membered saturated or unsaturated heterocyclyl, halogen, cyano, and amino; the substitution refers to being substituted by one or more substituents selected from halogen, hydroxy, cyano, and amino.

**5.** The compound of formula (I), and the enantiomer, the diastereoisomer, the racemate, the prodrug, the hydrate, the solvate, or the pharmaceutically acceptable salt thereof according to claim 1,

solvate, or the pharmaceutically acceptable salt thereof according to any one of claims 1-4, wherein $R_4$ is selected from hydrogen and $C_{1-4}$ alkyl.

**6.** The compound of formula (I), and the enantiomer, the diastereoisomer, the racemate, the prodrug, the hydrate, the solvate, or the pharmaceutically acceptable salt thereof according to claim 1, wherein the compound of formula (I) has the structures of formulas (I-1-1)-(I-1-7):

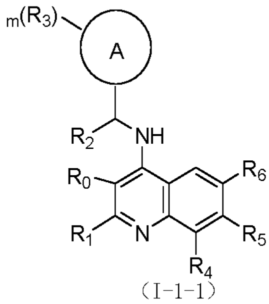
(I-1-1)

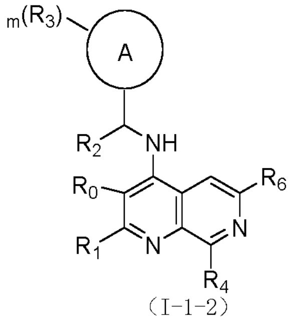
(I-1-2)

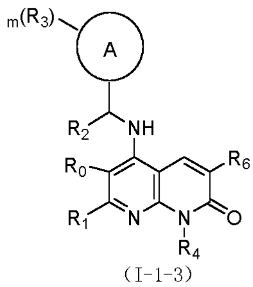
(I-1-3)

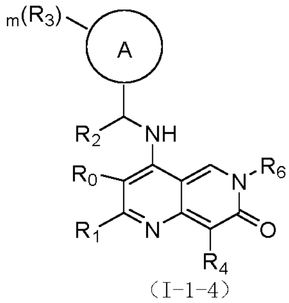
(I-1-4)

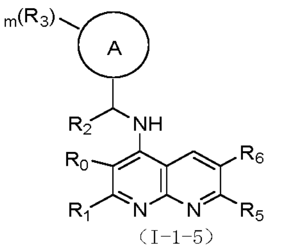
(I-1-5)

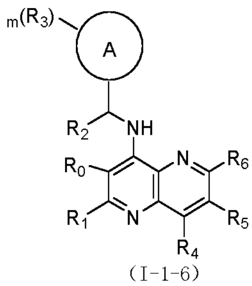
(I-1-6)

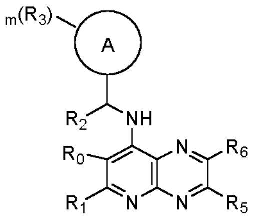
(I-1-7)

wherein,
ring A, $R_0$, $R_1$, $R_2$, $R_3$, $R_4$, $R_5$, $R_6$, and m are as defined in claim 1.

**7.** The compound of formula (I), and the enantiomer, the diastereoisomer, the racemate, the prodrug, the hydrate, the solvate, or the pharmaceutically acceptable salt thereof according to any one of claims 1-6,

wherein $R_5$ and $R_6$ are each independently hydrogen, unsubstituted or substituted $C_{1-10}$ alkyl, unsubstituted or substituted $C_{6-10}$ aryl, unsubstituted or substituted 5-10 membered heteroaryl, unsubstituted or substituted $C_{3-6}$ cycloalkyl, unsubstituted or substituted 4-10 membered saturated or unsaturated heterocyclyl, unsubstituted or substituted 5-membered heteroarylo-fused 6-membered heterocyclyl, halogen, -CN, -COOH, $-OR_7$, $-NH-R_7$, $-CONH-R_7$, $-NHCO-R_7$, $-SO_2-R_7$, or $-SO_2NH-R_7$;
$R_7$ is selected from hydrogen, unsubstituted or substituted $C_{1-10}$ alkyl, unsubstituted or substituted $C_{6-10}$ aryl, unsubstituted or substituted 5-10 membered heteroaryl, unsubstituted or substituted $C_{3-6}$ cycloalkyl, and unsubstituted or substituted 4-10 membered saturated or unsaturated heterocyclyl; and
the substitution in $R_5$, $R_6$, and $R_7$ refers to being substituted by one or more group A substituents, which include: $C_{1-6}$ alkyl, $C_{1-6}$ halogenated alkyl, hydroxy-substituted $C_{1-6}$ alkyl, amino-substituted $C_{1-6}$ alkyl, cyano-substituted $C_{1-6}$ alkyl, $C_{1-6}$ alkoxy-substituted $C_{1-6}$ alkyl, $C_{1-6}$ alkoxy, hydroxy, halogen, cyano, amino, carboxy, $-NH-C_{1-6}$ alkyl, $-NH-C_{3-6}$ cycloalkyl, unsubstituted or group B substituent-substituted $C_{6-10}$ aryl, unsubstituted or group B

substituent-substituted 5-10 membered heteroaryl, unsubstituted or group B-substituted $C_{3-6}$ cycloalkyl, unsubstituted or group B substituent-substituted 4-10 membered saturated or unsaturated heterocyclyl, -C(O)-$R_8$, -C(O)O-$R_8$, -C(O)-$CH_2$-$R_8$, -C(O)-NH-$R_8$, -NH-C(O)-$R_8$, -$SO_2$-$R_8$, and -$SO_2$NH-$R_8$; $R_8$ is selected from hydrogen, unsubstituted or group B substituent-substituted $C_{1-10}$ alkyl, unsubstituted or group B substituent-substituted $C_{6-10}$ aryl, unsubstituted or group B substituent-substituted 5-10 membered heteroaryl, unsubstituted or group B substituent-substituted $C_{3-6}$ cycloalkyl, and unsubstituted or group B substituent-substituted 4-10 membered saturated or unsaturated heterocyclyl; and the group B substituent includes: unsubstituted or halogenated $C_{1-6}$ alkyl, unsubstituted or halogenated $C_{1-6}$ alkoxy, hydroxy, halogen, cyano, amino, carboxy, and -NH-$C_{1-6}$ alkyl, for example, the substitution in $R_5$, $R_6$, and $R_7$ refers to being substituted by one or more group A substituents, which include: $C_{1-6}$ alkyl, $C_{1-6}$ halogenated alkyl, $C_{1-6}$ alkoxy, hydroxy, halogen, cyano, amino, carboxy, -NH-$C_{1-6}$ alkyl, -NH-$C_{3-6}$ cycloalkyl, unsubstituted or group B substituent-substituted $C_{6-10}$ aryl, unsubstituted or group B substituent-substituted 5-10 membered heteroaryl, unsubstituted or group B-substituted $C_{3-6}$ cycloalkyl, unsubstituted or group B substituent-substituted 4-10 membered saturated or unsaturated heterocyclyl, -C(O)-$R_8$, -C(O)-NH-$R_8$, -NH-C(O)-$R_8$, -$SO_2$-$R_8$, and -$SO_2$NH-$R_8$; $R_8$ is selected from hydrogen, unsubstituted or group B substituent-substituted $C_{1-10}$ alkyl, unsubstituted or group B substituent-substituted $C_{6-10}$ aryl, unsubstituted or group B substituent-substituted 5-10 membered heteroaryl, unsubstituted or group B substituent-substituted $C_{3-6}$ cycloalkyl, and unsubstituted or group B substituent-substituted 4-10 membered saturated or unsaturated heterocyclyl; and the group B substituent includes: $C_{1-6}$ alkyl, $C_{1-6}$ alkoxy, hydroxy, halogen, cyano, amino, and carboxy;

the heteroaryl in $R_5$, $R_6$, $R_7$, and the group A substituent is selected from

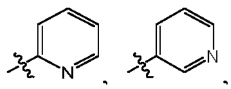

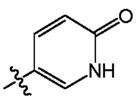, 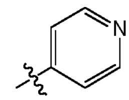, 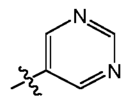, 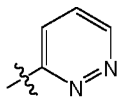, 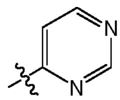, 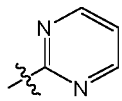, 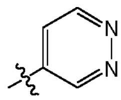, 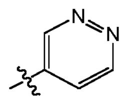,

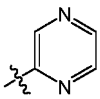, 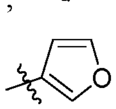, 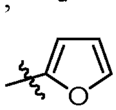, 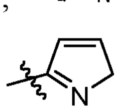, 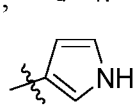, 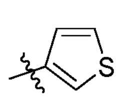, 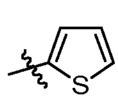, 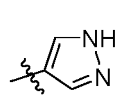,

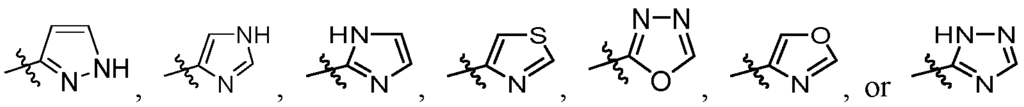

for example, selected from

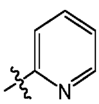, 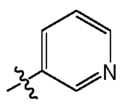, 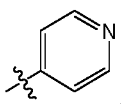, 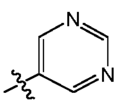, 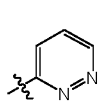, 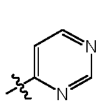, 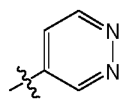, 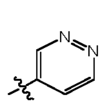,

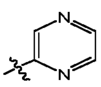, 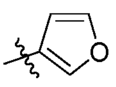, 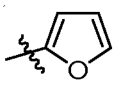, 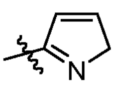, 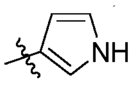, 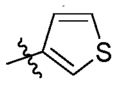, 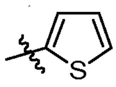, 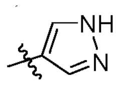,

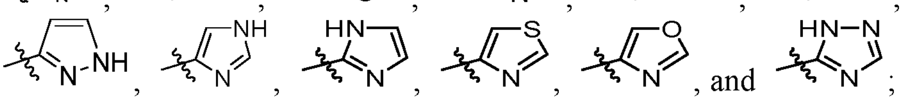

the heterocyclyl in $R_5$, $R_6$, $R_7$, and the group A substituent is selected from

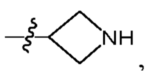,

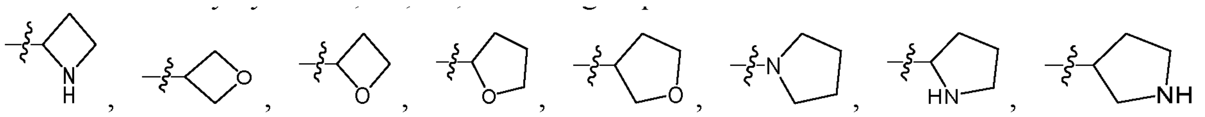,

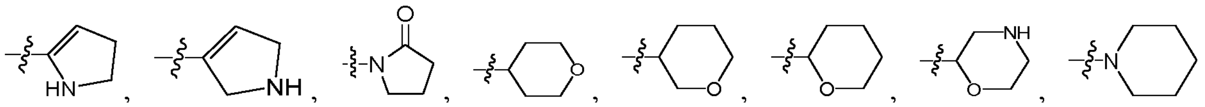,

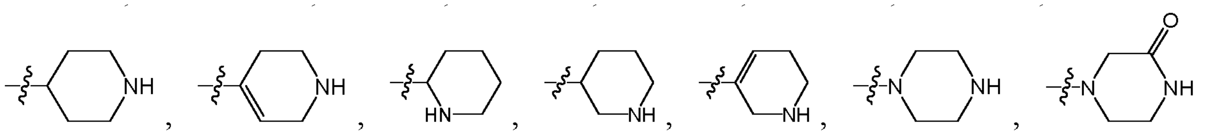,

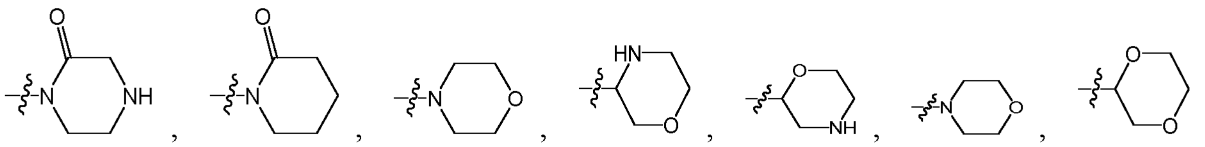,

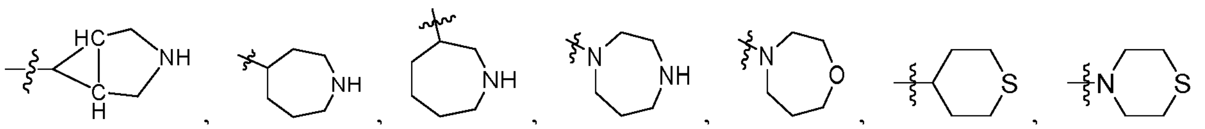,

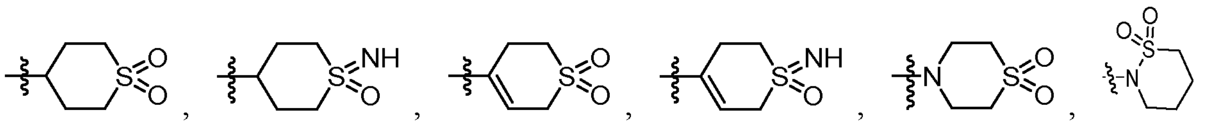,

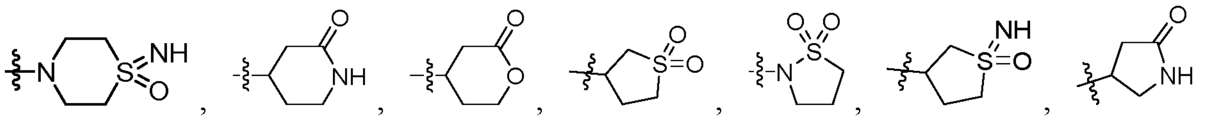,

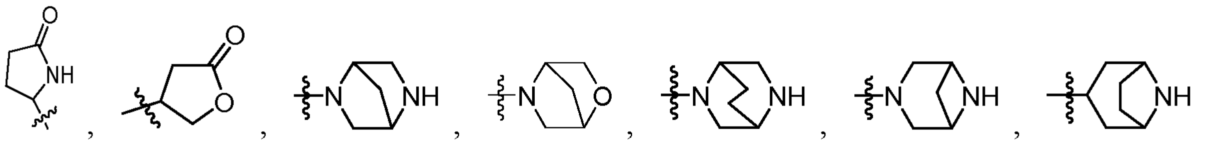,

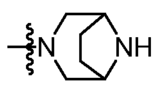, and 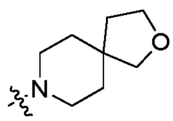,

for example, selected from

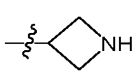, 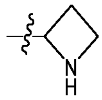, 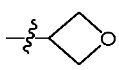,

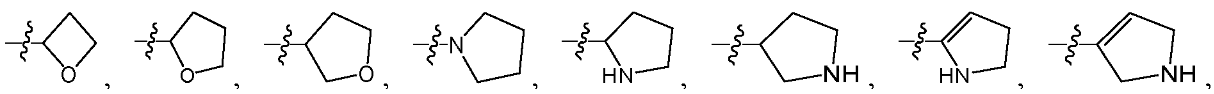
, , , , , , , ,

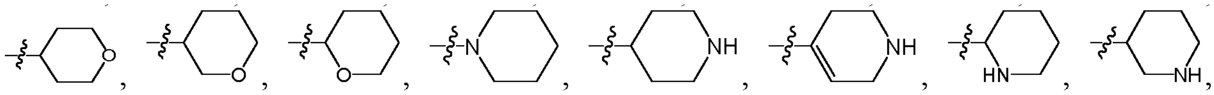
, , , , , , , ,

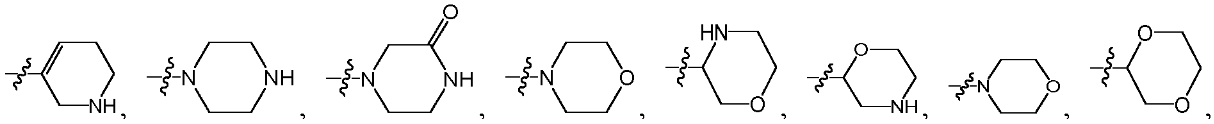
, , , , , , , ,

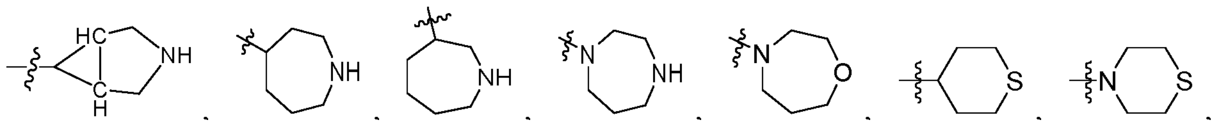
, , , , , , ,

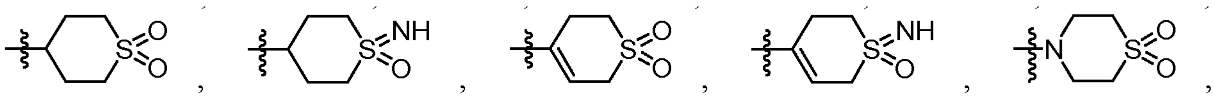
, , , , ,

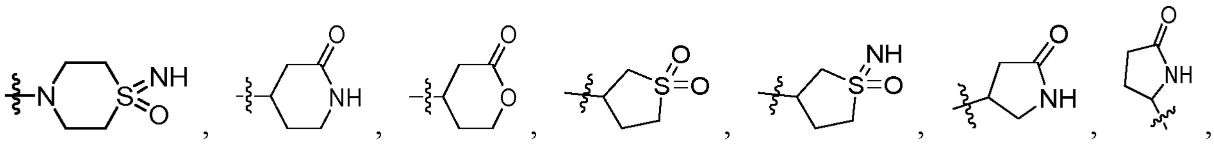
, , , , , , ,

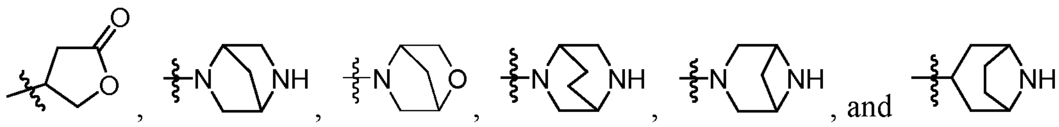
, , , , , and ;

and

the 5-membered heteroarylo-fused 6-membered heterocvclvl is selected from

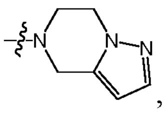
,

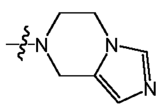
, and
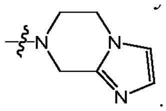
.

**8.** The compound of formula (I), and the enantiomer, the diastereoisomer, the racemate, the prodrug, the hydrate, the solvate, or the pharmaceutically acceptable salt thereof according to claim 7,

wherein $R_5$ is hydrogen or $C_{1-10}$ alkoxy;

$R_6$ is selected from unsubstituted or substituted $C_{1-10}$ alkyl, unsubstituted or substituted $C_{6-10}$ aryl, unsubstituted or substituted 5-10 membered heteroaryl, unsubstituted or substituted $C_{3-6}$ cycloalkyl, unsubstituted or substituted 4-10 membered saturated or unsaturated heterocyclyl, unsubstituted or substituted 5-10 membered heteroarylo-fused 4-10-membered heterocyclyl, halogen, -CN, -COOH, $-OR_7$, $-NH-R_7$, $-CONH-R_7$, $-NHCO-R_7$, $-SO_2-R_7$, and $-SO_2NH-R_7$;

$R_7$ is selected from hydrogen, unsubstituted or substituted $C_{1-10}$ alkyl, unsubstituted or substituted $C_{6-10}$ aryl, unsubstituted or substituted $C_{3-6}$ cycloalkyl, and unsubstituted or substituted 4-10 membered saturated or

unsaturated heterocyclyl; and

the substitution in $R_6$ and $R_7$ refers to being substituted by one or more group A substituents, which include: $C_{1-6}$ alkyl, $C_{1-6}$ halogenated alkyl, hydroxy-substituted $C_{1-6}$ alkyl, amino-substituted $C_{1-6}$ alkyl, cyano-substituted $C_{1-6}$ alkyl, $C_{1-6}$ alkoxy-substituted $C_{1-6}$ alkyl, $C_{1-6}$ alkoxy, hydroxy, halogen, cyano, amino, carboxy, -NH-$C_{1-6}$ alkyl, -NH-$C_{3-6}$ cycloalkyl, unsubstituted or group B substituent-substituted $C_{6-10}$ aryl, unsubstituted or group B substituent-substituted 5-10 membered heteroaryl, unsubstituted or group B-substituted $C_{3-6}$ cycloalkyl, unsubstituted or group B substituent-substituted 4-10 membered saturated or unsaturated heterocyclyl, -C(O)-Rs, -C(O)O-Rs, -C(O)-$CH_2$-$R_8$, -C(O)-NH-Rs, -NH-C(O)-Rs, -$SO_2$-$R_8$, and -$SO_2$NH-$R_8$; $R_8$ is selected from hydrogen, unsubstituted or group B substituent-substituted $C_{1-10}$ alkyl, unsubstituted or group B substituent-substituted $C_{6-10}$ aryl, unsubstituted or group B substituent-substituted 5-10 membered heteroaryl, unsubstituted or group B substituent-substituted $C_{3-6}$ cycloalkyl, and unsubstituted or group B substituent-substituted 4-10 membered saturated or unsaturated heterocyclyl; and the group B substituent includes: unsubstituted or halogenated $C_{1-6}$ alkyl, unsubstituted or halogenated $C_{1-6}$ alkoxy, hydroxy, halogen, cyano, amino, carboxy, and -NH-$C_{1-6}$ alkyl,

for example, the substitution in $R_6$ and $R_7$ refers to being substituted by one or more group A substituents, which include: $C_{1-6}$ alkyl, $C_{1-6}$ halogenated alkyl, $C_{1-6}$ alkoxy, hydroxy, halogen, cyano, amino, carboxy, -NH-$C_{1-6}$ alkyl, -NH-$C_{3-6}$ cycloalkyl, unsubstituted or group B substituent-substituted $C_{6-10}$ aryl, unsubstituted or group B substituent-substituted 5-10 membered heteroaryl, unsubstituted or group B-substituted $C_{3-6}$ cycloalkyl, unsubstituted or group B substituent-substituted 4-10 membered saturated or unsaturated heterocyclyl, -C(O)-$R_8$, -C(O)-NH-$R_8$, -NH-C(O)-$R_8$, -$SO_2$-$R_8$, and -$SO_2$NH-$R_8$; $R_8$ is selected from hydrogen, unsubstituted or group B substituent-substituted $C_{1-10}$ alkyl, unsubstituted or group B substituent-substituted $C_{6-10}$ aryl, unsubstituted or group B substituent-substituted 5-10 membered heteroaryl, unsubstituted or group B substituent-substituted $C_{3-6}$ cycloalkyl, and unsubstituted or group B substituent-substituted 4-10 membered saturated or unsaturated heterocyclyl; and the group B substituent includes: $C_{1-6}$ alkyl, $C_{1-6}$ alkoxy, hydroxy, halogen, cyano, amino, and carboxy.

**9.** The compound of formula (I), and the enantiomer, the diastereoisomer, the racemate, the prodrug, the hydrate, the solvate, or the pharmaceutically acceptable salt thereof according to claim 7,

wherein $R_5$ and $R_6$ are each independently unsubstituted or substituted $C_{1-10}$ alkyl, unsubstituted or substituted $C_{6-10}$ aryl, unsubstituted or substituted 5-10 membered heteroaryl, unsubstituted or substituted $C_{3-6}$ cycloalkyl, unsubstituted or substituted 4-10 membered saturated or unsaturated heterocyclyl, unsubstituted or substituted 5-membered heteroarylo-fused 6-membered heterocyclyl, halogen, -CN, -COOH, -$OR_7$, -NH-$R_7$, -CONH-$R_7$, -NHCO-$R_7$, -$SO_2$-$R_7$, or -$SO_2$NH-$R_7$;

$R_7$ is selected from hydrogen, unsubstituted or substituted $C_{1-10}$ alkyl, unsubstituted or substituted $C_{6-10}$ aryl, unsubstituted or substituted 5-10 membered heteroaryl, unsubstituted or substituted $C_{3-6}$ cycloalkyl, and unsubstituted or substituted 4-10 membered saturated or unsaturated heterocyclyl; and

the substitution in $R_5$, $R_6$, and $R_7$ refers to being substituted by one or more group A substituents, which include: $C_{1-6}$ alkyl, $C_{1-6}$ halogenated alkyl, hydroxy-substituted $C_{1-6}$ alkyl, amino-substituted $C_{1-6}$ alkyl, cyano-substituted $C_{1-6}$ alkyl, $C_{1-6}$ alkoxy-substituted $C_{1-6}$ alkyl, $C_{1-6}$ alkoxy, hydroxy, halogen, cyano, amino, carboxy, -NH-$C_{1-6}$ alkyl, -NH-$C_{3-6}$ cycloalkyl, unsubstituted or group B substituent-substituted $C_{6-10}$ aryl, unsubstituted or group B substituent-substituted 5-10 membered heteroaryl, unsubstituted or group B-substituted $C_{3-6}$ cycloalkyl, unsubstituted or group B substituent-substituted 4-10 membered saturated or unsaturated heterocyclyl, -C(O)-Rs, -C(O)O-Rs, -C(O)-$CH_2$-$R_8$, -C(O)-NH-Rs, -NH-C(O)-Rs, -$SO_2$-$R_8$, and -$SO_2$NH-$R_8$; $R_8$ is selected from hydrogen, unsubstituted or group B substituent-substituted $C_{1-10}$ alkyl, unsubstituted or group B substituent-substituted $C_{6-10}$ aryl, unsubstituted or group B substituent-substituted 5-10 membered heteroaryl, unsubstituted or group B substituent-substituted $C_{3-6}$ cycloalkyl, and unsubstituted or group B substituent-substituted 4-10 membered saturated or unsaturated heterocyclyl; and the group B substituent includes: unsubstituted or halogenated $C_{1-6}$ alkyl, unsubstituted or halogenated $C_{1-6}$ alkoxy, hydroxy, halogen, cyano, amino, carboxy, and -NH-$C_{1-6}$ alkyl,

the heteroaryl in $R_5$, $R_6$, $R_7$, and the group A substituent is selected from

 ,  ,

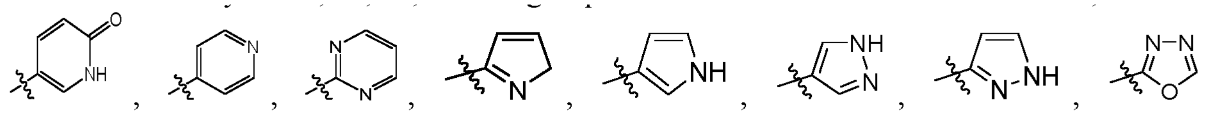

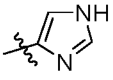, and 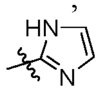;

the heterocyclyl in $R_5$, $R_6$, $R_7$, and the group A substituent is selected from

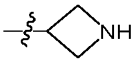,

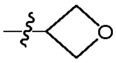, 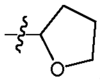, 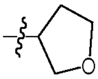, 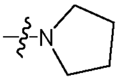, 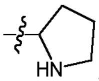, 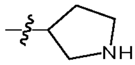, 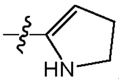, 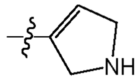, 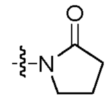,

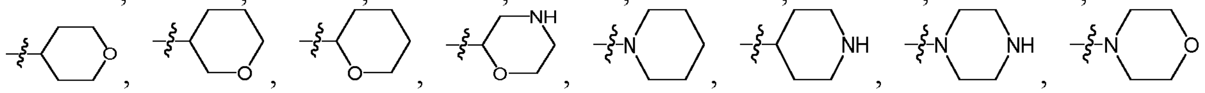

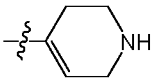, 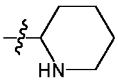, 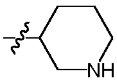, 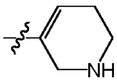, 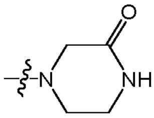, 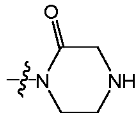, 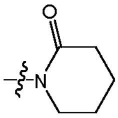, 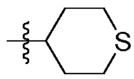, 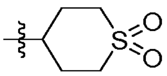, 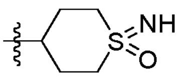, 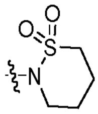, 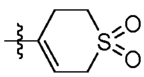, 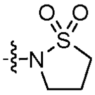, 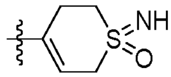, 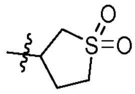, 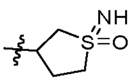, 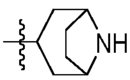, 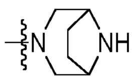, and 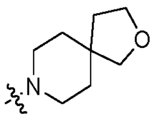;

and
the 5-membered heteroarylo-fused 6-membered heterocyclyl is selected from

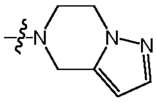,

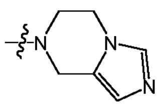, and 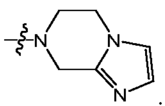.

**10.** The compound of formula (I), and the enantiomer, the diastereoisomer, the racemate, the prodrug, the hydrate, the solvate, or the pharmaceutically acceptable salt thereof according to claim 7,

wherein $R_5$ and $R_6$ are each independently unsubstituted or substituted $C_{1-10}$ alkyl, unsubstituted or substituted $C_{6-10}$ aryl, unsubstituted or substituted 5-10 membered heteroaryl, unsubstituted or substituted $C_{3-6}$ cycloalkyl, unsubstituted or substituted 4-10 membered saturated or unsaturated heterocyclyl, unsubstituted or substituted 5-membered heteroarylo-fused 6-membered heterocyclyl, halogen, -CN, -COOH, $-OR_7$, $-NH-R_7$, $-CONH-R_7$, $-NHCO-R_7$, $-SO_2-R_7$, or $-SO_2NH-R_7$;
$R_7$ is selected from hydrogen, unsubstituted or substituted $C_{1-10}$ alkyl, unsubstituted or substituted $C_{6-10}$ aryl, unsubstituted or substituted 5-10 membered heteroaryl, unsubstituted or substituted $C_{3-6}$ cycloalkyl, and unsubstituted or substituted 4-10 membered saturated or unsaturated heterocyclyl;

the substitution in $R_5$, $R_6$, and $R_7$ refers to being substituted by one or more group A substituents, which include: $C_{1-6}$ alkyl, $C_{1-6}$ halogenated alkyl, $C_{1-6}$ alkoxy, hydroxy, halogen, cyano, amino, carboxy, -NH-$C_{1-6}$ alkyl, -NH-$C_{3-6}$ cycloalkyl, unsubstituted or group B substituent-substituted $C_{6-10}$ aryl, unsubstituted or group B substituent-substituted 5-10 membered heteroaryl, unsubstituted or group B-substituted $C_{3-6}$ cycloalkyl, unsubstituted or group B substituent-substituted 4-10 membered saturated or unsaturated heterocyclyl, -C(O)-$R_8$, -C(O)-NH-$R_8$, -NH-C(O)-$R_8$, -$SO_2$-$R_8$, and -$SO_2$NH-$R_8$; $R_8$ is selected from hydrogen, unsubstituted or group B substituent-substituted $C_{1-10}$ alkyl, unsubstituted or group B substituent-substituted $C_{6-10}$ aryl, unsubstituted or group B substituent-substituted 5-10 membered heteroaryl, unsubstituted or group B substituent-substituted $C_{3-6}$ cycloalkyl, and unsubstituted or group B substituent-substituted 4-10 membered saturated or unsaturated heterocyclyl; and the group B substituent includes: $C_{1-6}$ alkyl, $C_{1-6}$ alkoxy, hydroxy, halogen, cyano, amino, and carboxy;

the heterocyclyl in $R_5$, $R_6$, $R_7$, and the group A substituent is selected from

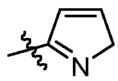,

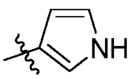, 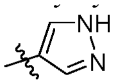, 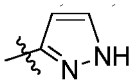, 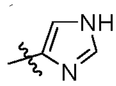, and 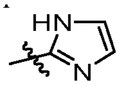;

the heteroaryl in $R_5$, $R_6$, $R_7$, and the group A substituent is selected from

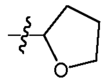,

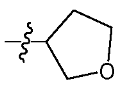, 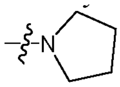, 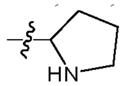, 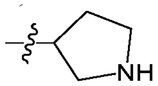, 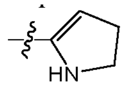, 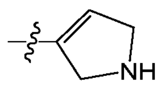, 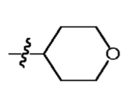, 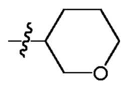, 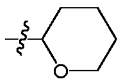, 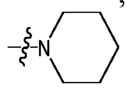, 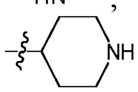, 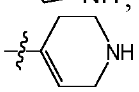, 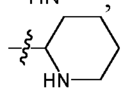, 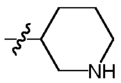, 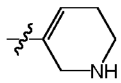, 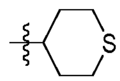, 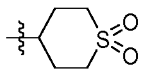, 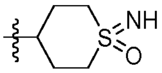, 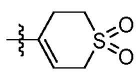, 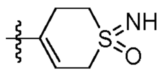, 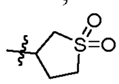, and 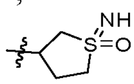; and

the 5-membered heteroarylo-fused 6-membered heterocyclyl is selected from

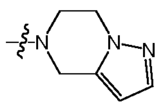,

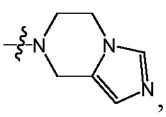, and 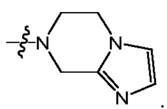.

**11.** The compound of formula (I), and the enantiomer, the diastereoisomer, the racemate, the prodrug, the hydrate, the solvate, or the pharmaceutically acceptable salt thereof according to claim 1, wherein the compound of formula (I) has the structure of formula (I-2) or formula (I-3), and each symbol and variable are as defined in claim 1 provided that $R_2$ is not hydrogen:

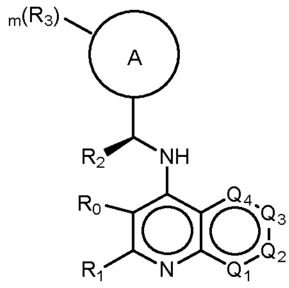
(I-2)

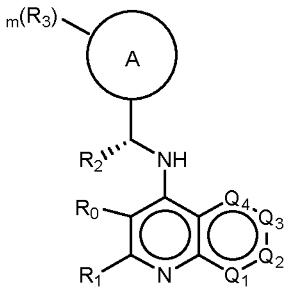
(I-3)

.

12. The compound of formula (I), and the enantiomer, the diastereoisomer, the racemate, the prodrug, the hydrate, the solvate, or the pharmaceutically acceptable salt thereof according to claim 1, wherein the compound of formula (I) is selected from the following compounds:

| No. | Structure | No. | Structure |
|---|---|---|---|
| A-1 | 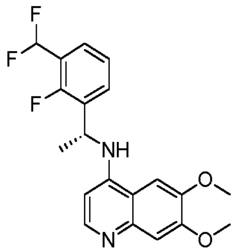 | A-2 | 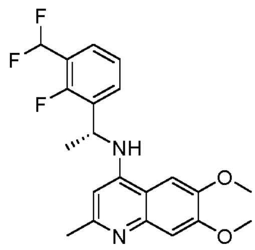 |
| A-3 | 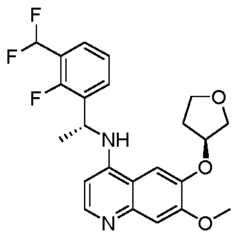 | A-4 | 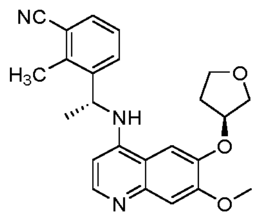 |
| A-5 | 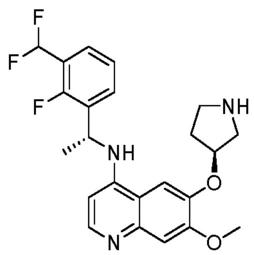 | A-6 | 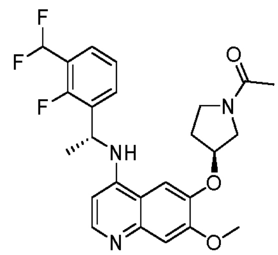 |
| A-7 | 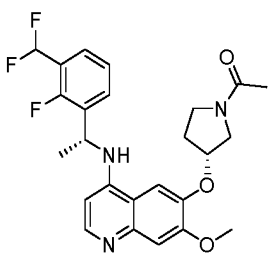 | A-8 | 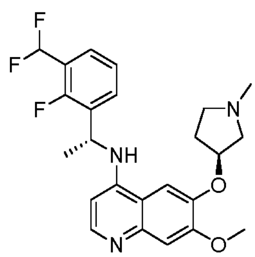 |

(continued)

| No. | Structure | No. | Structure |
|---|---|---|---|
| A-9 | 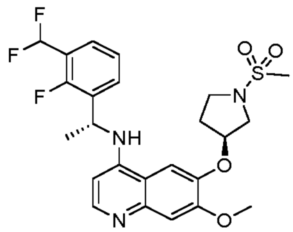 | A-10 | 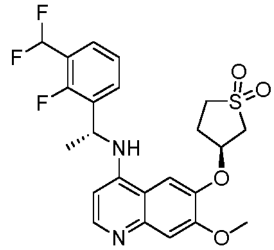 |
| A-11 | 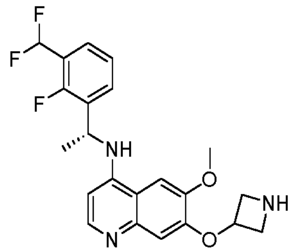 | A-12 | 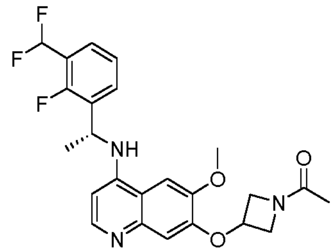 |
| A-13 | 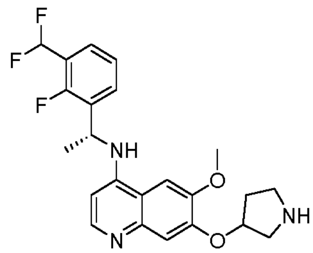 | A-14 | 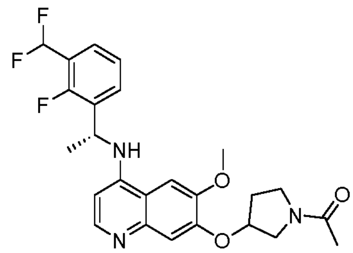 |
| A-15 | 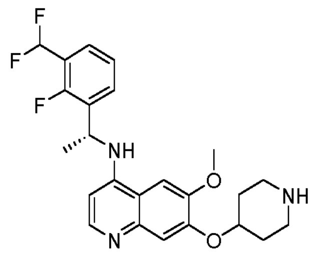 | A-16 | 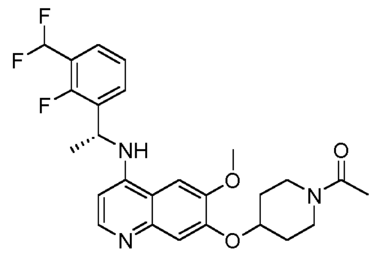 |
| A-17 | 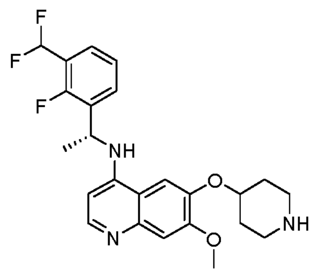 | A-18 | 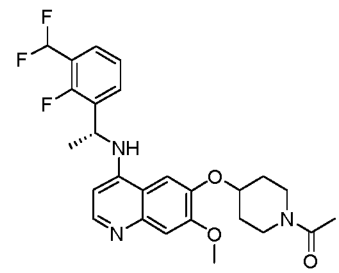 |
| A-19 | 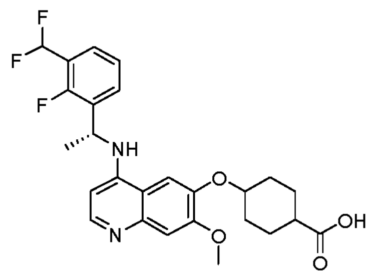 | A-20 | 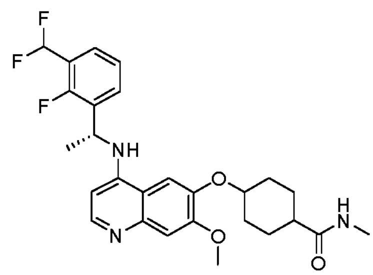 |

(continued)

| No. | Structure | No. | Structure |
|---|---|---|---|
| A-21 | 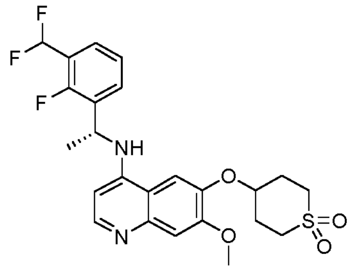 | A-22 | 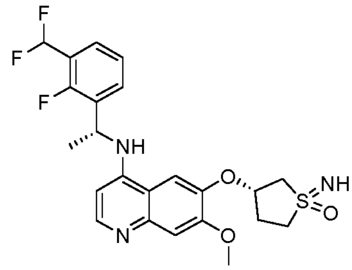 |
| A-23 | 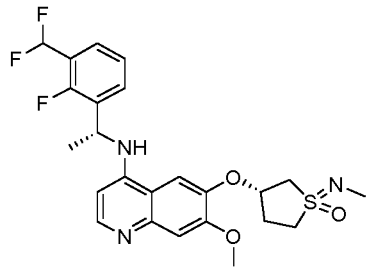 | A-24 | 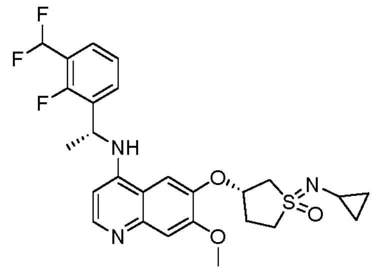 |
| A-25 | 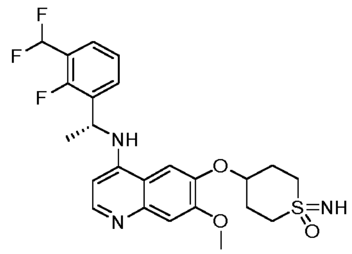 | A-26 | 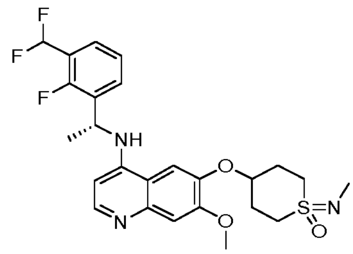 |
| A-27 | 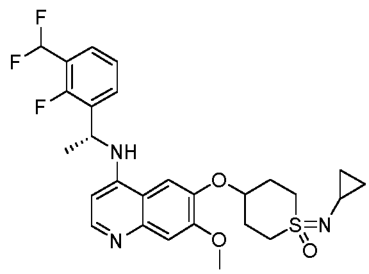 | A-28 | 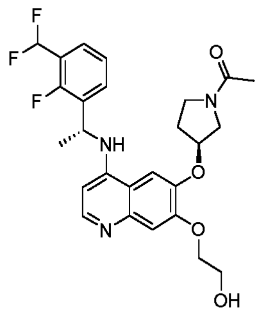 |
| A-29 | 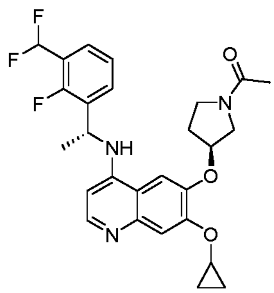 | A-30 | 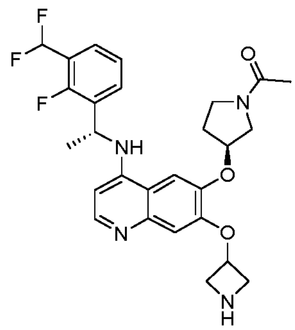 |

(continued)

| No. | Structure | No. | Structure |
|---|---|---|---|
| A-31 | 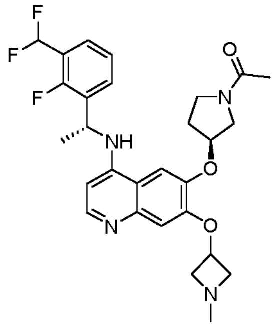 | A-32 | 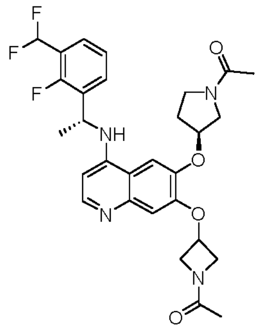 |
| A-33 | 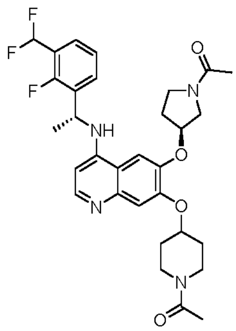 | A-34 | 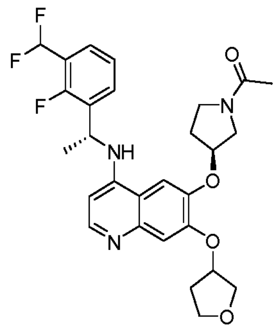 |
| A-3 5 | 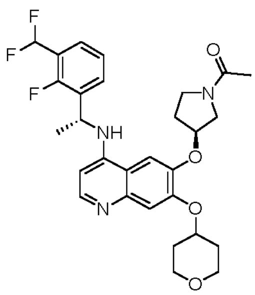 | A-36 | 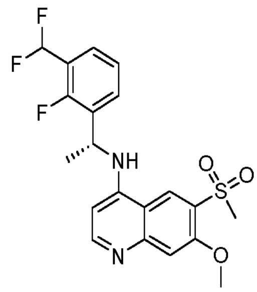 |
| A-37 | 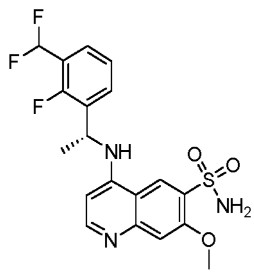 | A-38 | 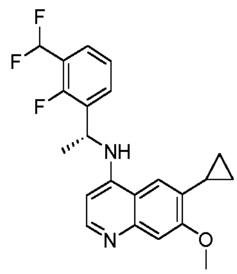 |
| A-39 | 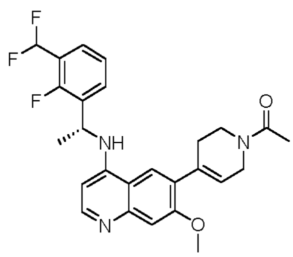 | A-40 | 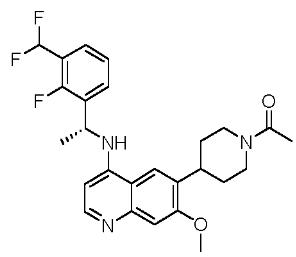 |

(continued)

| No. | Structure | No. | Structure |
|---|---|---|---|
| A-41 | 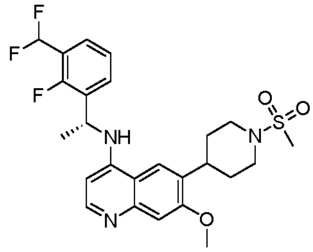 | A-42 | 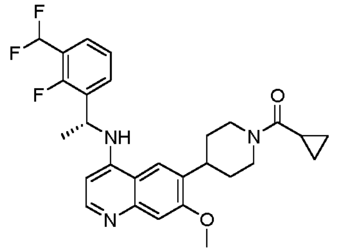 |
| A-43 | 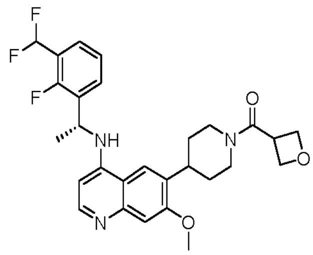 | A-44 | 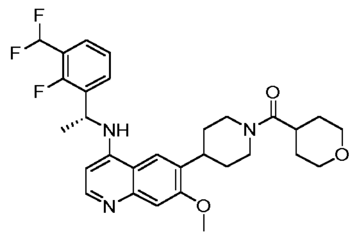 |
| A-45 | 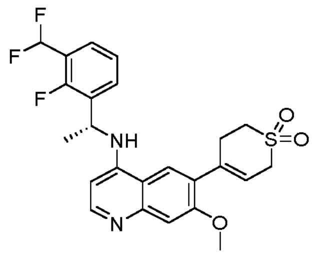 | A-46 | 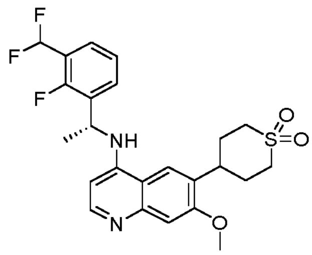 |
| A-47 | 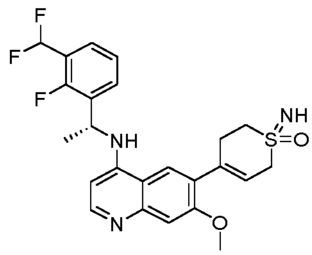 | A-48 | 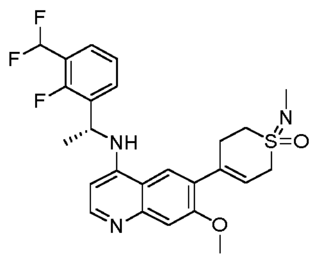 |
| A-49 | 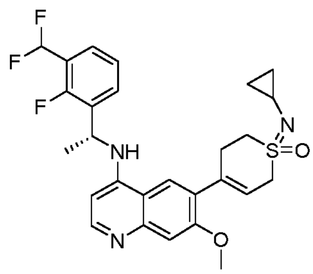 | A-50 | 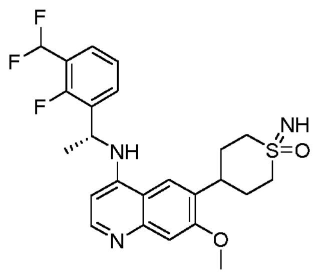 |
| A-51 | 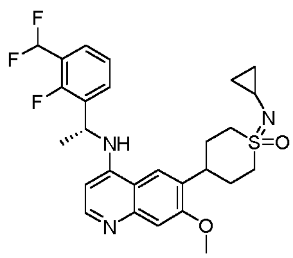 | A-52 | 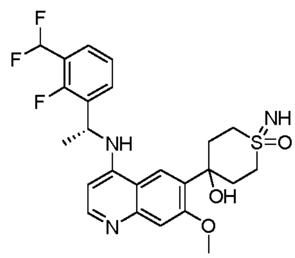 |

(continued)

| No. | Structure | No. | Structure |
|---|---|---|---|
| A-53 | 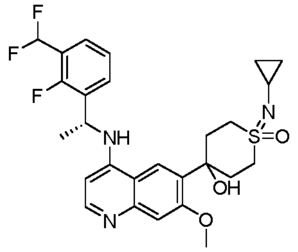 | A-54 | 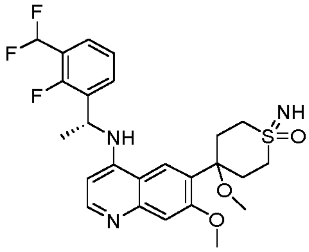 |
| A-55 | 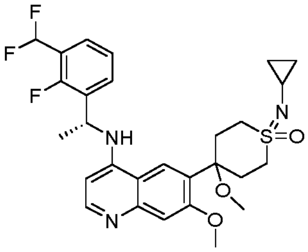 | A-56 | 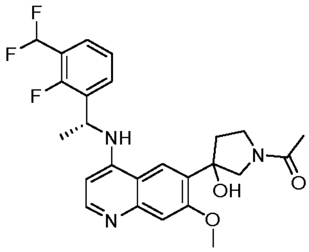 |
| A-57 | 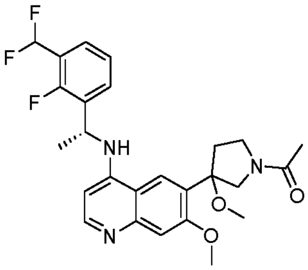 | A-58 | 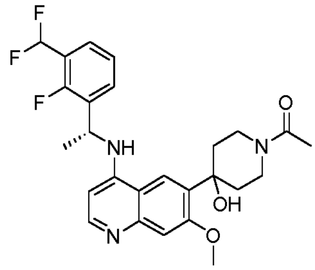 |
| A-59 | 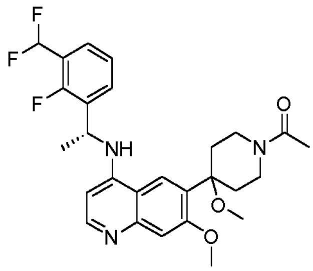 | A-60 | 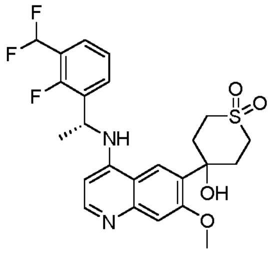 |
| A-61 | 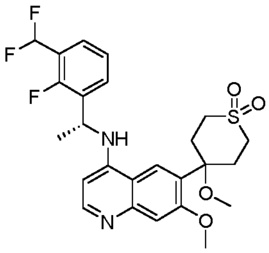 | A-62 | 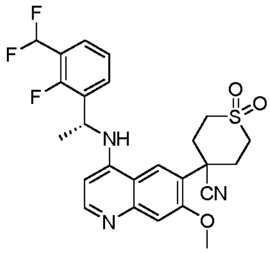 |
| A-63 | 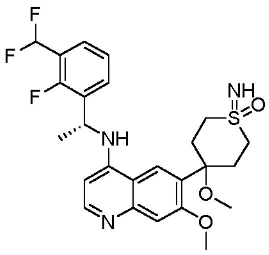 | A-64 | 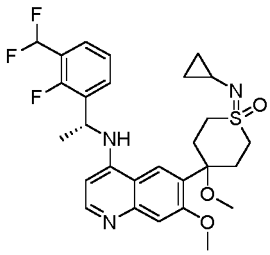 |

(continued)

| No. | Structure | No. | Structure |
|---|---|---|---|
| A-65 | 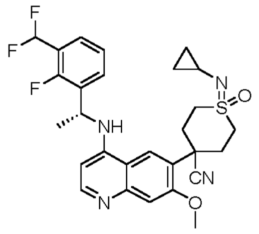 | A-66 | 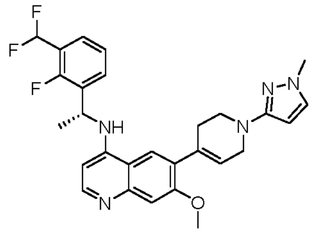 |
| A-67 | 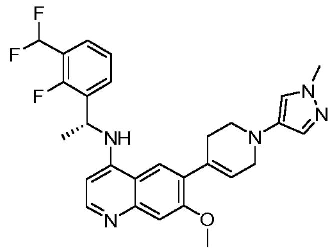 | A-68 | 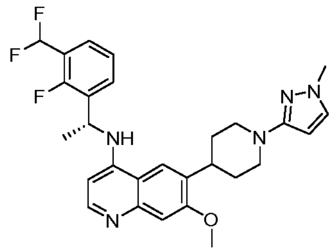 |
| A-69 | 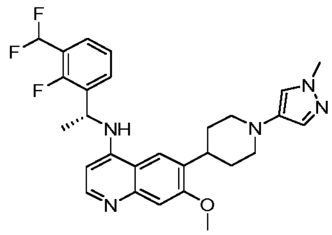 | A-70 | 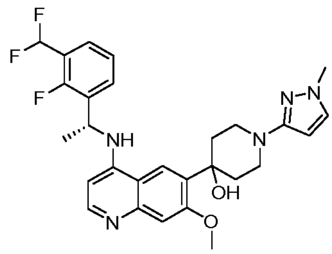 |
| A-71 | 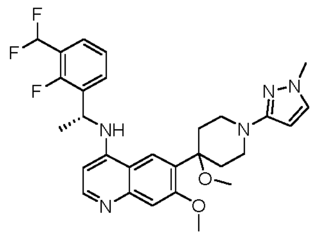 | A-72 | 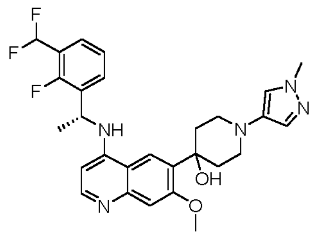 |
| A-73 | 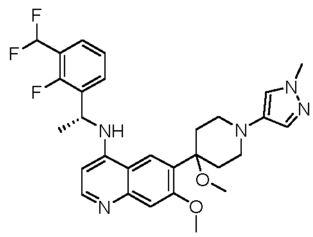 | A-74 | 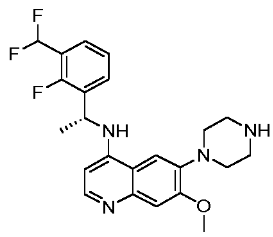 |
| A-75 | 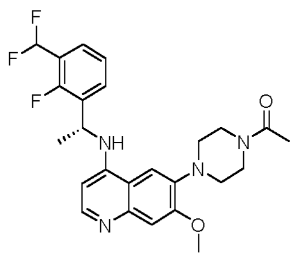 | A-76 | 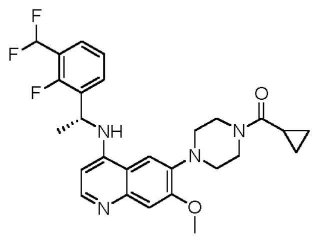 |

(continued)

| No. | Structure | No. | Structure |
|---|---|---|---|
| A-77 | 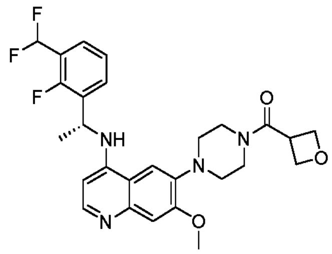 | A-78 | 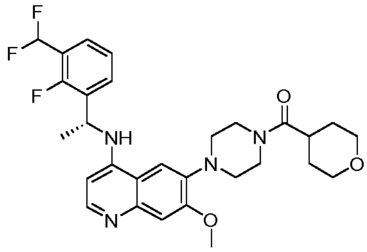 |
| A-79 | 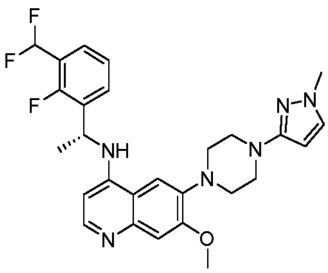 | A-80 | 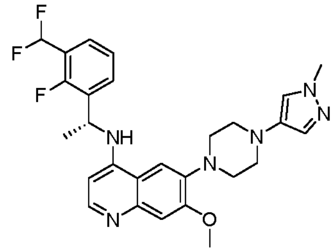 |
| A-81 | 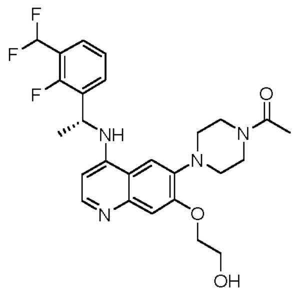 | A-82 | 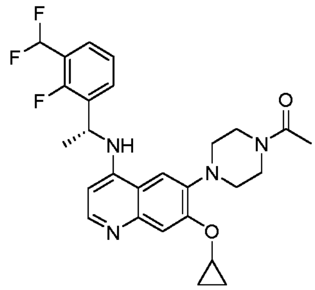 |
| A-83 | 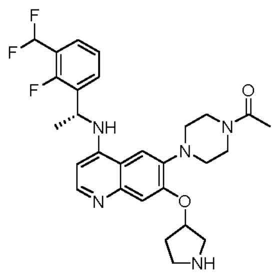 | A-84 | 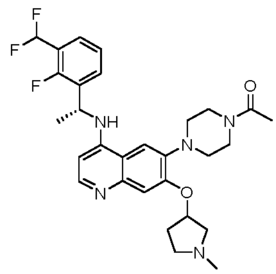 |
| A-85 | 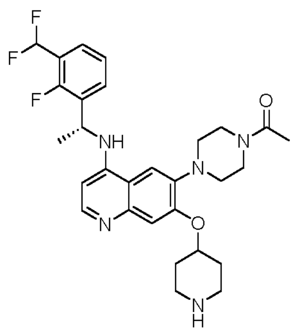 | A-86 | 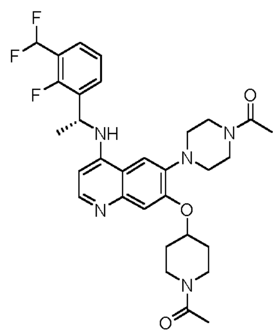 |

(continued)

| No. | Structure | No. | Structure |
|---|---|---|---|
| A-87 | 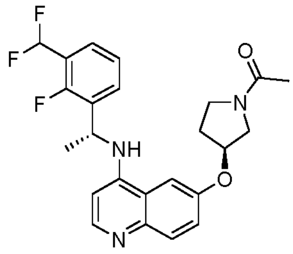 | A-88 | 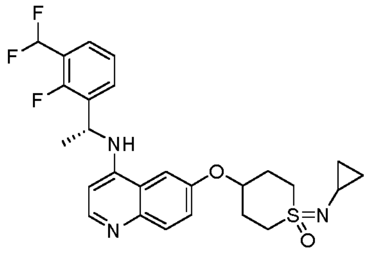 |
| A-89 | 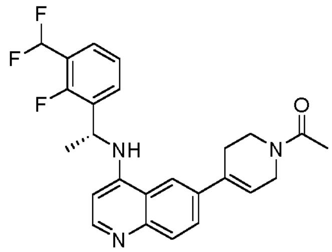 | A-90 | 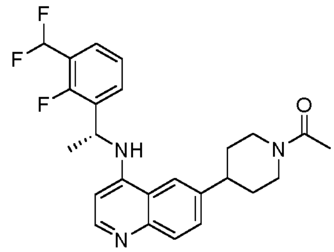 |
| A-91 | 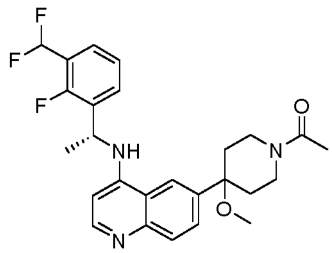 | A-92 | 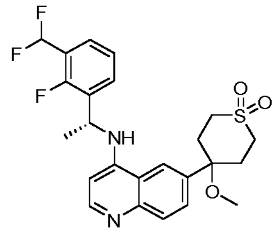 |
| A-93 | 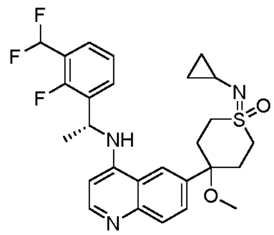 | A-94 | 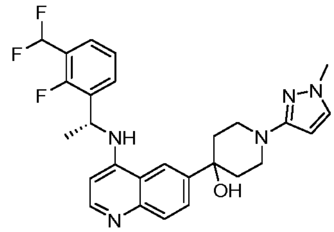 |
| A-95 | 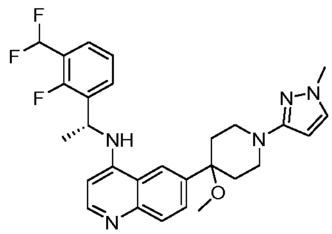 | A-96 | 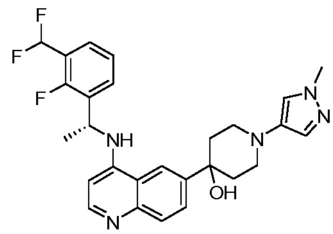 |
| A-97 | 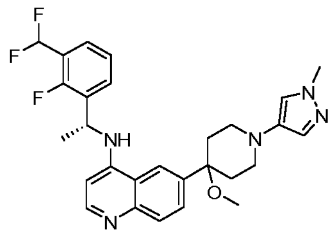 | A-98 | 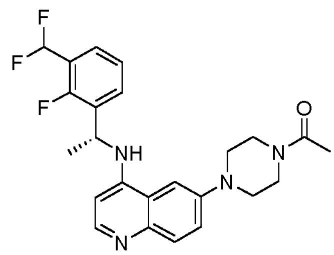 |

(continued)

| No. | Structure | No. | Structure |
|---|---|---|---|
| A-99 | 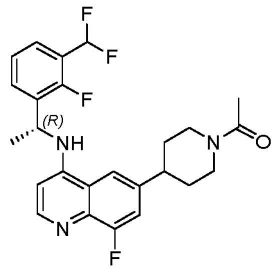 | A-100 | 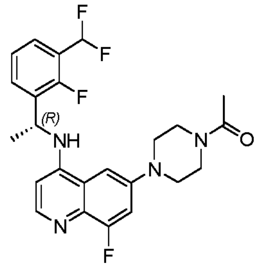 |
| A-101 | 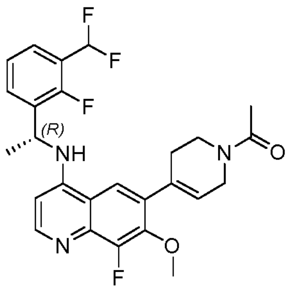 | A-102 | 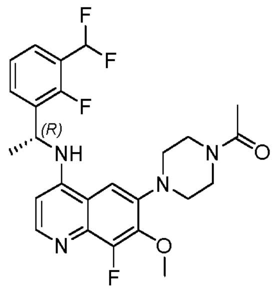 |
| B-1 | 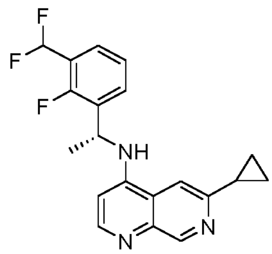 | B-2 | 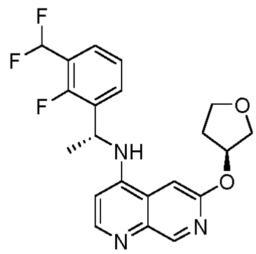 |
| B-3 | 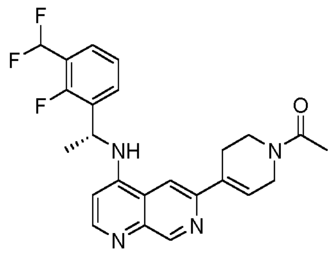 | B-4 | 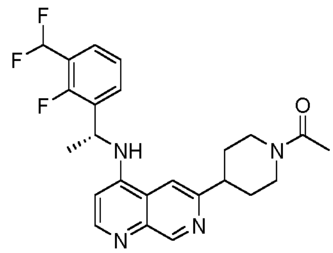 |
| B-5 | 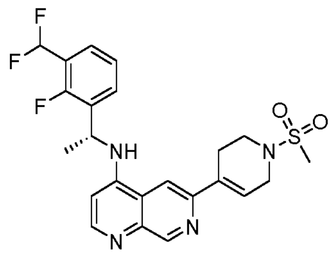 | B-6 | 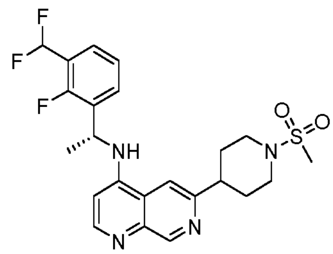 |
| B-7 | 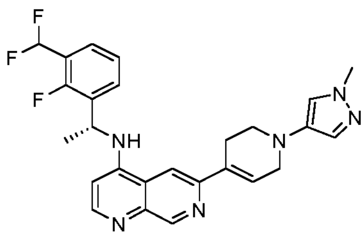 | B-8 | 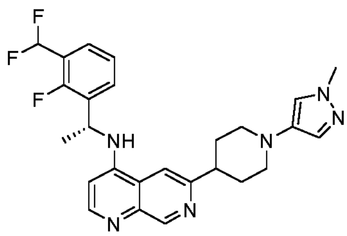 |

(continued)

| No. | Structure | No. | Structure |
|---|---|---|---|
| B-9 | 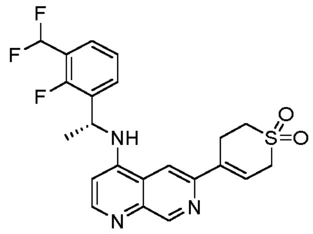 | B-10 | 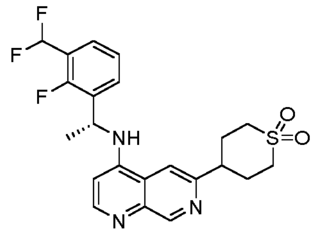 |
| B-11 | 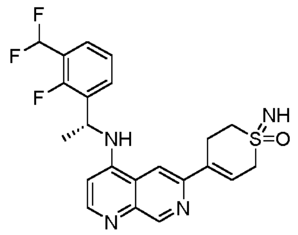 | B-12 | 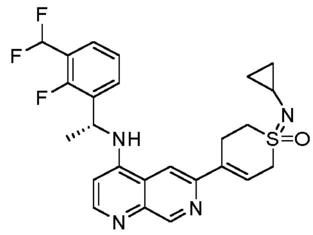 |
| B-13 | 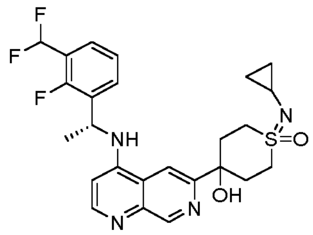 | B-14 | 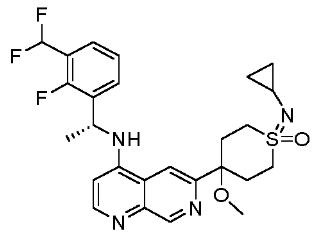 |
| B-15 | 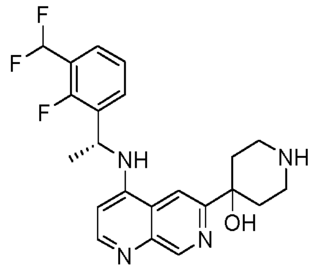 | B-16 | 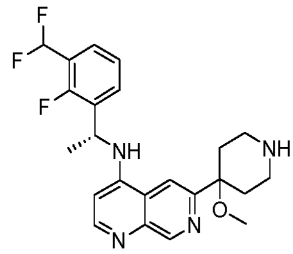 |
| B-17 | 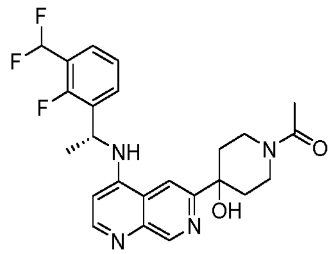 | B-18 | 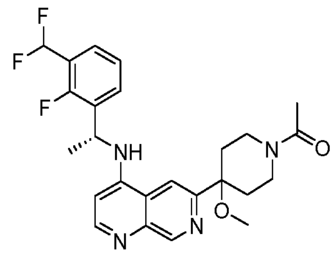 |
| B-19 | 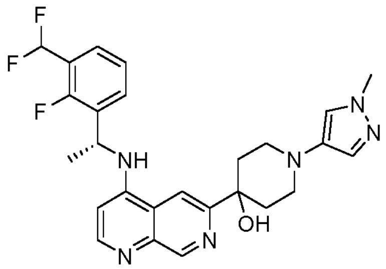 | B-20 | 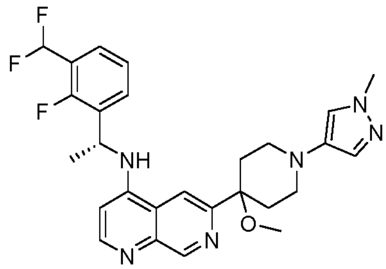 |

(continued)

| No. | Structure | No. | Structure |
|---|---|---|---|
| B-21 | 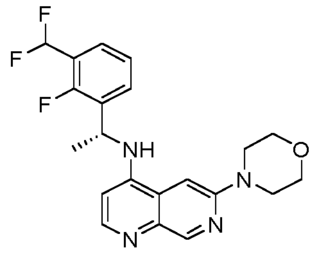 | B-22 | 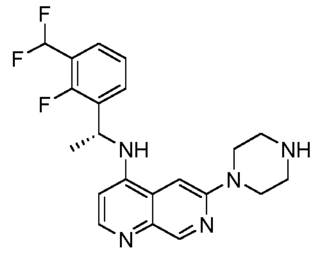 |
| B-23 | 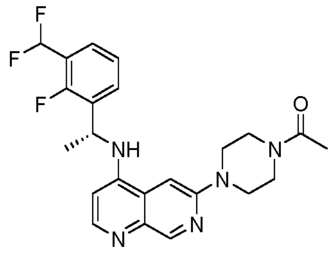 | B-24 | 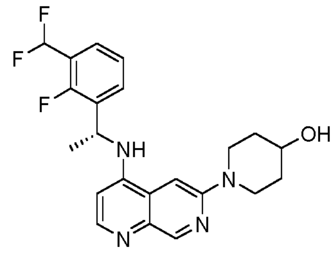 |
| B-25 | 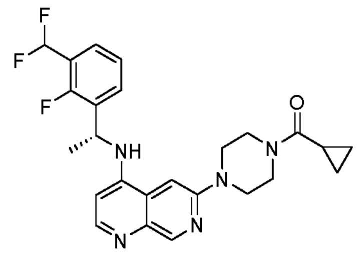 | B-26 | 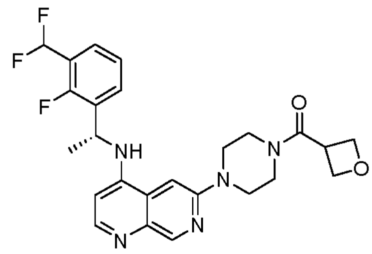 |
| B-27 | 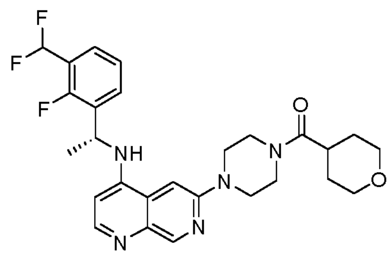 | B-28 | 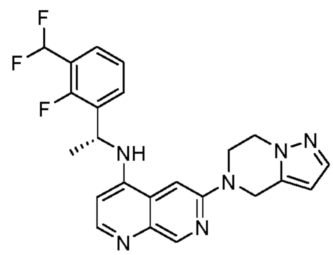 |
| B-29 | 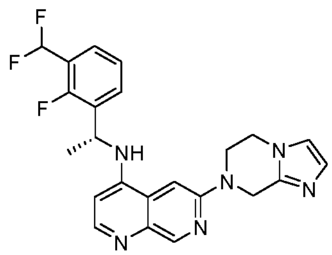 | B-30 | 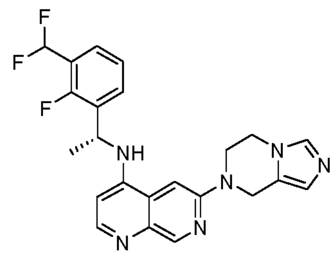 |
| B-31 | 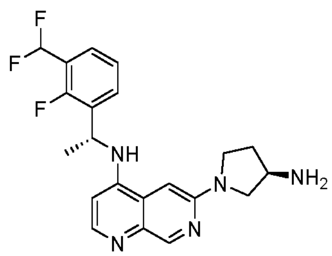 | B-32 | 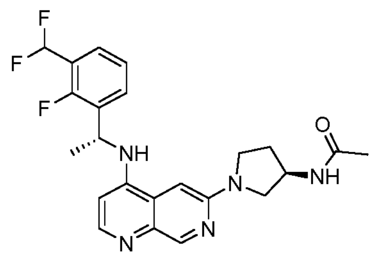 |

(continued)

| No. | Structure | No. | Structure |
|---|---|---|---|
| B-33 | 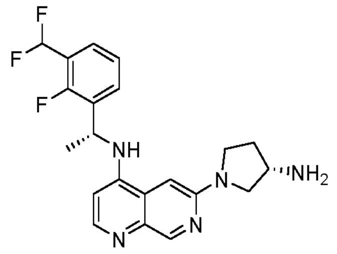 | B-34 | 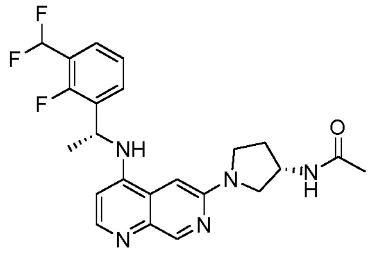 |
| B-35 | 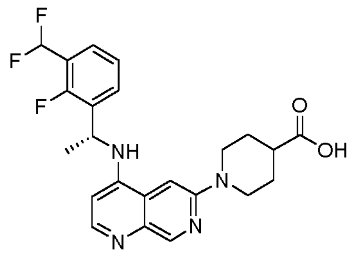 | B-36 | 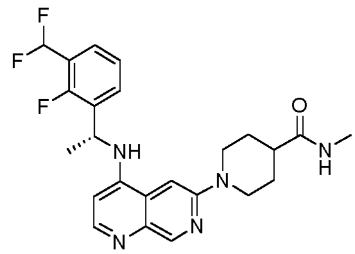 |
| B-37 | 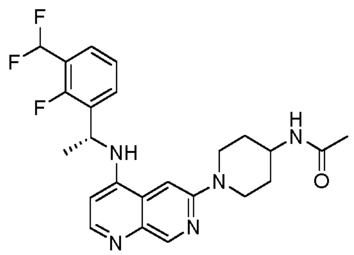 | B-38 | 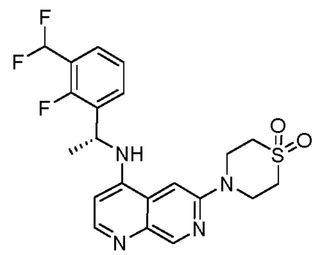 |
| B-39 | 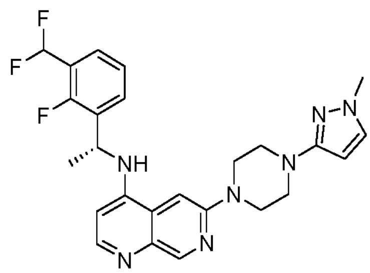 | B-40 | 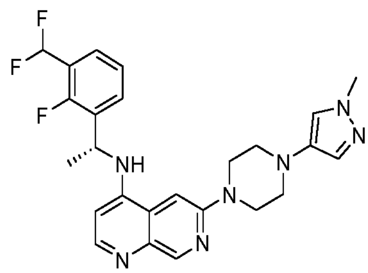 |
| B-41 | 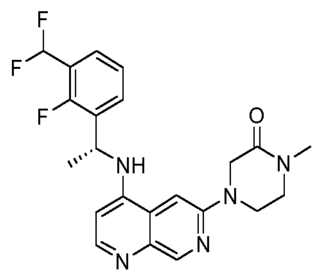 | B-42 | 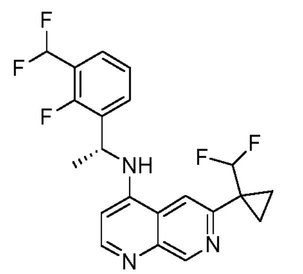 |
| B-43 | 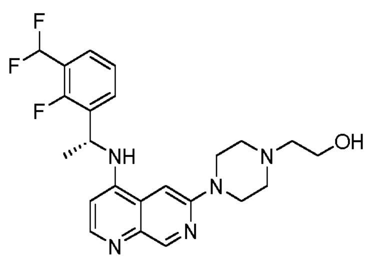 | B-44 | 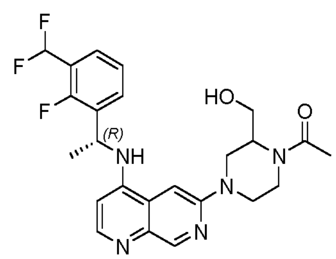 |

(continued)

| No. | Structure | No. | Structure |
|---|---|---|---|
| B-45 | 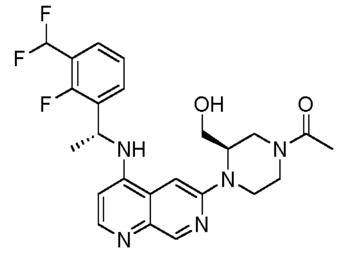 | B-46 | 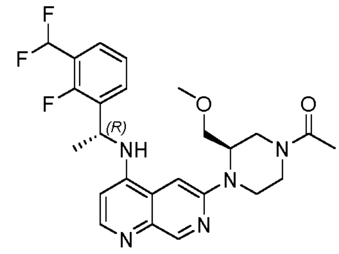 |
| B-47 | 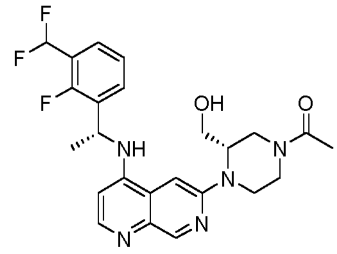 | B-48 | 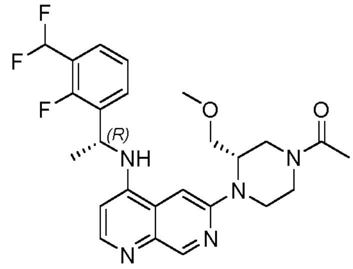 |
| B-49 | 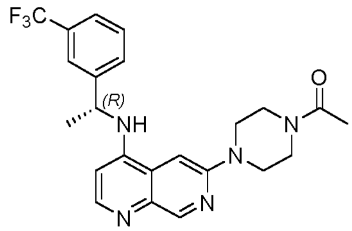 | B-50 | 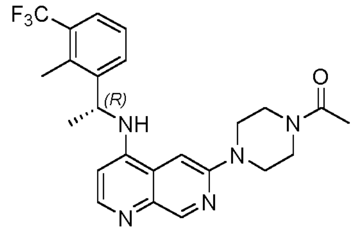 |
| B-51 | 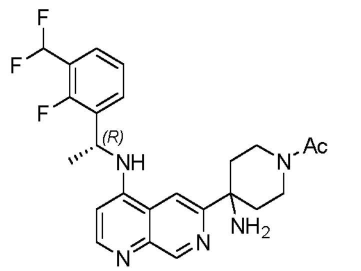 | B-52 | 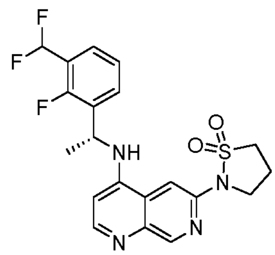 |
| B-53 | 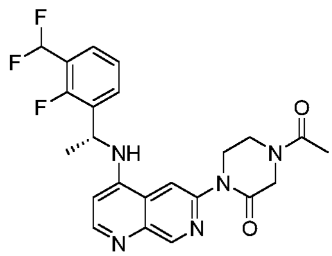 | B-54 | 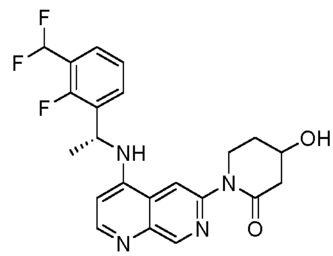 |
| B-55 | 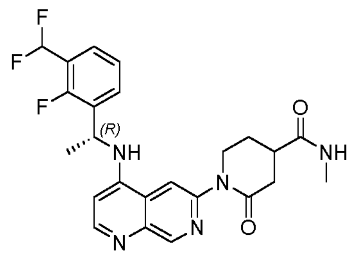 | B-56 | 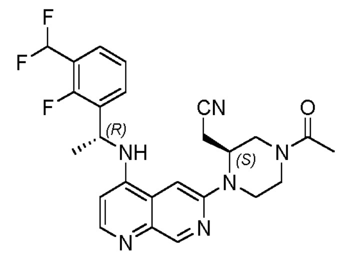 |

(continued)

| No. | Structure | No. | Structure |
|---|---|---|---|
| B-57 | 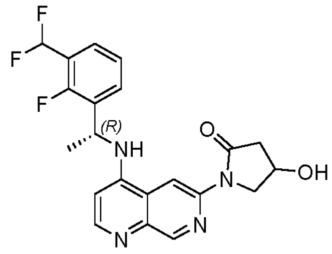 | B-58 | 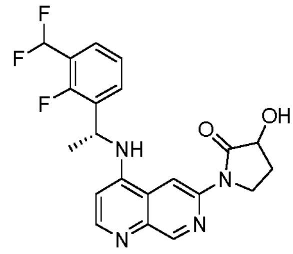 |
| B-59 | 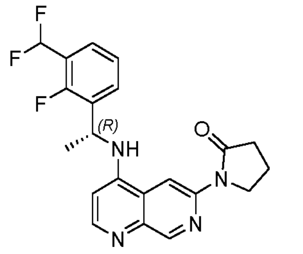 | B-60 | 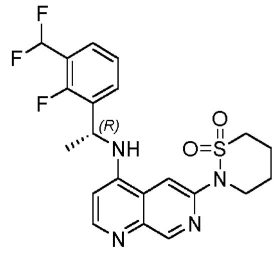 |
| B-61 | 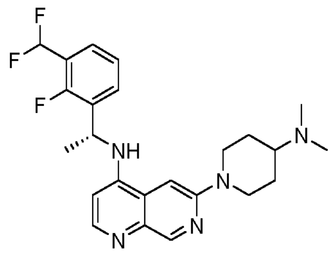 | B-62 | 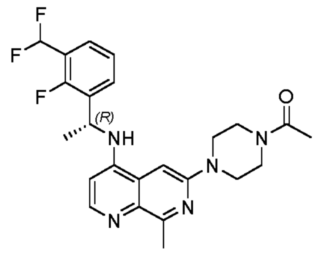 |
| B-63 | 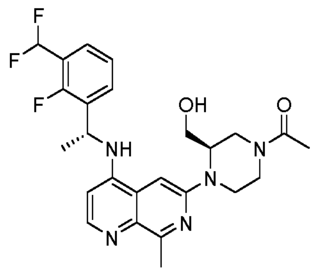 | B-64 | 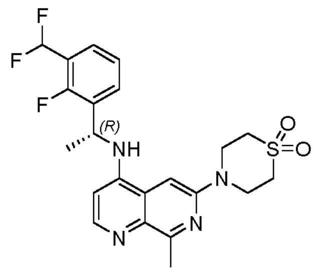 |
| B-65 | 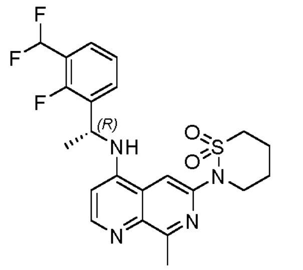 | B-66 | 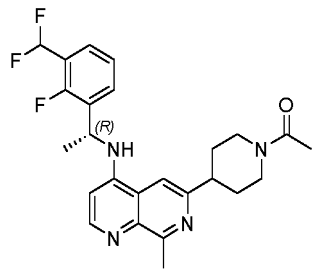 |
| B-67 | 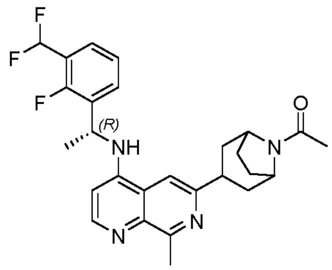 | B-68 | 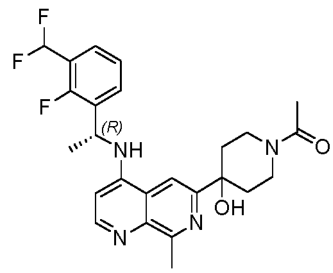 |

(continued)

| No. | Structure | No. | Structure |
|---|---|---|---|
| B-69 | 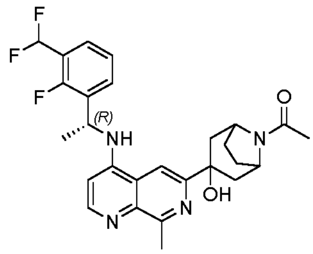 | B-70 | 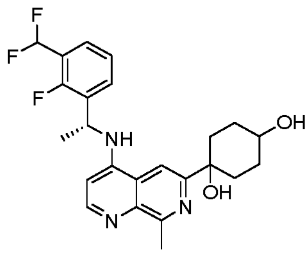 |
| B-71 | 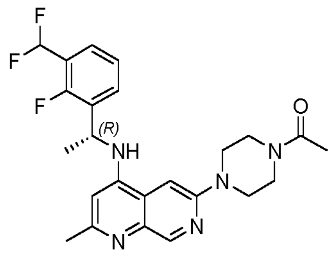 | B-72 | 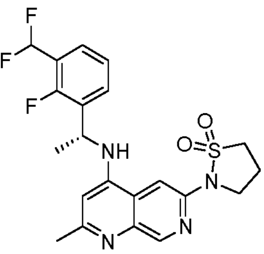 |
| B-73 | 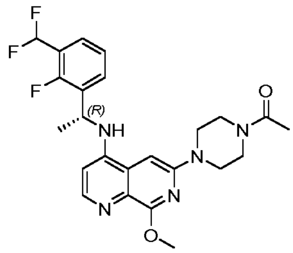 | B-74 | 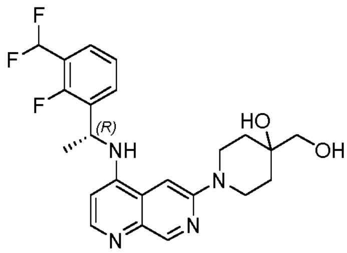 |
| B-75 | 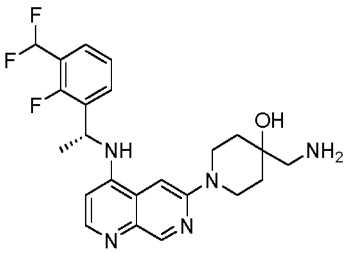 | B-76 | 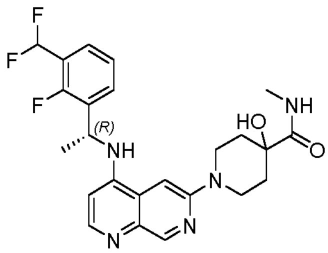 |
| B-77 | 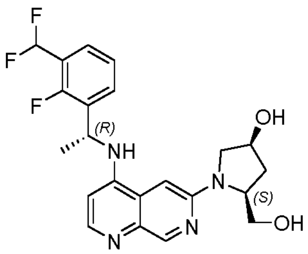 | B-78 | 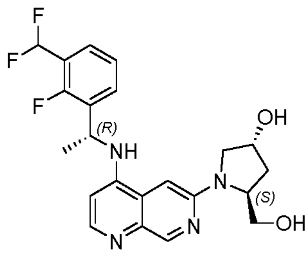 |
| B-79 | 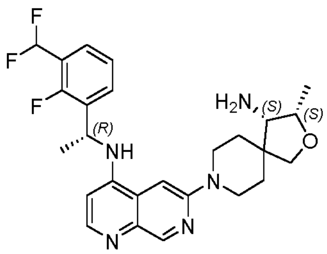 | B-80 | 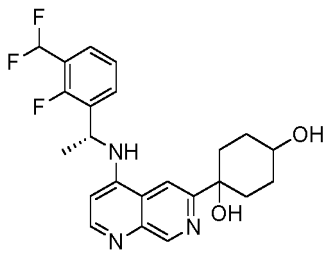 |

(continued)

| No. | Structure | No. | Structure |
|---|---|---|---|
| B-81 | 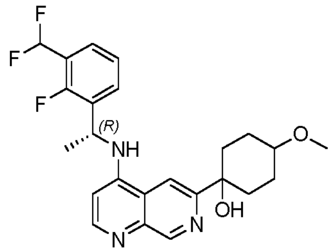 | B-82 | 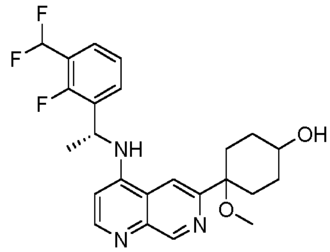 |
| B-83 | 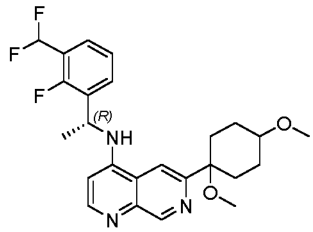 | B-84 | 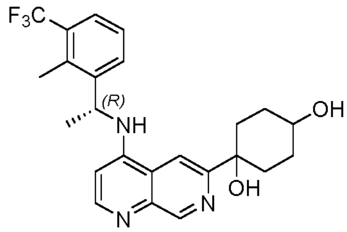 |
| B-85 | 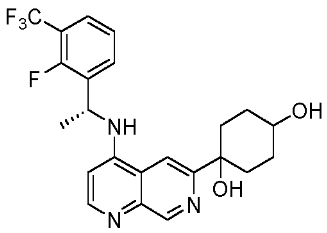 | B-86 | 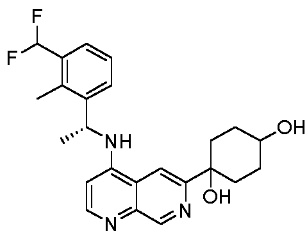 |
| B-87 | 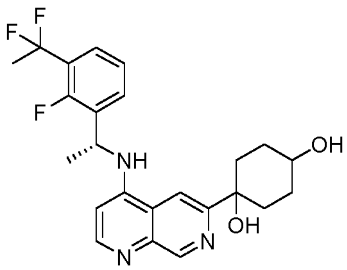 | B-88 | 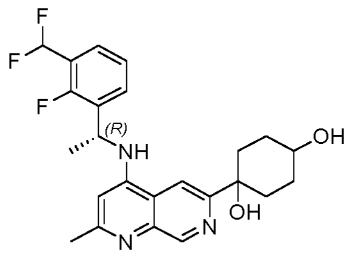 |
| B-89 | 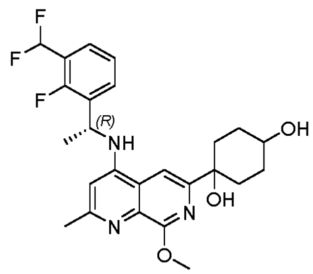 | B-90 | 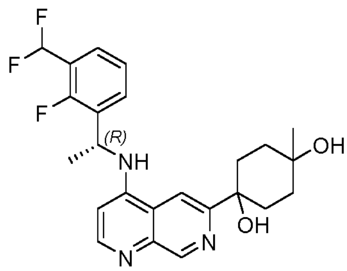 |
| B-91 | 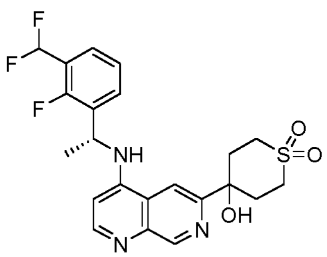 | B-92 | 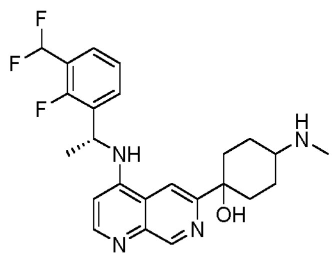 |

(continued)

| No. | Structure | No. | Structure |
|---|---|---|---|
| B-93 | 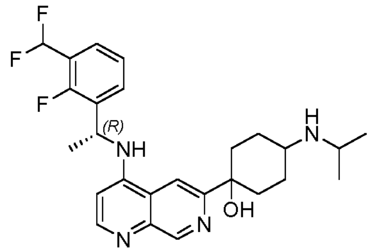 | B-94 | 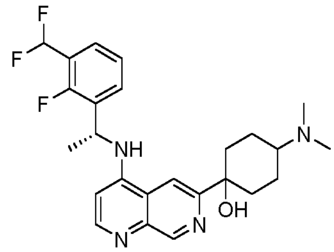 |
| B-95 | 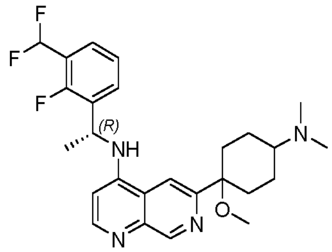 | B-96 | 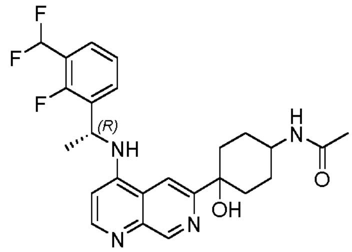 |
| B-97 | 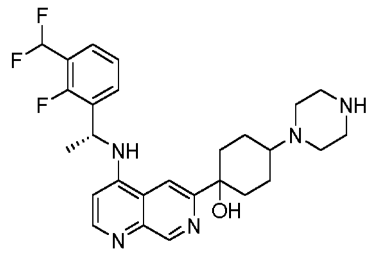 | B-98 | 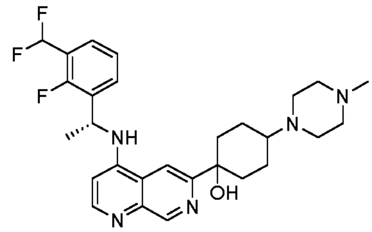 |
| B-99 | 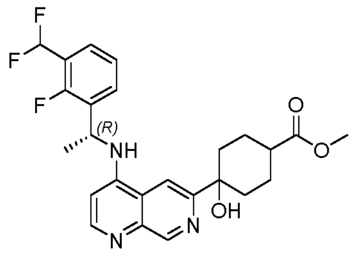 | B-100 | 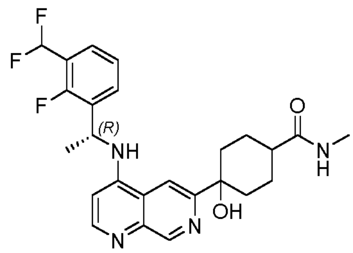 |
| B-101 | 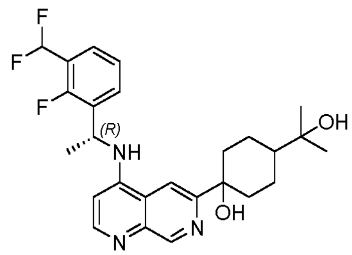 | B-102 | 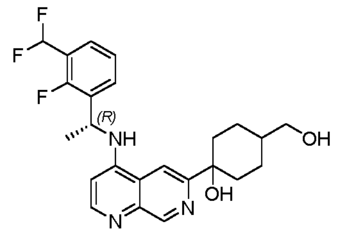 |
| B-103 | 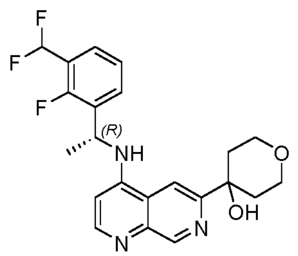 | B-104 | 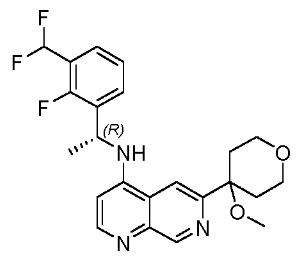 |

(continued)

| No. | Structure | No. | Structure |
|---|---|---|---|
| B-105 | 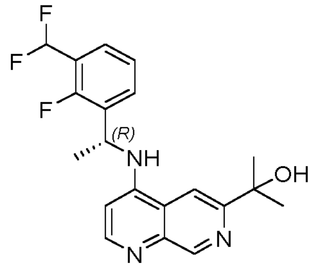 | B-106 | 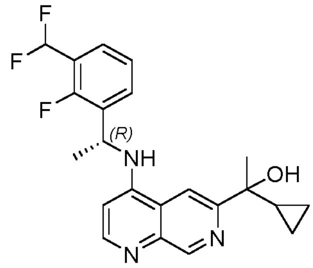 |
| B-107 | 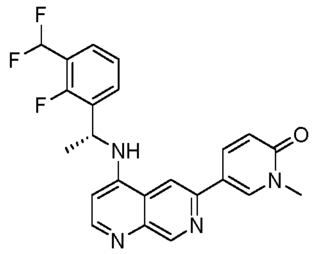 | B-108 | 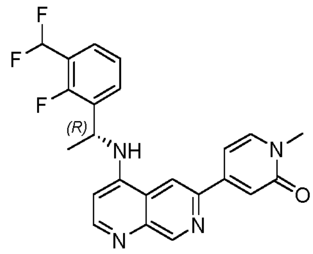 |
| B-109 | 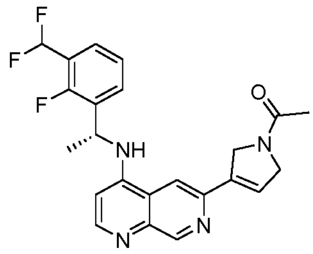 | B-110 | 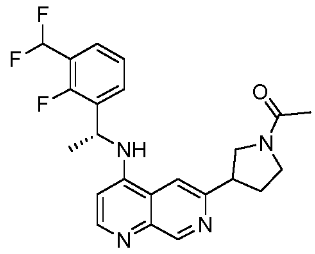 |
| B-111 | 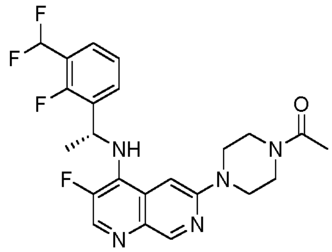 | B-112 | 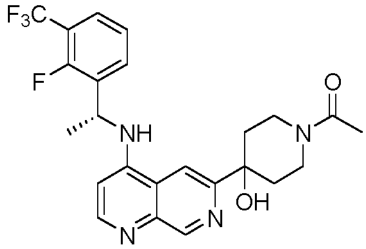 |
| B-113 | 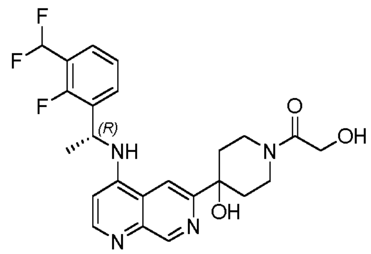 | B-114 | 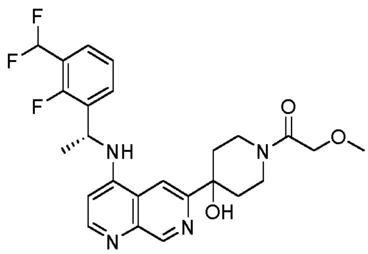 |
| B-115 | 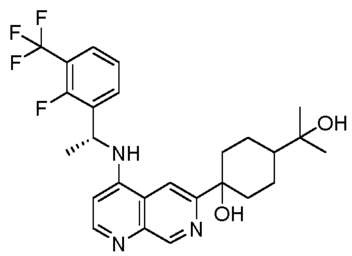 | B-116 | 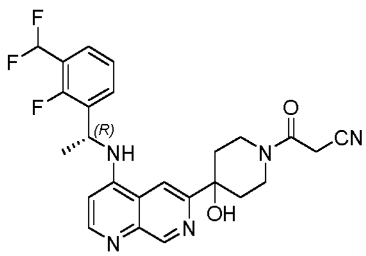 |

(continued)

| No. | Structure | No. | Structure |
|---|---|---|---|
| B-117 | 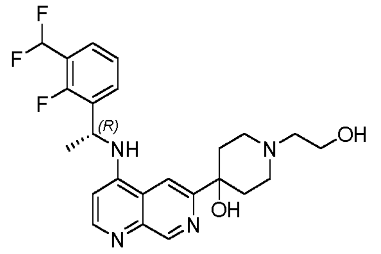 | B-118 | 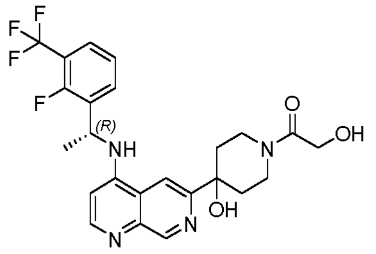 |
| B-119 | 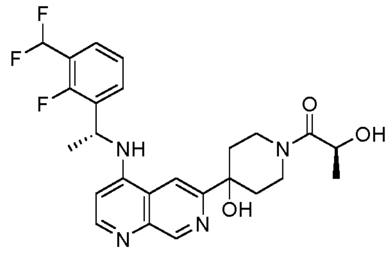 | B-120 | 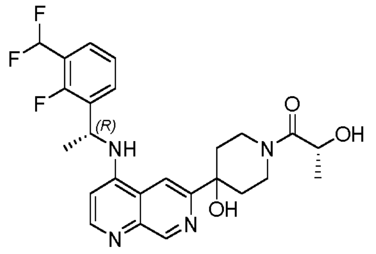 |
| B-121 | 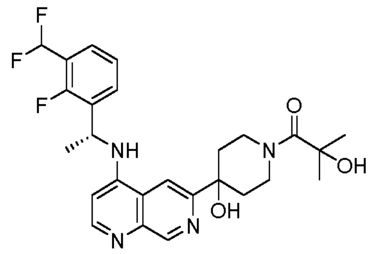 | B-122 | 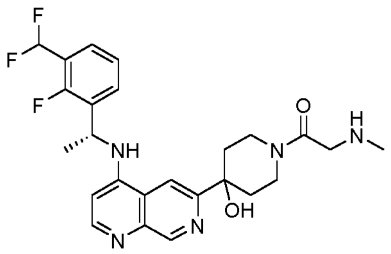 |
| B-123 | 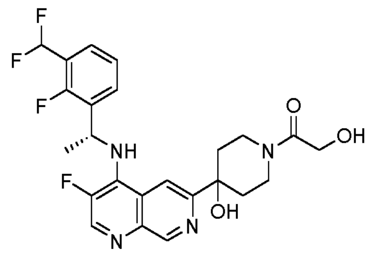 | B-124 | 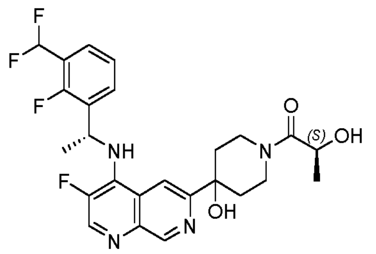 |
| B-125 | 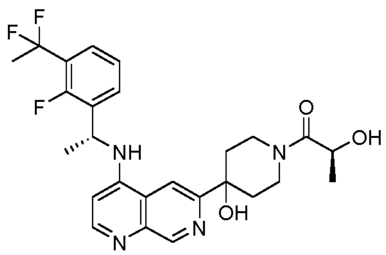 | B-126 | 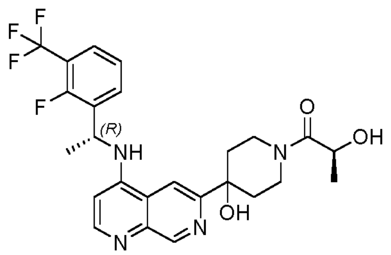 |
| B-127 | 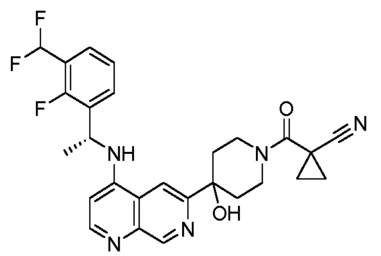 | B-128 | 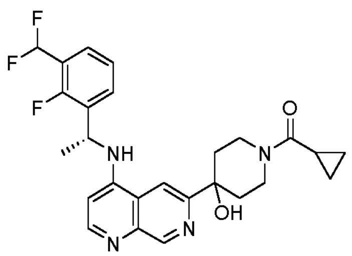 |

(continued)

| No. | Structure | No. | Structure |
|---|---|---|---|
| B-129 | 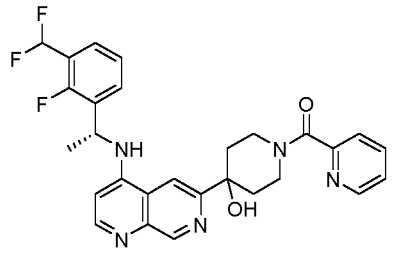 | B-130 | 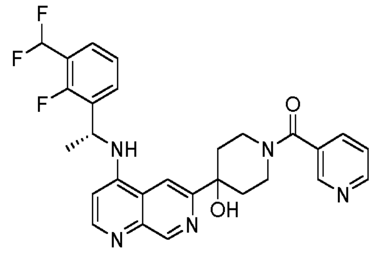 |
| B-131 | 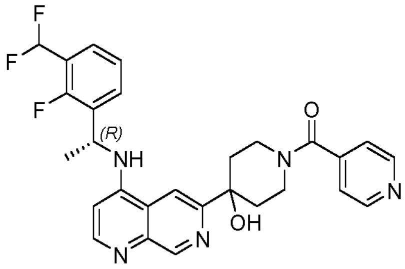 | B-132 | 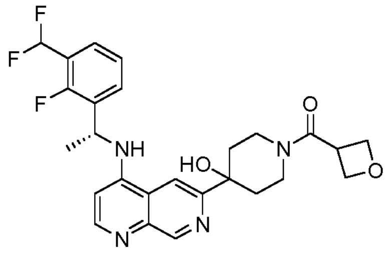 |
| B-133 | 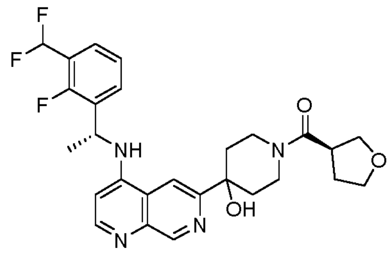 | B-134 | 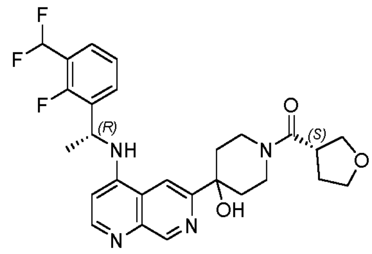 |
| B-135 | 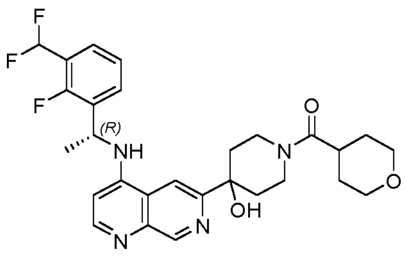 | B-136 | 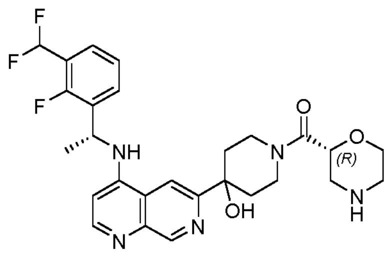 |
| B-137 | 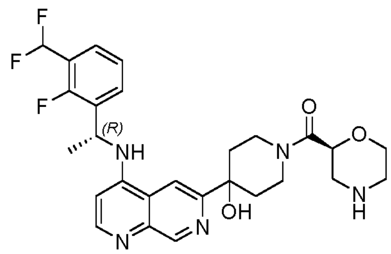 | B-138 | 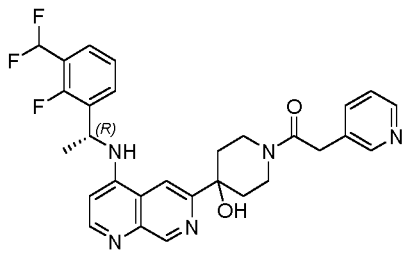 |
| B-139 | 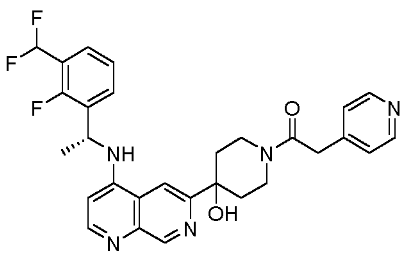 | B-140 | 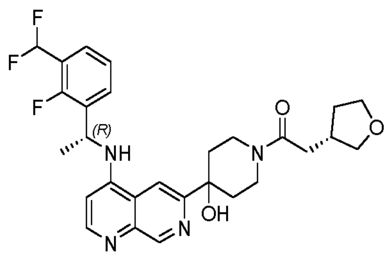 |

(continued)

| No. | Structure | No. | Structure |
|---|---|---|---|
| B-141 | 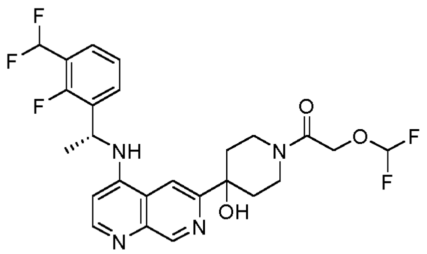 | B-142 | 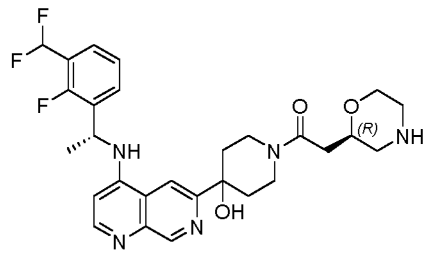 |
| B-143 | 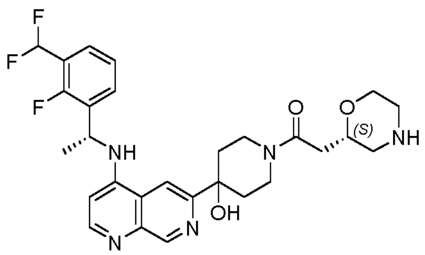 | B-144 | 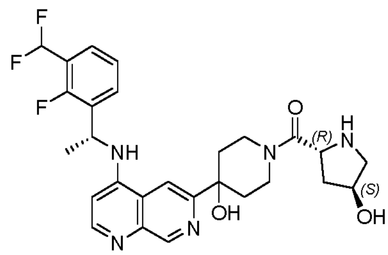 |
| B-145 | 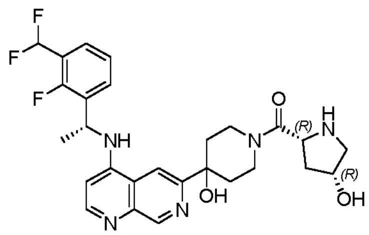 | B-146 | 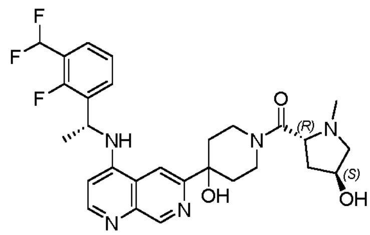 |
| B-147 | 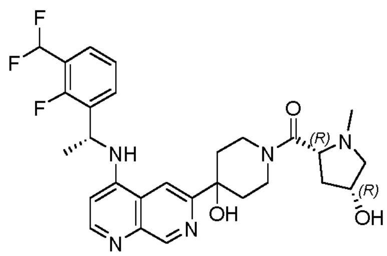 | B-148 | 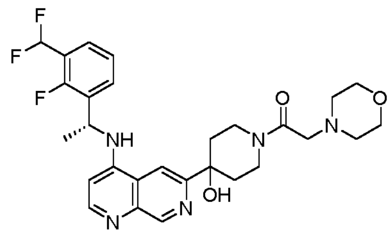 |
| B-149 | 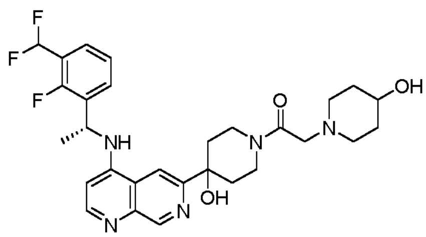 | B-150 | 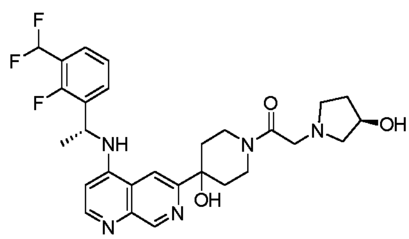 |
| B-151 | 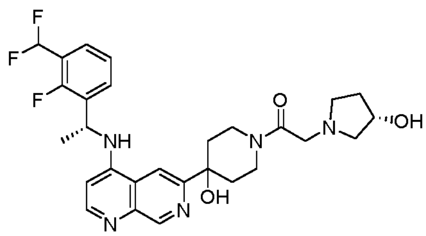 | B-152 | 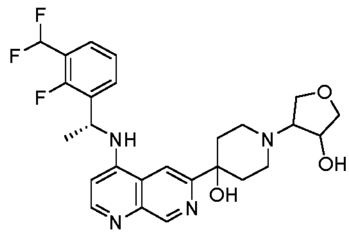 |

(continued)

| No. | Structure | No. | Structure |
|---|---|---|---|
| B-153 | 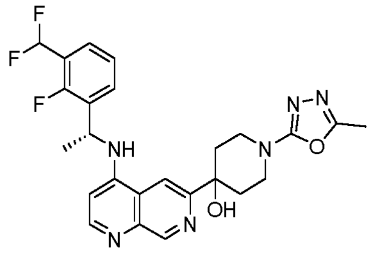 | B-154 | 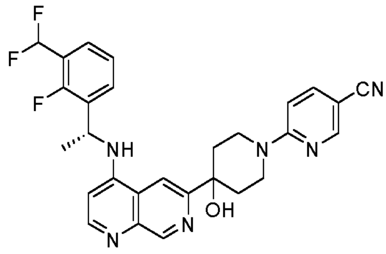 |
| B-155 | 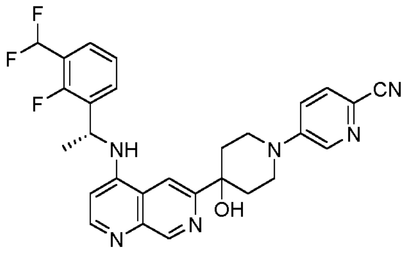 | B-156 | 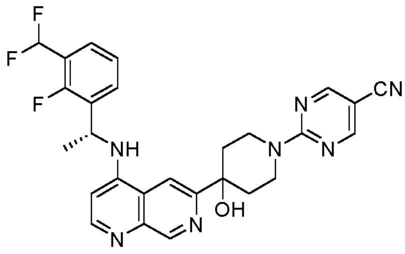 |
| B-157 | 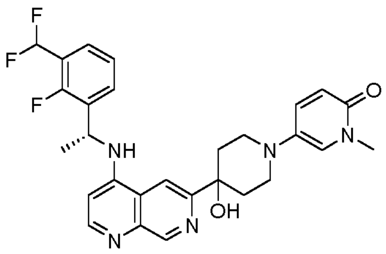 | B-158 | 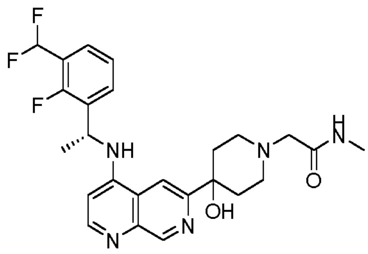 |
| B-159 | 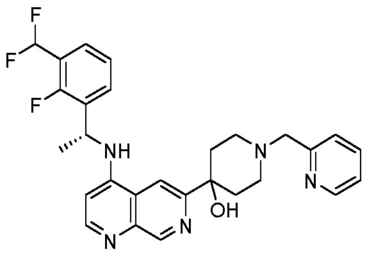 | B-160 | 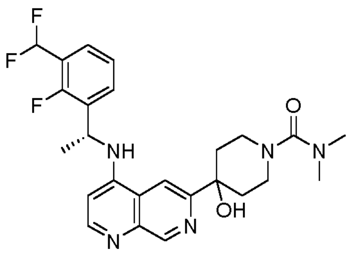 |
| B-161 | 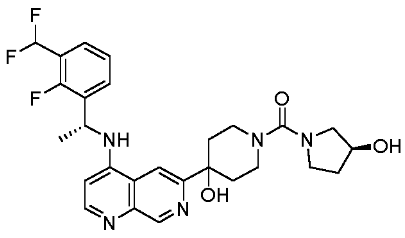 | B-162 | 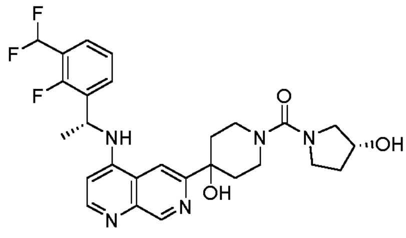 |
| B-163 | 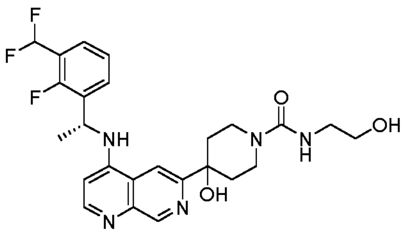 | B-164 | 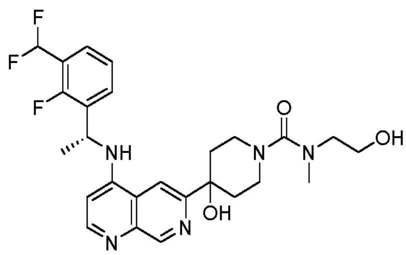 |

(continued)

| No. | Structure | No. | Structure |
|---|---|---|---|
| B-165 | 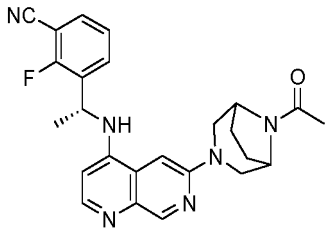 | B-166 | 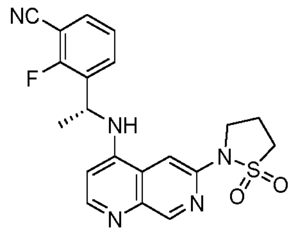 |
| B-167 | 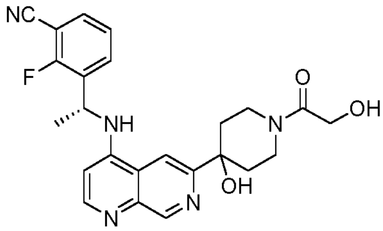 | B-168 | 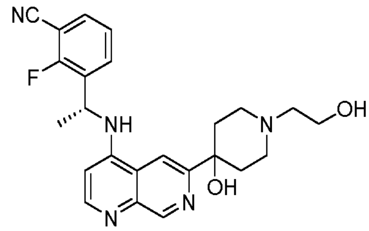 |
| B-169 | 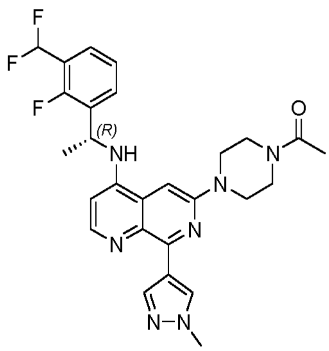 | B-170 | 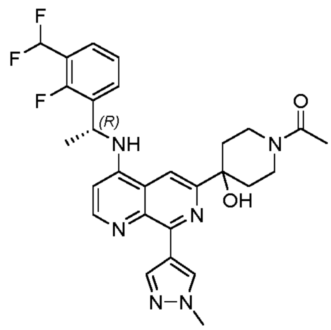 |
| B-171 | 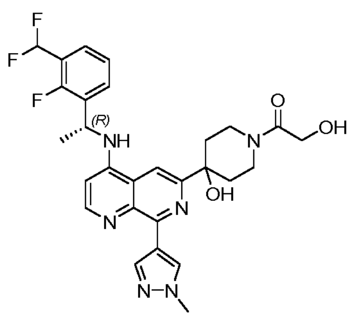 | B-172 | 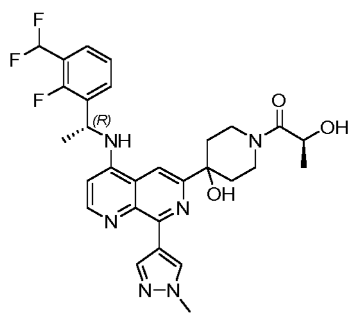 |
| B-173 | 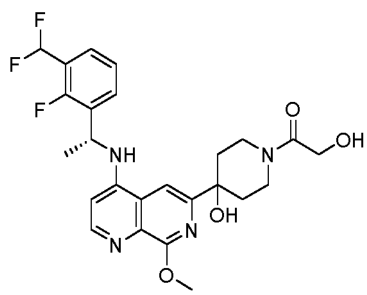 | B-174 | 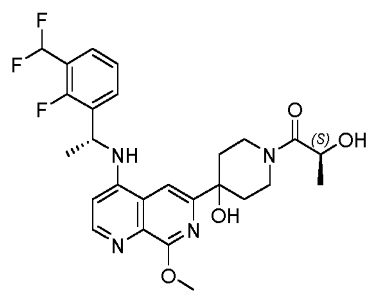 |

(continued)

| No. | Structure | No. | Structure |
|---|---|---|---|
| B-175 | 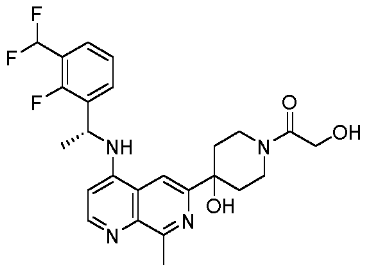 | B-176 | 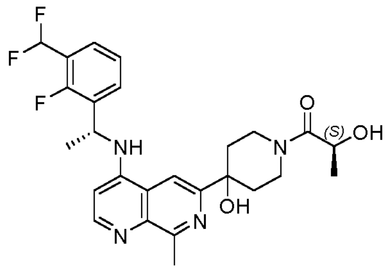 |
| C-1 | 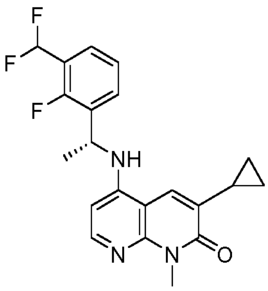 | C-2 | 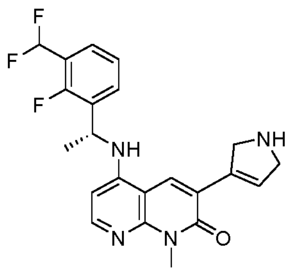 |
| C-3 | 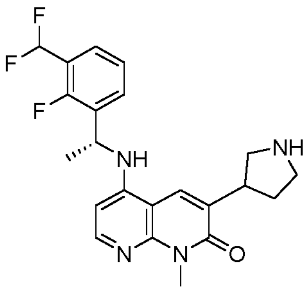 | C-4 | 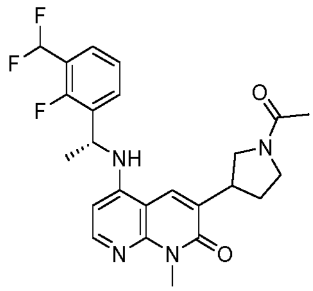 |
| C-5 | 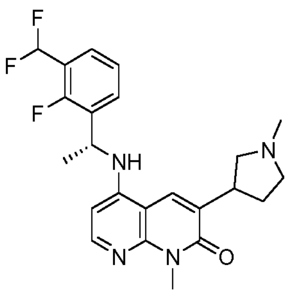 | C-6 | 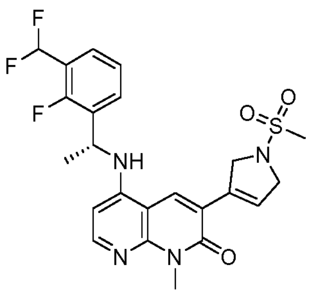 |
| C-7 | 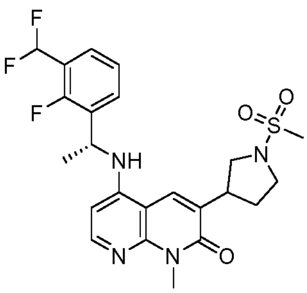 | C-8 | 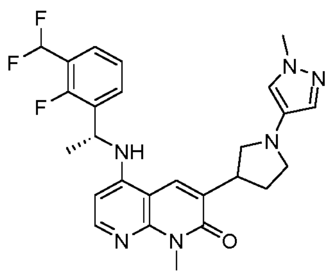 |

(continued)

| No. | Structure | No. | Structure |
|---|---|---|---|
| C-9 | 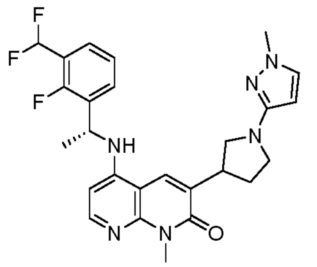 | C-10 | 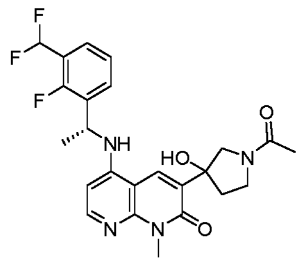 |
| C-11 | 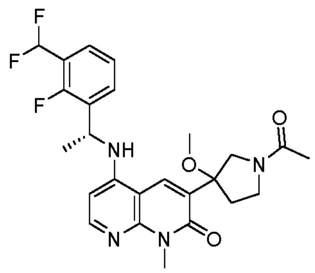 | C-12 | 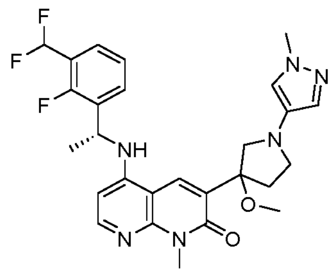 |
| C-13 | 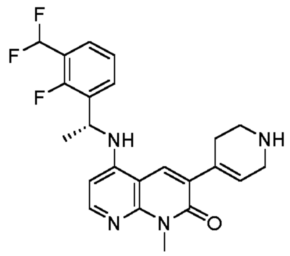 | C-14 | 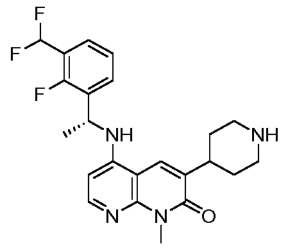 |
| C-15 | 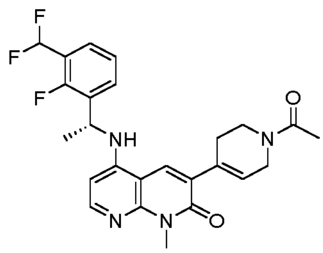 | C-16 | 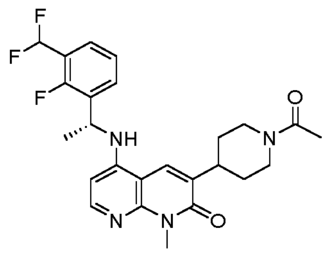 |
| C-17 | 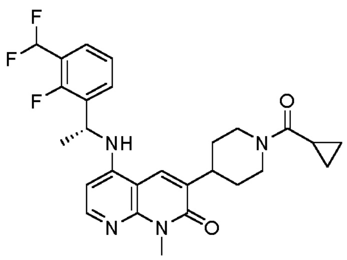 | C-18 | 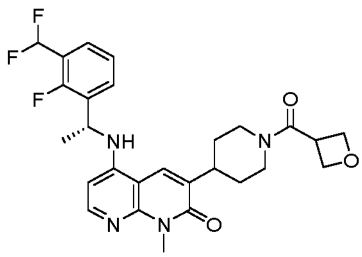 |
| C-19 | 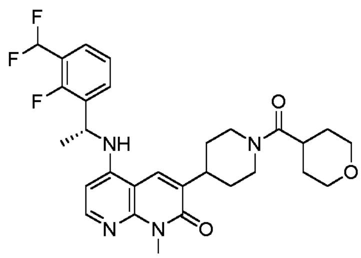 | C-20 | 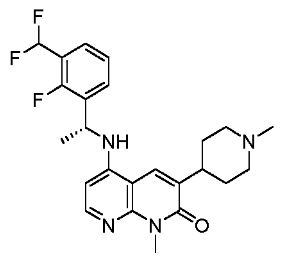 |

(continued)

| No. | Structure | No. | Structure |
|---|---|---|---|
| C-21 | 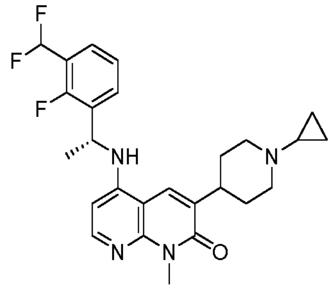 | C-22 | 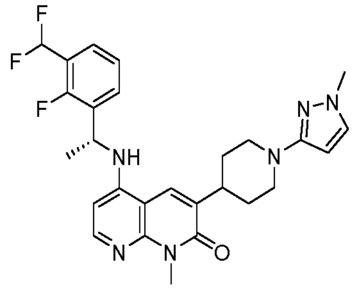 |
| C-23 | 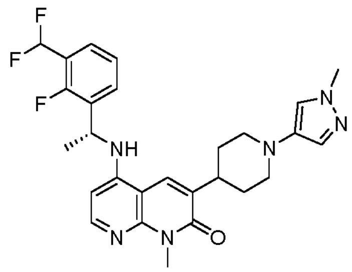 | C-24 | 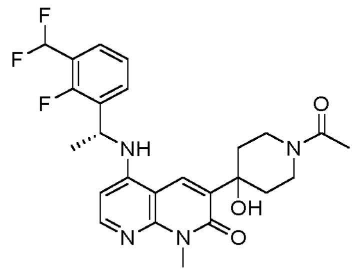 |
| C-25 | 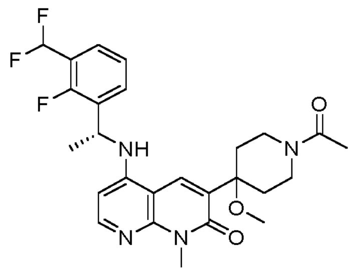 | C-26 | 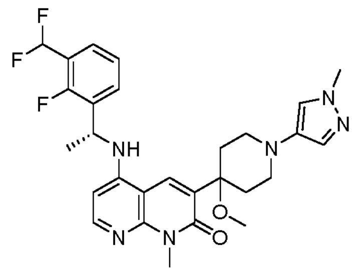 |
| C-27 | 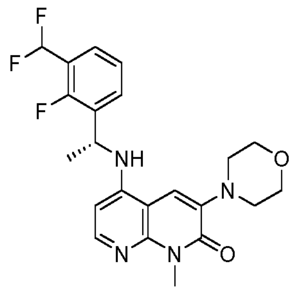 | C-28 | 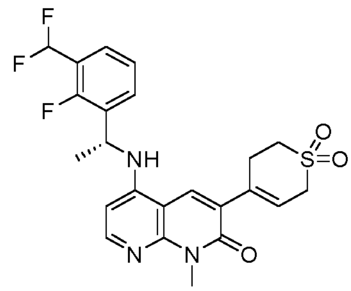 |
| C-29 | 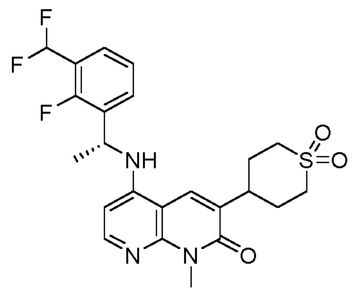 | C-30 | 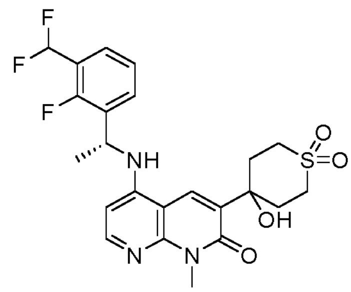 |

(continued)

| No. | Structure | No. | Structure |
|---|---|---|---|
| C-31 | 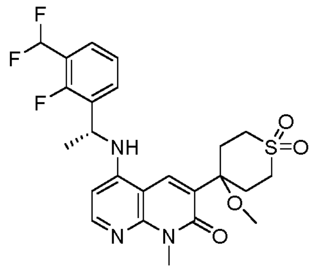 | C-32 | 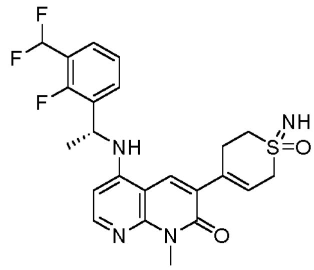 |
| C-33 | 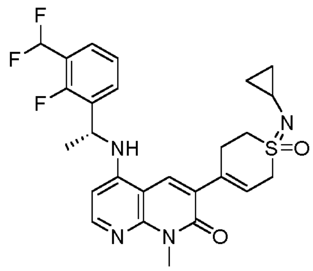 | C-34 | 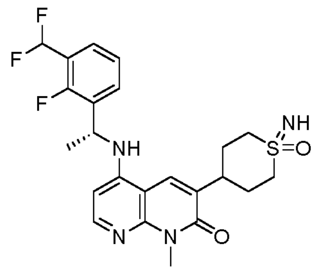 |
| C-35 | 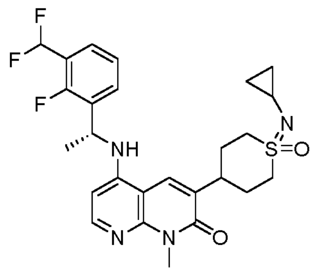 | C-36 | 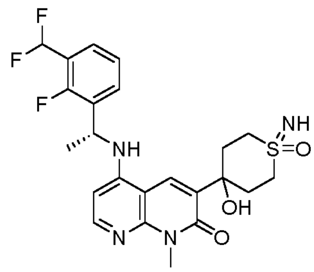 |
| C-37 | 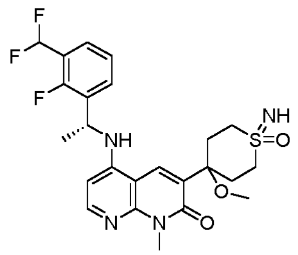 | C-38 | 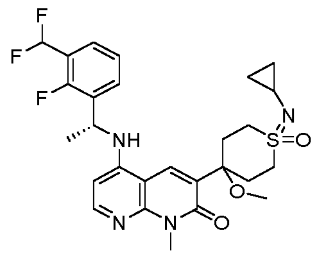 |
| D-1 | 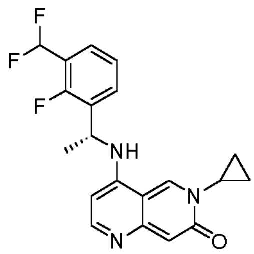 | D-2 | 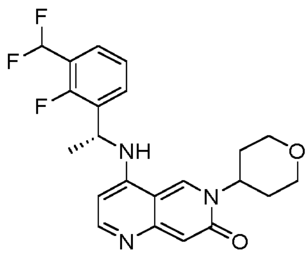 |
| D-3 | 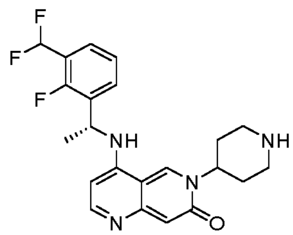 | D-4 | 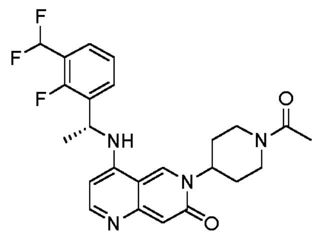 |

(continued)

| No. | Structure | No. | Structure |
|---|---|---|---|
| D-5 | 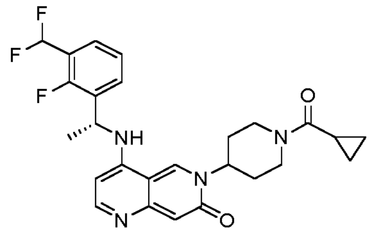 | D-6 | 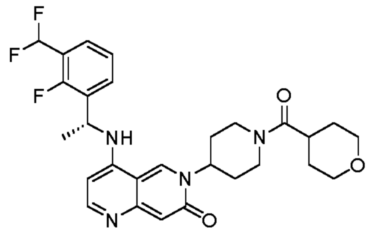 |
| D-7 | 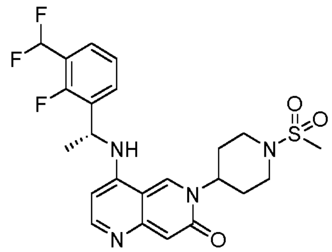 | D-8 | 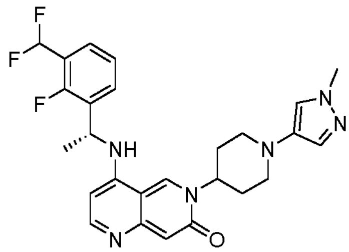 |
| D-9 | 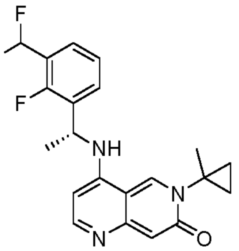 | D-10 | 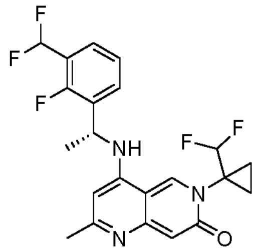 |
| D-11 | 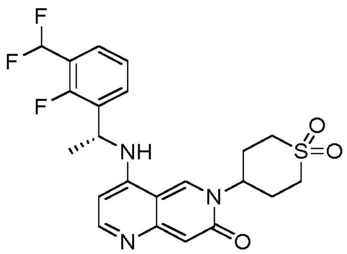 | D-12 | 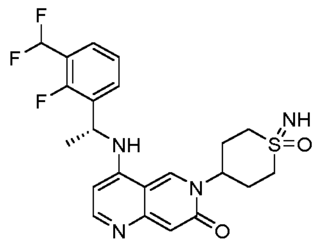 |
| D-13 | 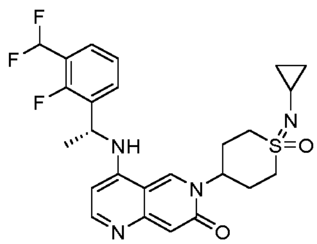 | | |

**13.** A pharmaceutical composition, comprising

(1) a therapeutically effective amount of the compound of formula (I), and the enantiomer, the diastereoisomer, the racemate, the prodrug, the hydrate, the solvate, or the pharmaceutically acceptable salt thereof according to any one of claims 1-12 as an active ingredient; and
(2) a pharmaceutically acceptable carrier.

**14.** Use of the compound of formula (I), and the enantiomer, the diastereoisomer, the racemate, the prodrug, the hydrate, the solvate, or the pharmaceutically acceptable salt thereof according to any one of claims 1-12, or the pharmaceutical composition according to claim 13 in the preparation of a drug for preventing and/or treating a disease associated

with mutation, activity, or expression amount of SOS1.

**15.** The use according to claim 14, wherein the disease associated with mutation, activity, or expression amount of SOS1 includes head and neck cancer, lung cancer, a mediastinal tumor, a gastrointestinal tumor, prostate cancer, testicular cancer, a gynecological tumor, breast cancer, renal and bladder cancer, an endocrine tumor, soft tissue sarcoma, osteosarcoma, rhabdomyosarcoma, mesothelioma, skin cancer, a peripheral nerve tumor, a central nervous system tumor, lymphoma, leukemia, Noonan syndrome, cardio-facio-cutaneous syndrome, and hereditary gingival fibromatosis and a related syndrome thereof.

**INTERNATIONAL SEARCH REPORT**

International application No.
**PCT/CN2022/122205**

**A. CLASSIFICATION OF SUBJECT MATTER**

C07D 471/04(2006.01)i; C07D 487/04(2006.01)i; A61K 31/519(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D A61K A61P

Documentation searched other than minimum documentation to the extent that such documents are included in the fields searched

Electronic data base consulted during the international search (name of data base and, where practicable, search terms used)

CNABS, CNKI, EPODOC, WPI, REGISTRY(STN), CAPLUS(STN): 吡啶, SOS1, 抑制, 癌, 肿瘤, pyridine, inhibit, cancer, tumor, tumour

**C. DOCUMENTS CONSIDERED TO BE RELEVANT**

| Category* | Citation of document, with indication, where appropriate, of the relevant passages | Relevant to claim No. |
|---|---|---|
| X | HILLIG, Roman C. et al. "Discovery of Potent SOS1 Inhibitors that Block RAS Activation via Disruption of the RAS-SOS1 Interaction." *Proceedings of the National Academy of Sciences of the United States of America,* Vol. 116, No. 7, 12 February 2019 (2019-02-12), pages 2551-2560 | 1-15 |
| X | DEGORCE, Sébastien L et al. "Discovery of Novel 3-Quinoline Carboxamides as Potent, Selective, and Orally Bioavailable Inhibitors of Ataxia Telangiectasia Mutated (ATM) Kinase." *Journal of Medicinal Chemistry,* Vol. 59, No. 13, 03 June 2016 (2016-06-03), pages 6281-6292 | 1-8, 11 |
| X | CN 1829695 A (SANOFI AVENTIS) 06 September 2006 (2006-09-06) description, page 17, embodiment 1, step f, and page 19, embodiment 2, step f | 1, 3, 5-8 |
| PX | CN 115043842 A (SUZHOU ZELGEN BIOPHARMACEUTICALS CO., LTD. et al.) 13 September 2022 (2022-09-13) claims 1, 6, and 8-9, and description, paragraphs [0109]-[0111] | 1-15 |
| PX | WO 2022156792 A1 (GUANGDONG NEWOPP BIOPHARMACEUTICALS CO., LTD.) 28 July 2022 (2022-07-28) claims 1 and 16-19 | 1-15 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 December 2022** | **15 December 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**

International application No.
**PCT/CN2022/122205**

**C. DOCUMENTS CONSIDERED TO BE RELEVANT**

| Category* | Citation of document, with indication, where appropriate, of the relevant passages | Relevant to claim No. |
|---|---|---|
| PX | WO 2022161461 A1 (JIANGSU SIMCERE PHARMACEUTICAL CO., LTD.) 04 August 2022 (2022-08-04)<br>claims 1 and 17-21 | 1-15 |
| PX | LUXENBURGER, Andreas et al. "Discovery of AL-GDa62 as a Potential Synthetic Lethal Lead for the Treatment of Gastric Cancer."<br>*Journal of Medicinal Chemistry,* Vol. 64, No. 24, 08 December 2021 (2021-12-08),<br>pages 18114-18142 | 1, 3-10 |

Form PCT/ISA/210 (second sheet) (January 2015)

**INTERNATIONAL SEARCH REPORT**
**Information on patent family members**

International application No.
**PCT/CN2022/122205**

| Patent document cited in search report | | | Publication date (day/month/year) | Patent family member(s) | | | Publication date (day/month/year) |
|---|---|---|---|---|---|---|---|
| CN | 1829695 | A | 06 September 2006 | HU | 0302440 | D0 | 29 September 2003 |
| | | | | ZA | 200600854 | A | 30 May 2007 |
| | | | | CN | 101239945 | A | 13 August 2008 |
| CN | 115043842 | A | 13 September 2022 | None | | | |
| WO | 2022156792 | A1 | 28 July 2022 | None | | | |
| WO | 2022161461 | A1 | 04 August 2022 | None | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2021092115 A

### Non-patent literature cited in the description

- **SAMBROOK et al.** Molecular Cloning: A Laboratory Manual. Cold Spring Harbor Laboratory Press, 1989